# EUROPEAN PATENT SPECIFICATION

(11) **EP 1 853 718 B1**
(45) Date of publication and mention of the grant of the patent: **05.08.2015**
(21) Application number: 06735370.6
(22) Date of filing: 15.02.2006
(51) Int. Cl.: A61K 39/395, C12P 21/08, C07K 16/00, C07K 16/28, A61P 35/00

(54) **ANTI-CD19 ANTIBODIES AND USES IN ONCOLOGY**
ANTI-CD19-ANTIKÖRPER UND IHRE VERWENDUNG IN DER ONKOLOGIE
ANTICORPS ANTI-CD19 ET LEUR UTILISATION EN ONCOLOGIE

(30) Priority: 15.02.2005 US 653587 P; 22.07.2005 US 702063 P
(43) Date of publication of application: 14.11.2007
(60) Divisional application: 12164266.4
(73) Proprietor: Duke University, Durham, North Carolina 27708-0083 (US)
(72) Inventor: YAZAWA, Norihito, Tokyo 113-0001 (JP); GRON, Hanne, Durham, NC 27707 (US); HAMAGUCHI, Yasuhito, Kanazawa, Ishikawa 920-8641 (JP); TEDDER, Thomas F., Durham, NC 27705 (US)
(74) Representative: Lee, Nicholas John
(86) International application number: PCT/US2006/005676
(87) International publication number: WO 2006/089133

(56) References cited:
- WO-A-2005/012493
- US-A- 5 530 101
- US-B1- 6 306 393
- G. PIETERSZ ET AL.: "In vitro and in vivo antitumour activity of a chimeric anti-CD19 antibody." CANCER IMMUNOLOGY AND IMMUNOTHERAPY, vol. 41, no. 1, 1 January 1995 (1995-01-01), pages 53-60, XP009109647 Berlin, Germany
- P. LANG ET AL.: "Chimeric CD19 antibody mediates cytotoxic activity against leukemic blasts with effector cells from pedriatic patients who received T-cell-depleted allografts." BLOOD, vol. 103, no. 10, 15 May 2004 (2004-05-15), pages 3982-3985, XP002443272 U.S.A.
- L. ZHOU ET AL.: "Tissue-specific expression of the human CD19 gene in transgenic mice inhibits antigen-independent B-lymphocyte development." MOLECULAR AND CELLULAR BIOLOGY, vol. 14, no. 6, June 1994 (1994-06), pages 3884-3894, XP002916501 Washington, DC, U.S.A.
- G. KANSAS ET AL.: "Transmembrane signals generated through MHC class II, CD19, CD20, CD39, and CD40 antigens induce LFA-1 dependent and independent adhesion in human B cells through a tyrosine kinase-dependent pathway." THE JOURNAL OF IMMUNOLOGY, vol. 147, no. 12, 15 December 1991 (1991-12-15), pages 4094-4102, XP002557706 Baltimore, MD, U.S.A.
- N. YAZAWA ET AL.: "Immunotherapy using unconjugated CD19 monoclonal antibodies in animal models for B lymphocyte malignancies and autoimmune disease." PROCEEDINGS OF THE NATIONAL ACADEMY OF SCIENCES OF THE U.S.A., vol. 102, no. 42, 18 October 2005 (2005-10-18), pages 15178-15183, XP002426225 Washington, DC, U.S.A.

### SEQUENCE LISTING

<110> MedImmune, Inc.
<120> ANTI-CD19 ANTIBODIES AND USES IN ONCOLOGY
<130> 11605-005-228
<140>
   <141>
<150> 60/653, 587
   <151> 2005-02-15
<150> 60/702,063
   <151> 2005-07-22
<160> 21
<170> FastSEQ for Windows Version 4.0
<210> 1
   <211> 373
   <212> DNA
   <213> Murine
<220>
   <223> mouse anti-(human)CD19 antibody HB12a Heavy chain VH-D-JH junctional sequence.
<400>1
   gaattcgagg tgcagctgca ggagtctgga cctgagctgg taaagcctgg ggcttcagtg 60
   aagatgtcct gcaaggcttc tggatacaca ttcactagct atgttatgca ctgggtgaag 120
   cagaagcctg ggcagggcct tgagtggatt ggatatttta atccttacaa tgatggtact 180
   gattactatg agaagttcaa aggcaaggcc acactgactt cagacaaatc ctccagcaca 240
   gcctacatgg cgctcagcag cctgacctct gaggactctg cggtctatta ctgtgcaaga 300
   gggacctatt actacggtag tagctacccc tttgactact ggggccaagg caccactctc 360
   acagtctcct cag                                                    373
<210> 2
   <211> 124
   <212> PRT
   <213> Murine
<220>
   <223> mouse anti-(human)CD19 antibody HB12a Heavy chain VH-D-JH junctional sequence
<400> 2
   Glu Phe Glu Val Gln Leu Gln Glu Ser Gly Pro Glu Leu Val Lys Pro
    1               5                  10                  15
   Gly Ala Ser Val Lys Met Ser Cys Lys Ala Ser Gly Tyr Thr Phe Thr
               20                  25                  30
   Ser Tyr Val Met His Trp Val Lys Gln Lys Pro Gly Gln Gly Leu Glu
           35                  40                  45
   Trp Ile Gly Tyr Phe Asn Pro Tyr Asn Asp Gly Thr Asp Tyr Tyr Glu
       50                  55                  60
   Lys Phe Lys Gly Lys Ala Thr Leu Thr Ser Asp Lys Ser Ser Ser Thr
   65                  70                  75                  80
   Ala Tyr Met Ala Leu Ser Ser Leu Thr Ser Glu Asp Ser Ala Val Tyr
                   85                  90                  95
   Tyr Cys Ala Arg Gly Thr Tyr Tyr Tyr Gly Ser Ser Tyr Pro Phe Asp
               100                 105                 110
   Tyr Trp Gly Gln Gly Thr Thr Leu Thr Val Ser Ser
           115                 120
<210> 3
   <211> 370
   <212> DNA
   <213> Murine
<220>
   <223> mouse anti-(human)CD19 antibody HB12b Heavy chain VH-D-JH junctional sequence
<400> 3
   gaattcgagg tgcagctgca ggagtctgga cctgagctgg tgaagcctgg ggcctcagtg 60
   aagatttcct gcaaagcttc tggctacgca ttcagtagct cttggatgaa ctgggtgata 120
   cagaggcctg gacagggtct tgagtggatt ggacggattt atcctggaga tggagatact 180
   aactacaatg ggaagttcaa gggcaaggcc acactgactg cagacaaatc ctccagtaca 240
   gcctacatgc agctcagcag cctgacctct gtggactctg cggtctattt ctgtgcaaga 300
   tcaggattta ttactacggt tttagacttt gactactggg gccacggcac cactctcaca 360
   gtctcctcag                                                        370
<210> 4
   <211> 123
   <212> PRT
   <213> Murine
<220>
   <223> mouse anti-(human)CD19 antibody HB12b Heavy chain VH-D-JH junctional sequence
<400> 4,
<210> 5
   <211> 124
   <212> PRT
   <213> Artificial Sequence
<220>
   <223> Consensus sequence of mouse anti-(human)CD19 antibody heavy chain junction VH-D-JH
<400> 5
   Glu Val Gln Leu Gln Glu Ser Gly Pro Glu Leu Val Lys Pro Gly Ala
   1 5 10 15
   Ser Val Lys Met Ser Cys Lys Ala Ser Gly Tyr Thr Phe Thr Ser Tyr
   20 25 30
   Val Met His Trp Val Lys Gln Lys Pro Gly Gln Gly Leu Glu Trp Ile
   35 40 45
   Gly Tyr Ile Asn Pro Tyr Asn Asp Gly Thr Asp Tyr Asn Glu Lys Phe
   50 55 60
   Lys Gly Lys Ala Thr Leu Thr Ser Asp Lys Ser Ser Ser Thr Ala Tyr
   65 70 75 80
   Met Ala Leu Ser Ser Leu Thr Ser Glu Asp Ser Ala Val Tyr Tyr Cys
   85 90 95
   Ala Arg Gly Thr Tyr Tyr Tyr Gly Ser Ser Tyr Tyr Tyr Pro Phe Asp
   100 105 110
   Tyr Trp Gly Gln Gly Thr Thr Leu Thr Val Ser Ser
   115 120
<210> 6
   <211> 121
   <212> PRT
   <213> Murine
<220>
   <223> mouse anti-(human)CD19 antibody 4G7 heavy chain junction VH-D-JH
<400> 6
   Glu Val Gln Leu Gln Gln Ser Gly Pro Glu Leu Ile Lys Pro Gly Ala
   1 5 10 15
   Ser Val Lys Met Ser Cys Lys Ala Ser Gly Tyr Thr Phe Thr Ser Tyr
   20 25 30
   Val Met His Trp Val Lys Gln Lys Pro Gly Gln Gly Leu Glu Trp Ile
   35 40 45
   Gly Tyr Ile Asn Pro Tyr Asn Asp Gly Thr Lys Tyr Asn Glu Lys Phe
   50 55 60
   Lys Gly Lys Ala Thr Leu Thr Ser Asp Lys Ser Ser Ser Thr Ala Tyr
   65 70 75 80
   Met Ala Leu Ser Ser Leu Thr Ser Glu Asp Ser Ala Val Tyr Tyr Cys
   85 90 95
   Ala Arg Gly Thr Tyr Tyr Tyr Gly Ser Arg Val Phe Asp Tyr Trp Gly
   100 105 110
   Gln Gly Thr Thr Leu Thr Val Ser Ser
   115 120
<210> 7
   <211> 124
   <212> PRT
   <213> Murine
<220>
   <223> mouse anti-(human)CD19 antibody HD37 heavy chain junction VH-D-JH
<400>7
   Gln Val Gln Leu Gln Gln Ser Gly Ala Glu Leu Val Arg Pro Gly Ser
   1 5 10 15
   Ser Val Lys Ile Ser Cys Lys Ala Ser Gly Tyr Ala Phe Ser Ser Tyr
   20 25 30
   Trp Met Asn Trp Val Lys Gln Arg Pro Gly Gln Gly Leu Glu Trp Ile
   35 40 45
   Gly Gln Ile Trp Pro Gly Asp Gly Asp Thr Asn Tyr Asn Gly Lys Phe
   50 55 60
   Lys Gly Lys Ala Thr Leu Thr Ala Asp Glu Ser Ser Ser Thr Ala Tyr
   65 70 75 80
   Met Gln Leu Ser Ser Leu Ala Ser Glu Asp Ser Ala Val Tyr Phe Cys
   85 90 95
   Ala Arg Arg Glu Thr Thr Thr Val Gly Arg Tyr Tyr Tyr Ala Met Asp
   100 105 110
   Tyr Trp Gly Gln Gly Thr Ser Val Thr Val Ser Ser
   115 120
<210> 8
   <211> 124
   <212> PRT
   <213> Murine
<220>
   <223> mouse anti-(human)CD19 antibody B43 heavy chain junction VH-D-JH
<400> 8
   Gln Val Gln Leu Leu Glu Ser Gly Ala Glu Leu Val Arg Pro Gly Ser
   1 5 10 15
   Ser Val Lys Ile Ser Cys Lys Ala Ser Gly Tyr Ala Phe Ser Ser Tyr
   20 25 30
   Trp Met Asn Trp Val Lys Gln Arg Pro Gly Gln Gly Leu Glu Trp Ile
   35 40 45
   Gly Gln Ile Trp Pro Gly Asp Gly Asp Thr Asn Tyr Asn Gly Lys Phe
   50 55 60
   Lys Gly Lys Ala Thr Leu Thr Ala Asp Glu Ser Ser Ser Thr Ala Tyr
   65 70 75 80
   Met Gln Leu Ser Ser Leu Arg Ser Glu Asp Ser Ala Val Tyr Ser Cys
   85 90 95
   Ala Arg Arg Glu Thr Thr Thr Val Gly Arg Tyr Tyr Tyr Ala Met Asp
   100 105 110
   Tyr Trp Gly Gln Gly Thr Thr Val Thr Val Ser Ser
   115 120
<210> 9
   <211> 121
   <212> PRT
   <213> Murine
<220>
   <223> mouse anti-(human)CD19 antibody FMC63 heavy chain junction VH-D-JH
<400> 9
   Glu Val Lys Leu Gln Glu Ser Gly Pro Gly Leu Val Ala Pro Ser Gln
   1               5                   10                  15
   Ser Leu Ser Val Thr Cys Thr Val Ser Gly Val Ser Leu Pro Asp Tyr
               20                  25                  30
   Gly Val Ser Trp Ile Arg Gln Pro Pro Arg Lys Gly Leu Glu Trp Leu
           35                  40                  45
   Gly Val Ile Trp Gly Ser Glu Asp Thr Thr Tyr Tyr Asn Ser Ala Leu
       50                  55                  60
   Lys Ser Arg Leu Thr Ile Ile Lys Asp Asn Ser Lys Ser Gln Val Phe
   65                  70                  75                  80
   Leu Lys Met Asn Ser Leu Gln Thr Asp Asp Thr Ala Ile Tyr Tyr Cys
                   85                  90                  95
   Ala Lys His Tyr Tyr Tyr Gly Gly Ser Tyr Ala Met Asp Tyr Trp Gly
               100                 105                 110
   Gln Gly Thr Ser Val Thr Val Ser Ser
           115                 120
<210> 10
   <211> 114
   <212> PRT
   <213> Artificial Sequence
<220>
   <223> consensus sequence of mouse anti-(human)CD19 antibody light chain V-kappa
<400> 10
   Asp Ile Val Met Thr Gln Thr Pro Ala Ser Leu Ala Val Ser Leu Gly
   1               5                   10                  15
   Gln Arg Ala Thr Ile Ser Cys Lys Ala Ser Gln Ser Val Asp Tyr Asn
               20                  25                  30
   Gly Asp Ser Tyr Leu Asn Trp Tyr Gln Gln Arg Pro Gly Gln Pro Pro
           35                  40                  45
   Lys Leu Leu Ile Tyr Asp Ala Ser Asn Leu Val Ser Gly Val Pro Asp
       50                  55                  60
   Arg Phe Ser Gly Ser Gly Ser Gly Thr Asp Phe Thr Leu Asn Ile His
   65                  70                  75                  80
   Pro Val Glu Lys Glu Asp Ala Ala Thr Tyr Tyr Cys Gln Gln Ser Thr
                   85                  90                  95
   Glu Asp Pro Tyr Thr Phe Gly Gly Gly Thr Lys Leu Glu Ile Lys Arg
               100                 105                 110
   Ala Asp
<210> 11
   <211> 112
   <212> PRT
   <213> Murine
<220>
   <223> mouse anti-(human)CD19 antibody HD37 light chain V-kappa
<400> 11
   Asp Ile Leu Leu Thr Gln Thr Pro Ala Ser Leu Ala Val Ser Leu Gly
   1               5                   10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Arg
               100                 105                 110
<210> 12
   <211> 114
   <212> PRT
   <213> Murine
<220>
   <223> mouse anti-(human)CD19 antibody B43 light chain
   V-kappa
<400> 12
   Glu Leu Val Leu Thr Gln Ser Pro Ala Ser Leu Ala Val Ser Leu Gly
   1               5                   10                  15
   Gln Arg Ala Thr Ile Ser Cys Lys Ala Ser Gln Ser Val Asp Tyr Asp
               20                  25                  30
   Gly Asp Ser Tyr Leu Asn Trp Tyr Gln Gln Ile Pro Gly Gln Pro Pro
           35                  40                  45
   Lys Leu Leu Ile Tyr Asp Ala Ser Asn Leu Val Ser Gly Ile Pro Pro
       50                  55                  60
   Arg Phe Ser Gly Ser Gly Ser Gly Thr Asp Phe Thr Leu Asn Ile His
   65                  70                  75                  80
   Pro Val Glu Lys Val Asp Ala Ala Thr Tyr His Cys Gln Gln Ser Thr
                   85                  90                  95
   Glu Asp Pro Trp Thr Phe Gly Gly Gly Thr Lys Leu Glu Ile Lys Arg
               100                 105                 110
   Arg Ser
<210> 13
   <211> 107
   <212> PRT
   <213> Murine
<220>
   <223> mouse anti-(human)CD19 antibody FMC63 light chain V-kappa
<400> 13
   Asp Ile Gln Met Thr Gln Thr Thr Ser Ser Leu Ser Ala Ser Leu Gly
   1               5                   10                  15
   Asp Arg Val Thr Ile Ser Cys Arg Ala Ser Gln Asp Ile Ser Lys Tyr
               20                  25                  30
   Leu Asn Trp Tyr Gln Gln Lys Pro Asp Gly Thr Val Lys Leu Leu Ile
           35                  40                  45
   Tyr His Thr Ser Arg Leu His Ser Gly Val Pro Ser Arg Phe Ser Gly
       50                  55                  60
   Ser Gly Ser Gly Thr Asp Tyr Ser Leu Thr Ile Ser Asn Leu Glu Gln
   65                  70                  75                  80
   Glu Asp Ile Ala Thr Tyr Phe Cys Gln Gln Gly Asn Thr Leu Pro Tyr
                   85                  90                  95
   Thr Phe Gly Gly Gly Thr Lys Leu Glu Ile Thr
               100                 105
<210> 14
   <211> 115
   <212> PRT
   <213> Murine
<220>
   <223> mouse anti-(human)CD19 antibody 4G7 light chain V-kappa
<400> 14
   Asp Ile Val Met Thr Gln Ala Ala Pro Ser Ile Pro Val Thr Pro Gly
   1               5                   10                  15
   Glu Ser Val Ser Ile Ser Cys Arg Ser Ser Lys Ser Leu Leu Asn Ser
               20                  25                  30
   Asn Gly Asn Thr Tyr Leu Tyr Trp Phe Leu Gln Arg Pro Gly Gln Ser
           35                  40                  45
   Pro Gln Leu Leu Ile Tyr Arg Met Ser Asn Leu Ala Ser Gly Val Pro
       50                  55                  60
   Asp Arg Phe Ser Gly Ser Gly Ser Gly Thr Ala Phe Thr Leu Arg Ile
   65                  70                  75                  80
   Ser Arg Val Glu Ala Glu Asp Val Gly Val Tyr Tyr Cys Met Gln His
                   85                  90                  95
   Leu Glu Tyr Pro Phe Thr Phe Gly Ala Gly Thr Lys Leu Glu Leu Lys
               100                 105                 110
   Arg Ala Asp
           115
<210> 15
   <211> 494
   <212> DNA
   <213> Murine
<220>
   <221> CDS
   <222> (57)...(494)
   <223> mouse anti-(human)CD19 antibody HB12a light chain
<400> 15
   catggactga aggagtagaa aactgatcac tctcctatgt ttatttcctc aaaatg atg 59
                                                                  Met
                                                                   1
   agt cct gcc cag ttc ctg ttt ctg tta gtg ctc tgg att cag gaa acc  107
   Ser Pro Ala Gln Phe Leu Phe Leu Leu Val Leu Trp Ile Gln Glu Thr
                   5                   10                  15
   aac ggt gat gtt ggg atg acc cag act cca ctc act ttg tcg gtc acc  155
   Asn Gly Asp Val Gly Met Thr Gln Thr Pro Leu Thr Leu Ser Val Thr
               20                  25                  30
   att gga caa cca gcc tct ttc tct tgc aag tca agt cag agc ctc tta  203
   Ile Gly Gln Pro Ala Ser Phe Ser Cys Lys Ser Ser Gln Ser Leu Leu
           35                  40                  45
   tat agt aat gga aaa acc tat ttg aat tgg tta tta cag agg cca ggc  251
   Tyr Ser Asn Gly Lys Thr Tyr Leu Asn Trp Leu Leu Gln Arg Pro Gly
    50                  55                  60                  65
   cag tct cca aag cgc cta atc cat ctg gtg tct aaa ctg gac tct gga  299
   Gln Ser Pro Lys Arg Leu Ile His Leu Val Ser Lys Leu Asp Ser Gly
                       70                  75                  80
   gtc cct gac agg ttc act ggc agt gga tca gga aca gat ttt aca ctg  347
   Val Pro Asp Arg Phe Thr Gly Ser Gly Ser Gly Thr Asp Phe Thr Leu
                   85                  90                  95
   aaa atc ggc aga gtg gag gct gag gat ttg gga gtt tat tac tgc gtg  395
   Lys Ile Gly Arg Val Glu Ala Glu Asp Leu Gly Val Tyr Tyr Cys Val
               100                 105                 110
   caa ggt aca cat ttt ccg tac acg ttc gga ggg ggg acc aaa cta gaa  443
   Gln Gly Thr His Phe Pro Tyr Thr Phe Gly Gly Gly Thr Lys Leu Glu
           115                 120                 125
   ata aaa cgg gct gat gct gca cca act gta tcc atc ttc cca cca tcc  491
   Ile Lys Arg Ala Asp Ala Ala Pro Thr Val Ser Ile Phe Pro Pro Ser
   130                 135                 140                 145
   agt                                                              494
   Ser
<210> 16
   <211> 146
   <212> PRT
   <213> Murine
<220>
   <223> predicted mouse anti- (human) CD19 antibody HB12a light chain
<400> 16
   Met Ser Pro Ala Gln Phe Leu Phe Leu Leu Val Leu Trp Ile Gln Glu
    1               5                  10                  15
   Thr Asn Gly Asp Val Gly Met Thr Gln Thr Pro Leu Thr Leu Ser Val
               20                  25                  30
   Thr Ile Gly Gln Pro Ala Ser Phe Ser Cys Lys Ser Ser Gln Ser Leu
           35                  40                  45
   Leu Tyr Ser Asn Gly Lys Thr Tyr Leu Asn Trp Leu Leu Gln Arg Pro
       50                  55                  60
   Gly Gln Ser Pro Lys Arg Leu Ile His Leu Val Ser Lys Leu Asp Ser
   65                  70                  75                  80
   Gly Val Pro Asp Arg Phe Thr Gly Ser Gly Ser Gly Thr Asp Phe Thr
                   85                  90                  95
   Leu Lys Ile Gly Arg Val Glu Ala Glu Asp Leu Gly Val Tyr Tyr Cys
               100                 105                 110
   Val Gln Gly Thr His Phe Pro Tyr Thr Phe Gly Gly Gly Thr Lys Leu
           115                 120                 125
   Glu Ile Lys Arg Ala Asp Ala Ala Pro Thr Val Ser Ile Phe Pro Pro
       130                 135                 140
   Ser Ser
   145
<210> 17
   <211> 485
   <212> DNA
   <213> Murine
<220>
   <221> CDS
   <222> (48)...(485)
   <223> mouse anti-(human)CD19 antibody HB12b light chain
<400> 17
   catggactga aggagtagaa aagcattctc tcttccagtt ctcagag atg gag aaa    56
                                                       Met Glu Lys
                                                       1
   gac aca ctc ctg cta tgg gtc ctg ctt ctc tgg gtt cca ggt tcc aca   104
   Asp Thr Leu Leu Leu Trp Val Leu Leu Leu Trp Val Pro Gly Ser Thr
       5                   10                  15
   ggt gac att gtg ctg acg cag tct cca acc tct ttg gct gtg tct cta   152
   Gly Asp Ile Val Leu Thr Gln Ser Pro Thr Ser Leu Ala Val Ser Leu
   20                  25                  30                  35
   ggg cag agg gcc acc atc tcc tgc aga gcc agc gaa agt gtt gat act   200
   Gly Gln Arg Ala Thr Ile Ser Cys Arg Ala Ser Glu Ser Val Asp Thr
                   40                  45                  50
   ttt ggc att agt ttt atg aac tgg ttc caa cag aaa cca gga cag cca   248
   Phe Gly Ile Ser Phe Met Asn Trp Phe Gln Gln Lys Pro Gly Gln Pro
               55                  60                  65
   ccc aaa ctc ctc atc cat gct gca tcc aat caa gga tcc ggg gtc cct   296
   Pro Lys Leu Leu Ile His Ala Ala Ser Asn Gln Gly Ser Gly Val Pro
           70                  75                  80
   gcc agg ttt agt ggt agt ggg tct ggg acg gac ttc agc ctc aac atc   344
   Ala Arg Phe Ser Gly Ser Gly Ser Gly Thr Asp Phe Ser Leu Asn Ile
       85                  90                  95
   cat cct atg gag gag gat gat agt gca atg tat ttc tgt cag caa agt   392
   His Pro Met Glu Glu Asp Asp Ser Ala Met Tyr Phe Cys Gln Gln Ser
   100                 105                 110                 115
   aag gag gtt cca ttc acg ttc ggc tcg ggg aca aag ttg gaa ata aaa   440
   Lys Glu Val Pro Phe Thr Phe Gly Ser Gly Thr Lys Leu Glu Ile Lys
                   120                 125                 130
   cgg gct gat gct gca cca act gta tcc atc ttc cca cca tcc agt       485
   Arg Ala Asp Ala Ala Pro Thr Val Ser Ile Phe Pro Pro Ser Ser
               135                 140                 145
<210> 18
   <211> 146
   <212> PRT
   <213> Murine
<220>
   <223> mouse anti-(human)CD19 antibody HB12b light chain
<400> 18
   Met Glu Lys Asp Thr Leu Leu Leu Trp Val Leu Leu Leu Trp Val Pro
   1               5                   10                  15
   Gly Ser Thr Gly Asp Ile Val Leu Thr Gln Ser Pro Thr Ser Leu Ala
               20                  25                  30
   Val Ser Leu Gly Gln Arg Ala Thr Ile Ser Cys Arg Ala Ser Glu Ser
           35                  40                  45
   Val Asp Thr Phe Gly Ile Ser Phe Met Asn Trp Phe Gln Gln Lys Pro
       50                  55                  60
   Gly Gln Pro Pro Lys Leu Leu Ile His Ala Ala Ser Asn Gln Gly Ser
   65                  70                  75                  80
   Gly Val Pro Ala Arg Phe Ser Gly Ser Gly Ser Gly Thr Asp Phe Ser
                   85                  90                  95
   Leu Asn Ile His Pro Met Glu Glu Asp Asp Ser Ala Met Tyr Phe Cys
               100                 105                 110
   Gln Gln Ser Lys Glu Val Pro Phe Thr Phe Gly Ser Gly Thr Lys Leu
           115                 120                 125
   Glu Ile Lys Arg Ala Asp Ala Ala Pro Thr Val Ser Ile Phe Pro Pro
       130                 135                 140
   Ser Ser
   145
<210> 19
   <211> 31
   <212> DNA
   <213> Artificial Sequence
<220>
   <223> promiscuous sense 5' VH primer(MsVHE)
<400> 19
   gggaattcga ggtgcagctg caggagtctg g 31
<210> 20
   <211> 31
   <212> DNA
   <213> Artificial Sequence
<220>
   <223> antisense primer complementary to the C coding region (primer C 1)
<400> 20
   gagttccagg tcactgtcac tggctcaggg a 31
<210> 21
   <211> 27
   <212> DNA
   <213> Artificial Sequence
<220>
   <223> constant region specific antisense 3' primer
<400> 21
   gactgaggca cctccagatg ttaactg 27

## Description

### 1. INTRODUCTION

The present invention is directed to an anti-CD 19 antibody for use in methods for the treatment of B cell disorders or diseases in human subjects, including B cell malignancies, using therapeutic antibodies that bind to the human CD19 antigen. In a preferred embodiment, the therapeutic anti-CD19 antibodies of the compositions and for use in the methods of the invention preferably mediate human antigen-dependent-cell-mediated-cytotoxicity (ADCC). The present invention is further directed to compositions Comprising humanized or chimeric anti-CD19 antibodies of the IgG1 and/or IgG3 human isotype. The present invention is further directed to such compositions that preferably mediate human ADCC. The present invention, also encompasses monoclonal humanized, or chimeric anti-CD 19 antibodies.

### 2. BACKGROUND OF THE INVENTION

B cell surface markers have been generally suggested as targets for the treatment of B cell disorders or diseases, autoimmune disease, and transplantation rejection. Examples of B cell surface markers include CD10, CD19, CD20, CD21, CD22, CD23, CD24, CD37, CD53, CD72, CD74, CD75, CD77, CD79a, CD79b, CD80, CD81, CD82, CD83, CD84, CD85, and CD86 leukocyte surface markers. Antibodies that specifically bind certain of these markers have been developed, and some have been tested for the treatment of diseases and disorders.

For example, chimeric or radiolabeled monoclonal antibody (mAb)-based therapies directed against the CD20 cell surface molecule specific for mature B cells and their malignant counterparts have been shown to be an effective *in vivo* treatment for non-Hodgkin's lymphoma (Tedder et al., Immunol. Today 15:450-454 (1994); Press et al., Hematology, 221-240 (2001); Kaminski et al., N. Engl. J. Med., 329:459-465 (1993); Weiner, Semin. Oncol., 26:43-51 (1999); Onrust et al., Drugs, 58:79-88 (1999); McLaughlin et al., Oncology, 12:1763-1769 (1998); Reff et al., Blood, 83:435-445 (1994); Maloney et al., Blood, 90:2188-2195 (1997); Maloney et al., J. Clin. Oncol., 15:3266-3274 (1997); Anderson et al., Biochem. Soc. Transac., 25:705-708 (1997)). Anti-CD20 monoclonal antibody therapy has also been found to ameliorate the manifestations of rheumatoid arthritis, systemic lupus erythematosus, idiopathic thrombocytopenic purpura and hemolytic anemia, as well as other immune-mediated diseases (Silverman et al, Arthritis Rheum., 48:1484-1492 (2002); Edwards et al., Rheumatology, 40:1-7 (2001); De Vita et al., Arthritis Rheumatism, 46:2029-2033 (2002); Leandro et al., Ann. Rheum. Dis., 61:883-888 (2002); Leandro et al., Arthritis Rheum., 46:2673-2677 (2001)). The anti-CD22 , monoclonal antibody LL-2 was shown to be effective in treating aggressive and relapsed lymphoma patients undergoing chemotherapeutic treatment (Goldenberg U.S. Patent Nos: 6,134,982 and 6,306,393). The anti-CD20 (IgG1) antibody, RITUXAN™, has successfully been used in the treatment of certain diseases such as adult immune thrombocytopenic purpura, rheumatoid arthritis, and autoimmune hemolytic anemia (Cured et al., WO 00/67796). Despite the effectiveness of this therapy, most acute lymphoblastic leukemias (ALL) and many other B cell malignancies either do not express CD20, express CD20 at low levels, or have lost CD20 expression following CD20 immunotherapy (Smith et al., Oncogene, 22:7359-7368 (2003)). Moreover, the expression of CD20 is not predictive of response to anti-CD20 therapy as only half of non-Hodgkin's lymphoma patients respond to CD20-directed immunotherapy.

The human CD 19 molecule is a structurally distinct cell surface receptor expressed on the surface of human B cells, including, but not limited to, pre-B cells, B cells in early development (*i.e.,* immature B cells), mature B cells through terminal differentiation into plasma cells, and malignant B cells. CD 19 is expressed by most pre-B acute lymphoblastic leukemias (ALL), non-Hodgkin's lymphomas, B cell chronic lymphocytic leukemias (CLL), pro-lymphocytic leukemias, hairy cell leukemias, common acute lymphocytic leukemias, and some Null-acute lymphoblastic leukemias (Nadler et al., J. Immunol., 131:244-250 (1983), Loken et al., Blood, 70:1316-1324 (1987), Uckun et al., Blood, 71:13-29 (1988), Anderson et al., 1984. Blood, 63:1424-1433 (1984), Scheuermann, Leuk. Lymphoma, 18:385-397(1995)). The expression of CD19 on plasma cells further suggests it may be expressed on differentiated B cell tumors such as multiple myeloma, plasmacytomas, Waldenstrom's tumors (Grossbard et al., Br. J. Haematol., 102:509-15(1998); Treon et al., Semin. Oncol., 30:248-52(2003)). Unlike CD20, the CD19 antigen was thought to be expressed at higher levels and internalized by cells when bound by an anti-CD19 antibody.

The CD 19 antigen has also been one of the many proposed targets for immunotherapy. However, the perceived unavailability as a target due to cellular internalization, was thought to have presented obstacles to the development of therapeutic protocols that could be successfully used in human subjects. The CLB-CD19 antibody (anti-CD 19 murine IgG2a mAb) was shown to inhibit growth of human tumors implanted in athymic mice (Hooijberg et al., Cancer Research, 55:840-846 (1995)). In another study, the monoclonal murine antibody FMC63 (IgG2a) was chimerized using a human IgG1 Fc region. Administration of this chimeric antibodies to SCID mice bearing a human B cell lymphoma (xenotransplantation model) did not induce complement-mediated cytotoxicity or ADCC, but resulted in significant killing of the transplanted tumor cells (Geoffrey et al., Cancer Immunol. Immunother., 41:53-60 (1995)).

The results obtained using xenotransplantation mouse models of tumor implantation led to studies using murine anti-CD19 antibodies in human patients. The murine CLB-CD19 antibody was administered to six patients diagnosed with a progressive non-Hodgkin's lymphoma who had failed previous conventional therapy (chemotherapy or radiotherapy). These patients were given total antibody doses ranging from 225 to 1,000 mg (Hekman et al., Cancer Immunol. immunotherapy, 32:364-372 (1991)). Although circulating tumor cells were temporarily reduced in two patients after antibody infusion, only one patient achieved partial remission after two periods of antibody treatment. No conclusions regarding therapeutic efficacy could be drawn from this small group of refractory patients.

Subsequently, these investigators showed that the anti-tumor effects of unconjugated CD20 mAbs are far superior to those of CD 19 mAbs in transplantation models (Hooijberg et al., Cancer Res., 55:840-846 (1995); and Hooijberg et al.., Cancer Res., 55:2627-2634 (1995)). Moreover, they did not observe additive or synergistic effects on tumor incidence when using CD19 and CD20 mAbs in combination (Hooijberg et al., Cancer Res., 55:840-846 (1995)). Although the xenottansplantation animal models were recognized to be poor prognostic indicators for efficacy in human subjects, the negative results achieved in these animal studies discouraged interest in therapy with naked anti-CD 19 antibodies.

The use of anti-CD 19 antibody-based immunotoxins produced equally discouraging results. In early clinical trials, the B4 anti-CD19 antibody (murine IgG1 mAb) was conjugated to the plant toxin ricin and administered to human patients having multiple myeloma who had failed previous conventional therapy (Grossbard et al., British Journal of Haematology, 102:509-515(1998)), advanced non-Hodgkin's lymphoma (Grossbard et al., Clinical Cancer Research, 5:2392-2398 (1999)), and refractory B cell malignancies, (Grossbard et al., Blood, 79:576-585 (1992)). These trials generally demonstrated the safety of administering the B4-ricin conjugate to humans; however, results were mixed and response rates were discouraging in comparison to clinical trials with RITUXAN™ (Grossbard et al., Clinical Cancer Research, 5:2392-2398 (1999)). In addition, a significant portion of the patients developed a human anti-mouse antibody (HAMA) response or a human anti-ricin antibody (HARA) response.

In another trial, seven low-grade non-Hodgkin's lymphoma patients previously treated with conventional therapy were treated with the murine CLB-CD19 antibody in combination with continuous infusion of low-dose interleukin-2 (Vlasveld et al., Cancer, Immunol. Immunotherapy, 40:37-47 (1995)). A partial remission occurred in one leukemic patient, and a greater than 50% reduction of circulating B cells was observed. Circulating B cell numbers were not changed in 4 of 5 remaining patients assessed. Thus, the therapeutic evaluation of murine anti-CD 19 antibodies and anti-CD 19 antibody-based immunotoxins in humans, generated anecdotal data that could not be evaluated for efficacy.

International Publication No. WO 2005/012493 provides humanized, chimeric and human anti-CD 19 antibody fusion proteins, and fragments thereof that bind to a human B cell marker. Such antibodies, fusion proteins arid fragments thereof are useful for the treatment and diagnosis of various B cell disorders, including B-cell malignancies and autoimmune diseases.

### 3. SUMMARY OF THE INVENTION

The invention relates to immunotherapeutic compositions and antibodies for use in methods for the treatment of B cell diseases and disorders in human subjects, such as, but not limited to, B cell malignancies, using therapeutic antibodies that bind to the human CD 19 antigen and that preferably mediate human ADCC. The present invention relates to pharmaceutical compositions comprising humanized anti-CD 19 antibodies of the IgG1 or IgG3 human isotype. The present invention relates to pharmaceutical compositions comprising humanized anti-CD19 antibodies of the IgG1 or IgG3 human isotype that preferably mediate human ADCC. The present invention relates to pharmaceutical compositions comprising chimerized anti-CD 19 antibodies of the IgG1 or IgG3 isotype that mediate human ADCC. In preferred embodiments, the present invention relates to pharmaceutical compositions comprising monoclonal humanized, or chimeric anti-CD19. antibodies.

Therapeutic formulations and regimens are described for treating human subjects diagnosed with B cell malignancies that derive from B cells and their precursors, including but not limited to, acute lymphoblastic leukemias (ALL), Hodgkin's lymphomas, non-Hodgkin's lymphomas, B cell chronic lymphocytic leukemias (CLL), multiple myeloma, follicular lymphoma, mantle cell lymphoma, pro-lymphocytic leukemias, hairy cell leukemias, common acute lymphocytic leukemias and some Null-acute lymphoblastic leukemias.

The antibodies for use in the methods of the invention are demonstrated by way of example, using a transgenic mouse model for evaluating CD19-directed immunotherapies in human subjects.

The invention provides a pharmaceutical composition comprising a monoclonal chimerized, or humanized anti CD 19 antibody that (a) is of the IgG1 or IgG3 human isotype, or (b) mediates human antibody-dependent cellular cytotoxicity (ADCC), in a pharmaceutically acceptable carrier, wherein the antibody comprises the sequences as set out as amino acids 33 to 37, amino acids 51 to 68 and amino acids 101 to 115 of SEQ ID NO:2 and the sequences as set out as amino acids 43 to 58, amino acids 74 to 80 and amino acids 113 to 121 ofSEQ ID NO:16, or the sequences as set out as amino acids 33 to 37, amino acids 51 to 68 and amino acids 101 to 114 of SEQ ID NO:4 and the sequences as set out as amino acids 44 to 58, amino acids 74 to 80 and amino acids 113 to 121 of SEQ ID NO:18, wherein one amino acid substitution is introduced into one or more of said sequences and wherein the antibody is able to deplete CD 19 expressing B cells from the bone marrow.

A therapeutically effective amount of the monoclonal chimerized anti-CD19 antibody of the IgG1 or IgG3 human isotype may be less than about 1 mg/kg of patient body weight.

A therapeutically effective amount of a monoclonal chimerized anti-CD19 antibody of the IgG1 or IgG3 human isotype may be greater than about 2 mg/kg of patient body weight.

The anti-CD19 antibody that mediates ADCC may be of the IgG1, or IgG3. human isotype.

The anti-CD 19 antibody may have a half-life of at least 4 to 7 days.

The composition may comprise a dose of 1500 mg/m2 or less of the anti-CD 19 antibody and the anti-CD 19 antibody may be administered by a parenteral, intraperitoneal, intramuscular, intravenous or subcutaneous route.

The composition may comprise a dose of 375 mg/m2 or less of the anti-CD 19 antibody and the anti-CD 19 antibody may be administered by a parenteral, intraperitoneal, intramuscular, intravenous or subcutaneous route.

The composition may comprise a dose of 1.5 mg/m2 or less of the anti-CD19 antibody and the anti-CD19 antibody may be administered by a parenteral, intraperitoneal, intramuscular, intravenous or subcutaneous, route.

The composition may comprise a dose of 0.5 ug/m2 or less of the anti-CD 19 antibody and the anti-CD19 antibody may be administered by a parenteral, intraperitoneal, intramuscular, intravenous or subcutaneous route.

The anti-CD19 antibody may be detectably labelled, may be a naked antibody, may be conjugated to a therapeutic compound, may be conjugated to a cytotoxic agent, or may be conjugated to a diagnostic agent.

The anti-CD 19 antibody may be bispecific.

The bispecific anti-CD 19 antibody may have specificity for binding effector cells.

The ADCC function of the anti-CD 19 antibody may be assessed by measuring the ability of the anti-CD 19 antibody to mediate target cell lysis by effector cells *in vitro*.

The anti-CD 19 antibody may comprise a heavy chain consisting of a sequence having at least 90% identity to SEQ ID NO:2 or SEQ ID NO:4.

The anti-CD 19 antibody may comprise the light chain consisting of a sequence having at least 95% identity to SEQ ID NO:16 or SEQ ID NO:18.

The invention also provides a monoclonal humanized anti-CD19 antibody that (a) is of the IgG1 or IgG3 human isotype, or (b) mediates human antibody-dependent cellular cytotoxicity (ADCC), wherein the antibody comprises the sequences as set out as amino acids 33 to 37, 51 to 68 and 101 to 115 of SEQ ID NO:2 and the sequences as set out as amino acids 43 to 58, 74 to 80 and 113 to 121 of SEQ ID NO:16, or the sequences as set out as amino acids 33 to 37, 51 to 68 and 101 to 114 of SEQ ID NO:4 and the sequences as set out as amino acids 44 to 58, 74 to 80 and 113 to 121 of SEQ ID NO:18, wherein one amino acid substitution is introduced into one or more of said sequences, for use in a method of treating a B cell malignancy in a human patient, wherein the antibody is able to deplete CD 19 expressing B cells from the bone marrow.

The anti-CD 19 antibody may comprise a heavy chain consisting of a sequence having at least 90% identity to SEQ ID NO:2 or SEQ ID NO:4.

The antibody may comprise the light chain consisting of a sequence having at least 95% identity to SEQ ID NO:16 or SEQ ID NO:18.

The antibody may be able to deplete circulating B cells.

The B cell malignancy may be acute lymphoblastic leukaemia, mantle cell lymphoma, pre-B cell acute lymphoblastic leukaemia, or precursor B cell lymphoblastic lymphoma.

The anti-CD19 antibody that mediates ADCC may be of the IgG1 or IgG3 human isotype.

The B cell malignancy may be treated prior to the administration of the anti-CD19 antibody.

The B cell malignancy may be treated with a therapy other than an anti-CD19 antibody therapy subsequent to the administration of the anti-CD19 antibody.

The treatment for the malignancy may be chemotherapy, radioimmunotherapy, toxin therapy, prodrug-activating enzyme therapy, antibody therapy, monocyte or macrophage enhancing therapy, immunoregulatory therapy, tumor neovasculature statin therapy, calicheamicin therapy, surgical therapy, or any combination thereof.

The invention also provides a monoclonal anti-CD19 antibody that mediates human antibody-dependent cellular cytotoxicity (ADCC) comprising the sequences as set out as amino acids 33 to 37, amino acids 51 to 68 and amino acids 101 to 115 of SEQ ID NO:2 and the sequences as set out as amino acids 43 to 58, amino acids 74 to 80 and amino acids 113 to 121 of SEQ ID NO:16, or the sequences as set out as amino acids 33 to 37, amino acids 51 to 68 and amino acids 101 to 114 of SEQ ID NO:4 and the sequences as set out as amino acids 44 to 58, amino acids 74 to 80 and amino acids 113 to 121 of SEQ ID NO:18, wherein one amino acid substitution is introduced into one or more of said sequences and wherein the antibody is able to deplete CD 19 expressing B cells from the bone marrow for use in a regimen for (a) treating an early stage disease resulting from a B cell malignancy in a human patient, or (b) treating a B cell malignancy in a human subject wherein the human subject has not previously received treatment for the malignancy, the B cell malignancy is CD 19 positive or the human has a monocyte count of at least 1 per dl, wherein the regimen administered depletes circulating B cells.

The antibody may comprise a heavy chain consisting of a sequence having at least 90% identity to SEQ ID NO:2 or SEQ ID NO:4.

The anti-CD 19 antibody may comprise the light chain consisting of a sequence having at least 95% identity to SEQ ID NO:16 or SEQ ID NO:18.

A therapy other than an anti-CD 19 antibody therapy may be subsequently administered to the human.

The therapy may be chemotherapy, radiotherapy, toxin based therapy, radiochemical based therapy or surgical therapy.

The regimen may comprise the administration of the antibody as a single therapeutic agent, in combination with another therapeutic agent, or in combination with an agent that reduces toxic side effects.

The regimen may comprise the repeat administration of the antibody.

The anti-CD 19 antibody may have a half-life that is at least 4 to 7 days.

The anti-CD 19 antibody may be a human IgG1 or IgG3 isotype antibody.

The anti-CD 19 antibody may be a humanized antibody.

The B cell malignancy may be a B cell subtype non-Hodgkin's lymphoma (NHL) including low grade/follicular NHL, small lymphocytic (SL) NHL, intermediate grade/follicular NHL, intermediate grade diffuse NHL, high grade immunoblastic NHL, high grade lymphoblastic NHL, high grade small non-cleaved cell NHL and bulky disease NHL; Burkitt's lymphoma; multiple myeloma; pre-B acute lymphoblastic leukemia and other malignancies that derive from early B cell precursors; common acute lymphocytic leukemia; chronic lymphocytic leukemia; hairy cell leukemia; Null-acute lymphoblastic leukemia; Waldenstrom's Macroglobulinemia; and pro-lymphocytic leukemia; light chain disease; plasmacytoma; osteosclerotic myeloma; plasma cell leukemia; monoclonal gammopathy of undetermined significance (MGUS); smoldering multiple myeloma (SMM); indolent multiple myeloma (IMM); or Hodgkin's lymphoma.

The regimen may further comprise administering a compound that enhances monocyte or macrophage function.

The human may be immunocompromised.

The anti-CD19 antibody may be administered by a parenteral, intraperitoneal, or intramuscular route.

The anti-CD19 antibody may be administered by an intravenous or subcutaneous route.

The anti-CD19 antibody may be administered by a subcutaneous route in a dose of 37.5 mg/m2 or less.

The anti-CD19 antibody may be administered in a dose of 1.5 mg/m2 or less.

The regimen may comprise more than one administration of the anti-CD19 antibody to the human.

The regimen may further comprise administering an anti-CD20 antibody, an anti-CD22 antibody, an anti-CD52 antibody, or any combination thereof.

In one embodiment, the invention provides for a pharmaceutical composition comprising a monoclonal humanized, anti-CD 19 antibody of the IgG1 or IgG3 human isotype in a pharmaceutically acceptable carrier. In another embodiment, the invention provides for a pharmaceutical composition comprising a therapeutically effective amount of a monoclonal chimerized anti-CD19 antibody of the IgG1 or IgG3 human isotype in a pharmaceutically acceptable carrier. In related embodiments, a therapeutically effective amount of a monoclonal chimerized anti-CD 19 antibody of the IgG1 or IgG3 human isotype is less than 1 mg/kg of patient body weight. In other related embodiments, a therapeutically effective amount of a monoclonal chimerized anti-CD19 antibody of the IgG1 or IgG3 human isotype is greater than 2 mg/kg of patient body weight.

According to one aspect, the invention provides for a pharmaceutical composition comprising a therapeutically effective amount of monoclonal humanized anti-CD19 antibody that mediates human antibody-dependent cellular cytotoxicity (ADCC), in a pharmaceutically acceptable carrier. The pharmaceutical composition may comprise a monoclonal chimerized anti-CD19 antibody that mediates human antibody-dependent cellular cytotoxicity (ADCC), and/or complement dependent cytotoxicty (CDC) and/or apoptotic activity in a pharmaceutically acceptable carrier.

The present invention concerns antibodies for use in a method of treating a B cell malignancy in a human comprising administering to a human in need thereof a monoclonal humanized anti-CD19 antibody of the IgG1 or IgG3 human isotype in an amount sufficient to deplete circulating B cells. The present invention also concerns antibodies for use in a method of treating a B cell malignancy in a human comprising administering to a human in need thereof a monoclonal humanized anti-CD 19 antibody that mediates human antibody-dependent cellular cytotoxicity (ADCC) in an amount sufficient to deplete circulating B cells. The present invention concerns antibodies for use in a method of treating a B cell malignancy in a human patient comprising the administration of a therapeutically effective regimen of a monoclonal humanized anti-CD 19 antibody of the IgG1 or IgG3 human isotype to a human patient in need of such treatment.

In one embodiment, the present invention is directed to the treatment of a B cell malignancy in a human patient comprising the administration of a therapeutically effective regimen of a monoclonal humanized anti-CD 19 antibody that mediates human antibody-dependent cellular cytotoxicity (ADCC), to a human patient in need of such treatment. In another embodiment, the present invention is directed to the treatment of an early stage disease resulting from a B cell malignancy in a human patient comprising administration of a therapeutically effective regimen of a monoclonal anti-CD 19 antibody that mediates human antibody-dependent cellular cytotoxicity (ADCC), to a human in need of such treatment. In a further embodiment, the present invention is directed to the treatment of a B cell malignancy in a human patient comprising administration of a therapeutically effective regimen of a monoclonal anti-CD 19 antibody that mediates human antibody-dependent cellular cytotoxicity (ADCC), to a human subject in need thereof, wherein the human subject has not previously received treatment for the malignancy. Yet another embodiment, of the present invention is directed to the treatment of a B cell malignancy in a human patient comprising administration of a therapeutically effective regimen of a monoclonal anti-CD 19 antibody that mediates human antibody-dependent cellular cytotoxicity (ADCC), to a human patient in need of such treatment, wherein the B cell malignancy is CD 19 positive. In a further embodiment, the present invention is directed to the treatment of a B cell malignancy in a human patient comprising administration of a therapeutically effective regimen of a monoclonal anti-CD19 antibody that mediates human antibody-dependent cellular cytotoxicity (ADCC), to a human patient in need of such treatment, wherein the human patient has a monocyte count of at least 1 per dL of circulating blood.

### 3.1 DEFINITIONS

As used herein, the terms "antibody" and "antibodies" (immunoglobulins) refer to monoclonal antibodies (including full-length monoclonal antibodies), polyclonal antibodies, multispecific antibodies (e.g., bispecific antibodies) formed from at least two intact antibodies, human antibodies, humanized antibodies, camelised antibodies, chimeric antibodies, single-chain Fvs (scFv), single-chain antibodies, single domain antibodies, domain antibodies, Fab fragments, F(ab')2 fragments, antibody fragments that exhibit the desired biological activity, disulfide-linked Fvs (sdFv), and anti-idiotypic (anti-Id) antibodies (including, e.g., anti-Id antibodies to antibodies of the invention), intrabodies, and epitope-binding fragments of any of the above. In particular, antibodies include immunoglobulin molecules and immunologically active fragments of immunoglobulin molecules, *i.e.,* molecules that contain an antigen-binding site. Immunoglobulin molecules can be of any type (*e.g.,* IgG, IgE, IgM, IgD, IgA and IgY), class (*e.g.,* IgGI, IgG2, IgG3, IgG4, IgA1 and IgA2) or subclass.

Native antibodies are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains. Such antibodies may be derived from any mammal, including, but not limited to, humans, monkeys, pigs, horses, rabbits, dogs, cats, mice, etc.

The term "variable" refers to the fact that certain portions of the variable domains differ extensively in sequence among antibodies and are responsible for the binding specificity of each particular antibody for its particular antigen. However, the variability is not evenly distributed through the variable domains of antibodies. It is concentrated in segments called Complementarity Determining Regions (CDRs) both in the light chain and the heavy chain variable domains. The more highly conserved portions of the variable domains are called the framework regions (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies (*see*, Kabat et al., Sequences of Proteins of Immunological Interest, 5th Ed. Public Health Service, National Institutes of Health, Bethesda, MD (1991)). The constant domains are generally not involved directly in antigen binding, but may influence antigen binding affinity and may exhibit various effector functions, such as participation of the antibody in ADCC.

The term "hypervariable region" when used herein refers to the amino acid residues of an antibody which are responsible for binding to its antigen. The hypervariable region comprises amino acid residues from a "complementarity determining region" or "CDR" (*e.g.,* residues 24-34 (L1), 50-56 (L2) and 89-97 (L3) in the light chain variable domain and 31-35 (H1), 50-65 (H2) and 95-102 (H3) in the heavy chain variable domain; Kabat *et al., Sequences of Proteins of Immunological Interest,* 5th Ed. Public Health Service, National Institutes of Health, Bethesda, MD (1991)) and/or those residues from a "hypervariable loop" (*e.g.*, residues 26-32 (L1), 50-52 (L2) and 91-96 (L3) in the light chain variable domain and 26-32 (H1), 53-55 (H2) and 96-101 (H3) in the heavy chain variable domain; Chothia and Lesk, J. Mol. Biol., 196:901-917 (1987)). "Framework" or "FR" residues are those variable domain residues other than the hypervariable region residues as herein defined, and include chimeric, humanized, human, domain antibodies, diabodies, vaccibodies, linear antibodies, and bispecific antibodies.

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma cells, uncontaminated by other immunoglobulin producing cells. Alternatively, the monoclonal antibody may be produced by cells stably or transiently transfected with the heavy and light chain genes encoding the monoclonal antibody.

The modifier "monoclonal" indicates the character of the antibody as being obtained from a substantially homogeneous population of antibodies, and is not to be construed as requiring engineering of the antibody by any particular method. The term "monoclonal" is used herein to refer to an antibody that is derived from a clonal population of cells, including any eukaryotic, prokaryotic, or phage clone, and not the method by which the antibody was engineered. For example, the monoclonal antibodies to be used in accordance with the present invention may be made by the hybridoma method first described by Kohler et al., Nature, 256:495 (1975), or may be made by any recombinant DNA method (*see, e.g.,* U.S. Patent No. 4,816,567), including isolation from phage antibody libraries using the techniques described in Clackson et al., Nature, 352:624-628 (1991) and Marks et al., J. Mol. Biol., 222:581-597 (1991), for example. These methods can be used to produce monoclonal mammalian, chimeric, humanized, human, domain antibodies, diabodies, vaccibodies, linear antibodies, and bispecific antibodies.

The term "chimeric" antibodies includes antibodies in which at least one portion of the heavy and/or light chain is identical with or homologous to corresponding sequences in antibodies derived from a particular species or belonging to a particular antibody class or subclass, and at least one other portion of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)). Chimeric antibodies of interest herein include "primatized" antibodies comprising variable domain antigen-binding sequences derived from a nonhuman primate (*e.g.,* Old World Monkey, such as baboon, rhesus or cynomolgus monkey) and human constant region sequences (U.S. Patent No. 5,693,780).

"Humanized" forms of nonhuman (*e.g.,* murine) antibodies are chimeric antibodies that contain minimal sequence derived from nonhuman immunoglobulin. For the most part, humanized antibodies are human immunoglobulins (recipient antibody) in which residues from a hypervariable region of the recipient are replaced by residues from a hypervariable region of a nonhuman species (donor antibody) such as mouse, rat, rabbit or nonhuman primate having the desired specificity, affinity, and capacity. In some instances, framework region (FR) residues of the human immunoglobulin are replaced by corresponding nonhuman residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a nonhuman immunoglobulin and all or substantially all of the FRs are those of a human immunoglobulin sequence. In certain embodiments, the humanized antibody will comprise at least a portion of an immunoglobulin constant region (Fc), typically that of a human immunoglobulin. For further details, *see,* Jones et al., Nature, 321:522-525 (1986); Riechmann et al., Nature, 332:323-329 (1988); and Presta, Curr. Op. Struct. Biol., 2:593-596 (1992).

A "human antibody" can be an antibody derived from a human or an antibody obtained from a transgenic organism that has been "engineered" to produce specific human antibodies in response to antigenic challenge and can be produced by any method known in the art. According to preferred techniques, elements of the human heavy and light chain loci are introduced into strains of the organism derived from embryonic stem cell lines that contain targeted disruptions of the endogenous heavy chain and light chain loci. The transgenic organism can synthesize human antibodies specific for human antigens, and the organism can be used to produce human antibody-secreting hybridomas. A human antibody can also be an antibody wherein the heavy and light chains are encoded by a nucleotide sequence derived from one or more sources of human DNA. A fully human antibody also can be constructed by genetic or chromosomal transfection methods, as well as phage display technology, or *in vitro* activated B cells, all of which are known in the art.

The "CD19" antigen refers to an antigen of about 90 kDa identified, for example, by the HD237 or B4 antibody (Kiesel et al., Leukemia Research II, 12:1119 (1987)). CD19 is found on cells throughout differentiation of B-lineage cells from the stem cell stage through terminal differentiation into plasma cells, including but not limited to, pre-B cells, B cells (including naïve B cells, antigen-stimulated B cells, memory B cells, plasma cells, and B lymphocytes) and follicular dendritic cells. CD19 is also found on B cells in human fetal tissue. In preferred embodiments, the CD 19 antigen targeted by the antibodies of the invention is the human CD19 antigen.

"Antibody-dependent cell-mediated cytotoxicity" and "ADCC" refer to a cell-mediated reaction in which non-specific cytotoxic cells (*e.g.,* Natural Killer (NK) cells, neutrophils, and macrophages) recognize bound antibody on a target cell and subsequently cause lysis of the target cell. In preferred embodiments, such cells are human cells. While not wishing to be limited to any particular mechanism of action, these cytotoxic cells that mediate ADCC generally express Fc receptors (FcRs). The primary cells for mediating ADCC, NK cells, express FcγRIII, whereas monocytes express FcγRI, FcγRII, FcγRIII and/or FcγRIV. FcR expression on hematopoietic cells is summarized in Ravetch and Kinet, Annu. Rev. Immunol., 9:457-92 (1991). To assess ADCC activity of a molecule, an *in vitro* ADCC assay, such as that described in U.S. Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecules of interest may be assessed *in vivo, e.g.,* in an animal model such as that disclosed in Clynes et al., PNAS (USA), 95:652-656 (1998).

"Complement dependent cytotoxicity" or "CDC" refers to the ability of a molecule to initiate complement activation and lyse a target in the presence of complement. The complement activation pathway is initiated by the binding of the first component of the complement system (C1q) to a molecule (*e.g.,* an antibody) complexed with a cognate antigen. To assess complement activation, a CDC assay, *e.g.,* as described in Gazzano-Santaro et al., J. Immunol. Methods, 202:163 (1996), may be performed.

"Effector cells" are leukocytes which express one or more FcRs and perform effector functions. Preferably, the cells express at least FcγRI, FCγRII, FcγRIII and/or FcγRIV arid carry out ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. In preferred embodiments the effector cells are human cells.

The terms "Fc receptor" or "FcR" are used to describe a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, FcγRIII, and FcγRIV subclasses, including allelic variants and alternatively spliced forms of these receptors. FcyRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (*See,* Daëron, Annu. Rev. Immunol., 15:203-234 (1997)). FcRs are reviewed in Ravetech and Kinet, Annu. Rev. Immunol., 9:457-92 (1991); Capel et al., Immunomethods, 4:25-34 (1994); and de Haas et al., J. Lab. Clin. Med., 126:330-41 (1995). Other FcRs, including those to be identified in the future, are encompassed by the term "FcR" herein. The term also includes the neonatal receptor, FcRn, which is responsible for the transfer of maternal IgGs to the fetus *(*Guyer et al., Immunol., 117:587 (1976) and Kim et al., J. Immunol., 24:249 (1994)).

"Fv" is the minimum antibody fragment which contains a complete antigen-recognition and binding site. This region consists of a dimer of one heavy and one light chain variable domain in tight, non-covalent or covalent association. It is in this configuration that the three CDRs of each variable domain interact to define an antigen-binding site on the surface of the V_{H}-V_{L} dimer. Collectively, the six CDRs. confer antigen-binding specificity to the antibody. However, even a single variable domain (or half of an Fv comprising only three CDRs specific for an antigen) has the ability to recognize and bind antigen, although at a lower affinity than the entire binding site.

"Affinity" of an antibody for an epitope to be used in the treatment(s) described herein is a term well understood in the art and means the extent, or strength, of binding of antibody to epitope. Affinity may be measured and/or expressed in a number of ways known in the art, including, but not limited to, equilibrium dissociation constant (KD or Kd), apparent equilibrium dissociation constant (KD' or Kd'), and IC50 (amount needed to effect 50% inhibition in a competition assay). It is understood that, for purposes of this invention, an affinity is an average affinity for a given population of antibodies which bind to an epitope. Values of KD' reported herein in terms of mg IgG per mL or mg/mL indicate mg Ig per mL of serum, although plasma can be used. When antibody affinity is used as a basis for administration of the treatment methods described herein, or selection for the treatment methods described herein, antibody affinity can be measured before and/or during treatment, and the values obtained can be used by a clinician in assessing whether a human patient is an appropriate candidate for treatment.

An "epitope" is a term well understood in the art and means any chemical moiety that exhibits specific binding to an antibody. An "epitope" can also comprise an antigen, which is a moiety or molecule that contains an epitope, and, as such, also specifically binds to antibody.

A "B cell surface marker" as used herein is an antigen expressed on the surface of a B cell which can be targeted with an agent which binds thereto. Exemplary B cell surface markers include the CD10, CD19, CD20, CD21, CD22, CD23, CD24, CD25, CD37, CD53; CD72, CD73, CD74, CD75, CD77, CD79a, CD79b, CD80, CD81, CD82, CD83, CD84, CD85, and CD86 leukocyte surface markers. The B cell surface marker of particular interest is preferentially expressed on B cells compared to other non-B cell tissues of a mammal and may be expressed on both precursor B cells and mature B cells. In one embodiment, the preferred marker is CD 19, which is found on B cells throughout differentiation of the lineage from the pro/pre-B cell stage through the terminally differentiated plasma cell stage.

The term "antibody half-life" as used herein means a pharmacokinetic property of an antibody that is a measure of the mean survival time of antibody molecules following their administration. Antibody half-life can be expressed as the time required to eliminate 50 percent of a known quantity of immunoglobulin from the patient's body or a specific compartment thereof, for example, as measured in serum, *i*.*e.,* circulating half-life, or in other tissues. Half life may vary from one immunoglobulin or class of immunoglobulin to another. In general, an increase in antibody half-life results in an increase in mean residence time (MRT) in circulation for the antibody administered.

The term "isotype" refers to the classification of an antibody. The constant domains of antibodies are not involved in binding, to antigen, but exhibit various effector functions. Depending on, the amino acid sequence of the heavy chain constant region, a given antibody, or immunoglobulin can be assigned to one of five major classes of immunoglobulins: IgA, IgD, IgE, IgG, and IgM. Several of these classes may be further divided into subclasses (isotypes), *e.g.,* IgG1 (gamma 1), IgG2 (gamma 2), IgG3 (gamma 3), and IgG4 (gamma 4), and IgA1 and IgA2. The heavy chain constant regions that correspond to the different classes of immunoglobulins are called α, δ, ε, γ, and µ, respectively. The structures and three-dimensional configurations of different classes of immunoglobulins are well-known. Of the various human immunoglobulin classes, only human IgG1, IgG2, IgG3, IgG4, and IgM are known to activate complement. Human IgG1 and IgG3 are known to mediate ADCC in humans.

As used herein, the term "immunogenicity" means that a compound is capable of provoking an immune response (stimulating production of specific antibodies and/or proliferation of specific T cells).

As used herein, the term "antigenicity" means that a compound is recognized by an antibody or may bind to an antibody and induce an immune response.

As used herein, the term "avidity" is a measure of the overall binding strength (*i.e*., both antibody arms) with which an antibody binds an antigen. Antibody avidity can be determined by measuring the dissociation of the antigen-antibody bond in antigen excess using any means known in the art, such as, but not limited to, by the modification of indirect fluorescent antibody as described by Gray et al., J. Virol. Meth., 44:11-24. (1993).

By the terms "treat," "treating" or "treatment of" (or grammatically equivalent terms) it is meant that the severity of the subject's condition is reduced or at least partially improved or ameliorated and/or that some alleviation, mitigation or decrease in at least one clinical symptom is achieved and/or there is an inhibition or delay in the progression of the condition and/or prevention or delay of the onset of a disease or illness. Thus, the terms "treat," "treating" or "treatments of" (or grammatically equivalent terms) refer to both prophylactic and therapeutic treatment regimes.

As used herein, a "sufficient amount" or "an amount sufficient to" achieve a particular result refers to an amount of an antibody or composition of the invention that is effective to produce a desired effect, which is optionally a therapeutic effect (*i.e.,* by administration of a therapeutically effective amount). For example, a "sufficient amount" or "an amount sufficient to" can be an amount that is effective to deplete B cells.

A "therapeutically effective" amount as used herein is an amount that provides some improvement or benefit to the subject. Alternatively stated, a "therapeutically effective" amount is an amount that provides some alleviation, mitigation, and/or decrease in at least one clinical symptom. Clinical symptoms associated with the disorders that can be treated by the methods of the invention are well-known to those skilled in the art. Further, those skilled in the art will appreciate that the therapeutic effects need not be complete or curative, as long as some benefit is provided to the subject.

### 4. BRIEF DESCRIPTION OF THE DRAWINGS

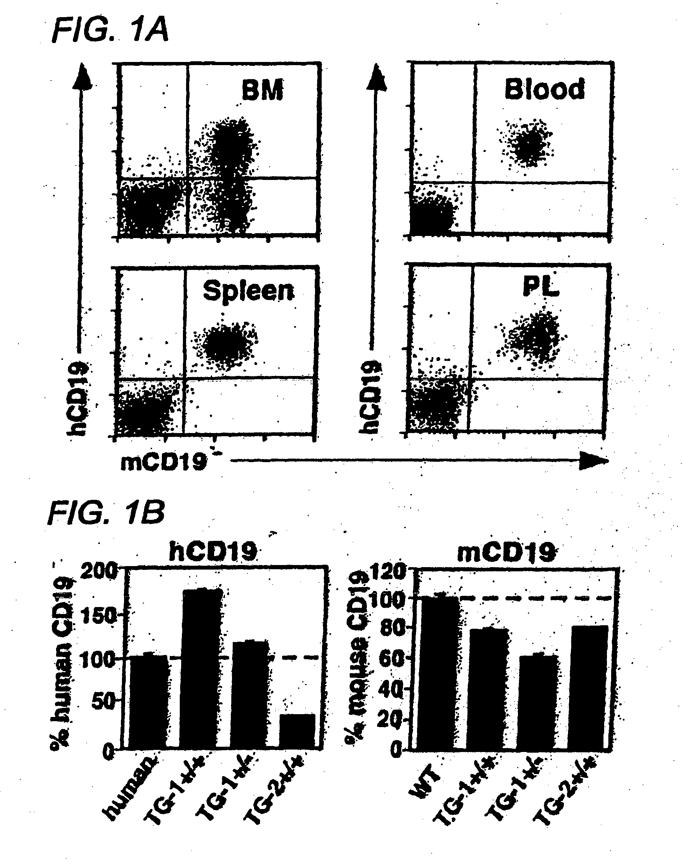

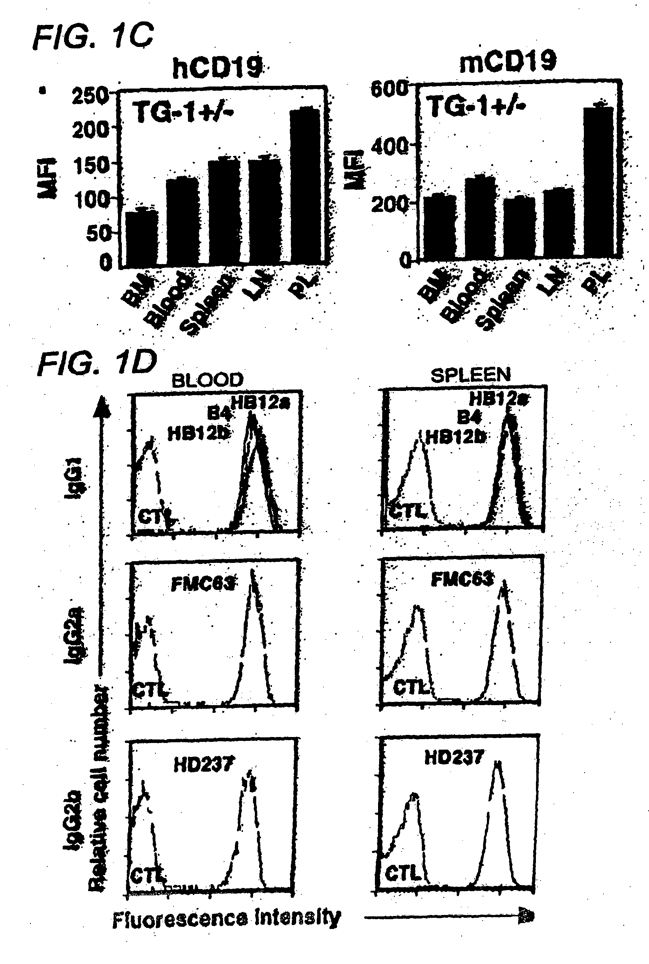

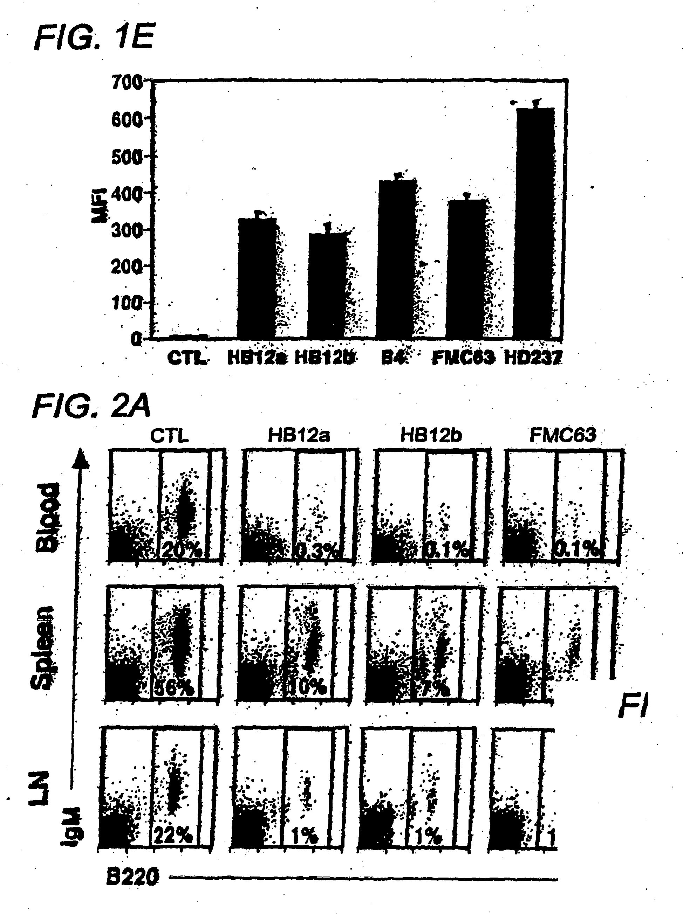

**Figs. 1A-1E** illustrate CD19 expression by hCD19TG mouse lines. **Fig. 1A** shows human and mouse CD19 expression by B cells from hCD19TG (TG-^{1+/-}) mice. **Fig. 1B** shows the relative mean densities of human and mouse CD19 expression by CD19⁺ blood B cells from hCD19TG mice. **Fig. 1C** shows the relative densities of hCD19 and mCD19 expression by CD19⁺ B cells from TG-1^{+/-} mouse tissues. **Fig. 1D** shows CDC19 antibody binding density on mouse blood and spleen B220⁺ B cells from TG-1^{+/-} mice. **Fig. 1E** shows anti-CD19 antibody binding to hCD19 cDNA-transfected 300.19 cells.

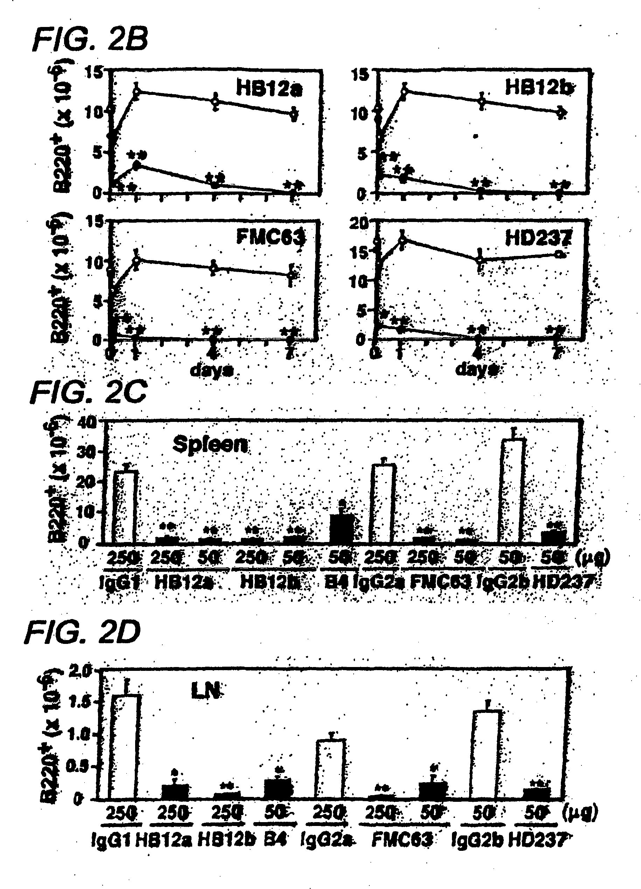

**Figs. 2A-2D** show blood, spleen, and lymph node B cell depletion in hCD19TG mice. **Fig. 2A** demonstrates representative B cell depletion from blood, spleen, and lymph node 7 days following CD 19 or isotype-matched control (CTL) antibody treatment of TG-1^{+/-} mice. **Fig. 2B** shows a time course of circulating B cell depletion by anti-CD19 antibodies. **Fig. 2C** and **Fig. 2D** show spleen and lymph node B cell numbers (±SEM), respectively, after treatment of TG-1^{+/-} mice with CD 19 (filled bars) or control (open bars) antibody at the indicated doses.

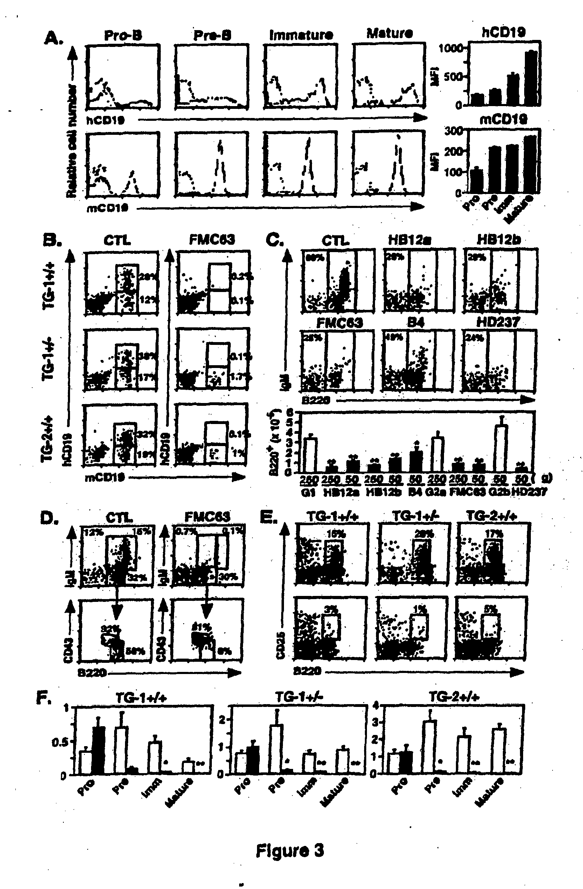

**Figs. 3A-3F** depict bone marrow B cell depletion following anti-CD 19 antibody treatment. **Fig. 3A** shows representative hCD19 and mCD19 expression by TG-1^{+/-} bone marrow B cell subpopulations assessed by four-color immunofluorescence staining with flow cytometry analysis. **Fig. 3B** shows depletion of hCD19⁺ cells in the bone marrow of hCD19TG mice seven days following FMC63 or isotype-matched control antibody (250 µg) treatment assessed by two-color immunofluorescence staining with flow cytometry analysis. **Fig. 3C** shows representative B220⁺ B cell depletion in the bone marrow seven days following CD19 or isotype-matched control antibody (250 µg) treatment of TG-1^{+/-} mice. **Fig. 3D** shows representative B cell subset depletion seven days following FMC63 or isotype-matched control antibody (250 µg) treatment of TG-1^{+/-} mice as assessed by three-color immunofluorescence staining. IgM⁻B220^{lo} pro-/pre-B cells were further subdivided based on CD43 expression (lower panels). **Fig. 3E** shows representative depletion of CD25⁺a220^{lo} pre-B cells seven days following FMC63 or isotype-matched control antibody (250 µg) treatment of hCD 19TG mouse lines as assessed by two-color immunofluorescence staining. **Fig. 3F** shows bar graphs indicating numbers (±SEM) of pro-B, pre-B, immature, and mature B cells within bilateral femurs seven days following FMC63 (closed bars) or control (open bars) antibody treatment of≥3 littermate pairs.

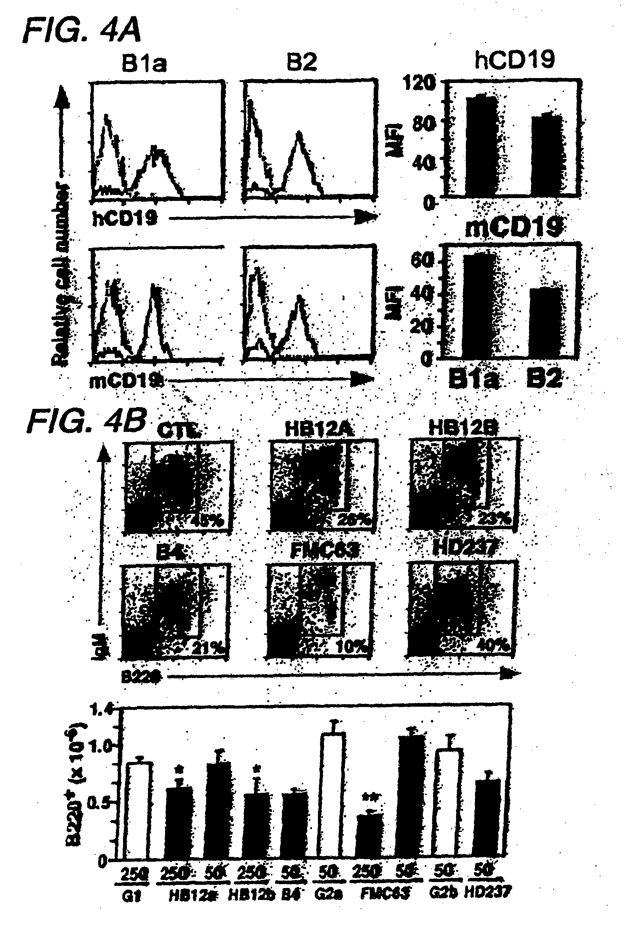

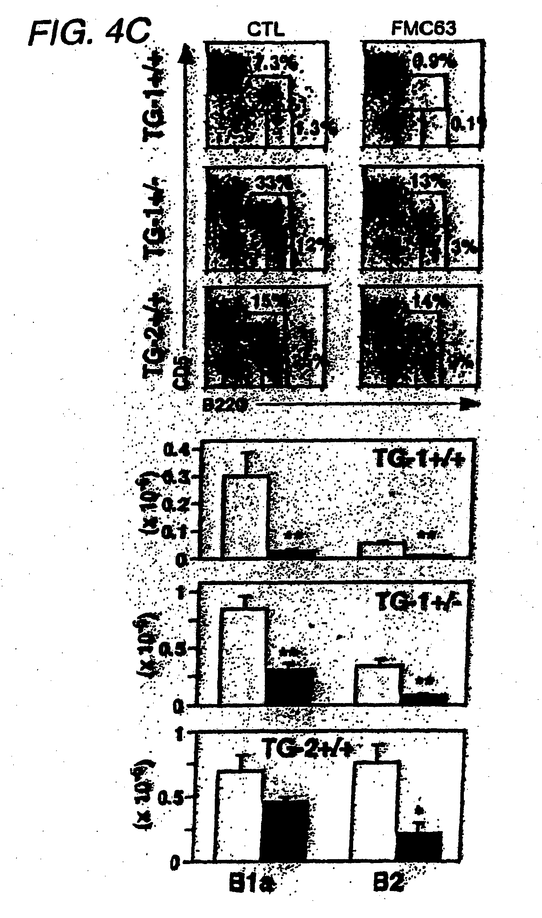

**Figs. 4A-4C** demonstrate that peritoneal cavity B cells are sensitive to anti-CD 19 antibody treatment. **Fig. 4A** shows human and mouse CD19 expression by peritoneal cavity CD5⁺B220⁺ B1a and CD5⁻B220^{hi} B2 (conventional) B cells. **Fig. 4B** shows depletion of peritoneal cavity B220⁺ cells from TG-1^{+/-} mice treated with CD19 (HB12a, HB12b, and FMC63 at 250 µg; B4 and HD237 at 50 µg) antibodies or control antibody (250 µg). **Fig. 4C** shows representative depletion of CD5⁺B220⁺ B1a and CD5-B220^{hi} B2 B cells seven days following anti-CD19 or control antibody treatment of hCD19TG mice.

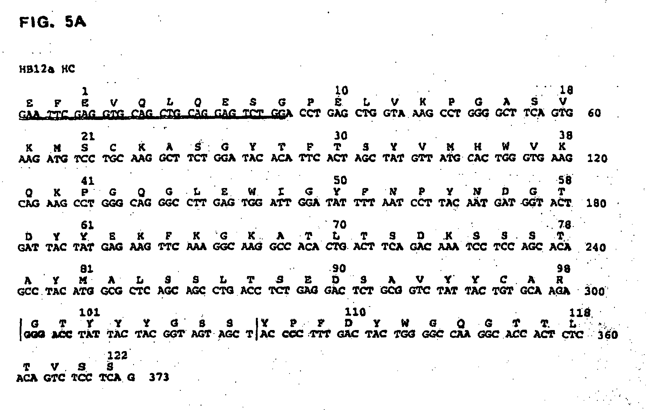

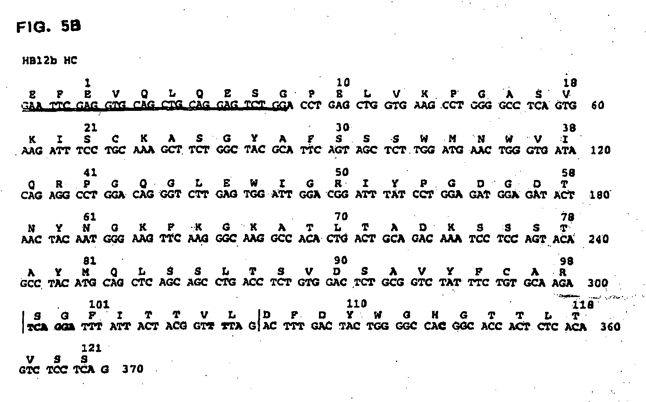

**Fig. 5A** depicts the nucleotide (SEQ ID NO:1) and predicted amino acid (SEQ ID NO:2) sequences for heavy chain V_{H}-D-J_{H} junctional sequences of the HB12a anti-CD 19 antibody. **Fig. 5B** depicts the nucleotide (SEQ ID NO:3) and predicted amino acid (SEQ ID NO:4) sequences for heavy chain V_{H}-D-J_{H} functional sequences of the HB12b anti-CD19 antibody.

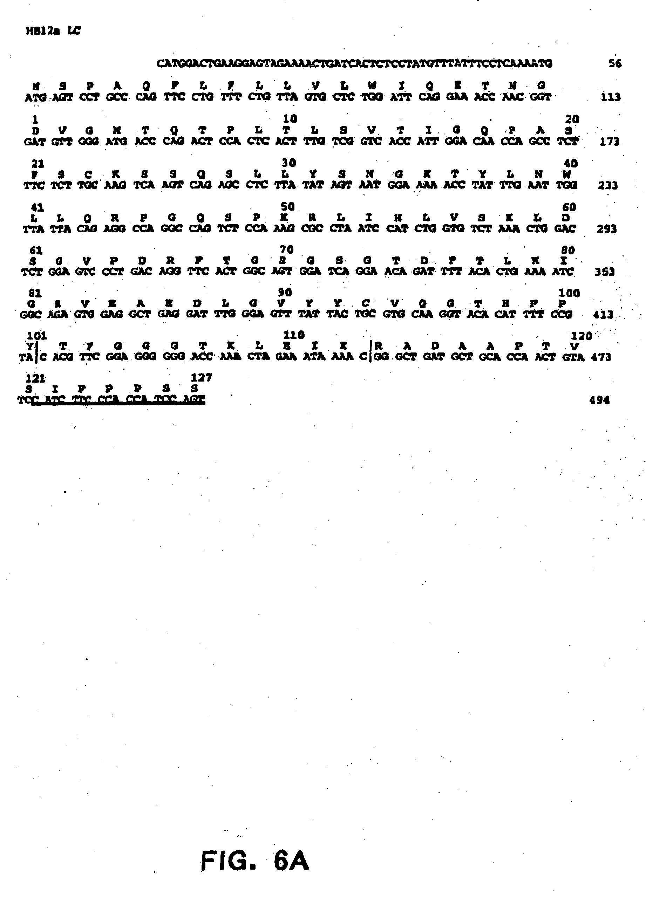

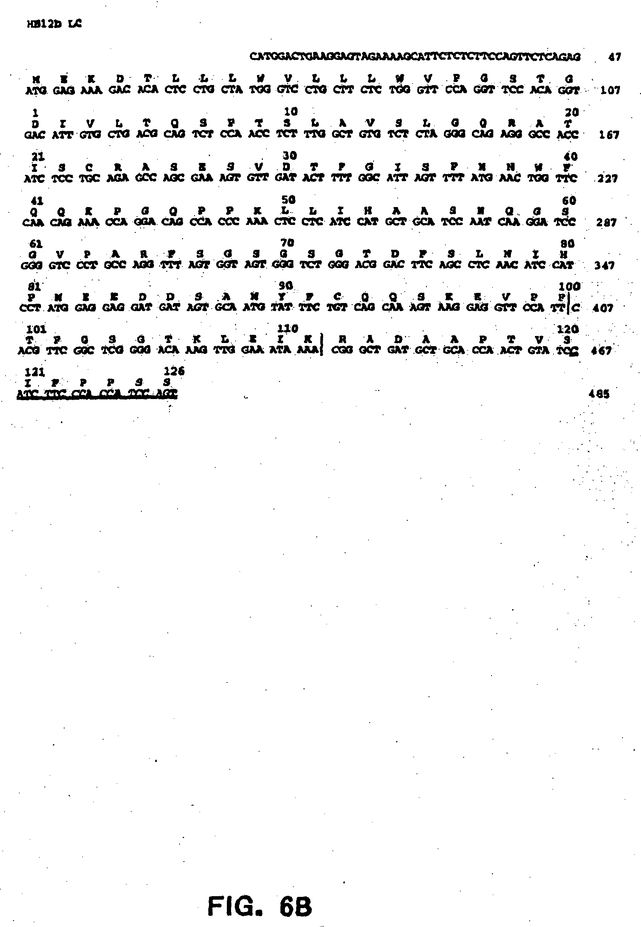

**Fig. 6A** depicts the nucleotide (SEQ ID NO:15) and predicted amino acid (SEQ ID NO:16) sequences for light chain sequences of the HB12a anti-CD19 antibody. **Fig. 6B** depicts the nucleotide (SEQ ID NO:17) and predicted amino acid (SEQ ID NO:18) sequences for light chain sequences of the HB12b anti-CD 19 antibody.

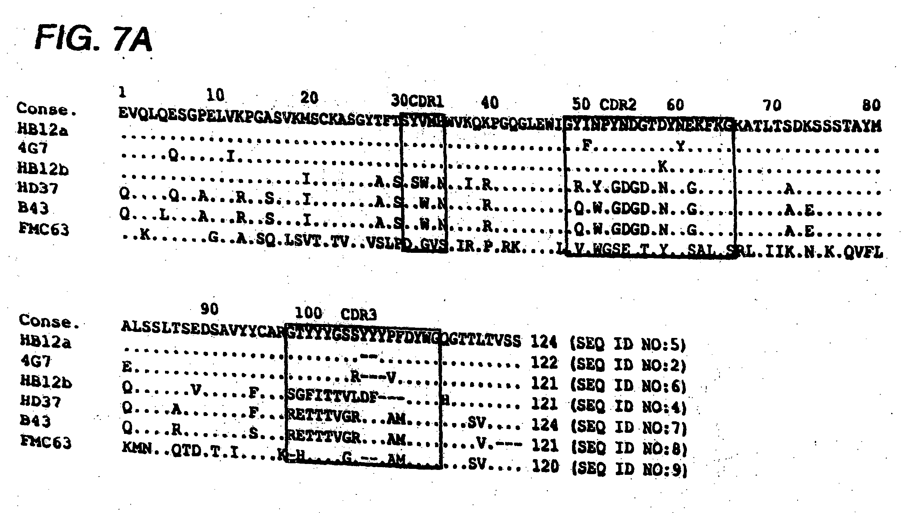

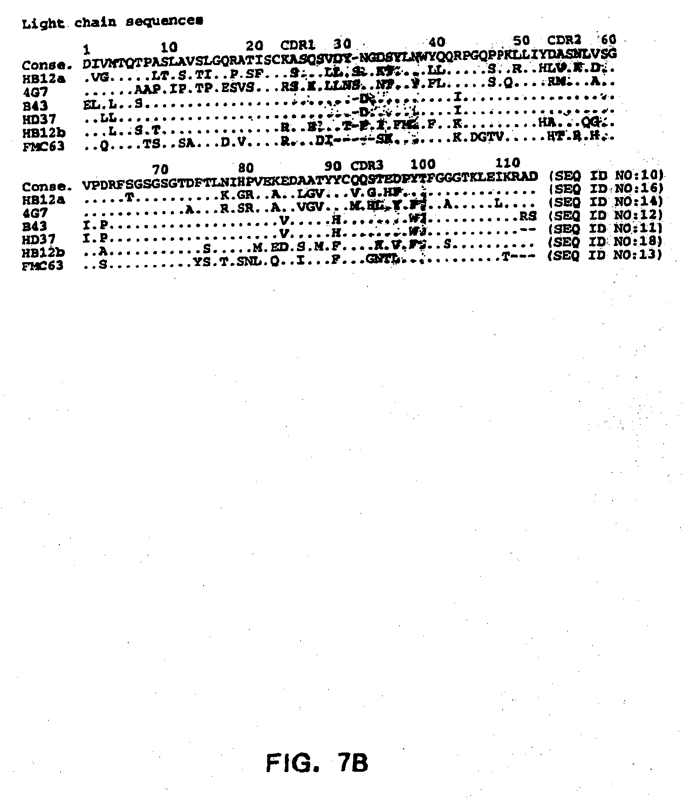

**Figs. 7A-7B** depict the amino acid sequence alignment of published mouse anti-(human) CD19 antibodies. **Fig. 7A** shows a sequence alignment for heavy chain V_{H}-D-J_{H} junctional sequences including a consensus sequence (SEQ ID NO:5), HB12a (SEQ ID NO:2), 4G7 (SEQ ID NO:6), HB12b (SEQ ID NO:4), HD37 (SEQ ID NO:7), B43 (SEQ ID NO:8), and FMC63 (SEQ ID NO:9). **Fig. 7B** shows light chain Vκ amino acid sequence analysis of anti-CD19 antibodies. Consensus sequence (SEQ ID NO:10), HB12a (SEQ ID NO:16), HB12b (SEQ ID NO:18), HD37 (SEQ ID NO:11), B43 (SEQ ID NO:12), FMC63 (SEQ ID NO:13), and 4G7 (SEQ ID NO:14) are aligned.

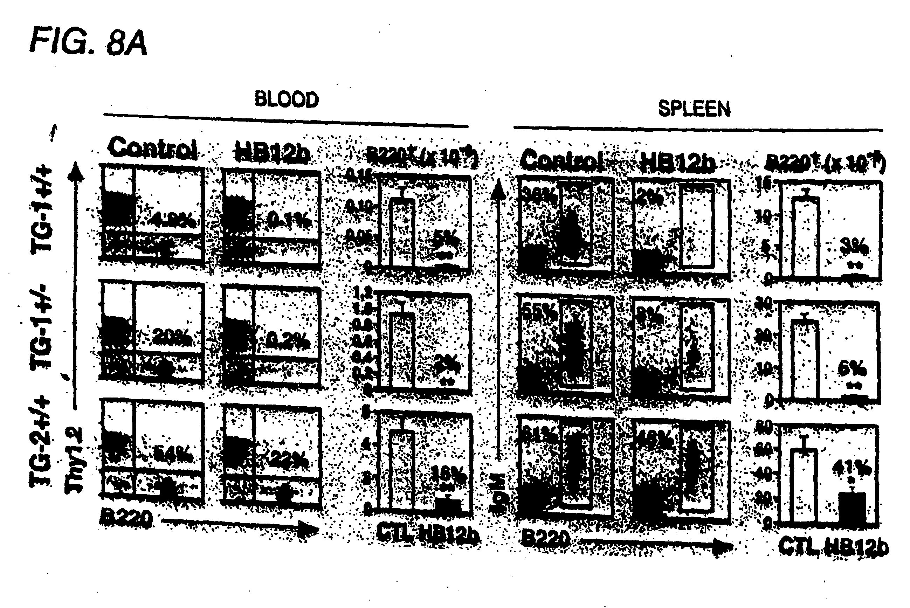

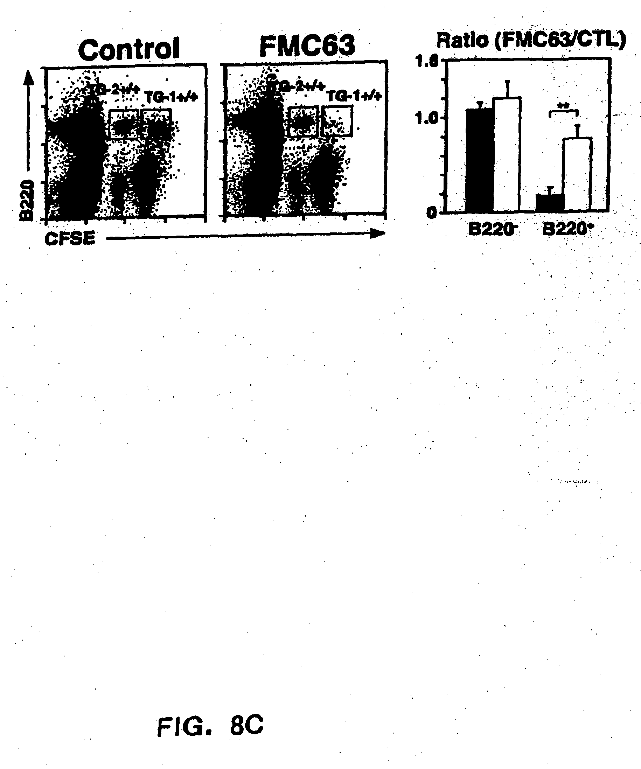

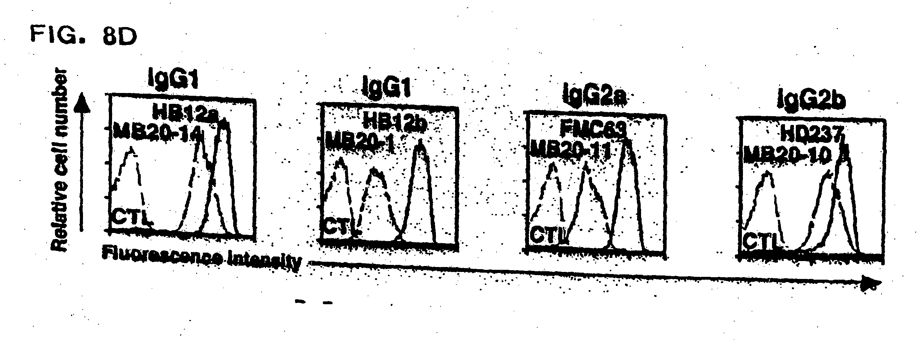

**Figs. 8A-8C** demonstrate that CD 19 density influences the efficiency of B cell depletion by anti-CD 19 antibodies *in vivo.* Representative blood and spleen B cell depletion in hCD19TG mice are shown following HB12b (**Fig**. **8A**) or FMC63 (**Fig**. **8B**) antibody treatment (seven days, 250 µg/mouse). **Fig. 8C** shows the relative anti-CD19 and anti-CD20 antibody-binding densities on blood B220⁺ B cells from TG-1^{+/-} mice. **Fig. 8D** shows the relative anti-CD19 and anti-CD20 antibody-binding densities on spleen B220⁺ B cells from TG-1^{+/-} mice.

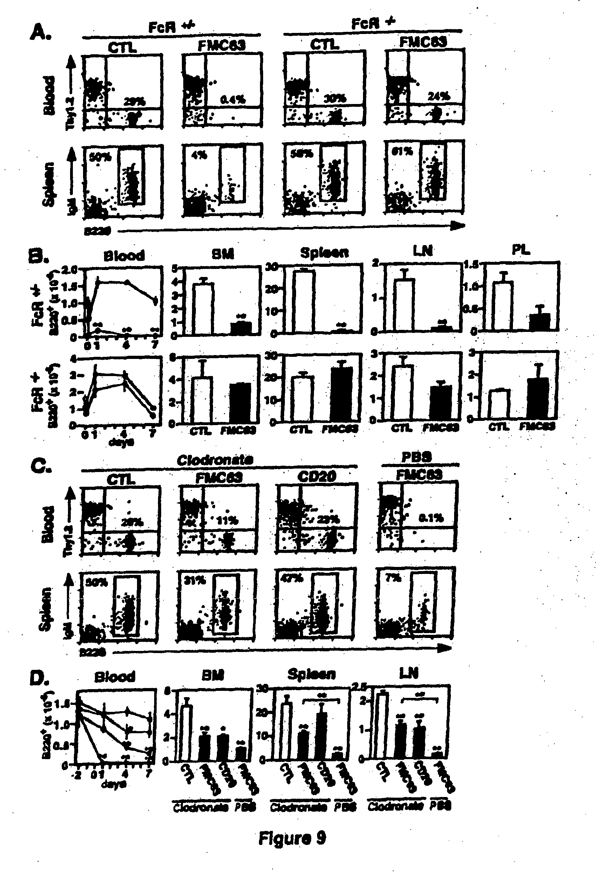

**Figs. 9A-9D** demonstrate B cell depletion following anti-CD19 antibody treatment is FcRy- and monocyte-dependent. **Fig. 9A** Representative blood and spleen B cell depletion 7 days after CD 19 or isotype-control antibody treatment of hCD 19 TG-1^{+/-} FcRγ^{+/-} or TG-1^{+/-} FcRγ^{-/-} littermates. **Fig. 9B** Blood and tissue B cell depletion seven days after antibody treatment of FcRγ^{-/-} littermates on day zero. **Fig. 9C** Representative B cell numbers in monocyte-depleted hCD19TG-1^{+/-} mice. **Fig. 9D** Blood and tissue B cell depletion seven days after antibody treatment.

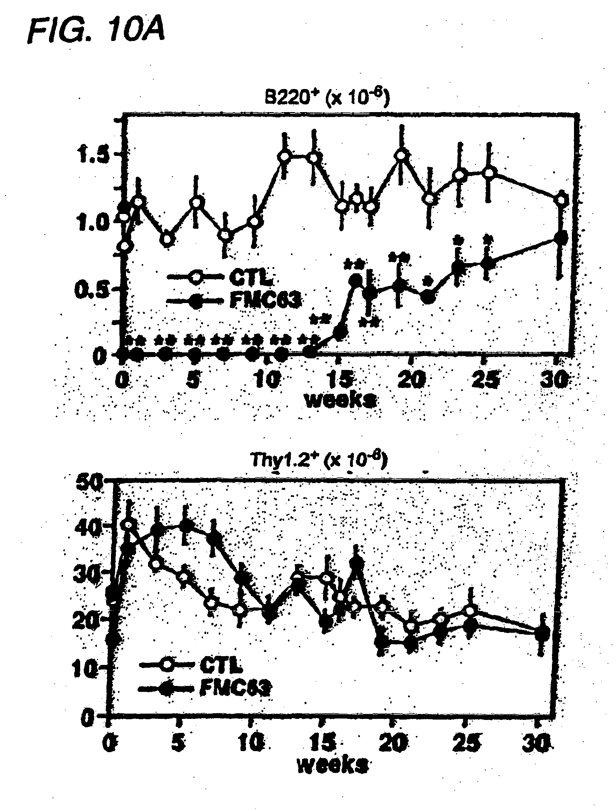

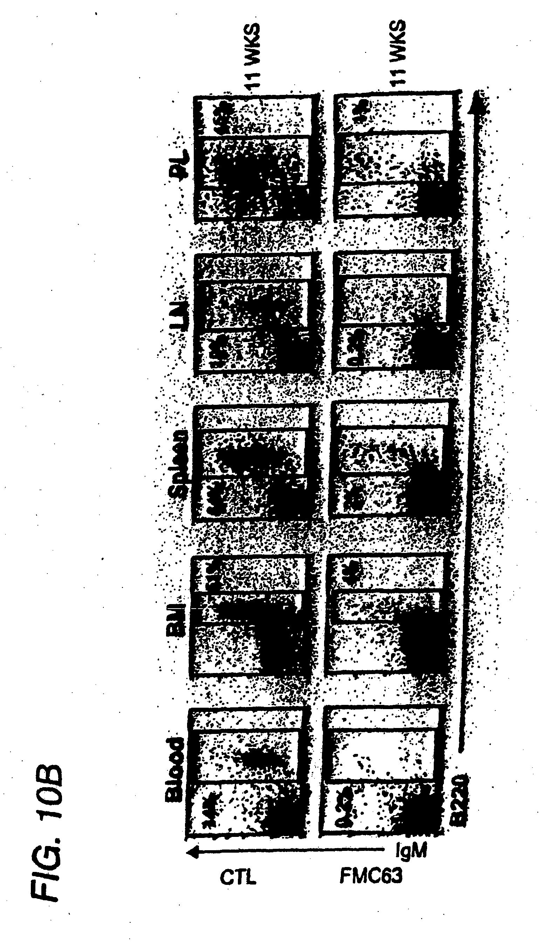

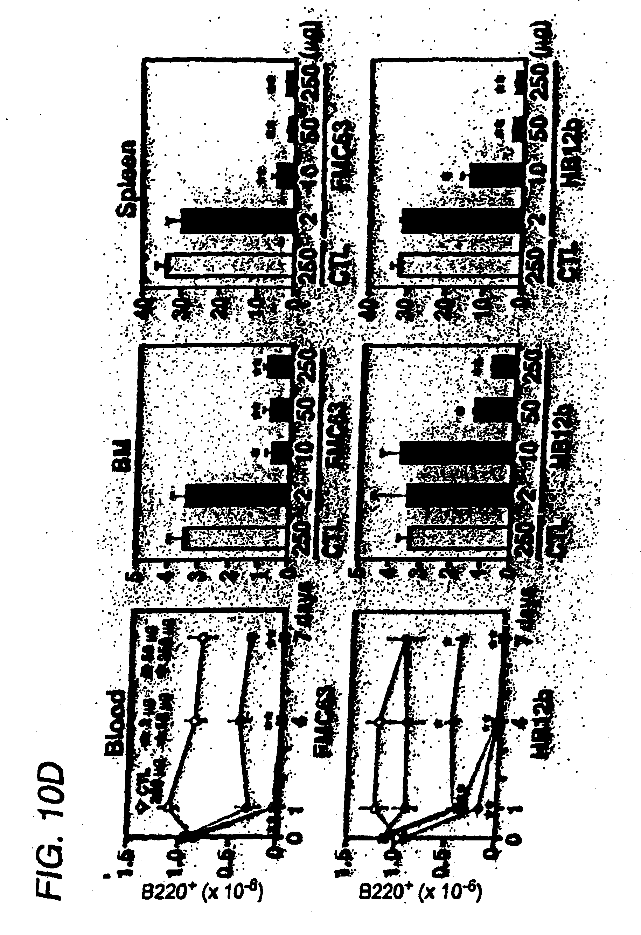

**Figs. 10A-10D** demonstrate duration and dose response of B cell depletion following anti-CD19 antibody treatment. **Fig. 10A** shows numbers of blood B220⁺ B cells and Thy-1⁺ T cells following FMC63 or isotype-control antibody treatment of TG-1^{+/-} mice on day zero. **Figs. 10B-C** show representative tissue B cell depletion in mice shown in **Fig. 10A** at 11, 16, and 30 weeks following antibody treatment, **Fig. 10D** shows anti-CD19 antibody dose responses for blood, bone marrow, and spleen B cell depletion.

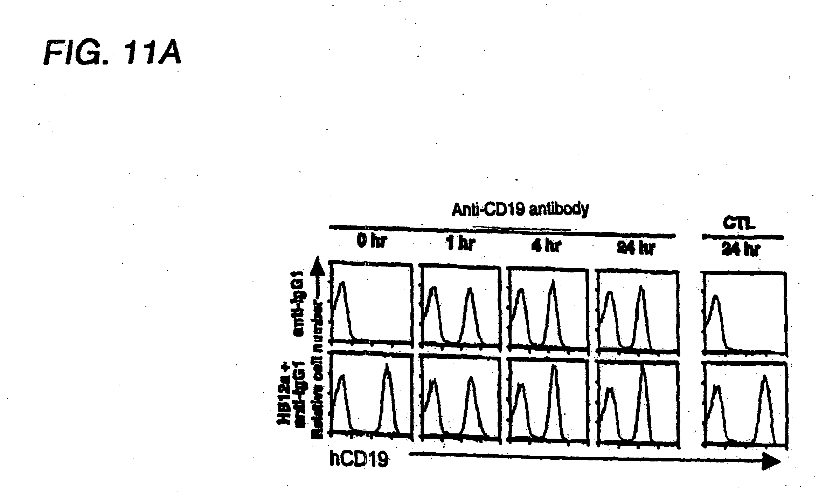

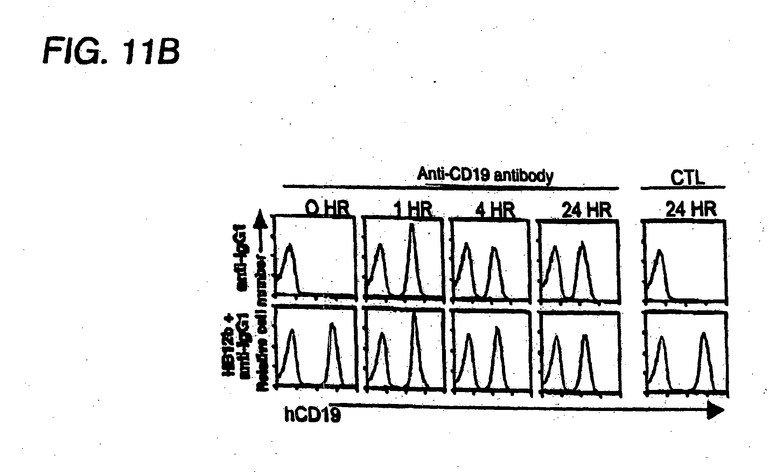

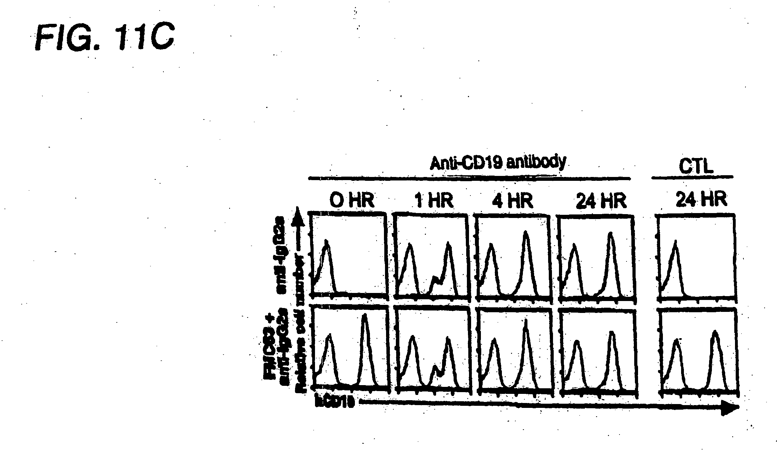

**Figs. 11A-11C** demonstrate that CD 19 is not internalized following antibody binding *in vivo.* Cell surface CD19 expression and B cell clearance in TG-1^{+/-} mice treated with HB12a (**Fig. 11A**), HB12b (**Fig. 11B**), FMC63 (**Fig. 11C**) or isotype-matched control antibody. (250 µg) *in vivo*.

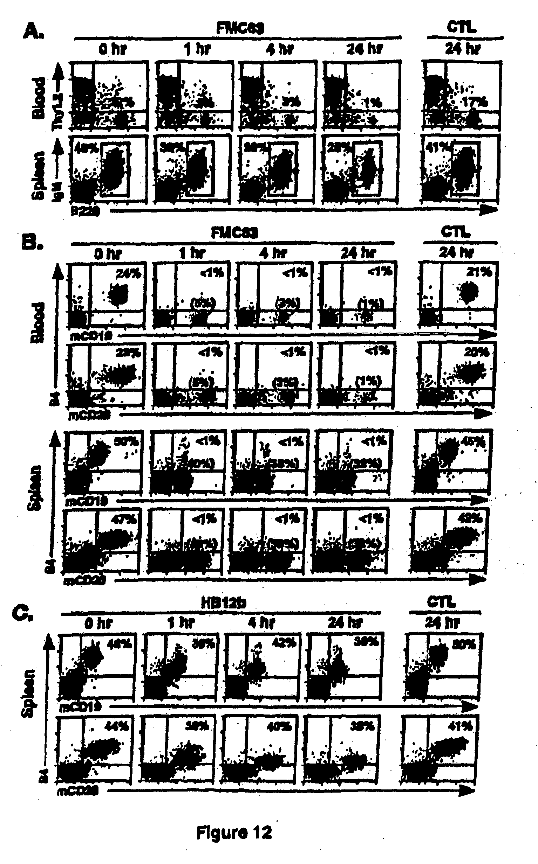

**Figs. 12A-12C** demonstrate CDC19 saturation following anti-CD19 antibody binding *in vivo*. **Fig. 12A** shows B cell clearance in TG-1^{+/-} mice treated with FMC63 or isotype-matched control antibody (250 µg) *in vivo.* **Fig. 12B** shows FMC63 antibody treatment (250 µg) saturates antibody-binding sites on hCD 19 within 1 hour of administration. **Fig. 12C** shows HB12b anti-CD19 antibody treatment (250 µg) saturates antibody-binding sites on hCD19 within 1 hour of administration as assessed in **Fig. 12B**.

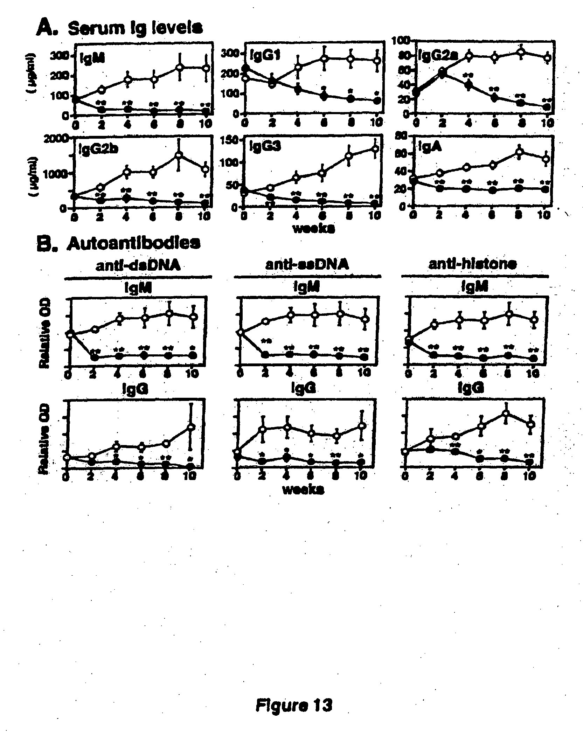

**Figs. 13A-13B** demonstrate anti-CD19 antibody treatment reduces serum immunoglobulin and autoantibody levels in TG-1^{+/-} mice. **Fig. 13A** depicts serum immunoglobulin levels and **Fig. 13B** anti-dsDNA, anti-ssDNA and anti-histone autoantibody levels after anti-CD 19 antibody treatment.

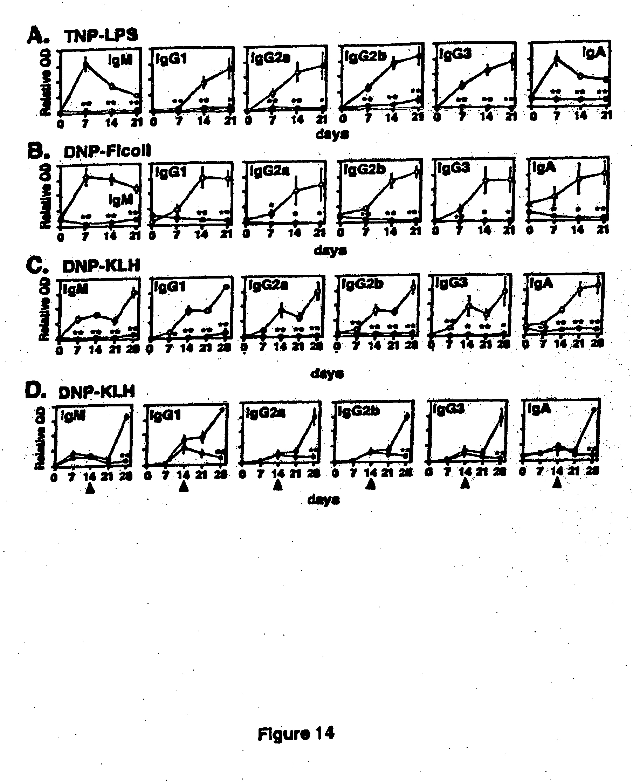

**Figs. 14A-14B** demonstrate anti-CD19 antibody treatment blocks humoral immune responses in TG-1^{+/-} mice. Antibody-treated mice were immunized with **Fig. 14A** TNP-LPS, **Fig. 14B** DNP-Ficoll and **Figs. 14C-14D** DNP-KLH. Littermates were treated with FMC63 (closed circles) or control (open circles) antibody (250 µg) either (A-C) 7 days before or:(D) 14 days after primary immunizations on day 0.

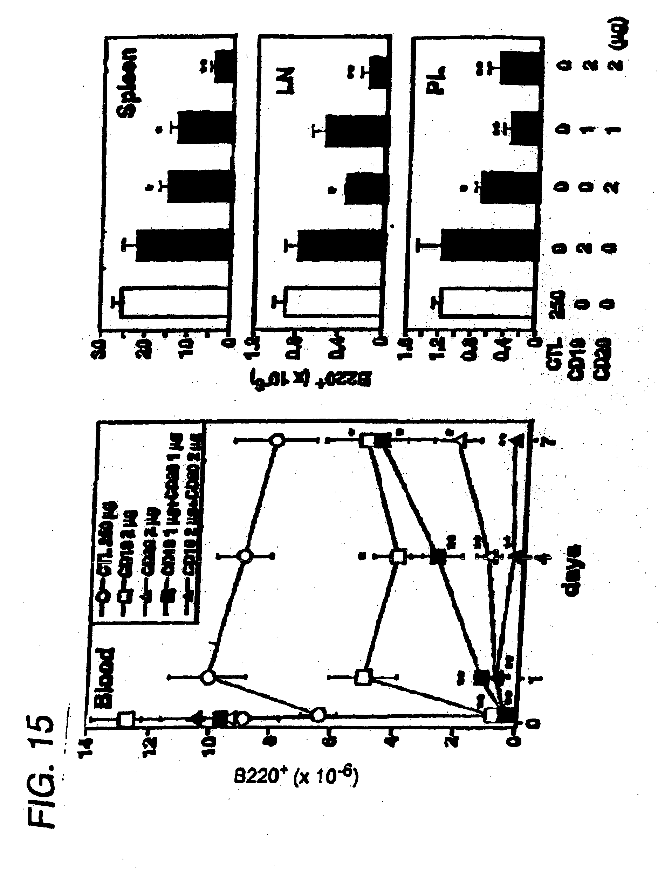

**Fig. 15** demonstrates that simultaneous anti-CD19 and anti-CD20 antibody treatments are additive.

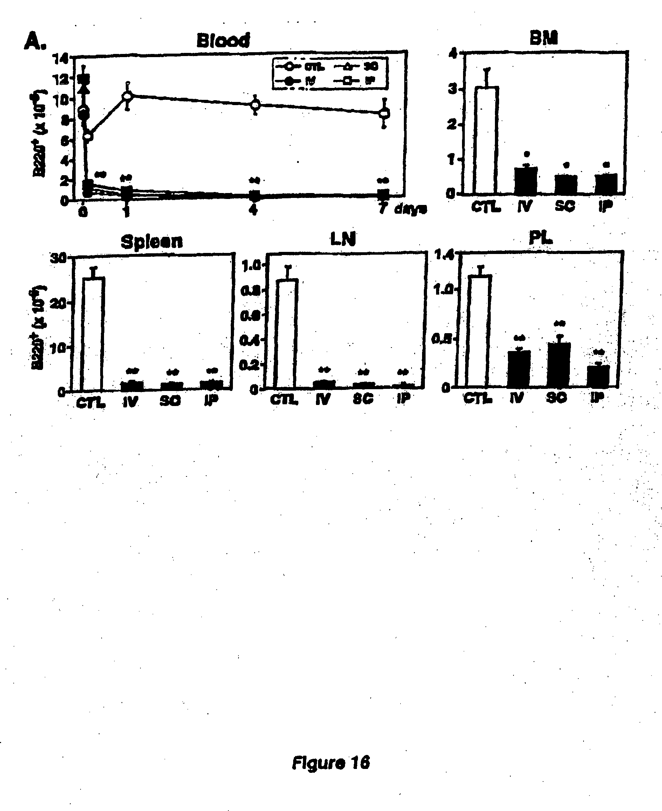

**Fig. 16** demonstrates that subcutaneous (s.c.), intraperitoneal (i.p.) and i.v. administration of anti-CD19 antibody effectively depletes circulating and tissue B cells *in vivo*.

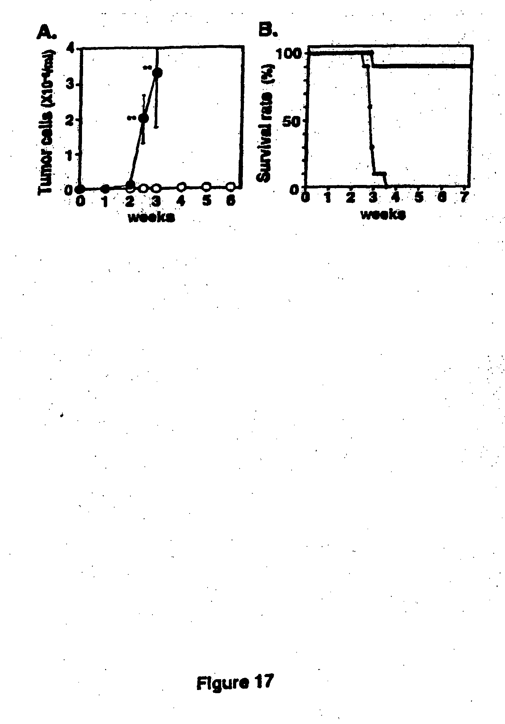

**Fig. 17A-17B****.** Anti-CD19 antibody treatment prevents hCD19⁺ lymphoma growth *in vivo* (**Fig**. **17A**) and increases survival rate (**Fig**. **17B**).

### 5. DETAILED DESCRIPTION OF THE INVENTION

The invention relates to immunotherapeutic compositions and antibodies for use in methods for the treatment of B cell diseases and disorders in human subjects, such as, but not limited to, B cell malignancies, using therapeutic antibodies that bind to the CD19 antigen and preferably mediate human ADCC. The present invention relates to pharmaceutical compositions comprising humanized, or chimeric anti-CD19 antibodies of the IgG1 or IgG3 human isotype. The present invention also relates to pharmaceutical compositions comprising humanized anti-CD19 antibodies of the IgG2 or IgG4 human isotype that preferably mediate human ADCC. In certain embodiments, the present invention also relates to pharmaceutical compositions comprising monoclonal humanized, or chimerized anti-CD 19 antibodies that can be produced by means known in the art.

Therapeutic formulations and regimens are described for treating human subjects diagnosed with B cell malignancies that derive from B cells and their precursors, including but not limited to, acute lymphoblastic leukemias (ALL), Hodgkin's lymphomas, non-Hodgkin's lymphomas, B cell chronic lymphocytic leukemias (CLL), multiple myeloma, follicular lymphoma, mantle cell lymphoma, pro-lymphocytic leukemias, hairy cell leukemias, common acute lymphocytic leukemias and some Null-acute lymphoblastic leukemias.

### 5.1 GENERATION OF ANTI-CD 19 ANTIBODIES

### 5.1.1. POLYCLONAL ANTI-CD19 ANTIBODIES

Polyclonal antibodies are preferably raised in animals by multiple subcutaneous (sc) or intraperitoneal (i.p:) injections of the relevant antigen and an adjuvant. It may be useful to conjugate the relevant antigen to a protein that is immunogenic in the species to be immunized, *e.g*., keyhole limpet hemocyanin, serum albumin, bovine thyroglobulin, or soybean trypsin inhibitor using a bifunctional or derivatizing agent, for example, maleimidobertzoyl sulfosuccinimide ester (conjugation through cysteine residues), N-hydroxysuccinimide (through lysine residues), glutaraldehyde, succunic anhydride, SOCl₂,

Animals are immunized against the antigen, immunogenic conjugates, or derivatives by combining, *e.g*., 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's incomplete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### 5.12. MONOCLONAL ANTI-CD 19 ANTIBODIES

The monoclonal anti-CD 19 antibodies of the invention exhibit binding specificity to human CD 19 antigen and can preferably mediate human ADCC. These antibodies can be generated using a wide variety of techniques known in the art including the use of hybridoma, recombinant, and phage display technologies, or a combination thereof. Antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the human CD 19 antigen. For example, the monoclonal antibodies to be used in accordance with the present invention may be made by the hybridoma method first described by Kohler et al., Nature, 256:495 (1975), which can be used to generate murine antibodies (or antibodies derived from other nonhuman mammals, *e.g.,* rat, goat, sheep, cows, camels, etc.), or human antibodies derived from transgenic animals *(see,* U.S. Patent Nos. 6,075,181, 6,114,598, 6,150,584, and 6,657,103). Alternatively, the monoclonal antibodies can be made by recombinant DNA methods *(see, e.g*., U.S. Patent No. 4,816,567) and include chimeric arid humanized antibodies. The "monoclonal antibodies" may also be isolated from phage antibody libraries using the techniques described in Clackson et al., Nature, 352:624-628 (1991) and Marks et al., J. Mol. Biol., 222:581-597 (1991), for example.

An engineered anti-CD 19 antibody can be produced by any means known in the art, including, but not limited to, those techniques described below and improvements to those techniques. Large-scale high-yield production typically involves culturing a host cell that produces the engineered anti-CD19 antibody and recovering the anti-CD19 antibody from the host cell culture.

### 5.1.3. HYBRIDOMA TECHNIQUE

Monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hatrimerling et al., in Monoclonal Antibodies and T Cell Hybridomas, 563-681 (Elsevier, N.Y., 1981). For example, in the hybridoma method, a mouse or other appropriate host animal, such as a hamster or macaque monkey, is immunized to elicit lymphocytes that produce or are capable of producing antibodies that will specifically bind to the protein used for immunization. Alternatively, lymphocytes may be immunized *in vitro.* Lymphocytes then are fused with myeloma cells using a suitable fusing agent, such as polyethylene glycol, to form a hybridoma cell (Goding, Monoclonal Antibodies: Principles and Practice, pp. 59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, CA, USA, and SP-2 or X63-Ag8.653 cells available from the American Type Culture Collection, Rockville, MD, USA. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the human CD 19 antigen. Preferably, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp. 59-103 (Academic Press, 1986)). Suitable culture media for this purpose include, for example, D-MEM or RPMI 1640 medium. In addition, the hybridoma cells may be grown *in vivo* as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

### 5.1.4. RECOMBINANT DNA TECHNIQUES

DNA encoding the anti-CD19 antibodies of the invention is readily isolated and sequenced using conventional procedures (*e.g*., by using oligonucleotide probes that are capable of binding specifically to genes encoding the heavy and light chains of the anti-CD19 antibodies). The hybridoma cells serve as a preferred source of such DNA. Once isolated, the DNA may be placed into expression vectors, which are then transfected into host cells such as *E. coli* cells, simian COS cells, Chinese hamster ovary (CHO) cells, or myeloma cells that do not otherwise produce immunoglobulin protein, to obtain the synthesis of anti-CD19 antibodies in the recombinant host cells.

In phage display methods, functional antibody domains are displayed on the surface of phage particles which carry the polynucleotide sequences encoding them. In particular, DNA sequences encoding V_{H} and V_{L} domains are amplified from animal cDNA libraries (*e.g*., human or murine cDNA libraries of affected tissues). The DNA encoding the V_{H} and V_{L} domains are recombined together with an scFv linker by PCR and cloned into a phagemid vector. The vector is electroporated in *E. coli* and the *E. coli* is infected with helper phage. Phage used in these methods are typically filamentous phage including fd and M13 and the V_{H} and V_{L} domains are usually recombinantly fused to either the phage gene III or gene VIII. Phage expressing an antigen-binding domain that binds to a particular antigen can be selected or identified with antigen, *e.g*., using labeled antigen or antigen bound or captured to a solid surface or bead. Examples of phage display methods that can be used to make the antibodies of the present invention include those disclosed in Brinkman et al., 1995, J. Immunol. Methods, 182:41-50; Ames et al., 1995, J. Immunol. Methods, 184:177-186; Kettleborough et al., 1994, Eur. J. Immunol., 24:952-958; Persic et al., 1997, Gene, 187:9-18; Burton et al., 1994, Advances in Immunology, 57:191-280; International Application No. PCT/GB91/O1134; International Publication Nos. WO 90/02809, WO 91/10737, WO 92/01047, WO 92/18619, WO 93/11236, WO 95/15982, WO 95/20401, and WO97/13844; and U.S. Patent Nos. 5,698,426, 5,223,409, 5,403,484, 5,580,717, 5,427,908, 5,750,753, 5,821,047, 5,571,698, 5,427,908, 5,516,637, 5,780,225, 5,658,727, 5,733,743, and 5,969,108.

As described in the above references, after phage selection, the antibody coding regions from the phage can be isolated and used to generate whole antibodies, including human antibodies, or any other desired antigen-binding fragment, and expressed in any desired host, including mammalian cells, insect cells, plant cells, yeast, and bacteria, *e.g*., as described below. Techniques to recombinantly produce Fab, Fab' and F(ab')₂ fragments can also be employed using methods known in the art such as those disclosed in PCT Publication No. WO 92/22324; Mullinax et al., 1992, BioTechniques, 12(6):864-869; Sawai et al., 1995, AJRI, 34:26-34; and Better et al., 1988, Science, 240:1041-1043.

Antibodies may be isolated from antibody phage libraries generated using the techniques described in McCafferty et al., Nature, 348:552-554 (1990). Clackson et al., Nature, 352:624-628 (1991). Marks et al., J. Mol. Biol., 222:581-597 (1991) describe the isolation of murine and human antibodies, respectively, using phage libraries. Chain shuffling can be used in the production of high affinity (nM range) human antibodies (Marks et al., Bio/Technology, 10:779-783 (1992)), as well as combinatorial infection and *in vivo* recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of anti-CD 19 antibodies.

To generate whole antibodies, PCR primers including V_{H} or V_{L} nucleotide sequences, a restriction site, and a flanking sequence to protect the restriction site can be used to amplify the V_{H} or V_{L} sequences in scFv clones. Utilizing cloning techniques known to those of skill in the art, the PCR amplified V_{H} domains can be cloned into vectors expressing a V_{H} constant region, *e.g*., the human gamma 4 constant region, and the PCR amplified V_{L} domains can be cloned into vectors expressing a V_{L} constant region, *e.g.*, human kappa or lamba constant regions. Preferably, the vectors for expressing the V_{H} or V_{L} domains comprise an EF-1α promoter, a secretion signal, a cloning site for the variable domain, constant domains, and a selection marker such as neomycin. The V_{H} and V_{L} domains may also be cloned into one vector expressing the necessary constant regions. The heavy chain conversion vectors and light chain conversion vectors are then co-transfected into cell lines to generate stable or transient cell lines that express full-length antibodies, *e.g*., IgG, using techniques known to those of skill in the art.

The DNA also may be modified, for example, by substituting the coding sequence for human heavy and light chain constant domains in place of the homologous murine sequences (U.S. Patent No. 4,81,6,567; Morrison et al., Proc. Natl. Acad. Sci. USA, 81:6851 (1984)), or by covalently joining to the immunoglobulin coding sequence all or part of the coding sequence for a non-immunoglobulin polypeptide.

### 5.1.5. CHIMERIC ANTIBODIES

The anti-CD19 antibodies herein specifically include chimeric antibodies (immunoglobulins) in which a portion of the heavy and/or light chain is identical with or homologous to corresponding sequences in antibodies derived from a particular species or belonging to a particular antibody class or subclass, while another portion of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:68,51-6855 (1984)). Chimeric antibodies of interest herein include "primatized" antibodies comprising variable domain antigen-binding sequences derived from a nonhuman primate (*e.g*., Old World Monkey, such as baboon, rhesus or cynomolgus monkey) and human constant region sequences (U.S. Patent No. 5,693,780).

### 5.1.6. HUMANIZED ANTIBODIES

A humanized antibody can be produced using a variety of techniques known in the art, including but not limited to, CDR-grafting (*see, e.g*., European Patent No. EP 239, 400; International Publication No. WO 91/09967; and U.S. Patent Nos. 5,225,539, 5,530,101, and 5,585,089), veneering or resurfacing (*see*, *e.g.*, European Patent Nos. EP 592,106 and EP 519,596; Padlan, 1991, Molecular Immunology 28(4/5):489-498; Studnicka et al., 1994, Protein Engineering, 7(6):805-814; and Roguska et al., 1994, PNAS, 91:969-973), chain shuffling *(see, e.g.*, U.S. Patent No. 5,565,332), and techniques disclosed in, *e.g.*, U.S. Patent No. 6,407,213, U.S. Patent No. 5,766,886, International Publication No. WO 9317105, Tan et al., J. Immunol., 169:1119-25 (2002), Caldas et al., Protein Eng., 13(5):353-60 (2000), Morea et al., Methods, 20(3):267-79 (2000), Baca et al., J. Biol. Chem., 272(16):10678-84 (1997), Roguska et al., Protein Eng., 9(10):895-904 (1996), Couto et al., Cancer Res., 55 (23 Supp):5973s-5977s (1995), Couto et al., Cancer Res., 55(8):1717-22 (1995), Sandhu JS, Gene, 150(2):409-10 (1994), and Pedersen et al., J. Mol. Biol., 235(3):959-73 (1994). Often, framework residues in the framework regions will be substituted with the corresponding residue from the CDR donor antibody to alter, preferably improve, antigen binding. These framework substitutions are identified by methods well-known in the art, *e.g*., by modeling of the interactions of the CDR and framework residues to identify framework residues important for antigen binding and sequence comparison to identify unusual framework residues at particular positions. (*See, e.g.*, Queen et al., U.S. Patent No. 5,585,089; and Riechmann et al., 1988, Nature, 332:323.)

A humanized anti-CD 19 antibody has one or more amino acid residues introduced into it from a source which is nonhuman. These nonhuman amino acid residues are often referred to as "import" residues, which are typically taken from an "import" variable domain. Thus, humanized antibodies comprise one or more CDRs from nonhuman immunoglobulin molecules and framework regions from human. Humanization of antibodies is well-known in the art and can essentially be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody, *i.e.*, CDR-grafting (EP 239,400; PCT Publication No. WO 91/09967; and U.S. Patent Nos. 4,816,567; 6,331,415; 5,225,539; 5,530,101; 5,585,089; 6,548,640), In such humanized chimeric antibodies, substantially less than an intact human variable domain has been substituted by the corresponding sequence from a nonhuman species. In practice, humanized antibodies are typically human antibodies in which some CDR residue and possibly some FR residues are substituted by residues from analogous sites in rodent antibodies. Humanization of anti-CD 19 antibodies can also be achieved by veneering or resurfacing (EP 592,106; EP 519,596; Padlan, 1991, Molecular Immunology 28(4/5):489-498; Studnicka et al., Protein Engineering, 7(6):805-814 (1994); and Roguska et al., PNAS, 91:969-973 (1994)) or chain shuffling (U.S. Patent No. 5,565,332).

The choice of human variable domains, both light and heavy, to be used in making the humanized antibodies is to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987)). Another method uses a particular framework derived from the consensus sequence of all human antibodies of a particular subgroup of light or heavy chains. The same framework may be used for several different humanized anti-CD 19 antibodies (Carter et al., Proc. Natl. Acad. Sci. USA, 89:4285 (1992); Presta et al., J. Immunol., 151:2623 (1993)).

Anti-CD 19 antibodies can be humanized with retention of high affinity for CD 19 and other favorable biological properties. The humanized antibodies may be prepared by a process of analysis of the parental sequences and various conceptual humanized products using three-dimensional models of the parental and humanized sequences. Three-dimensional immunoglobulin models are commonly available and-are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind CD19. In this way, FR residues can be selected and combined from the recipient and import sequences so that the desired antibody characteristic, such as increased affinity for CD19, is achieved. In general, the CDR residues are directly and most substantially involved in influencing antigen binding.

A "humanized" antibody retains a similar antigenic specificity as the original antibody, *i.e.*, in the present invention, the ability to bind human CD 19 antigen. However, using certain methods of humanization, the affinity and/or specificity of binding of the antibody for human CD19 antigen may be increased using methods of "directed evolution," as described by Wu et al., J. Mol. Biol., 294:151 (1999).

### 5.1.7. HUMAN ANTIBODIES

For *in vivo* use of antibodies in humans, it may be preferable to use human antibodies. Completely human antibodies are particularly desirable for therapeutic treatment of human subjects. Human antibodies can be made by a variety of methods known in the art including phage display methods described above using antibody libraries derived from human immunoglobulin sequences, including improvements to these techniques. *See, also*, U.S. Patent Nos. 4,444,887 and 4,716,111; and PCT publications WO 98/46645, WO 98/50433, WO 98/24893, WO98/16654, WO 96/34096, WO 96/33735, and WO 91/10741. A human antibody can also be an antibody wherein the heavy and light chains are encoded by a nucleotide sequence derived from one or more sources of human DNA.

Human anti-CD 19 antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. For example, it has been described that the homozygous deletion of the antibody heavy chain joining region (JH) gene in chimeric and germ-line mutant mice results in complete inhibition of endogenous antibody production. The modified embryonic stem cells are expanded and microinjected into blastocysts to produce chimeric mice. The chimeric mice are then bred to produce homozygous offspring which express human antibodies. The transgenic mice are immunized in the normal fashion with a selected antigen, *e.g*., all or a portion of a polypeptide of the invention. Anti-CD 19 antibodies directed against the human CD 19 antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies, including, but not limited to, IgG1 (gamma 1) and IgG3. For an overview of this technology for producing human antibodies, *see,* Lonberg and Huszar (Int. Rev. Immunol., 13:65-93 (1995)). For a detailed discussion of this technology for producing human antibodies and human monoclonal antibodies and protocols for producing such antibodies, *see*, *e.g.*, PCT Publication Nos. WO 98/24893, WO 96/34096, and WO 96/33735; and U.S. Patent Nos. 5,413,923; 5,625,126; 5,633,425; 5,569,825; 5,661,016; 5,545,806; 5,814,318; and 5,939,598. In addition, companies such as Abgenix, Inc. (Freemont, CA) and Genpharm (San Jose, CA) can be engaged to provide human antibodies directed against a selected antigen using technology similar to that described above. For a specific discussion of transfer of a human germ-line immunoglobulin gene array in germ-line mutant mice that will result in the production of human antibodies upon antigen challenge *see, e.g*., Jakobovits et al., Proc. Natl. Acad. Sci. USA, 90:2551 (1993); Jakobovits et al., Nature, 362:255-258 (1993); Bruggermann et al., Year in Immunol., 7:33 (1993); and Duchosal et al., Nature, 355:258 (1992).

Human antibodies can also be derived from phage-display libraries (Hoogenboom et al., J. Mol. Biol., 227:381 (1991); Marks et al., J. Mol. Biol., 222:581-597 (1991); Vaughan et al., Nature Biotech., 14:309 (1996)). Phage display technology (McCafferty et al., Nature, 348:552-553 (1990)) can be used to produce human antibodies and antibody fragments *in vitro*, from immunoglobulin variable (V) domain gene repertoires from unimmunized donors. According to this technique, antibody V domain genes are cloned inframe into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for their review *see, e.g.*, Johnson, Kevin S. and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of unimmunized mice. A repertoire of V genes from unimmunized human donors can be constructed and antibodies to a diverse array of antigens (including self-antigens) can be isolated essentially following the techniques described by Marks et al., J. Mol. Biol., 222:581-597 (1991), or Griffith et al., EMBO J., 12:725-734 (1993). *See, also,* U.S. Patent Nos. 5,565,332 and 5,573,905.

Human antibodies may also be generated by *in vitro* activated B cells *(see,* U.S. Patents 5,567,610 and 5,229,275). Human antibodies may also be generated *in vitro* using hybridoma techniques such as, but not limited to, that described by Roder et al. (Methods Enzymol., 121:140-167 (1986)).

### 5.1.8. ALTERED/MUTANT ANTIBODIES

The anti-CD 19 antibodies of the compositions and for use in the methods of the invention can be mutant antibodies. As used herein, "antibody mutant" or "altered antibody" refers to an amino acid sequence variant of an anti-CD 19 antibody wherein one or more of the amino acid residues of an anti-CD19 antibody have been modified. The modifications to the amino acid sequence of the anti-CD19 antibody, include modifications to the sequence to improve affinity or avidity of the antibody for its antigen, and/or modifications to the Fc portion of the antibody to improve effector function. The modifications may be made to any known anti-CD 19 antibodies or anti-CD 19 antibodies identified as described herein. Such altered antibodies necessarily have less than 100% sequence identity or similarity with a known anti-CD19 antibody. The altered antibody may have an amino acid sequence having at least 25%, 35%, 45%, 55%, 65%, or 75% amino acid sequence identity or similarity with the amino acid sequence of either the heavy or light chain variable domain of an anti-CD19 antibody, more preferably at least 80%, more preferably at least 85%, more preferably at least 90%, and most preferably at least 95%. The altered antibody may have an amino acid sequence having at least 25%, 35%, 45%, 55%, 65%, or 75% amino acid sequence identity or similarity with the amino acid sequence of the heavy chain CDR1, CDR2, or CDR3 of an anti-CD19 antibody, more preferably at least 80%, more preferably at least 85%, more preferably at least 90%, and most preferably at least 95%. In a preferred embodiment, the altered antibody will maintain human CD 19 binding capability. The anti-CD 19 antibody of the invention may comprise a heavy chain that is about 10%, 15%, 20%, 25%, 30%, 35%, 40%, 45%, 50%, 55%, 60%, 65%, 70%, 75%, 80%, 85%, 90%, 95% or more identical to an amino acid sequence ofSEQ ID NO:2 (**Fig. 5A**) corresponding to the heavy chain of HB12a. The anti-CD19 antibody of the invention may comprise a heavy chain that is about 10%, 15%, 20%, 25%, 30%, 35%, 40%, 45%, 50%, 55%, 60%, 65%, 70%, 75%, 80%, 85%, 90%, 95% or more identical to ain amino acid sequence of SEQ ID NO:4 (**Fig. 5B**) corresponding to the heavy chain of HB12b. The anti-CD19 antibody of the invention may comprise a light chain that is about 10%, 15%, 20%, 25%, 30%, 35%, 40%, 45%, 50%, 55%, 60%, 65%, 70%, 75%, 80%, 85%, 90%, 95% or more identical to an amino acid sequence of SEQ ID NO:16 (**Fig. 6A**) corresponding to the light chain of HB12a. The anti-CD19 antibody of the invention may comprise a light chain that is about 10%, 15%, 20%, 25%, 30%, 35%, 40%, 45%, 50%, 55%, 60%, 65%, 70%, 75%, 80%, 85%, 90%, 95% or more identical to an amino acid sequence of SEQ ID NO:18 (**Fig. 6B**) corresponding to the light chain of HB12b. Hybridomas producing HB 12a and HB 12b anti-CD 19 antibodies have been deposited under ATCC deposit nos. PTA-6580 and PTA-6581.

Identity or similarity with respect to this sequence is defined herein as the percentage of amino acid residues in the candidate sequence that are identical (*i.e*., same residue) or similar (*i.e.*, amino acid residue from the same group based on common side-chain properties, *see* below) with anti-CD19 antibody residues, after aligning the sequences and introducing gaps, if necessary, to achieve the maximum percent sequence identity. None of N-terminal, C-terminal, or internal extensions, deletions, or insertions into the antibody sequence outside of the variable domain shall be construed as affecting sequence identity or similarity.

"% identity," as known in the art, is a measure of the relationship between two polynucleotides or two polypeptides, as determined by comparing their sequences. In general, the two sequences to be compared are aligned to give a maximum correlation between the sequences. The alignment of the two sequences is examined and the number of positions giving an exact amino acid or nucleotide correspondence between the two sequences determined, divided by the total length of the alignment and multiplied by 100 to give a % identity figure. This % identity figure may be determined over the whole length of the sequences to be compared, which is particularly suitable for sequences of the same or very similar length and which are highly homologous, or over shorter defined lengths, which is more suitable for sequences of unequal length or which have a lower level of homology.

For example, sequences can be aligned with the software clustalw under Unix which generates a file with an ".aln" extension, this file can then be imported into the Bioedit program (Hall, T.A. 1999, BioEdit: a user-friendly biological sequence alignment editor and analysis program for Windows 95/98/NT. Nucl. Acids. Symp. Ser. 41:95-98) which opens the .aln file. In the Bioedit window, one can choose individual sequences (two at a time) and alignment them. This method allows for comparison of the entire sequence.

Methods for comparing the identity of two or more sequences are well-known in the art. Thus for instance, programs are available in the Wisconsin Sequence Analysis Package, version 9.1 (Devereux J. et al., Nucleic Acids Res., 12:387-395, 1984, available from Genetics Computer Group, Madison, WI, USA). The determination of percent identity between two sequences can be accomplished using a mathematical algorithm. For example, the programs BESTFIT and GAP, may be used to determine the % identity between two polynucleotides and the % identity between two polypeptide sequences. BESTFIT uses the "local homology" algorithm of Smith and Waterman (Advances in Applied Mathematics, 2:482-489, 1981) and finds the best single region of similarity between two sequences. BESTFIT is more suited to comparing two polynucleotide or two polypeptide sequences which are dissimilar in length, the program assuming that the shorter sequence represents a portion of the longer. In comparison, GAP aligns two sequences finding a "maximum similarity" according to the algorithm of Neddleman and Wunsch (J. Mol. Biol., 48:443-354, 1970). GAP is more suited to comparing sequences which are approximately the same length and an alignment is expected over the entire length. Preferably the parameters "Gap Weight" and "Length Weight" used in each program are 50 and 3 for polynucleotides and 12 and 4 for polypeptides, respectively. Preferably % identities and similarities are determined when the two sequences being compared are optimally aligned.

Other programs for determining identity and/or similarity between sequences are also known in the art, for instance the BLAST family of programs (Karlin & Altschul, 1990, Proc. Natl. Acad. Sci. USA, 87:2264-2268, modified as in Karlin & Altschul, 1993, Proc. Natl. Acad. Sci. USA, 90:5873-5877, available from the National Center for Biotechnology Information (NCB), Bethesda; MD, USA and accessible through the home page of the NCBI at www.ncbi.nlm.nih.gov). These programs exemplify a preferred, non-limiting example of a mathematical algorithm utilized for the comparison of two sequences. Such an algorithm is incorporated into the NBLAST and XBLAST programs of Altschul et al., 1990, J. Mol. Biol. 215:403-410. BLAST nucleotide searches can be performed with the NBLAST program, score = 100, wordlength = 12 to obtain nucleotide sequences homologous to a nucleic acid molecule encoding all or a portion if an anti-CD 19 antibody of the invention. BLAST protein searches can be performed with the XBLAST program, score = 50, wordlength = 3 to obtain amino acid sequences homologous to a protein molecule of the invention. To obtain gapped alignments for comparison purposes, Gapped BLAST can be utilized as described in Altschul et al., 1997, Nucleic Acids Res., 25:3389-3402. Alternatively, PSI-Blast can be used to perform an iterated search which detects distant relationships between molecules (*Id.*). When utilizing BLAST, Gapped BLAST, and PSI-Blast programs, the default parameters of the respective programs (*e.g*., XBLAST and NBLAST) can be used. *See,* http://www.ncbi.nlm.nih.gov. Another preferred, non-limiting example of a mathematical algorithm utilized for the comparison of sequences is the algorithm of Myers and Miller, 1988, CABIOS 4:11-17. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

Another non-limiting example of a program for determining identity and/or similarity between sequences known in the art is FASTA (Pearson W.R. and Lipman D.J., Proc. Nat. Acad. Sci. USA, 85:2444-2448, 1988, available as part of the Wisconsin Sequence Analysis Package). Preferably the BLOSUM62 amino acid substitution matrix (Henikoff S. and Henikoff J.G., Proc. Nat. Acad. Sci. USA, 89:10915-10919, 1992) is used in polypeptide sequence comparisons including where nucleotide sequences are first translated into amino acid sequences before comparison.

Yet another non-limiting example of a program known in the art for determining identity and/or similarity between amino acid sequences is SeqWeb Software (a web-based interface to the GCG Wisconsin Package: Gap program) which is utilized with the default algorithm and parameter settings of the program: blosum62, gap weight 8, length weight 2.

The percent identity between two sequences can be determined using techniques similar to those described above, with or without allowing gaps. In calculating percent identity, typically exact matches are counted.

Preferably the program BESTFIT is used to determine the % identity of a query polynucleotide or a polypeptide sequence with respect to a polynucleotide or a polypeptide sequence of the present invention, the query and the reference sequence being optimally aligned and the parameters of the program set at the default value.

To generate an altered antibody, one or more amino acid alterations (*e.g*., substitutions) are introduced in one or more of the hypervariable regions of the species-dependent antibody. Alternatively, or in addition, one or more alterations (*e.g*., substitutions) of framework region residues may be introduced in an anti-CD19 antibody where these result in an improvement in the binding affinity of the antibody mutant for the antigen from the second mammalian species. Examples of framework region residues to modify include those which non-covalently bind antigen directly (Amit et al., Science, 233:747-753 (1986)); interact with/effect the conformation of a CDR (Chothia et al., J. Mol. Biol., 196:901-917 (1987)); and/or participate in the V_{L}-V_{H} interface (EP 239 400B1). In certain embodiments, modification of one or more of such framework region residues results in an enhancement of the binding affinity of the antibody for the antigen from the second mammalian species. For example, from about one to about five framework residues may be altered in this embodiment of the invention. Sometimes, this may be sufficient to yield an antibody mutant suitable for use in preclinical trials, even where none of the hypervariable region residues have been altered. Normally, however, an altered antibody will comprise additional hypervariable region alteration(s).

The hypervariable region residues which are altered may be changed randomly, especially where the starting binding affinity of an anti-CD 19 antibody for the antigen from the second mammalian species is such that such randomly produced altered antibody can be readily screened.

One useful procedure for generating such an altered antibody is called "alanine scanning mutagenesis" (Cunningham and Wells, Science, 244:1081-1085 (1989)). Here, one or more of the hypervariable region residue(s) are replaced by alanine or polyalanine residue(s) to affect the interaction of the amino acids with the antigen from the second mammalian species. Those hypervariable region residue(s) demonstrating functional sensitivity to the substitutions then are refined by introducing additional or other mutations at or for the sites of substitution. Thus, while the site for introducing an amino acid sequence variation is predetermined, the nature of the mutation per se need not be predetermined. The Ala-mutants produced this way are screened for their biological activity as described herein.

Another procedure for generating such an altered antibody involves affinity maturation using phage display (Hawkins et al., J. Mol. Biol., 254:889-896 (1992) and Lowman et al., Biochemistry, 30(45):10832-10837 (1991)). Briefly, several hypervariable region sites (*e.g.*, 6-7 sites) are mutated to generate all possible amino acid substitutions at each site. The antibody mutants thus generated are displayed in a monovalent fashion from filamentous phage particles as fusions to the gene III product of M 13 packaged within each particle. The phage-displayed mutants are then screened for their biological activity (*e.g.*, binding affinity) as herein disclosed.

Mutations in antibody sequences may include substitutions, deletions, including internal deletions, additions, including additions yielding fusion proteins, or conservative substitutions of amino acid residues within and/or adjacent to the amino acid sequence, but that result in a "silent" change, in that the change produces a functionally equivalent anti-CD 19 antibody. Conservative amino acid substitutions may be made on the basis of similarity in polarity, charge, solubility, hydrophobicity, hydrophilicity, and/or the amphipathic nature of the residues involved. For example, non-polar (hydrophobic) amino acids include alanine, leucine, isoleucine; valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid. In addition, glycine and proline are residues that can influence chain orientation. Non-conservative substitutions will entail exchanging a member of one of these classes for another class. Furthermore, if desired, non-classical amino acids or chemical amino acid analogs can be introduced as a substitution or addition into the antibody sequence. Non-classical amino acids include, but are not limited to, the D-isomers of the common amino acids, α -amino isobutyric acid, 4-aminobutyric acid, Abu, 2-amino butyric acid, γ-Abu, ε-Ahx, 6-amino hexanoic acid, Aib, 2-amino isobutyric acid, 3-amino propionic acid, ornithine, norleucine, norvaline, hydroxyproline, sarcosine, citruiline, cysteic acid, t-butylglycine, t-butylalanine, phenylglycine, cyclohexylalanine, β-alanine, fluoro-amino acids, designer amino acids such as β-methyl amino acids, Cα-methyl amino acids, Nα-methyl amino acids, and amino acid analogs in general.

The sites selected for modification may be affinity matured using phage display *(see* above).

Any technique for mutagenesis known in the art can be used to modify individual nucleotides in a DNA sequence, for purposes of making amino acid substitution(s) in the antibody sequence, or for creating/deleting restriction sites to facilitate further manipulations. Such techniques include, but are not limited to, chemical mutagenesis, *in vitro* site-directed mutagenesis (Kunkel, Proc. Natl. Acad. Sci. USA, 82:488 (1985); Hutchinson, C. et al., J. Biol. Chem., 253:6551 (1978)), oligonucleotide-directed mutagenesis (Smith, Ann. Rev. Genet., 19:423-463 (1985); Hill et al., Methods Enzymol., 155:558-568 (1987)), PCR-based overlap extension (Ho et al., Gene, 77:51-59 (1989)), PCR-based megaprimer mutagenesis (Sarkar et al., Biotechniques, 8:404-407 (1990)), etc. Modifications can be confirmed by double-stranded dideoxy DNA sequencing.

In certain embodiments of the invention the anti-CD 19 antibodies can be modified to produce fusion proteins; *i.e.*, the antibody, or a fragment fused to a heterologous protein, polypeptide or peptide. The protein fused to the portion of an anti-CD19 antibody may be an enzyme component of ADEPT. Examples of other proteins or polypeptides that can be engineered as a fusion protein with an anti-CD 19 antibody include, but are not limited to toxins such as ricin, abrin, ribonuclease, DNase I, Staphylococcal enterotoxin-A, pokeweed anti-viral protein, gelonin, diphtherin toxin, Pseudomonas exotoxin, and Pseudomonas endotoxin. *See,* for example, Pastan et al.., Cell, 47:641 (1986), and Goldenberg et al., Cancer Journal for Clinicians, 44:43 (1994). Enzymatically active toxins and fragments thereof which can be used include diphtheria A chain, non-binding active fragments of diphtheria toxin, exotoxin A chain (from Pseudomonas aeruginosa), ricin A chain, abrin A chain, modeccin A chain, alpha-sarcin, *Aleurites fordii* proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. *See,* for example, WO 93/21232 published October 28, 1993.

Additional fusion proteins may be generated through the techniques of gene-shuffling, motif-shuffling, exon-shuffling, and/or codon-shuffling (collectively, referred to as "DNA shuffling"). DNA shuffling may be employed to alter the activities of SYNAGIS^{®} or fragments thereof (*e.g*., an antibody or a fragment thereof with higher affinities and lower dissociation rates). *See,* generally, U.S. Patent Nos. 5,605,793; 5,811,238; 5,830,721; 5,834,252; and 5,837,458, and Patten et al., 1997, Curr. Opinion Biotechnol., 8:724-33 ; Harayama, 1998, Trends Biotechnol. 16(2):76-82; Hansson et al., 1999, J Mol. Biol., 287:265-76; and Lorenzo and Blasco, 1998, Biotechniques 24(2):308-313. The antibody may be a binding-domain immunoglobulin fusion protein as described in U.S. Publication 20030118592, U.S. Publication 200330133939, and PCT Publication WO 02/056910, all to Ledbetter *et al.*

### 5.1.9. DOMAIN ANTIBODIES

The anti-CD19 antibodies of the compositions and for use in the methods of the invention can be domain antibodies, *e.g*., antibodies containing the small functional binding units of antibodies, corresponding to the variable regions of the heavy(V_{H}) or light (V_{L}) chains of human antibodies. Examples of domain antibodies include, but are not limited to, those available from Domantis Limited (Cambridge, UK) and Domantis Inc. (Cambridge, MA, USA) that are specific to therapeutic targets *(see,* for example, WO04/058821; WO04/003019; U.S. Patent Nos. 6,291,158; 6,582,915; 6,696,245; and 6,593,081). Commercially available libraries of domain antibodies can be used to identify anti-CD19 domain antibodies. The anti-CD19 antibodies of the invention may comprise a CD19 functional binding unit and a Fc gamma receptor functional binding unit.

### 5.1.10 DIABODIES

The term "diabodies" refers to small antibody fragments with two antigen-binding sites, which fragments comprise a heavy chain variable domain (V_{H}) connected to a light chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

### - 5.1.11. VACCIBODIES

The anti-CD19 antibodies may be Vaccibodies. Vaccibodies are dimeric polypeptides. Each monomer of a vaccibody consists of a scFv with specificity for a surface molecule on APC connected through a hinge region and a Cγ3 domain to a second scFv. Vaccibodies containing as one of the scFv's an anti-CD19 antibody fragment may be used to juxtapose those B cells to be destroyed and an effector cell that mediates ADCC. For example, *see,* Bogen et al., U.S. Patent Application Publication No. 20040253238.

### 5.1.12. LINEAR ANTIBODIES

The anti-CD19 antibodies may be linear antibodies. Linear antibodies comprise a pair of tandem Fd segments (V_{H}-C_{H1} -V_{H}-C_{H1}) which form a pair of antigen-binding regions. Linear antibodies can be bispecific or monospecific. *See*, Zapata et al., Protein Eng., 8(10):1057-1062 (1995).

### 5.1.13. PARENT ANTIBODY

In certain embodiments of the invention, the anti-CD 19 antibody is a parent antibody. A "parent antibody" is an antibody comprising an amino acid sequence which lacks, or is deficient in, one or more amino acid residues in or adjacent to one or more hypervariable regions thereof compared to an altered/mutant antibody as herein disclosed. Thus, the parent antibody has a shorter hypervariable region than the corresponding hypervariable region of an antibody mutant as herein disclosed. The parent polypeptide may comprise a native sequence (*i.e.*, a naturally occurring) antibody (including a naturally occurring allelic variant) or an antibody with pre-existing amino acid sequence modifications (such as other insertions, deletions and/or substitutions) of a naturally occurring sequence. Preferably the parent antibody is a humanized antibody or a human antibody.

### 5.1.14. ANTIBODY FRAGMENTS

"Antibody fragments" comprise a portion of a full-length antibody, generally the antigen binding or variable region thereof. Examples of antibody fragments include Fab, Fab' , F(ab')₂, and Fv fragments; diabodies; linear antibodies; single-chain antibody molecules; and multispecific antibodies formed from antibody fragments.

Traditionally, these fragments were derived via proteolytic digestion of intact antibodies (*see, e.g.*, Morimoto et al., Journal of Biochemical and Biophysical Methods, 24:107-117 (1992) and Brennan et al., Science, 229:81 (1985)). However, these fragments can now be produced directly by recombinant host cells. For example, the antibody fragments can be isolated from the antibody phage libraries discussed above. Alternatively, Fab' -SH fragments can be directly recovered from *E. coli* and chemically coupled to form F(ab')₂ fragments (Carter et al., Bio/Technology, 10:163-167 (1992)). According to another approach, F(ab')₂ fragments can be isolated directly from recombinant host cell culture. Other techniques for the production of antibody fragments will be apparent to the skilled practitioner. In other embodiments, the antibody of choice is a single-chain Fv fragment (scFv). *See*, for example, WO 93/16185. In certain embodiments, the antibody is not a Fab fragment.

### 5.1.15. BISPECIFIC ANTIBODIES

Bispecific antibodies are antibodies that have binding specificities for at least two different epitopes. Exemplary bispecific antibodies may bind to two different epitopes of the B cell surface marker. Other such antibodies may bind a first B cell marker and further bind a second B cell surface marker. Alternatively, an anti-B cell marker binding arm may be combined with an arm which binds to a triggering molecule on a leukocyte such as a T cell receptor molecule (*e.g*., CD2 or CD3), or Fc receptors for IgG (FcγR), so as to focus cellular defense mechanisms to the B cell. Bispecific antibodies may also be used to localize cytotoxic agents to the B cell. These antibodies possess a B cell marker-binding arm and an arm which binds the cytotoxic agent (*e.g*., saporin, anti-interferon-α, vinca alkaloid, ricin A chain, methola-exate or radioactive isotope hapten). Bispecific antibodies can be prepared as full-length antibodies or antibody fragments (*e.g*., F(ab'): bispecific antibodies).

Methods for making bispecific antibodies are known in the art. (*See,* for example, Millstein et al., Nature, 305:537-539 (1983); Traunecker et al., EMBO J., 10:3655-3659 (1991); Suresh et al., Methods in Enzymology, 121:210 (1986); Kostelny et al.., J. Immunol, 148(5):1547-1553 (1992); Hollinger et al., Proc. Natl Acad. Sci. USA, 90:6444-6448 (1993); Gruber et al., J. Immunol., 152:5368 (1994); U.S. Patent Nos. 4,474,893; 4,714,681; 4,925,648; 5,573,920; 5,601,81; 95,731,168; 4,676,980; and 4,676,980, WO 94/04690; WO 91/00360; WO 92/200373; WO 93/17715; WO 92/08802; and EP 03089.)

In certain embodiments of the invention, the compositions and antibodies for use in the methods do not comprise a bispecific murine antibody with specificity for human CD 19 and the CD3 epsilon chain of the T cell receptor such as the bispecific antibody described by Daniel et al., Blood, 92:4750-4757 (1998). Where the anti-CD19 antibody of the compositions and for use in the methods of the invention is bispecific, the anti-CD 19 antibody may behumanized and has specificity for human CD 19 and is capable of binding to a human effector cell such as, for example, a monocyte/macrophage and/or a natural killer cell to effect cell death.

### 5.1.16. ENGINEERING EFFECTOR FUNCTION

It may be desirable to modify the anti-CD19 antibody for use in the invention with respect to effector function, so as to enhance the effectiveness of the antibody in treating B cell malignancies, for example. For example, cysteine residue(s) may be introduced in the Fc region, thereby allowing interchain disulfide bond formation in this region. The homodimeric antibody thus generated may have improved internalization capability and/or increased complement-mediated cell killing and/or antibody-dependent cellular cytotoxicity (ADCC). *See*, Caron et al., J. Exp Med., 176:1191-1195 (1992) and Shopes, B., J. Immunol., 148:2918-2922 (1992). Homodimeric antibodies with enhanced anti-tumor activity may also be prepared using heterobifunctional cross-linkers as described in Wolff et al., Cancer Research, 53:2560-2565 (1993). Alternatively, an antibody can be engineered which has dual Fc regions and may thereby have enhanced complement lysis and ADCC capabilities. *See,* Stevenson et al., Anti-Cancer Drug Design, 3:219-230 (1989).

Other methods of engineering Fc regions of antibodies so as to alter effector functions are known in the art (*e.g*., U.S. Patent Publication No. 20040185045 and PCT Publication No. WO 2004/016750, both to Koenig et al., which describe altering the Fc region to enhance the binding affinity for FcγRIIB as compared with the binding affinity for FCγRIIA; *see, also*, PCT Publication Nos. WO 99/58572 to Armour et al., WO 99/51642 to Idusogie et al., and U.S. 6,395,272 to Deo et al.). Methods of modifying the Fc region to decrease binding affinity to FcγRIIB are also known in the art (*e.g*., U.S. Patent Publication No. 20010036459 and PCT Publication No. WO 01/79299, both to Ravetch et al.). Modified antibodies having variant Fc regions with enhanced binding affinity for FcγRIIIA and/or FcγRIIA as compared with a wildtype Fc region have also been described (*e.g.*, PCT Publication Nos. WO 2004/063351, to Stavenhagen et al.).

*In vitro* assays known in the art can be used to determine whether the anti-CD19 ' antibodies used in the compositions and for use in the methods of the invention are capable of mediating ADCC, such as those described in Section 5.3.2.

### 5.1.17. VARIANT Fc REGIONS

Described herein are formulations of proteins comprising a variant Fc region. That is, a non naturally occurring Fc region, for example an Fc region comprising one or more non naturally occurring amino acid residues. Also encompassed by the variant Fc regions are Fc regions which comprise amino acid deletions, additions and/or modifications.

It will be understood that Fc region as used herein includes the polypeptides comprising the constant region of an antibody excluding the first constant region immunoglobulin domain. Thus Fc refers to the last two constant region immunoglobulin domains of IgA, IgD, and IgG, and the last three constant region immunoglobulin domains of IgE and IgM, and the flexible hinge N-terminal to these domains. For IgA and IgM Fc may include the J chain. For IgG, Fc comprises immunoglobulin domains Cgamma2 and Cgamma3 (Cγ2 and Cγ3) and the hinge between Cgammal (Cγ1) and Camma2 (Cγ2). Although the boundaries of the Fc region may vary, the human IgG heavy chain Fc region is usually defined to comprise residues C226 or P230 to its carboxyl-terminus, wherein the numbering is according to the EU index as in Kabat et al. (1991, NIH Publication 91-3242, National Technical Information Service, Springfield, VA). The "EU index as set forth in Kabat" refers to the residue numbering of the human IgG1 EU antibody as described in Kabat et al. supra. Fc may refer to this region in isolation, or this region in the context of an antibody, antibody fragment, or Fc fusion protein. An Fc variant protein may be an antibody, Fc fusion, or any protein or protein domain that comprises an Fc region. Particularly preferred are proteins comprising variant Fc regions, which are non naturally occurring variants of an Fc. Note: Polymorphisms have been observed at a number of Fc positions, including but not limited to Kabat 270, 272, 312, 315, 356, and 358, and thus slight differences between the presented sequence and sequences in the prior art may exist.

Described herein are Fc variant proteins which have altered binding properties for an Fc ligand (e.g., an Fc receptor, C1q) relative to a comparable molecule (e.g., a protein having the same amino acid sequence except having a wild type Fc region). Examples of binding properties include but are not limited to, binding specificity, equilibrium dissociation constant (KD), dissociation and association rates (Koff and Kon respectively), binding affinity and/or avidity. It is generally understood that a binding molecule (e.g., a Fc variant protein such as an antibody) with a low KD is preferable to a binding molecule with a high KD. However, in some instances the value of the kon or koff may be more relevant than the value of the KD. One skilled in the art can determine which kinetic parameter is most important for a given antibody application.

The affinities and binding properties of an Fc domain for its ligand, may be determined by a variety of in vitro assay methods (biochemical or immunological based assays) known in the art for determining Fc-FcγR interactions, i.e., specific binding of an Fc region to an FcγR including but not limited to, equilibrium methods (e.g., enzyme-linked immunoabsorbent assay (ELISA), or radioimmunoassay (RIA)), or kinetics (e.g., BIACORE® analysis), and other methods such as indirect binding assays, competitive inhibition assays, fluorescence resonance energy transfer (FRET), gel electrophoresis and chromatography (e.g., gel filtration). These and other methods may utilize a label on one or more of the components being examined and/or employ a variety of detection methods including but not limited to chromogenic, fluorescent, luminescent, or isotopic labels. A detailed description of binding affinities and kinetics can be found in Paul, W.E., ed., Fundamental Immunology, 4th Ed., Lippincott-Raven, Philadelphia (1999), which focuses on antibody-immunogen interactions.

The Fc variant protein may have enhanced binding to one or more Fc ligand relative to a comparable molecule. The Fc variant protein may have an affinity for an Fc ligand that is at least 2 fold, or at least 3 fold, or at least 5 fold, or at least 7 fold, or a least 10 fold, or at least 20 fold, or at least 30 fold, or at least 40 fold, or at least 50 fold, or at least 60 fold, or at least 70 fold, or at least 80 fold, or at least 90 fold, or at least 100 fold, or at least 200 fold greater than that of a comparable molecule. The Fc variant protein may have enhanced binding to an Fc receptor. The Fc variant protein may have enhanced binding to the Fc receptor FcγRIIIA. The Fc variant protein may have enhanced binding to the Fc receptor FcRn. The Fc variant protein may have enhanced binding to C1q relative to a comparable molecule.

The serum half-life of proteins comprising Fc regions may be increased by increasing the binding affinity of the Fc region for FcRn. The Fc variant protein may have enhanced serum half life relative to comparable molecule.

"Antibody-dependent cell-mediated cytotoxicity" or "ADCC" refers to a form of cytotoxicity in which secreted Ig bound onto Fc receptors (FcRs) present on certain cytotoxic cells (e.g., Natural Killer (NK) cells, neutrophils, and macrophages) enables these cytotoxic effector cells to bind specifically to an antigen-bearing target cell and subsequently kill the target cell with cytotoxins. Specific high-affinity IgG antibodies directed to the surface of target cells "arm" the cytotoxic cells and are absolutely required for such killing. Lysis of the target cell is extracellular, requires direct cell-to-cell contact, and does not involve complement. It is contemplated that, in addition to antibodies, other proteins comprising Fc regions, specifically Fc fusion proteins, having the capacity to bind specifically to an antigen-bearing target cell will be able to effect cell-mediated cytotoxicity. For simplicity, the cell-mediated cytotoxicity resulting from the activity of an Fc fusion protein is also referred to herein as ADCC activity.

The ability of any particular Fc variant protein to mediate lysis of the target cell by ADCC can be assayed. To assess ADCC activity an Fc variant protein of interest is added to target cells in combination with immune effector cells, which may be activated by the antigen antibody complexes resulting in cytolysis of the target cell. Cytolysis is generally detected by the release of label (e.g. radioactive substrates, fluorescent dyes or natural intracellular proteins) from the lysed cells. Useful effector cells for such assays include peripheral blood mononuclear cells (PBMC) and Natural Killer (NK) cells. Specific examples of in vitro ADCC assays are described in Wisecarver et al., 1985 79:277-282; Bruggemann et al., 1987, J Exp Med 166:1351-1361; Wilkinson et al., 2001, J Immunol Methods 258:183-191; Patel et al., 1995 J Immunol Methods 184:29-38. Alternatively, or additionally, ADCC activity of the Fc variant protein of interest may be assessed in vivo, e.g., in a animal model such as that disclosed in Clynes et al., 1998, PNAS USA 95:652-656.

An Fc variant protein may have enhanced ADCC activity relative to a comparable molecule. An Fc variant protein may have ADCC activity that is at least 2 fold, or at least 3 fold, or at least 5 fold or at least 10 fold or at least 50 fold or at least 100 fold greater than that of a comparable molecule. An Fc variant protein may have enhanced binding to the Fc receptor FcγRIIIA and enhanced ADCC activity relative to a comparable molecule. The Fc variant protein may have both enhanced ADCC activity and enhanced serum half life relative to a comparable molecule.

"Complement dependent cytotoxicity" and "CDC" refer to the lysing of a target cell in the presence of complement. The complement activation pathway is initiated by the binding of the first component of the complement system (C1q) to a molecule, an antibody for example, complexed with a cognate antigen. To assess complement activation, a CDC assay, e.g. as described in Gazzano-Santoro et al., 1996, J. Immunol. Methods, 202: le63, may be performed. An Fc variant protein may have enhanced CDC activity relative to a comparable molecule. An Fc variant protein may have CDC activity that is at least 2 fold, or at least 3 fold, or at least 5 fold or at least 10 fold or at least 50 fold or at least 100 fold greater than that of a comparable molecule. The Fc variant protein may have both enhanced CDC activity and enhanced serum half life relative to a comparable molecule.

The present invention may provide formulations, wherein the Fc region comprises a non naturally occurring amino acid residue at one or more positions selected from the group consisting of 234, 235, 236, 239, 240, 241, 243, 244, 245, 247, 252, 254, 256; .262, 263, 264, 265, 266, 267, 269, 296, 297, 298, 299, 313, 325, 326, 327, 328, 329, 330, 332, 333, and 334 as numbered by the EU index as set forth in Kabat. Optionally, the Fc region may comprise a non naturally occurring amino acid residue at additional and/or alternative positions known to one skilled in the art (see, e.g., U.S. Patents 5,624,821; 6,277,375; 6,737,056; PCT Patent Publications WO 01/58957; WO 02/06919; WO 04/016750; WO 04/029207; WO 04/035752 and WO 05/040217).

The present invention may provide an Fc variant protein formulation, wherein the Fc region comprises at least one non naturally occurring amino acid residue selected from the group consisting of 234D, 234E, 234N, 234Q, 234T, 234H, 234Y, 234I, 234V, 234F, 235A, 235D, 235R, 235W, 235P, 235S, 235N, 235Q, 235T, 235H, 235Y, 235I, 235V, 235F, 236E, 239D, 239E, 239N, 239Q, 239F, 239T, 239H, 239Y, 240I, 240A, 240T, 240M, 241W, 241 L, 241Y, 241E, 241 R. 243W, 243L 243Y, 243R, 243Q, 244H, 245A, 247V, 247G, 252Y, 254T, 256E, 262I, 262A, 262T, 262E, 263I, 263A, 263T, 263M, 264L, 264I, 264W, 264T, 264R, 264F, 264M, 264Y, 264E, 265G, 265N, 265Q, 265Y, 265F, 265V, 265I, 265L, 265H, 265T, 266I, 266A, 266T, 266M, 267Q, 267L, 269H, 269Y, 269F, 269R, 296E, 296Q, 296D, 296N, 296S, 296T, 296L, 2961, 296H, 269G, 297S, 297D, 297E, 298H, 298I, 298T, 298F, 299I, 299L, 299A, 299S, 299V, 299H, 299F, 299E, 313F, 325Q, 325L, 3251, 325D, 325E, 325A, 325T, 325V, 325H, 327G, 327W, 327N, 327L, 328S, 328M, 328D, 328E, 328N; 328Q, 328F, 328I, 328V, 328T, 328H, 328A, 329F, 329H, 329Q, 330K, 330G, 330T, 330C, 330L, 330Y, 330V, 330I, 330F, 330R, 330H, 332D, 332S, 332W, 332F, 332E, 332N, 332Q, 332T, 332H, 332Y, and 332A as numbered by the EU index as set forth in Kabat. Optionally, the Fc region may comprise additional and/or alternative non naturally occurring amino acid residues known to one skilled in the art (see, e.g., U.S. Patents 5,624,821; 6,277,375; 6,737,056; PCT Patent Publications WO 01/58957; WO 02/06919; WO 04/016750; WO 04/029207; WO 04/035752 and WO 05/040217).

The present invention may provide an Fc variant protein formulation, wherein the Fc region comprises at least a non naturally occurring amino acid at one or more positions selected from the group consisting of 239, 330 and 332, as numbered by the EU index as set forth in Kabat. The present invention may provide an Fc variant protein formulation, wherein the Fc region comprises at least one non naturally occurring amino acid selected from the group consisting of 239D, 330L and 332E, as numbered by the EU index as set forth in Kabat. Optionally, the Fc region may further comprise addtional non naturally occurring amino acid at one or more positions selected from the group consisting of 252, 254, and 256, as numbered by the EU index as set forth in Kabat. The present invention may provide an Fc variant protein formulation, wherein the Fc region comprises at least one non naturally occurring amino acid selected from the group consisting of 239D, 330L and 332E, as numbered by the EU index as set forth in Kabat and at least one non naturally occurring amino acid at one or more positions are selected from the group consisting of 252Y, 254T and 256E, as numbered by the EU index as set forth in Kabat.

The Fc variants of the present invention may be combined with other known Fc variants such as those disclosed in Ghetie et al., 1997, Nat Biotech. 15:637-40; Duncan et al, 1988, Nature 332:563-564; Lund et al., 1991, J. Immunol 147:2657-2662; Lund et al, 1992, Mol Immunol 29:53-59; Alegre et al, 1994, Transplantation 57:1537-1543; Hutchins et al., 1995, Proc Natl. Acad Sci U S A 92:11980-11984; Jefferis et al, 1995, Immunol Lett. 44:111-117; Lund et al., 1995, Faseb J 9:115-119; Jefferis et al, 1996, Immunol Lett 54:101-104; Lund et al, 1996, J Immunol 157:4963-4969; Armour et al., 1999, Eur J Immunol 29:2613-2624; Idusogie et al, 2000, J Immunol 164:4178-4184; Reddy et al, 2000, J Immunol 164:1925-1933; Xu et al., 2000, Cell Immunol 200:16-26; Idusogie et al, 2001, J Immunol 166:2571-2575; Shields et al., 2001, J Biol Chem 276:6591-6604; Jefferis et al, 2002, Immunol Lett 82:57-65; Presta et al., 2002, Biochem Soc Trans 30:487-490); U.S. Patent Nos. 5,624,821; 5,885,573; 5,677,425; 6,165,745; 6,277,375; 5,869,046; 6,121,022; 5,624,821; 5,648,260; 6,528,624; 6,194,551; 6,737,056; 6,821,505; 6,277,375; U.S. Patent Publication Nos. 2004/0002587 and PCT Publications WO 94/29351; WO 99/58572; WO 00/42072; WO 02/060919; WO 04/029207; WO 04/099249; WO 04/063351. Also encompassed by the present disclosure are Fc regions which comprise deletions, additions and/or modifications. Still other modifications/substitutions/additions/deletions of the Fc domain will be readily apparent to one skilled in the art.

Methods for generating non naturally occurring Fc regions are known in the art. For example, amino acid substitutions and/or deletions can be generated by mutagenesis methods, including, but not limited to, site- directed mutagenesis (Kunkel, Proc. Natl. Acad. Sci. USA 82:488-492 (1985)), PCR mutagenesis (Higuchi, in "PCR Protocols: A Guide to Methods and Applications", Academic Press, San Diego, pp. 177-183 (1990)), and cassette mutagenesis (Wells et al., Gene 34:315-323 (1985)). Preferably, site-directed mutagenesis is performed by the overlap-extension PCR method (Higuchi, in "PCR Technology: Principles and Applications for DNA Amplification", Stockton Press, New York, pp. 61-70 (1989)). Alternatively, the technique of overlap-extension PCR (Higuchi, ibid.) can be used to introduce any desired mutation(s) into a target sequence (the starting DNA). For example, the first round of PCR in the overlap- extension method involves amplifying the target sequence with an outside primer (primer 1) and an internal mutagenesis primer (primer 3), and separately with a second outside primer (primer 4) and an internal primer (primer 2), yielding two PCR segments (segments A and B). The internal mutagenesis primer (primers 3) is designed to contain mismatches to the target sequence specifying the desired mutation(s). In the second round of PCR, the products of the first round, of PCR (segments A and B) are amplified by PCR using the two outside primers (primers 1 and 4). The resulting full-length PCR segment (segment C) is digested with restriction enzymes and the resulting restriction fragment is cloned into an appropriate vector. As the first step of mutagenesis, the starting DNA (e.g., encoding an Fc fusion protein, an antibody or simply an Fc region), is operably cloned into a mutagenesis vector. The primers are designed to reflect the desired amino acid substitution. Other methods useful for the generation of variant Fc regions are known in the art (see, e.g., U.S. Patent Nos. 5,624,821; 5,885,573; 5,677,425; 6,165,745; 6,277,375; 5,869,046; 6,121,022; 5,624,821; 5,648,260; 6,528,624; 6,194,551; 6,737,056; 6,821,505; 6,277,375; U.S. Patent Publication Nos. 2004/0002587 and PCT Publications WO 94/29351; WO 99/58572; WO 00/42072; WO 02/060919; WO 04/029207; WO 04/099249; WO 04/063351).

An Fc variant protein may comprise one or more engineered glycoforms, i.e., a carbohydrate composition that is covalently attached to the molecule comprising an Fc region. Engineered glycoforms may be useful for a variety of purposes, including but not limited to enhancing or reducing effector function. Engineered glycoforms may be generated by any method known to one skilled in the art, for example by using engineered or variant expression strains, by co-expression with one or more enzymes, for example DI N-acetylglucosaminyltransferase III (GnTI11), by expressing a molecule comprising an Fc region in various organisms or cell lines from various organisms, or by modifying carbohydrate(s) after the molecule comprising Fc region has been expressed. Methods for generating engineered glycoforms are known in the art, and include but are not limited to those described in Umana et al, 1999, Nat. Biotechnol 17:176-180; Davies et al., 20017 Biotechnol Bioeng 74:288-294; Shields et al, 2002, J Biol Chem 277:26733-26740; Shinkawa et al., 2003, J Biol Chem 278:3466-3473) U.S. Pat. No. 6,602,684; U.S. Ser. No. 10/277,370; U.S. Ser. No. 10/113,929; PCT WO 00/61739A1; PCT WO 01/292246A1; PCT WO 02/311140A1; PCT WO 02/30954A1; Potillegent™ technology (Biowa, Inc. Princeton, N.J.); GlycoMAb™ glycosylation engineering technology (GLYCART biotechnology AG, Zurich, Switzerland). See, e.g., WO 00061739; EA01229125; US 20030115614; Okazaki et al., 2004, JMB, 336: 1239-49.

### 5.1.18. GLYCOSYLATION OF ANTIBODIES

The glycosylation of antibodies utilized in accordance with the invention can be modified. For example, a glycoslated antibody can be made (*i.e*., the antibody lacks glycosylation). Glycosylation can be altered to, for example, increase the affinity of the antibody for a target antigen. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for antigen. Such an approach is described in further detail in U.S. Patent Nos. 5,714,350 and 6,350,861. Alternatively, one or more amino acid substitutions can be made that result in elimination of a glycosylation site present in the Fc region (*e.g*., Asparagine 297 of IgG). Furthermore, a glycosylated antibodies may be produced in bacterial cells which lack the necessary glycosylation machinery.

Additionally or alternatively, an antibody can be made that has an altered type of glycosylation, such as a hypofucosylated antibody having reduced amounts of fucosyl residues or an antibody having increased bisecting GlcNAc structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies of the invention to thereby produce an antibody with altered glycosylation. See, for example, Shields, R.L. et al. (2002) J. Biol. Chem. 277:26733-26740; Umana et al. (1999) Nat. Biotech. 17:176-1, as well as, European Patent No: EP 1,176,195; PCT Publications WO 03/035835; WO 99/54342.

### 5.2. MANUFACTURE/PRODUCTION OF ANTI-CD19 ANTIBODIES

Once a desired anti-CD19 antibody is engineered, the anti-CD 19 antibody can be produced on a commercial scale using methods that are well-known in the art for large scale manufacturing of antibodies. For example, this can be accomplished using recombinant expressing systems such as, but not limited to, those described below.

### 5.2.1. RECOMBINANT EXPRESSION SYSTEMS

Recombinant expression of an antibody for use in the invention or variant thereof, generally requires construction of an expression vector containing a polynucleotide that encodes the antibody. Once a polynucleotide encoding an antibody molecule or a heavy or light chain of an antibody, or portion thereof (preferably, but not necessarily, containing the heavy or light chain variable domain), of the invention has been obtained, the vector for the production of the antibody molecule may be produced by recombinant DNA technology using techniques well-known in the art. *See, e.g.,* U.S. Patent No. 6,331,415. Thus, methods for preparing a protein by expressing a polynucleotide containing an antibody encoding nucleotide sequence are described herein. Methods which are well-known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination. The invention, thus, provides replicable vectors comprising a nucleotide sequence encoding an antibody molecule of the invention, a heavy or light chain of an antibody, a heavy or light chain variable domain of an antibody or a portion thereof, or a heavy or light chain CDR, operably linked to a promoter. Such vectors may include the nucleotide sequence encoding the constant region of the antibody molecule *(see, e.g.,* International Publication Nos. WO 86/05807 and WO 89/01036; and U.S. Patent No. 5,122,464) and the variable domain of the antibody may be cloned into such a vector for expression of the entire heavy, the entire light chain, or both the entire heavy and light chains.

Alternatively, the anti-CD 19 antibodies of the compositions and for use in the methods of the invention can be made using targeted homologous recombination to produce all or portions of the anti-CD19 antibodies *(see,* U.S. Patent Nos. 6,063,630,6,187,305, and 6,692,737). The anti-CD19 antibodies of the compositions and for use in the methods of the invention can be made using random recombination techniques to produce all or portions of the anti-CD19 antibodies (*see*, U.S. Patent Nos. 6,361,972, 6,524,818, 6,541,221, and 6,623,958). Anti-CD19 antibodies can also be produced in cells expressing an antibody from a genomic sequence of the cell comprising a modified immunoglobulin locus using Cre-mediated site-specific homologous recombination *(see,* U.S. Patent No. 6,091,001). Where human antibody production is desired, the host cell should be a human cell line. These methods may advantageously be used to engineer stable cell lines which permanently express the antibody molecule.

Once the expression vector is transferred to a host cell by conventional techniques, the transfected cells are then cultured by conventional techniques to produce an antibody of the invention. Thus, the invention includes host cells containing a polynucleotide encoding an antibody of the invention or fragments thereof, or a heavy or light chain thereof, or portion thereof, or a single-chain antibody of the invention, operably linked to a heterologous promoter. The expression of double-chained antibodies, vectors encoding both the heavy and light chains may be co-expressed in the host cell for expression of the entire immunoglobulin molecule, as detailed below.

A variety of host-expression vector systems may be utilized to express the anti-CD19 antibodies of the invention or portions thereof that can be used in the engineering and generation of anti-CD19 antibodies (*see, e.g.,* U.S. Patent No. 5,807,715). For example, mammalian cells such as Chinese hamster ovary cells (CHO), in conjunction with a vector such as the major intermediate early gene promoter element from human cytomegalovirus is an effective expression system for antibodies (Foecking et al., Gene, 45:101 (1986); and Cockett et al., Bio/Technology, 8:2 (1990)). In addition, a host cell strain may be chosen which modulates the expression of inserted antibody sequences, or modifies and processes the antibody gene product in the specific fashion desired. Such modifications (*e.g.*, glycosylation) and processing (*e.g*., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the antibody or portion thereof expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 3T3, W138, BT483, Hs578T, HTB2, BT2O and T47D, NS0 (a murine myeloma cell line that does not endogenously produce any immunoglobulin chains), CRL7O30 and HsS78Bst cells.

Human cell lines developed by immortalizing human lymphocytes can be used to recombinantly produce monoclonal human anti-CD19 antibodies. The human cell line PER.C6. (Crucell, Netherlands) can be used to recombinantly produce monoclonal human anti-CD 19 antibodies.

In bacterial systems, a number of expression vectors may be advantageously selected depending upon the use intended for the antibody molecule being expressed. For example, when a large quantity of such an antibody is to be produced, for the generation of pharmaceutical compositions comprising an anti-CD19 antibody, vectors which direct the expression of high levels of fusion protein products that are readily purified may be desirable. Such vectors include, but are not limited to, the *E. coli* expression vector pUR278 (Ruther et al., EMBO, 12:1791 (1983)), in which the antibody coding sequence may be ligated individually into the vector in frame with the lac Z coding region so that a fusion protein is produced; pIN vectors (Inouye & Inouye, 1985, Nucleic Acid Res. 13:3101-3109 (1985); Van Heeke & Schuster, 1989, J. Biol. Chem., 24:5503-5509 (1989)); and the like. pGEX vectors may also be used to express foreign polypeptides as fusion proteins with glutathione 5-transferase (GST). In general, such fusion proteins are soluble and can easily be purified from lysed cells by adsorption and binding to matrix glutathione agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example, the polyhedrin gene) of the virus and placed under control of an AcNPV promoter (for example, the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, *e.g*., the late promoter and tripartite leader sequence. This chimeric gene may then be inserted in the adenovirus genome by *in vitro* or *in vivo* recombination. Insertion in a non-essential region of the viral genome (*e.g*., region E1 or E3) will result in a recombinant virus that is viable and capable of expressing the antibody molecule in infected hosts (*e.g., see,* Logan & Shenk, Proc. Natl. Acad. Sci. USA, 81:355-359 (1984)). Specific initiation signals may also be required for efficient translation of inserted antibody coding sequences. These signals include the ATG initiation codon and adjacent sequences. Furthermore, the initiation codon should generally be in phase with the reading frame of the desired coding sequence to ensure translation of the entire insert. These exogenous translational control signals and initiation codons can be of a variety of origins, both natural and synthetic. The efficiency, of expression may be enhanced by the inclusion of appropriate transcription enhancer elements, transcription terminators, etc. (*see, e.g.,* Bittner et al., Methods in Enzymol., 153:51-544(1987)).

For long-term, high-yield production of recombinant proteins, stable expression is preferred. For example, cell lines which stably express the antibody molecule may be engineered. Rather than transient expression systems that use replicating expression vectors which contain viral origins of replication, host cells can be transformed with DNA controlled by appropriate expression control elements (*e.g*., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. Plasmids that encode the anti-CD19 antibody can be used to introduce the gene/cDNA into any cell line suitable for production in culture. Alternatively, plasmids called "targeting vectors" can be used to introduce expression control elements (*e.g*., promoters, enhancers, etc.) into appropriate chromosomal locations in the host cell to "activate" the endogenous gene for anti-CD 19 antibodies.

A number of selection systems may be used, including, but not limited to, the herpes simplex virus thymidine kinase (Wigler et al., Cell, 11:223 (1977)), hypoxanthineguanine phosphoribosyltransferase (Szybalska & Szybalski, Proc. Natl. Acad. Sci. USA, 48:202 (1992)), and adenine phosphoribosyltransferase (Lowy et al., Cell, 22:8-17 (1980)) genes can be employed in tk⁻, hgprt- or aprT⁻cells, respectively. Also, antimetabolite resistance can be used as the basis of selection for the following genes: *dhfr,* which confers resistance to methotrexate (Wigler et al., Natl. Acad. Sci. USA, 77:357 (1980); O'Hare et al., Proc. Natl. Acad. Sci. USA, 78:1527 (1981)); *gpt,* which confers resistance to mycophenolic acid (Mulligan & Berg, Proc. Natl. Acad. Sci. USA, 78:2072 (1981)); *neo,* which confers resistance to the aminoglycoside G-418 (Wu and Wu, Biotherapy 3:87-95 (1991); Tolstoshev, Ann. Rev. Pharmacol. Toxicol. 32:573-596 (1993); Mulligan, Science 260:926-932 (1993); and Morgan and Anderson, Ann. Rev. Biochem. 62:191-217 (1993); May, TIB TECH 11 (5):155-2 15 (1993)); and *hygro,* which confers resistance to hygromycin (Santerre et al., Gene, 30:147 (1984)). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds.), Current Protocols in Human Genetics, John Wiley & Sons, NY (1994); Colberre-Garapin et al., 1981, J. Mol. Biol., 150:1.

The expression levels of an antibody molecule can be increased by vector amplification (for a review, *see,* Bebbington and Hentschel, The use of vectors based on gene amplification for the expression of cloned genes in mammalian cells in DNA cloning, Vol. 3. Academic Press, New York (1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., Mol. Cell. Biol., 3:257 (1983)). Antibody expression levels may be amplified through the use recombinant methods and tools known to those skilled in the art of recombinant protein production, including technologies that remodel surrounding chromatin and enhance transgene expression in the form of an active artificial transcriptional domain.

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes, and is capable of expressing, both heavy and light chain polypeptides. In such situations, the light chain should be placed before the heavy chain to avoid an excess of toxic free heavy chain (Proudfoot, Nature 322:562-65 (1986); and Kohler, 1980, Proc. Natl. Acad. Sci. USA, 77:2197 (1980)). The coding sequences for the heavy and light chains may comprise cDNA or genomic DNA.

Once an antibody molecule of the invention has been produced by recombinant expression, it may be purified by any method known in the art for purification of an immunoglobulin molecule, for example, by chromatography (*e.g*., ion exchange, affinity, particularly by affinity for the specific antigen after Protein A, and sizing column chromatography), centrifugation, differential solubility, or by any other standard technique for the purification of proteins. Further, the antibodies of the present invention or fragments thereof may be fused to heterologous polypeptide sequences described herein or otherwise known in the art to facilitate purification.

### 5.2.2. ANTIBODY PURIFICATION AND ISOLATION

When using recombinant techniques, the antibody can be produced intracellularly, in the periplasmic space, or directly secreted into the medium. If the antibody is produced intracellularly, as a first step, the particulate debris, either host cells or lysed fragments, is removed, for example, by centrifugation or ultrafiltration. Carter et al., Bio/Technology, 10:163-167 (1992) describe a procedure for isolating antibodies which are secreted into the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody mutant is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, hydrophobic interaction chromatography, ion exchange chromatography, gel electrophoresis, dialysis, and/or affinity chromatography either alone or in combination with other purification steps. The suitability of protein A as an affinity ligand depends on the species and isotype of any immunoglobulin Fc domain that is present in the antibody mutant. Protein A can be used to purify antibodies that are based on human γ1, γ2, or γ 4 heavy chains (Lindmark et al., J. Immunol. Methods, 62:1-13 (1983)). Protein G is recommended for all mouse isotypes and for human γ3 (Guss et al., EMBO J., 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a CH₃ domain, the Bakerbond ABX resin (J.T. Baker, Phillipsburg, NJ) is useful for purification. Other techniques for protein purification such as fractionation on an ion-exchange column, ethanol precipitation, Reverse Phase HPLC, chromatography on silica, chromatography on heparin, SEPHAROSE chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to low pH hydrophobic interaction chromatography using an elution buffer at a pH between about 2.5-4.5, preferably performed at low salt concentrations (*e.g*., from about 0-0.25 M salt).

### 5.3. THERAPEUTIC ANTI-CD19 ANTIBODIES

The anti-CD 19 antibody used in the compositions and for use in the methods of the invention is preferably a a humanized antibody that preferably mediates human ADCC. In certain embodiments, the anti-CD19 antibodies can be chimeric antibodies. In preferred embodiments, anti-CD 19 antibody is a monoclonal humanized, or chimeric anti-CD 19 antibody. The anti-CD19 antibody used in the compositions and for use in the methods of the invention is preferably a humanized antibody of the IgG1 or IgG3 human isotype. The anti-CD19 antibody used in the compositions and for use in the methods of the invention may be a human antibody or a humanized antibody of the IgG2 or IgG4 human isotype that preferably mediates ADCC.

While such antibodies can be generated using the techniques described above, the murine antibodies HB12a and HB12b as described herein or other commercially available anti-CD19 antibodies can be chimerized, or humanized.

For example, known anti-CD19 antibodies that can be used include, but are not limited to, HD37 (IgG1) (DAKO, Carpinteria, CA), BU12 (G.D. Johnson, University of Birmingham, Birmingham, United Kingdom), 4G7 (IgG1) (Becton-Dickinson, Heidelberg, Germany), J4.119 (Beckman Coulter, Krefeld, Germany), B43 (PharMingen, San Diego, CA), SJ25C1 (BD PharMingen, San Diego, CA), FMC63 (IgG2a) (Chemicon Int'l., Temecula, CA) (Nicholson et al., Mol. Immunol., 34:1157-1165 (1997); Pietersz et al., Cancer Immunol. Immunotherapy, 41:53-60 (1995); and Zola et al., Immunol. Cell Biol., 69:411-422 (1991)), B4 (IgG1) (Beckman Coulter, Miami, FL) Nadler et al., J. Immunol., 131:244-250 (1983), and/or HD237 (IgG2b) (Fourth International Workshop on Human Leukocyte Differentiation Antigens, Vienna, Austria, 1989; and Pezzutto et al., J. Immunol., 138:2793-2799 (1987)).

In certain embodiments, the anti-CD19 antibody of the invention comprises the heavy chain of HB12a comprising an amino acid sequence of SEQ ID NO:2 (**Fig. 5A**). In other embodiments, the anti-CD19 antibody of the invention comprises the heavy chain of HB12b comprising an amino acid sequence of SEQ ID NO:4 (**Fig. 5B**).

In certain embodiments, the anti-CD19 antibody of the invention comprises the right chain of HB12a comprising an amino acid sequence of SEQ ID NO:16 (**Fig. 6A**)**.** In other embodiments, the anti-CD19 antibody of the invention comprises the light chain of HB12b comprising an amino acid sequence of SEQ ID NO:18 (**Fig. 6B**).

In certain embodiments, the antibody is an isotype switched variant of a know antibody (*e.g.,* to an IgG1 or IgG3 human isotype) such as those described above (*e.g.,* HB12a or HB12b).

The anti-CD 19 antibodies used in the compositions and for use in the methods of the invention can be naked antibodies, immunoconjugates or fusion proteins. Preferably the anti-CD19 antibodies described above for use in the compositions and for use in the methods of the invention are able to reduce or deplete B cells in a human treated therewith. Depletion of B cells can be in circulating B cells, or in particular tissues such as, but not limited to, bone marrow, spleen, gut-associated lymphoid tissues, and/or lymph nodes. Such depletion may be achieved via various mechanisms such as antibody-dependent cell-mediated cytotoxicity (ADCC) and/or complement dependent cytotoxicity (CDC), inhibition of B cell proliferation and/or induction of B cell death (*e.g*., via apoptosis). By "depletion" of B cells it is meant a reduction in circulating B cells and/or B cells in particular tissue(s) by at least about 25%, 40%, 50%, 65%, 75%, 80%, 85%, 90%, 95% or more as described in Section 5.4.3. In particular embodiments, virtually all detectable B cells are depleted from the circulation and/or particular tissue(s). By "depletion" of circulating immunoglobulin (Ig) it is meant a reduction by at least about 25%, 40%, 50%, 65%, 75%, 80%, 85%, 90%, 95% or more as described in Section 5.4.3. As described herein, virtually all detectable Ig may be depleted from the circulation.

### 5.3.1. SCREENING OF ANTIBODIES FOR HUMAN CD 19 BINDING

Binding assays can be used to identify antibodies that bind the human-CD19 antigen. Binding assays may be performed either as direct binding assays or as competition-binding assays. Binding can be detected using standard ELISA or standard Flow Cytometry assays. In a direct binding assay, a candidate antibody is tested for binding to human CD19 antigen. The screening assays comprise, in a second step, determining the ability to cause cell death or apoptosis of B cells expressing human CD 19. Competition-binding assays, on the other hand, assess the ability of a candidate antibody to compete with a known anti-CD19 antibody or other compound that binds human CD 19.

In a direct binding assay, the human CD19 antigen is contacted with a candidate antibody under conditions that allow binding of the candidate antibody to the human CD19 antigen. The binding may take place in solution or on a solid surface. Preferably, the candidate antibody is previously labeled for detection. Any detectable compound may be used for labeling, such as but not limited to, a luminescent, fluorescent, or radioactive isotope or group containing same, or a nonisotopic label, such as an enzyme or dye. After a period of incubation sufficient for binding to take place, the reaction is exposed to conditions and manipulations that remove excess or non-specifically bound antibody. Typically, it involves washing with an appropriate buffer. Finally, the presence of a CD19-antibody complex is detected.

In a competition-binding assay, a candidate antibody is evaluated for its ability to inhibit or displace the binding of a known anti-CD 19 antibody (or other compound) to the human CD 19 antigen. A labeled known binder of CD 19 may be mixed with the candidate antibody, and placed under conditions in which the interaction between them would normally occur, with and without the addition of the candidate antibody. The amount of labeled known binder of CD 19 that binds the human CD 19 may be compared to the amount bound in the presence or absence of the candidate antibody.

To facilitate antibody antigen complex formation and detection, the binding assay may be carried out with one or more components immobilized on a solid surface. The solid support could be, but is not restricted to, polycarbonate, polystyrene, polypropylene, polyethylene, glass, nitrocellulose, dextran, nylon, polyacrylamide and agarose. The support configuration can include beads, membranes, microparticles, the interior surface of a reaction vessel such as a microtiter plate, test tube or other reaction vessel. The immobilization of human CD 19, or other component, can be achieved through covalent or non-covalent attachments. The attachment may be indirect, *i.e.,* through an attached antibody. The human CD 19 antigen and negative controls may be tagged with an epitope, such as glutathione S-transferase (GST) so that the attachment to the solid surface can be mediated by a commercially available antibody such as anti-GST (Santa Cruz Biotechnology).

For example, such an affinity binding assay may be performed using the human CD 19 antigen which is immobilized to a solid support. Typically, the non-mobilized component of the binding reaction, in this case the candidate anti-CD 19 antibody, is labeled to enable detection. A variety of labeling methods are available and may be used, such as luminescent, chromophore, fluorescent, or radioactive isotope or group containing same, and nonisotopic labels, such as enzymes or dyes. The candidate anti-CD19 antibody may be labeled with a fluorophore such as fluorescein isothiocyanate (FITC, available from Sigma Chemicals, St. Louis).

Finally, the label remaining on the solid surface may be detected by any detection method known in the art. For example, if the candidate anti-CD 19 antibody is labeled with a fluorophore, a fluorimeter may be used to detect complexes.

The human CD 19 antigen may be added to binding assays in the form of intact cells that express human CD19 antigen, or isolated membranes containing human CD19 antigen. Thus, direct binding to human CD19 antigen may be assayed in intact cells in culture or in animal models in the presence and absence of the candidate anti-CD19 antibody. A labeled candidate anti-CD19 antibody may be mixed with cells that express human CD19 antigen, or with crude extracts obtained from such cells, and the candidate anti-CD 19 antibody may be added. Isolated membranes may be used to identify candidate anti-CD 19 antibodies that interact with human CD 19. For example, in a typical experiment using isolated membranes, cells may be genetically engineered to express human CD 19 antigen. Membranes can be harvested by standard techniques and used in an *in vitro* binding assay. Labeled candidate anti-CD19 antibody (e.g., fluorescent labeled antibody) may be bound to the membranes and assayed for specific activity; specific binding may be determined by comparison with binding assays performed in the presence of excess unlabeled (cold) candidate anti-CD19 antibody. Alternatively, soluble human CD 19 antigen may be recombinantly expressed and utilized in non-cell based assays to identify antibodies that bind to human CD 19 antigen. The recombinantly expressed human CD19 polypeptides can be used in the non-cell based screening assays. Alternatively, peptides corresponding to one or more of the binding portions of human CD 19 antigen, or fusion proteins containing one or more of the binding portions of human CD19 antigen can be used in non-cell based assay systems to identify antibodies that bind to portions of human CD19 antigen. In non-cell based assays the recombinantly expressed human CD19 is attached to a solid substrate such as a test tube, microtiter well or a column, by means well-known to those in the art (*see,* Ausubel *et al., supra*). The test antibodies are then assayed for their ability to bind to human CD 19 antigen.

Alternatively, the binding reaction may be carried out in solution. In this assay, the labeled component is allowed to interact with its binding partner(s) in solution. If the size differences between the labeled component and its binding partner(s) permit such a separation, the separation can be achieved by passing the products of the binding reaction through an ultrafilter whose pores allow passage of unbound labeled component but not of its binding partner(s) or of labeled component bound to its partner(s). Separation can also be achieved using any reagent capable of capturing a binding partner of the labeled component from solution, such as an antibody against the binding partner and so on.

For example, a phage library can be screened by passing phage from a continuous phage display library through a column containing purified human CD19 antigen, or derivative, analog, fragment, or domain, thereof, linked to a solid phase, such as plastic beads. By altering the stringency of the washing buffer, it is possible to enrich for phage that express peptides with high affinity for human CD19 antigen. Phage isolated from the column can be cloned and affinities can be measured directly. Knowing which antibodies and their amino acid sequences confer the strongest binding to human CD 19 antigen, computer models can be used to identify the molecular contacts between CD 19 antigen and the candidate antibody.

The solid support is membrane containing human CD 19 antigen attached to a microtiter dish. Candidate antibodies, for example, can bind cells that express library antibodies cultivated under conditions that allow expression of the library members in the microtiter dish. Library members that bind to the human CD 19 are harvested. Such methods, are generally described by way of example in Parmley and Smith, 1988, Gene, 73:305-318; Fowlkes et al., 1992, BioTechniques, 13:422-427; PCT Publication No. WO94/18318; and in references cited hereinabove. Antibodies identified as binding to human CD19 antigen can be of any of the types or modifications of antibodies described above.

### 5.3.2. SCREENING OF ANTIBODIES FOR HUMAN ADCC EFFECTOR FUNCTION

Antibodies of the human IgG class are preferred for use in the invention because they have functional characteristics such a long half-life in serum and can mediate various effector functions (Monoclonal Antibodies: Principles and Applications, Wiley-Liss, Ind., Chapter 1 (1995)). The human IgG class antibody is further classified into the following 4. subclasses: IgG1, IgG2, IgG3 and IgG4. A large number of studies have so far been conducted for ADCC and CDC and apoptotic activity as effector functions of the IgG class antibody, and it has been reported that among antibodies of the human IgG class, the IgG1 subclass has the highest ADCC activity and CDC activity in humans (chemical Immunology, 65, 88 (1997)).

Expression of ADCC activity and CDC activity and apoptotic activity of the human IgG1 subclass antibodies generally involves binding of the Fc region of the antibody to a receptor for an antibody (hereinafter referred to as "FcγR") existing on the surface of effector cells such as killer cells, natural killer cells or activated macrophages. Various complement components can be bound. Regarding the binding, it has been suggested that several amino acid residues in the hinge region and the second domain of C region (hereinafter referred to as "Cγ2 domain") of the antibody are important (Eur. J. Immunol., 23, 1098 (1993), Immunology, 86, 319 (1995), Chemical Immunology, 65, 88 (1997)) and that a sugar chain in the Cγ2 domain (Chemical Immunology, 65, 88 (1997)) is also important.

The anti-CD 19 antibodies of the invention can be modified with respect to effector function, *e.g*., so as to enhance ADCC and/or complement dependent cytotoxicity (CDC) and/or apoptotic activity of the antibody. This may be achieved by introducing one or more amino acid substitutions in the Fc region of an antibody. Alternatively or additionally, cysteine residue(s) may be introduced in the Fc region, allowing for interchain disulfide bond formation in this region. In this way a homodimeric antibody can be generated that may have improved internalization capability and or increased complement-mediated cell killing and ADCC (Caron et al., J. Exp. Med., 176:1191-1195 (1992) and Shopes, J. Immunol., 148:2918-2922 (1992)). Heterobifunctional cross-linkers can also be used to generate homodimeric antibodies with enhanced anti-tumor activity (Wolff et al., Cancer Research, 53:2560-2565 (1993)). Antibodies can also be engineered to have two or more Fc regions resulting in enhanced complement lysis and ADCC capabilities (Stevenson et al., Anti-Cancer Drug Design, (3)219-230 (1989)).

Other methods of engineering Fc regions of antibodies so as to alter effector functions are known in the art (*e.g*., U.S. Patent Publication No. 20040185045 and PCT Publication No. WO 2004/016750; both to Koenig et al.*,* which describe altering the Fc region to enhance the binding affinity for FcγRIIB as compared with the binding affinity for FCγRIIA; *see also* PCT Publication Nos. WO 99/58572 to Armour et al.*,* WO 99/51642 to Idusogie et al.*,* and U.S. 6,395,272 to Deo et al.). Methods of modifying the Fc region to decrease binding affinity to FcγRIIB are also known in the art (*e.g.,* U.S. Patent Publication No. 20010036459 and PCT Publication No. WO 01/79299, both to Ravetch et al.). Modified antibodies having variant Fc regions with enhanced binding affinity for FcγRIIIA. and/or FcγRIIA as compared with a wildtype Fc region have also been described (e.g., PCT Publication No. WO 2004/063351, to Stavenhagen et al.).

At least four different types of FcγR have been found, which are respectively called FcγRI (CD64), FcγRII (CD32), FcγRIII (CD 16), and FcγRIV. In human, FcγRII and FcγRIII are further classified into FcγRIIa and FcγRIIb, and FcγRIIIa and FcγRIIIb, respectively. FcγR is a membrane protein belonging to the immunoglobulin superfamily, FcγRII, FcγRIII, and FcγRIV have an α chain having an extracellular region containing two immunoglobulin-like domains, FcγRI has an α chain having an extracellular region containing three immunoglobulin-like domains, as a constituting component, and the α chain is involved in the IgG binding activity. In addition, FcγRI and FcγRIII have a γ chain or ζ chain as a constituting component which has a signal transduction function in association with the α chain (Annu. Rev. Immunol., 18, 709 (2000), Annu. Rev. Immunol., 19,275 (2001)). FcγRIV has been described by Bruhns et al., Clin. Invest. Med., (Canada) 27:3D (2004).

To assess ADCC activity of an anti-CD 19 antibody of interest, an *in vitro* ADCC assay can be used, such as that described in U.S. Patent No. 5,500,362 or 5,821,337. Useful effector cells for such assays include peripheral blood mononuclear cells (PBMC) and Natural Killer (NK) cells. For example, the ability of any particular antibody to mediate lysis of the target cell by complement activation and/or ADCC can be assayed. The cells of interest are grown and labeled *in vitro;* the antibody is added to the cell culture in combination with immune cells which may be activated by the antigen antibody complexes; *i.e.,* effector cells involved in the ADCC response. The antibody can also be tested for complement activation. In either case, cytolysis of the target cells is detected by the release of label from the lysed cells. In fact, antibodies can be screened using the patient's own serum as a source of complement and/or immune cells. The antibodies that are capable of mediating human ADCC in the *in vitro* test can then be used therapeutically in that particular patient. Alternatively, or additionally, ADCC activity of the molecule of interest may be assessed *in vivo, e.g.,* in an animal model such as that disclosed in Clynes et al., PNAS (USA) 95:652-656 (1998). Moreover, techniques for modulating (*i.e.,* increasing or decreasing) the level of ADCC, and optionally CDC activity, and optionally apoptotic activity of an antibody are well-known in the art. *See, e.g.,* U.S. Patent No. 6,1994,551; *(see, e.g.,* Chaouchi et al., J. Immunol., 154(7): 3096-104 (1995); Pedersen et al., Blood, 99(4): 1314-1318 (2002); Alberts et al., Molecular Biology of the Cell; Steensma et al., Methods Mol Med., 85: 323-32, (2003)). Antibodies of the present invention may be capable or may have been modified to have the ability of inducing ADCC and/or CDC and%r an apoptotic response. Preferably, such assays to determine ADCC function are practiced using human effector cells to assess human ADCC function.

### 5.3.3. IMMUNOCONJUGATES AND FUSION PROTEINS

According to certain aspects of the invention, therapeutic agents or toxins can be conjugated to chimerized, or humanized anti-CD19 antibodies for use in the compositions and for use in the methods of the invention. In certain embodiments, these conjugates can be generated as fusion proteins *(see,* Section 5.1.8). Examples of therapeutic agents and toxins include, but are not limited to, members of the enediyne family of molecules, such as calicheamicin and esperamicin. Chemical toxins can also be taken from the group consisting of duocarmycin, *(see, e.g.,* U.S. Patent No. 5,703,080 and U.S. Patent No. 4,923,990), methotrexate, doxorubicin, melphalan, chlorambucil, ARA-C, vindesine, mitomycin C, cis-platinum, etoposide, bleomycin and 5-fluorouracil. Examples of chemotherapeutic agents also include Adriamycin, Doxorubicin, 5-Fluorouracil, Cytosine arabinoside (Ara-C), Cyclophosphamide, Thiotepa, Taxotere (docetaxel), Busulfan, Cytoxiri, Taxol, Methotrexate, Cisplatin, Melphalan, Vinblastine, Bleomycin, Etoposide, Ifosfamide, Mitomycin C, Mitoxantrone, Vincreistine, Vinorelbine, Carboplatin, Teniposide, Daunomycin, Carminomycin, Aminopterin, Dactinomycin, Mitomycins, Esperamicins *(see,* U.S. Patent No. 4,675,187), Melphalan, and other related nitrogen mustards.

For example, "CVB" (1.5 g/m² cyclophosphamide, 200-400 mg/m² etoposide, and 150-200 mg/m² carmustine) can be used in the combination therapies of the invention. CVB is a regimen used to treat non-Hodgkin's lymphoma (Patti et al., Eur. J. Haematol., 51:18 (1993)). Other suitable combination chemotherapeutic regimens are well-known to those of skill in the art. *See,* for example, Freedman et al., "Non-Hodgkin's Lymphontas," in Cancer Medicine, Volume 2, 3rd Edition, Holland et al. (eds.), pp. 2028-2068 (Lea & Febiger 1993). As an illustration, first generation chemotherapeutic regimens for treatment of intermediate-grade non-Hodgkin's lymphoma include C-MOPP (cyclophosphamide, vincristine, procarbazine and prednisone) and CHOP (cyclophosphamide, doxorubicin, vincristine, and prednisone). A useful second generation chemotherapeutic regimen is m-BACOD (methotrexate, bleomycin, doxorubicin, cyclophosphamide, vincristine, dexamethasone, and leucovorin), while a suitable third generation regimen is MACOP-B (methotrexate, doxorubicin, cyclophosphamide, vincristine, prednisone, bleomycin, and, leucovorin). Additional useful drugs include phenyl butyrate and brostatin-1.

Other toxins that can be used in the immunoconjugates of the invention include, poisonous lectins, plant toxins such as ricin, abrin, modeccin, botulina, and diphtheria toxins. Of course, combinations of the various toxins could also be coupled to one antibody molecule thereby accommodating variable cytotoxicity. Illustrative of toxins which are suitably employed in the combination therapies of the invention are ricin, abrin, ribonuclease, DNase I, Staphylococcal enterotoxin-A, pokeweed anti-viral protein, gelonin, diphtherin toxin, Pseudomonas exotoxin, and Pseudomonas endotoxin. *See,* for example, Pastan et al., Cell, 47:641 (1986), and Goldenberg et al., Cancer Journal for Clinicians, 44:43 (1994). Enzymatically active toxins and fragments thereof which can be used include diphtheria A chain, non-binding active fragments of diphtheria toxin, exotoxin A chain (from *Pseudomonas aeruginosa),* ricin A chain, abrin A chain, modeccin A chain, Alpha-sarcin, *Aleurites fordii* proteins, dianthin proteins, *Phytolaca americana* proteins (PAPI, PAPII, and PAP-S), *Momordica charantia* inhibitor, curcin, crotin, *Sapaonaria officinalis* inhibitor, gelonin, mitogellin, restrictocin, phenomycin, enomycin and the tricothecenes. *See,* for example, WO 93/21232 published October 28, 1993.

Suitable toxins and chemotherapeutic agents are described in Remington's Pharmaceutical Sciences, 19th Ed. (Mack Publishing Co. 1995), and in Goodman And Gilman's The Pharmacological Basis of Therapeutics, 7th Ed. (MacMillan Publishing Co. 1985). Other suitable toxins and/or chemotherapeutic agents are known to those of skill in the art.

The anti-CD 19 antibody of the present invention may also be used in ADEPT by conjugating the antibody to a prodrug-activating enzyme which converts a prodrug (*e.g*., a peptidyl chemotherapeutic agent, *see,* WO81/01145) to an active anti-cancer drug. *See,* for example, WO 88/07378 and U.S. Patent No. 4,975,278. The enzyme component of the immunoconjugate useful for ADEPT includes any enzyme capable of acting on a prodrug in such a way so as to covert it into its more active, cytotoxic form:

Enzymes that are useful in the methods described herein include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as β-galactosidase and neuraminidase useful for converting glycosylated prodrugs into free drugs; β-lactamase useful for converting drugs derivatized with α-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes," can be used to convert the prodrugs of the invention into free active drugs *(see, e.g.,* Massey, Nature 328:457-458 (1987)). Antibody-abzyme conjugates can be prepared as described herein for delivery of the abzyme as desired to portions of a human affected by a B cell malignancy.

The enzymes described herein can be covalently bound to the antibody by techniques well-known in the art such as the use of the heterobifunctional crosslinking reagents discussed above. Alternatively, fusion proteins comprising at least the antigen-binding region of an antibody of the invention linked to at least a functionally active portion of an enzyme of the invention can be constructed using recombinant DNA techniques well-known in the art *(see, e.g*., Neuberger et al., Nature, 312:604-608 (1984)).

Covalent modifications of the anti-CD 19 antibody are included within the scope of this disclosure. They may be made by chemical synthesis or by enzymatic or chemical cleavage of the antibody, if applicable. Other types of covalent modifications of the anti-CD 19 antibody are introduced into the molecule by reacting targeted amino acid residues of the antibody with an organic derivatizing agent that is capable of reacting with selected side chains or the N- or C-terminal residues.

Cysteinyl residues most commonly are reacted with α-haloacetates (and corresponding amines), such as, chloroacetic acid or chloroacetainide, to give carboxymethyl or carboxyamidomethyl derivatives. Similarly, iodo-reagents may also be used. Cysteinyl residues also are derivatized by reaction with bromotrifluoroacetone, α-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylpyrocarbonate at pH 5.5-7.0 because this agent is relatively specific for the histidyl side chain. Para-bromophenacyl - bromide also is useful; the reaction is preferably performed in 0.1 M sodium cacodylate at pH 6.0.

Lysyl and amino-terminal residues are reacted with succinic or other carboxylic acid anhydrides. Derivatization with these agents has the effect of reversing the charge of the lysinyl residues. Other suitable reagents for derivatizing α-amino-containing residues and/or ε-amino-containing residues include imidoesters such as methyl picolinimidate, pyridoxal phosphate, pyridoxal, chloroborohydride, trinitrobenzenesulfonic acid, 0-methylisourea, 2,4-pentanedione, and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginyl residues generally requires that the reaction be performed in alkaline conditions because of the high pKa of the guanidine functional group. Furthermore, these reagents may react with the ε-amino groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using ¹²⁵I or ¹³¹I to prepare labeled proteins for use in radioimmunoassay.

Carboxyl side groups (aspartyl or glutamyl) are selectively modified by reaction with carbodiimides (R--N=C=N--R'), where R and R' are different alkyl groups, such as 1-cyclohexyl-3-(2-morpholinyl-- 4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues, respectively. These residues are deamidated under neutral or basic conditions. The deamidated form of these residues falls within the scope of this disclosure.

Other modifications include hydroxylation of proline and lysine, phosphorylation of hydroxyl groups of seryl or threonyl residues, methylation of the α-amino groups of lysine, arginine, and histidine side chains (T.E. Creighton, Proteins: Structure and Molecular Properties, W.H. Freeman & Co., San Francisco, pp. 79-86 (1983)), acetylation of the N-terminal amine, and amidation of any C-terminal carboxyl group.

Another type of covalent modification involves chemically or enzymatically coupling glycosides to the antibody. These procedures are advantageous in that they do not require production of the antibody in a host cell that has glycosylation capabilities for N- or O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330 published 11 Sep. 1987, and in Aplin and Wriston, CRC Crit. Rev. Biochem., pp. 259-306 (1981).

### 5.4. PHARMACEUTICAL FORMULATIONS, ADMINISTRATION AND DOSING

The pharmaceutical formulations of the invention contain as the active ingredient humanized, or chimeric anti-CD19 antibodies. The formulations contain naked antibody, immunoconjugate, or fusion protein in an amount effective for producing the desired response in a unit of weight or volume suitable for administration to a human patient, and are preferably sterile. The response can, for example, be measured by determining the physiological effects of the anti-CD 19 antibody composition, such as, but not limited to, circulating B cell depletion, tissue B cell depletion, regression of a B cell malignancy, or decrease of disease symptoms. Other assays will be known to one of ordinary skill in the art and can be employed for measuring the level of the response.

### 5.4.1. PHARMACEUTICAL FORMULATIONS

An anti-CD 19 antibody composition may be formulated with a pharmaceutically acceptable carrier. The term "pharmaceutically acceptable" means one or more non-toxic materials that do not interfere with the effectiveness of the biological activity of the active ingredients. Such preparations may routinely contain salts, buffering agents, preservatives, compatible carriers, and optionally other therapeutic agents. Such pharmaceutically acceptable preparations may also routinely contain compatible solid or liquid fillers, diluents or encapsulating substances which are suitable for administration into a human. When used in medicine, the salts should be pharmaceutically acceptable, but non-pharmaceutically acceptable salts may conveniently be used to prepare pharmaceutically acceptable salts thereof and are not excluded from the scope of the disclosure. Such pharmacologically and pharmaceutically acceptable salts include, but are not limited to, those prepared from the following acids: hydrochloric, hydrobromic, sulfuric, nitric, phosphoric, maleic, acetic, salicylic, citric, boric, formic, malonic, succinic, and the like. Also, pharmaceutically acceptable salts can be prepared as alkaline metal or alkaline earth salts, such as sodium, potassium or calcium salts. The term "carrier" denotes an organic or inorganic ingredient, natural or synthetic, with which the active ingredient is combined to facilitate the application. The components of the pharmaceutical compositions also are capable of being co-mingled with the antibodies of the present disclosure, and with each other, in a manner such that there is no interaction which would substantially impair the desired pharmaceutical efficacy.

As described herein, the anti-CD 19 antibody compositions can be prepared for storage by mixing the antibody or immunoconjugate having the desired degree of purity with optional physiologically acceptable carriers, excipients or stabilizers (Remington's Pharmaceutical Sciences, 16th edition, Osol, A. Ed. (1999)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptide; proteins, such as serum albumin, gelatin, or immunoglobulins; hydrophilic polymers such as polyvinylpyrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*., Zn-protein complexes); and/or non-ionic surfactants such as TWEEN, PLURONICS™ or polyethylene glycol (PEG).

The anti-CD 19 antibody compositions also may contain, optionally, suitable preservatives, such as: benzalkonium chloride; chlorobutanol; parabens and thimerosal.

The anti-CD19 antibody compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for parenteral administration conveniently comprise a sterile aqueous or non-aqueous preparation of anti-CD 19 antibody, which is preferably isotonic with the blood of the recipient. This preparation may be formulated according to known methods using suitable dispersing or wetting agents and suspending agents. The sterile injectable preparation also may be a sterile injectable solution or suspension in a non-toxic parenterally acceptable diluent or solvent, for example, as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono-or di-glycerides. In addition, fatty acids such as oleic acid may be used in the preparation of injectables. Carrier formulation, suitable for oral, subcutaneous, intravenous, intramuscular, etc. administration can be found in Remington's Pharmaceutical Sciences, Mack Publishing Co., Easton, PA. Carrier formulations suitable for various routes of administration can be the same or similar to that described for RITUXAN™. *See,* Physicians' Desk Reference (Medical Economics Company, Inc., Montvale, NJ, 2005), pp. 958-960 and 1354-1357. As described herein, the anti-CD19 antibody compositions are formulated for intravenous administration with sodium chloride, sodium citrate dihydrate, polysorbate 80, and sterile water where the pH of the composition is adjusted to approximately 6.5. Those of skill in the art are aware that intravenous injection provides a useful mode of administration due to the thoroughness of the circulation in rapidly distributing antibodies. Intravenous administration, however, is subject to limitation by a vascular barrier comprising endothelial cells of the vasculature and the subendothelial matrix. Still, the vascular barrier is a more notable problem for the uptake of therapeutic antibodies by solid tumors. Lymphomas have relatively high blood flow rates, contributing to effective antibody delivery. Intralymphatic routes of administration, such as subcutaneous or intramuscular injection, or by catheterization of lymphatic vessels, also provide a useful means of treating B cell lymphomas. Anti-CD19 antibodies of the compositions and for use in the methods of the invention may be self-administered subcutaneously. The composition may be formulated as a lyophilized drug or in a liquid buffer (*e.g.*, PBS and/or citrate) at about 50 mg/mL.

The formulation herein may also contain more than one active compound as necessary for the particular indication being treated, preferably those with complementary activities that do not adversely affect each other. For example, it may be desirable to further provide an immunosuppressive agent. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration are typically sterile. This is readily accomplished by filtration through sterile filtration membranes.

Sustained-release preparations may be prepared. Suitable examples of sustained-release preparations include semipermeable matrices of solid hydrophobic polymers containing the anti-CD19 antibody, which matrices are in the form of shaped articles, *e.g*., films, or microcapsule. Examples of sustained-release matrices include polyesters, hydrogels (for example, poly(2-hydroxyethyl-methacrylate), or poly(vinylalcohol)), polylactides (U.S. Patent No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions. The pharmaceutically acceptable carriers used in the compositions of the invention do not affect human ADCC or CDC.

The anti-CD 19 antibody compositions disclosed herein may-also be formulated as immunoliposomes. A "liposome" is a small vesicle composed of various types of lipids, phospholipids and/or surfactant which is useful for delivery of a drug (such as the anti-CD 19 antibodies disclosed herein) to a human. The components of the liposome are commonly arranged in a bilayer formation, similar to the lipid arrangement of biological membranes. Liposomes containing the antibodies of the invention are prepared by methods known in the art, such as described in Epstein et al., Proc. Natl. Acad. Sci. USA, 82:3688 (1985); Hwang et al., Proc. Natl. Acad. Sci. USA, 77:4030 (1980); and U.S. Patent Nos. 4,485,045 and 4,544,545. Liposomes with enhanced circulation time are disclosed in U.S. Patent No. 5,013,556. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. The antibody of the present invention can be conjugated to the liposomes as described in Martin et al., J. Biol. Chem., 257:286-288 (1982) via a disulfide interchange reaction. A therapeutic agent can also be contained within the liposome. *See,* Gabizon et al., J. National Cancer Inst., (19)1484 (1989).

Some of the preferred pharmaceutical formulations include, but are not limited to:
(a) a sterile, preservative-free liquid concentrate for intravenous (i.v.) administration of anti-CD19 antibody, supplied at a concentration of 10 mg/ml in either 100 mg (10 mL) or 500 mg (50 mL) single-use vials. The product can be formulated for i.v. administration using sodium chloride, sodium citrate dihydrate, polysorbate and sterile water for injection. For example, the product can be formulated in 9.0 mg/mL sodium chloride, 7.35 mg/mL sodium citrate dihydrate, 0.7 mg/mL polysorbate 80, and sterile water for injection. The pH is adjusted to 6.5.
(b) A sterile, lyophilized powder in single-use glass vials for subcutaneous (s.c.) injection. The product can be formulated with sucrose, L-histidine hydrochloride monohydrate, L-histidine and polysorbate 20. For example, each single-use vial can contain 150 mg anti-CD19 antibody, 123.2 mg sucrose, 6.8 mg L-histidine hydrochloride monohydrate, 4.3 mg L-histidine, and 3 mg polysorbate 20. Reconstitution of the single-use vial with 1.3 ml sterile water for injection yields approximately 1.5 ml solution to deliver 125 mg per 1.25 ml (100 mg/ml) of antibody.
(c) A sterile, preservative-free lyophilized powder for intravenous (i.v.) administration. The product can be formulated with α-trehalose dihydrate, L-histidine HCl, histidine and polysorbate 20 USP. For example, each vial can contain 440 mg anti-CD19 antibody, 400 mg α,α-trehalose dihydrate, 9.9 mg L-histidine HCl, 6.4 mg L-histidine, and 1.8 mg polysorbate 20, USP. Reconstitution with 20 ml of bacteriostatic water for injection (BWFI), USP, containing 1.1% benzyl alcohol as a preservative, yields a multi-dose solution containing 21 mg/ml antibody at a pH of approximately 6.
(d) A sterile, lyophilized powder for intravenous infusion in which the anti-CD19 antibody is formulated with sucrose, polysorbate, monobasic sodium phosphate monohydrate, and dibasic sodium phosphate dihydrate. For example, each single-use vial can contain 100 mg antibody, 500 mg sucrose, 0.5 mg polysorbate 80, 2.2 mg monobasic sodium phosphate monohydrate, and 6.1 mg dibasic sodium phosphate dihydrate. No preservatives are present. Following reconstitution with 10 ml sterile water for injection, USP, the resulting pH is approximately 7.2.
(e) A sterile, preservative-free solution for subcutaneous administration supplied in a single-use, 1 ml pre-filled syringe. The product can be formulated with sodium chloride, monobasic sodium phosphate dihydrate, dibasic sodium phosphate dihydrate, sodium citrate, citric acid monohydrate, mannitol, polysorbate 80 and water for injection, USP. Sodium hydroxide may be added to adjust pH to about 5.2.

For example, each syringe can be formulated to deliver 0.8 ml (40 mg) of drug product. Each 0.8 ml contains 40 mg anti-CD19 antibody, 4.93 mg sodium chloride, 0.69 mg monobasic sodium phosphate dihydrate, 1.22 mg dibasic sodium phosphate dihydrate, 0.24 mg sodium citrate, 1.04 citric acid monohydrate, 9.6 mg mannitol, 0.8 mg polysorbate 80 and water for injection, USP.
(f) A sterile, preservative-free, lyophilized powder contained in a single-use vial that is reconstituted with sterile water for injection (SWFI), USP, and administered as a subcutaneous (s.c.) injection. The product can be formulated with sucrose, histidine hydrochloride monohydrate, L-histidine, and polysorbate. For example, a 75 mg vial can contain 129.6 mg or 112.5 mg of the anti-CD19 antibody, 93.1 mg sucrose, 1.8 mg L-histidine hydrochloride monohydrate, 1.2 mg L-histidine, and 0.3 mg polysorbate 20, and is designed to deliver 75 mg of the antibody in 0.6 ml after reconstitution with 0.9 ml SWFI, USP. A 150 mg vial can contain 202.5 mg or 175 mg anti-CD19 antibody, 145.5 mg sucrose, 2.8 mg L-histidine hydrochloride monohydrate, 1.8 mg L-histidine, and 0.5 mg polysorbate 20, and is designed to deliver 150 mg of the antibody in 1.2 ml after reconstitution with 1.4 ml SWFI, USP.
(g) A sterile, hyophilized product for reconstitution with sterile water for injection. The product can be formulated as single-use vials for intramuscular (IM) injection using mannitol, histidine and glycine. For example, each single-use vial can contain 100 mg antibody, 67.5 mg of mannitol, 8.7 mg histidine and 0.3 mg glycine, and is designed to deliver 100 mg antibody in 1.0 ml when reconstituted with 1.0 ml sterile water for injection. Alternatively, each single-use vial can contain 50 mg antibody, 40.5 mg mannitol, 5.2 mg histidine and 0.2 mg glycine, and is designed to deliver 50 mg of antibody when reconstituted with 0.6 ml sterile water for injection.
(h) A sterile, preservative-free solution for intramuscular (IM) injection, supplied at a concentration of 100 mg/ml. The product can be formulated in single-use vials with histidine, glycine, and sterile water for injection. For example, each single-use vial can be formulated with 100 mg antibody, 4.7 mg histidine, and 0.1 mg glycine in a volume of 1.2 ml designed to deliver 100 mg of antibody in 1 ml. Alternatively, each single-use vial can be formulated with 50 mg antibody, 2.7 mg histidine and 0.08 mg glycine in a volume of 0.7 ml or 0.5 ml designed to deliver 50 mg of antibody in 0.5 ml.

The pharmaceutical composition of the invention may be stable at 4°C. The pharmaceutical composition of the invention may be stable at room temperature.

### 5.4.2. ANTIBODY HALF-LIFE

In certain embodiments, the half-life of an anti-CD19 antibody of the compositions and for use in the methods of the invention is at least about 4 to 7 days. The mean half-life of the anti-CD19 antibody of the compositions and for use in the methods of the invention may be at least about 2 to 5 days, 3 to 6 days, 4 to 7 days, 5 to 8 days, 6 to 9 days, 7 to 10 days, 8 to 11 days, 8 to 12, 9 to 13, 10 to 14, 11 to 15, 12 to 16, 13 to 17,14 to 18, 15 to 19, or 16 to 20 days. The half-life of an anti-CD 19 antibody of the compositions and for use in the methods of the invention can be up to about 50 days. The half-lives of the antibodies of the compositions and for use in the methods of the invention can be prolonged by methods, known in the art. Such prolongation can in turn reduce the amount and/or frequency of dosing of the antibody compositions of the invention. Antibodies with improved *in vivo* half-lives and methods for preparing them are disclosed in U.S. Patent No. 6,277,375; and, International Publication Nos. WO 98/23289 and WO 97/3461.

The serum circulation of the anti-CD19 antibodies of the invention *in vivo may* also be prolonged by attaching inert polymer molecules such as high molecular weight polyethyleneglycol (PEG) to the antibodies with or without a multifunctional linker either through site-specific conjugation of the PEG to the N- or C-terminus of the antibodies or via epsilon-amino groups present on lysyl residues. Linear or branched polymer derivatization that results in minimal loss of biological activity will be used. The degree of conjugation can be closely monitored by SDS-PAGE and mass spectrometry to ensure proper conjugation of PEG molecules to the antibodies. Unreacted PEG can be separated from antibody-PEG conjugates by size-exclusion or by ion-exchange chromatography. PEG-derivatized antibodies can be tested for binding activity as well as for *in vivo* efficacy using methods known to those of skill in the art, for example, by immunoassays described herein.

Further, the antibodies of the compositions and for use in the methods of the invention can be conjugated to albumin in order to make the antibody more stable *in vivo* or have a longer half-life *in vivo.* The techniques are well known in the art, *see, e.g.,* International Publication Nos. WO 93/15199, WO 93/15200, and WO 01/77137; and European Patent No. EP 413, 622.

### 5.4.3. ADMINISTRATION AND DOSING

Administration of the compositions of the invention to a human patient can be by any route, including but not limited to intravenous, intradermal, transdermal, subcutaneous, intramuscular, inhalation (*e.g.*, via an aerosol), buccal (*e.g*., sub-lingual), topical (*i.e.*, both skin and mucosal surfaces, including airway surfaces), intrathecal, intraarticular, intraplural, intracerebral, intra-arterial, intraperitoneal, oral, intralymphatic, intranasal, rectal or vaginal administration, by perfusion through a regional catheter, or by direct intralesional injection. The compositions of the invention may be administered by intravenous push or intravenous infusion given over defined period (*e.g.*, 0.5 to 2 hours). The compositions of the invention may be delivered by peristaltic means or in the form of a depot, although the most suitable route in any given case will depend, as is well known in the art, on such factors as the species, age, gender and overall condition of the subject, the nature and severity of the condition being treated and/or on the nature of the particular composition (*i.e.*, dosage, formulation) that is being administered. The route of administration may be via bolus or continuous infusion over a period of time, once or twice a week. In other particular embodiments, the route of administration is by subcutaneous injection, optionally once or twice weekly. The compositions, and/or methods of the invention may be administered on an outpatient basis.

In certain embodiments, the dose of a composition comprising anti-CD19 antibody is measured in units of mg/kg of patient body weight. The dose of a composition comprising anti-CD19 antibody may be measured in units of mg/kg of patient lean body weight (*i.e.*, body weight minus body fat content). In yet other embodiments, the dose of a composition comprising anti-CD 19 antibody is measured in units of mg/m² of patient body surface area. The dose of a composition comprising anti-CD 19 antibody may be measured in units of mg per dose administered to a patient. Any measurement of dose can be used in conjunction with the compositions and antibodies for use in the methods of the invention and dosage units can be converted by means standard in the art.

Those skilled in the art will appreciate that dosages can be selected based on a number of factors including the age, sex, species and condition of the subject (*e.g*., stage of B cell malignancy), the desired degree of cellular depletion, the disease to be treated and/or the particular antibody or antigen-binding fragment being used and can be determined by one of skill in the art. For example, effective amounts of the compositions of the invention may be extrapolated from dose-response curves derived *in vitro test* systems or from animal model (*e.g*., the cotton rat or monkey) test systems. Models and methods for evaluation of the effects of antibodies are known in the art (Wooldridge et al., Blood, 89(8): 2994-2998 (1997)). For particular B cell malignancies, therapeutic regimens standard in the art for antibody therapy can be used with the compositions and antibodies for use in the methods of the invention.

Examples of dosing regimens that can be used in the methods described herein include, but are not limited to, daily, three times weekly (intermittent), weekly, or every 14 days. Dosing regimens include, but are not limited to, monthly dosing or dosing every 6-8 weeks.

Those skilled in the art will appreciate that dosages are generally higher and/or frequency of administration greater for initial treatment as compared with maintenance regimens.

The anti-CD19 antibodies described herein bind to B cells and, thus, can result in more efficient (*i.e.*, at lower dosage) depletion of B cells (as described herein). Higher degrees of binding may be achieved where the density of human CD 19 on the surface of a patient's B cells is high. Dosages of the antibody (optionally in a pharmaceutically acceptable carrier as part of a pharmaceutical composition) may be at least about 0.0005, 0.001, 0.05, 0.075, 0.1, 0.25, 0.375, 0.5, 1, 2.5, 5, 10, 20, 37.5, or 50 mg/m² and/or less than about 500, 475, 450, 425, 400, 375, 350, 325, 300, 275, 250, 225, 200, 175, 150, 125, 100, 75, 60, 50, 37.5, 20, 15, 10, 5, 2.5, 1, 0.5, 0.375, 0.1, 0.075 or 0.01 mg/m². The dosage may be between about 0.0005 to about 200 mg/m², between about 0.001 and 150 mg/m², between about 0.075 and 125 mg/m², between about 0.375 and 100 mg/m², between about 2.5 and 75 mg/m², between about 10 and 75 mg/m², and between about 20 and 50 mg/m². The dosage of anti-CD19 antibody used may be at least about 0.1, 0.2, 0.3, 0.4, 0.5, 0.6, 0.7, 0.8, 0.9, 1, 1.5,2,2.5, 3, 3.5, 4, 4.5, 5, 5.5, 6, 6.5, 7, 7.5, 8, 8.5, 9, 9.5, 10, 10.5, 11, 11.5, 12, 12.5,13,13.5, 14, 14.5, 15, 15.5, 16, 16.5, 17, 17.5, 18, 18.5, 19, 19.5, 20, 20.5 mg/kg of body weight of a patient. The dose of naked anti-CD 19 antibody used may be at least about 1 to 10, 5 to 15, 10 to 20, or 15 to 25 mg/kg of body weight of a patient. The dose of anti-CD19 antibody used may be at least about 1 to 20,3 to 15, or 5 to 10 mg/kg of body weight of a patient. The dose of anti-CD19 antibody used may be at least about 5, 6, 7, 8, 9, or 10 mg/kg of body weight of a patient. A single dosage unit of the antibody (optionally in a pharmaceutically acceptable carrier as part of a pharmaceutical composition) can be at least about 0.5, 1, 2, 4; 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118; 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 204, 206, 208, 210, 212, 214, 216, 218, 220, 222; 224, 226, 228, 230, 232, 234, 236, 238, 240, 242, 244, 246, 248, or 250 micrograms/m². The dose may be up to 1 g per single dosage unit.

All of the above doses are exemplary and can be used in conjunction with the compositions and antibodies for use in the methods of the invention, however where an anti-CD 19 antibody is used in conjunction with a toxin or radiotherapeutic agent the lower doses described above are preferred. Where the patient has low levels of CD 19 density, the lower doses described above are preferred.

Where chimeric anti-CD 19 antibodies are used, the dose or amount of the chimeric antibody may be greater than about 2, 3, 4, 5, 6, 7, 8, 9, 10, 11, 12, 13,14, 15, or 16 mg/kg of patient body weight. Where chimeric anti-CD19 antibodies are used, the dose or amount of the chimeric antibody may be less than about 1, 0.9, 0.8, 0.7, 0.6, 0.5, 0.4, 0.3, 0.2, or 0.1 mg/kg of patient body weight.
In some embodiments of this invention, antibodies and/or compositions of this invention can be administered at a dose lower than about 375 mg/m²; at a dose lower than about 37.5 mg/m²; at a dose lower than about 0.375 mg/m²; and/or at a dose between about 0.075 mg/m² and about 125 mg/m². Dosage regimens of the invention may comprise low doses, administered at repeated intervals. For example, the compositions of the invention can be administered at a dose lower than about 375 mg/m² at intervals of approximately every 1, 2, 3, 4, 5, 6, 7., 8, 9, 10, 15, 20, 25, 30, 35, 40, 45, 50, 60, 70, 80, 90, 100, 125, 150, 175, or 200 days.

The specified dosage can result in B cell depletion in the human treated using the compositions and antibodies for use in the methods of the invention for a period of at least about 1, 2, 3, 5, 7, 10, 14, 20, 30, 45, 60, 75, 90, 120, 150 or 180 days or longer. In certain embodiments, pre-B cells (not expressing surface immunoglobulin) are depleted. In certain embodiments, mature B cells (expressing surface immunoglobluin) are depleted. In other embodiments, all non-malignant types of B cells can exhibit depletion. Any of these types of B cells can be used to measure B cell depletion. B cell depletion can be measured in bodily fluids such as blood serum, or in tissues such as bone marrow. B cells may be depleted by at least 30%, 40%, 50%, 60%, 70%, 80%, 90%, or 100% in comparison to B cell levels in the patient being treated before use of the compositions and antibodies for use in the methods of the invention. B cells may be depleted by at least 30%, 40%, 50%, 60%, 70%, 80%, 90%, or 100% in comparison to typical standard B cell levels for humans. The typical standard B cell levels for humans are determined using patients comparable to the patient being treated with respect to age, sex, weight, and other factors.

A dosage of about 125 mg/m² or less of an antibody or antigen-binding fragment results in B cell depletion for a period of at least about 7, 14, 21, 30, 45, 60, 90, 120, 150, or 200 days. A dosage of about 37.5 mg/m² or less depletes B cells for a period of at least about 7, 14, 21, 30, 45, 60, 90, 120, 150, or 200 days. A dosage of about 0.375 mg/m² or less results in depletion of B cells for at least about 7, 14, 21, 30, 45 or 60 days. A dosage of about 0.075 mg/m² or less results in depletion of B cells for a period of at least about 7, 14, 21, 30, 45, 60, 90, 120, 150, or 200 days. A dosage of about 0.01 mg/m², 0.005 mg/m² or even 0.001 mg/m² or less results in depletion of B cells for at least about 3, 5, 7,10, 14, 21, 30, 45, 60, 90, 120, 150, or 200 days. The dosage can be administered by any suitable route, but is optionally administered by a subcutaneous route.

As another aspect, the invention provides the discovery that B cell depletion and/or treatment of B cell disorders can be achieved at lower dosages of antibody or antibody fragments than employed in currently available methods. The present invention may provide a method of depleting B cells and/or treating a B cell disorder, comprising administering to a human, an effective amount of an antibody that specifically binds to CD19, wherein a dosage of about 500, 475, 450, 425, 400, 375, 350, 325, 300, 275, 250, 225, 200, 175, 150, 125, 100, 75, 60, 50, 37.5,20, 10, 5, 2.5, 1, 0.5, 0.375, 0.25, 0.1, 0.075, 0.05, 0.001, 0.0005 mg/m² or less results in a depletion of B cells (circulating and/or tissue B cells) of 25%, 35%, 50%, 60%, 75%, 80%, 85%, 90%, 95%, 98% or more for a period at least about 3, 5, 7, 10, 14, 21, 30, 45, 60, 75, 90, 120, 150, 180, or 200 days or longer. A dosage of about 125 mg/m² or 75 mg/m² or less results in at least about 50%, 75%, 85% or 90% depletion of B cells for at least about 7,14, 21,30, 60, 75, 90, 120, 150 or 180 days. A dosage of about 50, 37.5 or 10 mg/m² results in at least about a 50%, 75%, 85% or 90% depletion of B cells for at least about 7, 14, 21, 30, 60, 75, 90, 120 or 180 days. A dosage of about 0.375 or 0.1 mg/m² results in at least about a 50%, 75%, 85% or 90% depletion of B cells for at least about 7, 14, 21, 30, 60, 75 or 90 days. A dosage of about 0.075, 0.01, 0.001, or 0.0005 mg/m² results in at least about a 50%, 75%, 85% or 90% depletion of B cells for at least about 7, 14, 21, 30 or 60 days.

The dose can be escalated or reduced to maintain a constant dose in the blood or in a tissue, such as, but not limited to, bone marrow. The dose may be escalated or reduced by about 2%, 5%, 8%, 10%, 15%, 20%, 30%, 40%, 50%, 60%, 70%, 80%, 90%, and 95% in order to maintain a desired level of the antibody of the compositions and for use in the methods of the invention.

The dosage can be adjusted and/or the infusion rate can be reduced based on patient's immunogenic response to the compositions and antibodies for use in the methods of the invention.

A loading dose of the anti-CD 19 antibody and/or composition of the invention can be administered first followed by a maintenance dose until the B cell malignancy being treated progresses or followed by a defined treatment course (*e.g*., CAMPATH™, MYLOTARG™, or RITUXAN™, the latter of which allow patients to be treated for a defined number of doses that has increased as additional data have been generated).

According to another aspect of the invention, a patient may be pretreated with the compositions and antibodies for use in the methods of the invention to detect, minimize immunogenic response, or minimize adverse effects of the compositions and antibodies for use in the methods of the invention.

### 5.4.4. TOXICITY TESTING

The tolerance, toxicity and/or efficacy of the compositions and/or treatment regimens of the present invention can be determined by standard pharmaceutical procedures in cell cultures or experimental animals, *e.g.,* for determining the LD50 (the dose lethal to 50% of the population), the ED50 (the dose therapeutically effective in 50% of the population), and IC50 (the dose effective to achieve a 50% inhibition). The dose may be a dose effective to achieve at least a 60%, 70%, 80%, 90%, 95%, or 99% depletion of circulating B cells or circulating immunogloblulin, or both. The dose ratio between toxic and therapeutic effects is the therapeutic index and it can be expressed as the ratio LD50/ED50. Therapies that exhibit large,therapeutic indices are preferred. While therapies that exhibit toxic side effects may be used, care should be taken to design a delivery system that targets such agents to CD 19-expressing cells in order to minimize potential damage to CD19 negative cells and, thereby, reduce side effects.

Data obtained from the cell culture assays and animal studies can be used in formulating a range of dosages of the compositions and/or treatment regimens for use in humans. The dosage of such agents lies preferably within a range of circulating concentrations that include the ED50 with little or no toxicity. The dosage may vary within this range depending upon the dosage form employed and the route of administration utilized. For any therapy used in the methods of the invention, the therapeutically effective dose can be estimated by appropriate animal models. Depending on the species of the animal model, the dose is scaled for human use according to art-accepted formulas, for example, as provided by Freireich et al., Quantitative comparison of toxicity of anticancer agents in mouse, rat, monkey, dog, and human, Cancer Chemotherapy Reports, NCI 1966 40:219-244. Data obtained from cell culture assays can be useful for predicting potential toxicity. Animal studies can be used to formulate a specific dose to achieve a circulating plasma concentration range that includes the IC50 (*i.e.*, the concentration of the test compound that achieves a half-maximal inhibition of symptoms) as determined in cell culture. Such information can be used to more accurately determine useful doses in humans. Plasma drug levels may be measured, for example, by high performance liquid chromatography, ELISA, or by cell based assays.

### 5.5. PATIENT DIAGNOSIS, STAGING AND THERAPEUTIC REGIMENS

The treatment regimen and dose used with the compositions and antibodies for use in the methods of the invention may be chosen based on a number of factors including, but not limited to, the stage of the B cell disease or disorder being treated. Appropriate treatment regimens can be determined by one of skill in the art for particular stages of a B cell disease or disorder in a patient or patient population. Dose response curves can be generated using standard protocols in the art in order to determine the effective amount of the compositions of the invention for treating patients having different stages of a B cell disease or disorder. In general, patients having more advanced stages of a B cell disease or disorder will require higher doses and/or more frequent doses which may be administered over longer periods of time in comparison to patients having an early stage B cell disease or disorder.

The anti-CD 19 antibodies of the compositions and for use in the methods of the invention can be used to treat B cell diseases, including B cell malignancies. The term "B cell malignancy" includes any malignancy that is derived from a cell of the B cell lineage. Exemplary B cell malignancies include, but are not limited to: B cell subtype non-Hodgkin's lymphoma (NHL) including low grade/follicular NHL, small lymphocytic (SL) NHL, intermediate grade/follicular NHL, intermediate grade diffuse NHL, high grade immunoblastic NHL, high grade lymphoblastic NHL, high grade small non-cleaved cell NHL; mantle-cell lymphoma, and bulky disease NHL; Burkitt's lymphoma; multiple myeloma; pre-B acute lymphoblastic leukemia and other malignancies that derive from early B cell precursors; common acute lymphocytic leukemia (ALL); chronic lymphocytic leukemia (CLL) including including immunoglobulin-mutated CLL and immunoglobulinunmutated CLL; hairy cell leukemia; Null-acute lymphoblastic leukemia; Waldenstrom's Macroglobulinemia; diffuse large B cell lymphoma (DLBCL) including germinal center B cell-like (GCB) DLBCL, activated B cell-like (ABC) DLBCL, and type 3 DLBCL; prolymphocytic leukemia; light chain disease; plasmacytoma; osteosclerotic myeloma; plasma cell leukemia; monoclonal gammopathy of undetermined-significance (MGUS); smoldering multiple myeloma (SMM); indolent multiple myeloma (IMM); Hodgkin's lymphoma including classical and nodular lymphocyte pre-dominant type; lymphoplasmacytic lymphoma (LPL); and marginal-zone lymphoma including gastric mucosal-associated lymphoid tissue (MALT) lymphoma.

The inventors have shown that the inventive antibodies and compositions can deplete mature B cells. The invention can be employed to treat mature B cell malignancies (*i.e.*, express Ig on the cell surface) including but not limited to follicular lymphoma, mantle-cell lymphoma, Burkitt's lymphoma, multiple myeloma, diffuse large B-cell lymphoma (DLBCL) including germinal center B cell-like (GCB) DLBCL, activated B cell-like (ABC) DLBCL, and type 3 DLBCL, Hodgkin's lymphoma including classical and nodular lymphocyte pre-dominant type, lymphoplasmacytic lymphoma (LPL), marginal-zone lymphoma including gastric mucosal-associated lymphoid tissue (MALT) lymphoma, and chronic lymphocytic leukemia (CLL) including immunoglobulin-mutated CLL and immunoglobulin-unmutated CLL.

Further, CD 19 is expressed earlier in B cell development than, for example, CD20, and is therefore particularly suited for treating pre-B cell and immature B cell malignancies *(i. e.,* do not express Ig on the cell surface), for example, in the bone marrow. Illustrative pre-B cell and immature B cell malignancies include, but are not limited to, acute lymphoblastic leukemia.

The invention may be practiced to treat extranodal tumors.

### 5.5.1. DIAGNOSIS AND STAGING OF B CELL MALIGNANCIES

The progression of cancer, such as a B cell disease or disorder capable of tumor formation (*e.g.*, non-Hodgkin lymphoma, diffuse large B cell lymphoma, follicular lymphoma, and Burkitt lymphoma) is typically characterized by the degree to which the cancer has spread through the body and is often broken into the following four stages which are prognostic of outcome. Stage I: The cancer is localized to a particular tissue and has not spread to the lymph nodes. Stage II: The cancer has spread to the nearby lymph nodes, *i.e.*, metastasis. Stage III: The cancer is found in the lymph nodes in regions of the body away from the tissue of origin and may comprise a mass or multiple tumors as opposed to one. Stage IV: The cancer has spread to a distant part of the body. The stage of a cancer can be determined by clinical observations and testing methods that are well known to those of skill in the art. The stages of cancer described above are traditionally used in conjunction with clinical diagnosis of cancers characterized by tumor formation, and can be used in conjunction with the compositions and antibodies for use in the methods of the present invention to treat B cell diseases and disorders. Typically early stage disease means that the disease remains localized to a portion of a patient's body or has not metastasized.

With respect to non-tumor forming B cell diseases and disorders such as, but not limited to, multiple myeloma, the criteria for determining the stage of disease differs. The Durie-Salmon Staging System has been widely used. In this staging system, clinical stage of disease (stage I, II, or III) is based on several measurements, including levels of M protein, the number of lytic bone lesions, hemoglobin values, and serum calcium levels. Stages are further divided according to renal (kidney) function (classified as A or B). According to the Durie-Salmon Staging System Stage I (low cell mass) is characterized by all of the following: Hemoglobin value >10 g/dL; Serum calcium value normal or ≤ 12 mg/dL; Bone x-ray, normal bone structure (scale 0) or solitary bone plasmacytoma only; and Low M-component production rate: IgG value <5 g/dL, IgA value <3 g/d, Bence Jones protein <4 g/24 h. Stage I patients typically have no related organ or tissue impairment or symptoms. Stage II (intermediate cell mass) is characterized by fitting neither stage I nor stage III. Stage III (high cell mass) is characterized by one or more of the following: Hemoglobin value <8.5 g/dL; Serum calcium value > 12 mg/dL; Advanced lytic bone lesions (scale 3); High M-component production rate: IgG value >7 g/dL, IgA value >5 g/dL, Bence Jones protein > 12 g/24 h Subclassification (either A or B), where A is Relatively normal renal function (serum creatinine value <2.0 mg/dL) and B is Abnormal renal function (serum creatinine value ≥ 2.0 mg/dL).

Another staging system for myeloma is the International Staging System (ISS) for myeloma. This system can more effectively discriminate between staging groups and is based on easily measured serum levels of beta 2-microglobulin (ß2-M) and albumin. According to the ISS for myeloma, Stage I is characterized by ß2-M <3.5 and Albumin ≥ 3.5, Stage II is characterized by ß2-M <3.5 and albumin <3.5 or ß2-M 3.5 - 5.5, and Stage III is characterized by ß2-M >5.5 (Multiple Myeloma Research Foundation, New Canaan, CT).

The stage of a B cell malignancy in a patient is a clinical determination. As indicated above, with respect to solid tumors, the spread, location, and number of tumors are the primary factors in the clinical determination of stage. Determination of stage in patients with non-tumor forming B cell malignancies can be more complex requiring serum level measurements as described above.

The descriptions of stages of B cell diseases and disorders above are not limiting. Other characteristics known in the art for the diagnosis of B cell diseases and disorders can be used as criteria for patients to determine stages of B cell diseases or disorders.

### 5.5.2. CLINICAL CRITERIA FOR DIAGNOSING B CELL MALIGNANCIES

Diagnostic criteria for different B cell malignancies are known in the art. Historically, diagnosis is typically based on a combination of microscopic appearance and immunophenotype. More recently, molecular techniques such as gene-expression profiling have been applied to develop molecular definitions of B cell malignancies (*see, e.g.*, Shaffer et al., Nature 2:920-932(2002)). Exemplary methods for clinical diagnosis of particular B cell malignancies are provided below. Other suitable methods will be apparent to those skilled in the art.

### 5.5.2.1. FOLLICULAR NHL

In general, most NHL (with the exception of mantle-cell lymphoma) have highly mutated immunoglobulin genes that appear to be the result of somatic hypermutation (SHM). The most common genetic abnormalities in NHL are translocations and mutations of the *BCL6* gene.

Follicular NHL is often an indolent B cell lymphoma with a follicular growth pattern. It is the second most common lymphoma in the United States and Western Europe. The median age at which this disease presents is 60 years and there is a slight female predominance. Painless lymphadenopathy is the most common symptom. Tests often indicate involvement of the blood marrow and sometimes the peripheral blood. Follicular NHL is divided into cytologic grades based on the proportion of large cells in the follicle with the grades forming a continuum from follicular small cleaved-cell to large-cell predominance. *(See,* S. Freeman, et al., Follicular Lymphoma, pp. 367-388, In Non-Hodgkin's Lymphomas, P. Mauch et al., eds., Lippincott Williams & Wilkins, Philadelphia, PA (2004); T. Lister et al., Follicular Lymphoma, pp. 309-324, In Malignant Lymphoma, B. Hancock et al., eds., Oxford University Press, New York, N.Y. (2000)).

Most follicular NHL is characterized by a translocation between chromosomes 14 and 18 resulting in overexpression of *BCL2.* Follicular NHL is also characterized by both SHM and ongoing SHM and a gene expression profile similar to germinal center (GC) B cells (*see, e.g.*, Shaffer et al., Nature 2:920-932 (2002)), which are the putative cells of origin for this malignancy. Heavy- and light chain rearrangements are typical. The tumor cells of this disease express monoclonal surface immunoglobulin with most expressing IgM. Nearly all follicular NHL tumor cells express the antigens CD19, CD20, CD79a, CD21, CD35 and CD10 but lack expression of CD5 and CD43. Paratrabecular infiltration with small cleaved cells is observed in the bone marrow. (*See,* S. Freedman et al., Follicular Lymphoma, pp. 367-388, In Non-Hodgkin's Lymphomas, P. Mauch et al., eds., Lippincott Williams & Wilkins, Philadelphia, PA (2004); T. Lister et al., Follicular Lymphoma, pp. 309-324, In Malignant Lymphoma, B. Hancock et al., eds., Oxford University Press, New York, N.Y. (2000)).

Diagnosis of follicular NHL generally relies on biopsy of an excised node in order to evaluate tissue architecture and cytological features. Fine-needle aspirations are usually not adequate since this procedure is less likely to provide tissue that can be evaluated and it fails to provide enough tissue for additional tests. Bilateral bone marrow biopsies are also indicated since involvement can be patchy. Additional diagnostic procedures include chest x-rays, chest, abdomen, neck and pelvis computed tomography (CT) scans, complete blood count, and chemistry profile. Flow cytometry and immunohistochemistry can be used to distinguish between follicular NHL and other mature B cell lymphomas. *(See,* S. Freedman et al., Follicular Lymphoma, pp. 367-388, In Non-Hodgkin's Lymphomas, P. Mauch et al., eds., Lippincott Williams & Wilkins, Philadelphia, PA (2004); T. Lister et al., Follicular Lymphoma, pp. 309-324, In Malignant Lymphoma, B. Hancock et al., eds., Oxford University Press, New York, N.Y. (2000)).

### 5.5.2.2. MANTLE-CELL LYMPHOMA

Mantle-cell lymphoma localizes to the mantle region of secondary follicles and is characterized by a nodular and/or diffuse growth pattern. Mantle-cell lymphoma patients have median age of 60-65 years with the disease affecting predominantly males. For diagnostic purposes, the usual presenting feature is a generalized lymphadenopathy. Additionally, the spleen is often enlarged. This B cell lymphoma is associated with a t(11;14) between the IgH locus and cyclin D1 gene, which results in overexpression of cyclin D1. More than 50% of cases show additional chromosomal abnormalities. Mantle-cell lymphoma is typically not characterized by SHM. (*See,* W. Hiddemann et al., Mantle Cell Lymphoma, pp. 461-476, In Non-Hodgkin's Lymphomas, P. Mauch et al., eds., Lippincott Williams & Wilkins, Philadelphia, PA (2004); D. Weisenburger et al., Mantle Cell Lymphoma, pp. 28-41, In Malignant Lymphoma, B. Hancock et al., eds., Oxford University Press, New York, N.Y: (2000)).

Immunophenotyping (flow cytometry or frozen section) immunohistochemistry of mantle cell lymphoma cells shows them to nearly always be monoclonal, bearing surface IgM. Mantle cell lymphoma cells have also been noted to bear surface IgD. The cells express the antigens CD19, CD20, CD22 and CD24, but not CD23. They also express surface antigens CD5 but not for CD10, distinguishing them from true follicle center-cell lymphomas which are almost always CD5 negative. Frequently, extranodal involvement is found including bone marrow infiltration and tumors of the liver and gastrointestinal tract. Mild anemia and leukemic expression is not uncommon with mantle-cell lymphoma. (*See,* A. Lal et al., Role of Fine Needle Aspiration in Lymphoma, pp. 181-220, In W. Finn et al., eds., Hematopathology in Oncology, Kluwer Academic Publishers, Norwell, MA (2004); W. Hiddemann et al., Mantle Cell Lymphoma, pp. 461-476, In Non-Hodgkin's Lymphomas, P. Mauch et al., eds., Lippincott Williams & Wilkins, Philadelphia, PA (2004)).

Diagnosis of mantle-cell lymphoma involves examination of the peripheral blood as well as bone marrow and lymph node biopsies. In addition, cytogenetic studies and immunophenotyping are useful in differential diagnosis. (*See,* W. Hiddemann, et al., Mantle Cell Lymphoma pp. 461-476, In Non-Hodgkin's Lymphomas, P. Mauch, et al., eds., Lippincott Williams & Wilkins, Philadelphia, PA (2004); D. Weisenburger, et al., Mantle Cell Lymphoma, pp. 28-41, In Malignant Lymphoma, B. Hancock, et al., eds., Oxford University Press, New York, N.Y. (2000)).

### 5.5.2.3. BURKITT'S LYMPHOMA

Burkitt's lymphoma is an aggressive B cell lymphoma typically observed in children and young adults and is usually associated with bulky disease of the jaw and/or abdomen. Approximately 20% of patients have bone marrow involvement. An endemic form of Burkitt's lymphoma involves Epstein-Barr virus (EBV) infection of malignant cells; the sporadic form is independent of EBV infection. A translocation of *c-myc* to immunoglobulin loci, which results in deregulation of the *c-myc* gene, is characteristic of this disease (t(8;14)(q24;q32)). Interestingly, deletions of the *c-myc* sequences appear to be involved in the sporadic form of the disease, while the endemic form usually involves point mutations or insertions. (See, V. Pappa, et al., Molecular Biology, pp. 133-157, In Malignant Lymphoma, B. Hancock, et al., eds., Oxford University Press, New York, N.Y. (2000)). Burkitt's lymphoma is also characterized by SHM, and the malignant cells have a gene expression profile similar to GC B cells, suggesting that this malignancy is derived from GC B cells.

Immunophenotype of Burkett's lymphoma shows the cells of this disease express CD19, CD20, CD22, and CD79a, but not CD5, CD23, cyclin D or terminal deoxynucleotidyl transferase. Frequently, these cells are positive for CD 10 and *BCL6* and usually negative for *BCL2.* (*See*, I. Magrath, et al., Burkitt's Lymphoma, pp. 477-501, In Non-Hodgkin's Lymphoma, P. Mauch, et al., eds., Lippincott Williams & Wilkins, Philadelphia, PA (2004)).

High grade B cell Burkitt's-like lymphoma is a lymphoma borderline between Burkitt's lymphoma and large B cell lymphoma. The cells of this lymphoma express CD19 and CD20 but expression of CD10, which is nearly always present in true Burkitt's lymphoma, is frequently absent. Because of this and other characteristics, some believe this lymphoma should be classified as a diffuse large B cell lymphoma. *(See,* K. Maclennan, Diffuse Aggressive B cell Lymphoma, pp. 49-54, In Malignant Lymphoma, B. Hancock, et al., eds., Oxford University Press, New York, N.Y. (2000)).

Diagnosis of Burkitt's lymphoma generally relies on detection of the translocation associated with this lymphoma; thus, conventional cytogenetic analysis is usually performed. Long distance polymerase chain reaction techniques and fluorescent in situ hybridization (FISH) have been used to detect Ig-myc junctions in the translocations and other genetic alterations associated with this disease. *(See,* R. Siebert, et al., Blood 91:984-990 (1998); T. Denyssevych, et al., Leukemia, 16:276-283 (2002)).

### 5.5.2.4. DIFFUSE LARGE B CELL LYMPHOMA (DLBCL)

DLBCL is the most common non-Hodgkin's lymphoma and can arise from small B cell lymphoma, follicular lymphoma or marginal zone lymphoma. Typically, patients present with lymphadenopathy; however, a large percent of patients present in extranodal sites as well, with gastrointestirlal involvement being the most common. Bone marrow involvement is observed in about 15% of patients. *(See,* Armitage, et al., Diffuse Large B cell Lymphoma, pp. 427-453, In Non-Hodgkin's Lymphomas, P. Mauch, et al., eds., Lippincott Williams & Wilkins, Philadelphia, PA (2004)). Heterogeneity in clinical, biological and morphological characteristics makes this group of lymphomas difficult to subclassify. However, two distinct subgroups have been identified with one expressing genes characteristic of germinal center B cells (GC-DLBCL) and the other overexpressing genes in peripheral blood B cells. Survival rates are significantly better for patients with GC-DLBCL than those with activated B cell type (ABC)-DLBCL. (*See*, W. Chan, Archives of Pathology and Laboratory Medicine 128(12):, 1379-1384 (2004)).

DLBCLs express the cell surface antigens CD 19, CD20, CD22, and CD79a. CD10 is expressed in the large majority of cases and CD5 expression is observed in about 10% of cases. *(See,* K. Maclennan, Diffuse Aggressive B cell Lymphoma, pp. 49-54, In Malignant Lymphoma, B. Hancock, et al., eds., Oxford University Press, New York, N.Y. (2000)). DLBCL is often marked by abnormalities of *BCL6* and/or translocations of *BCL2* to the IgH. locus. GC B cell like (GC) DLBCL is characterized by SHM with highly mutated immunoglobulin genes and ongoing SHM in malignant clones with a GC B cell-like gene expression profile. Most GC DLBCL have undergone immunoglobulin class switching. ABC-DLBCL is characterized by high level expression of NF-κB target genes including BCL2, interferon regulatory factor 4, CD44, FLIP and cyclin D. SHM, but not ongoing SHM, is present, and ABC-DLBCL does not have a GC B cell gene expression profile. Almost all ABC-DLBCL express a high level of IgM.

### 5.5.2.5. EXTRANODAL MARGINAL ZONE LYMPHOMA

Extranodal marginal-zone lymphoma is an extranodal lymphoma that occurs in organs normally lacking organized lymphoid tissue (*e.g*., stomach, salivary glands, lungs and thyroid glands). It is largely a disease that affects older adults with a median age of over 60 years. Often, chronic inflammation or autoimmune processes precede development of the lymphoma. Gastric mucosal-associated lymphoid tissue (MALT) lymphoma, the most common type of marginal-zone lymphoma, is associated with *Helicobacter pylori* infection. Studies have shown a resolution of symptoms with eradication of the *H. pylori* infection following an antibiotic regimen. The presenting symptoms for gastric MALT lymphoma include nonspecific dyspepsia, epigastric pain, nausea, gastrointestinal bleeding and anemia. Systemic symptoms are uncommon, as are elevated levels of lactate acid dehydrogenase. (*See,* J. Yahalom, et al., Extranodal Marginal Zone B cell Lymphoma of Mucosa- Associated Lymphoid Tissue, pp. 345-360, In Non-Hodgkin's Lymphomas, P. Mauch, et al., eds., Lippincott Williams & Wilkins, Philadelphia, PA (2004); J. Radford, Other Low-Grade Non-Hodgkin's Lymphomas, pp. 325-330, In Malignant Lymphoma, B. Hancock, et al., eds., Oxford University Press, New York, N.Y. (2000). Systemic B symptoms include fevers greater than 38°C for longer than 2 weeks without sign of infection, night sweats, extreme fatigue or unintentional weight loss of greater than or equal to 10% of body weight over the previous 6 months).

The immunophenotye of MALT lymphoma is characterized by expression of CD20, CD79a, CD21 and CD35 and lack of expression of CD5, CD23, and CD10. About half of MALT lymphomas express CD43. The immunoglobulin typically expressed in the tumor cells of this disease is IgM while IgD is not expressed. These features are critical in distinguishing this lymphoma from other small B cell lymphomas such as mantle cell lymphoma, lymphocytic lymphoma and follicular lymphoma. Trisomy 3 has been reported in 60% of MALT lymphoma cases. In 25-40% of gastric and pulmonary MALT lymphomas a t(11;18) is observed. This translocation is observed much less frequently in other MALT lymphomas. T(11;18) is associated with nuclear expression of *BCL10.* (*See,* J. Yahalom, et al., Extranodal Marginal Zone B cell Lymphoma of Mucosa- Associated Lymphoid Tissue, pp. 345-360, In Non-Hodgkin's Lymphomas, P. Mauch, et al., eds., Lippincott Williams & Wilkins, Philadelphia, PA (2004)). Marginal-zone lymphomas are generally characterized by SHM and ongoing SHM.

Diagnostic procedures include immunophenotyping or flow cytometry to determine the identity of the cell surface markers. In addition, molecular genetic analysis should be done to determine the presence of t(11;18) as this is an indicator that the disease will not respond to antibiotics. Histology can be used to determine the presence of *H. pylori.* Additional tests should include a complete blood count, basic biochemical tests including that for lactate acid dehydrogenase; CT scans of the abdomen, chest and pelvis and a bone marrow biopsy. (*See,* J. Yahalom, et al., Extranodal Marginal Zone B cell Lymphoma, of Mucosa- Associated Lymphoid Tissue, pp. 345-360, In Non-Hodgkin's Lymphomas, P. Mauch, et al., eds., Lippincott Williams & Wilkins, Philadelphia, PA (2004)).

### 5:5.2.6. NODAL MARGINAL ZONE B CELL LYMPHOMA

Nodal Marginal Zone B cell Lymphoma is a relatively newly classified lymphoma thus little has been published on it. It is a primary nodal B cell lymphoma sharing genetic and morphological characteristics with extranodal and splenic marginal zone lymphomas, but does not localize to the spleen or extranodally. Hepatitis C virus has been reported to be associated with this lymphoma as has Sjögren's syndrome. *(See,* F. Berger, et al., Nodal Marginal Zone B cell Lymphoma, pp. 361-365, In Non-Hodgkin's Lymphomas, P. Mauch, et al., eds., Lippincott Williams & Wilkins, Philadelphia, PA (2004)).

Nodal marginal zone lymphoma has a heterogeneous cytology and morphology. Due to its relatively high proportion of large cells this lymphoma, unlike the other marginal lymphomas (splenic and extranodal), cannot be classified as true low grade B cell lymphoma. The genetic and immunological phenotype of nodal marginal zone lymphoma includes expression of CD 19, CD20, *BCL2*, sIgM and cytoplasmic IgG (cIg). These cells do not express CD5, CD10, CD23, CD43 or cyclin D1. The translocation characteristic of MALT lymphoma, t(11;18), is not observed for nodal marginal zone lymphoma. These characteristics aid in the differential diagnosis of this lymphoma from other small B cell lymphomas. *(See,* F. Berger, et al., Nodal Marginal Zone B cell Lymphoma, pp. 361-365, In Non-Hodgkin's Lymphomas, P. Mauch, et al., eds., Lippincott Williams & Wilkins, Philadelphia, PA (2004)).

### 5.5.2.7. SPLENIC MARGINAL ZONE LYMPHOMA

Splenic Marginal Zone Lymphoma is an indolent micro-nodular B cell lymphoma with a characteristic clinical presentation of prominent splenomegaly and infiltration of the peripheral blood and the bone marrow. In addition, a relatively high level of liver involvement has been reported. A role for hepatitis C virus has been postulated for this lymphoma. The immunophenotype of splenic marginal zone lymphoma is typically CD20⁺, IgD⁺, *BCL2⁺,* p27⁺, CD3⁻, CD5⁻-,CD10⁻, CD23⁻, CD38⁻, CD43⁻, *BCL-6*⁻*,* and cyclin D1⁻. Genetic characteristics include a 7q deletion, p53 alterations and SHM. *(See,* M. Piris, et al., Splenic Marginal Zone Lymphoma, pp. 275-282, In Non-Hodgkin's Lymphomas, P. Mauch, et al., eds., Lippincott Williams & Wilkins, Philadelphia, PA (2004)).

Diagnosis generally relies on immunophenotyping to determine the identity of the cell surface markers. Genetic and biochemical analysis, in combination with data on cell surface markers, help to differentiate this lymphoma from other small B cell lymphomas. (*See,* M. Piris, et al., Splenic Marginal Zone Lymphoma, pp. 275-282, In Non-Hodgkin's Lymphomas, P. Mauch, et al., eds., Lippincott Williams & Wilkins, Philadelphia, PA (2004)).

### 5.5.2.8. ACUTE (B CELL) LYMPHOCYTIC LEUKEMIA (ALL)

ALL is a marrow-based neoplasm largely affecting children with the highest incidence between 1-5 years. Most common symptoms at presentation include fatigue, lethargy, fever and bone and joint pain. Fatigue and lethargy correlates with the degree of anemia present. An elevated white blood cell count is common at presentment. Radiographs of the chest often show skeletal lesions. Extramedullary spread is common and involves the central nervous system, testes, lymph nodes, liver, spleen and kidney. Anterior mediastinal masses are observed in only about 5-10% of newly diagnosed cases. (*See,* J. Whitlock, et al., Acute Lymphocytic Leukemia, pp. 2241-2271, In Wintrobe's Clinical Hematology, Tenth Edition, G. Lee, et al., eds. Williams & Wilkins, Baltimore, MD (1999)).

The immunophenotype of ALL is CD10⁺, CD19⁺, CD26⁺, and CD24⁺. Pre-B cell ALL cells express cytoplasmic but not surface immunoglobulin, while mature B cell ALL (which accounts for only 1-2% of ALL cases) is distinguished from other leukemias of B cell lineage by the expression of surface immunoglobulin. Cytogenetic characteristics of ALL includes t(8;14), t(2;8) and t(8;22). Although rarely detected at the cytogenetic level t(12;21) may be the most common cytogenetic abnormality associated with childhood ALL (observed in about 25% of cases). *(See,* M. Kinney, et al., Classification and Differentiation of the Acute Leukemias, pp. 2209-2240, In Wintrobe's Clinical Hematology, Tenth Edition, G. Lee, et al., eds. Williams & Wilkins, Baltimore, MD (1999); J Whitlock, et al., Acute Lymphocytic Leukemia, pp. 2241-2271; In Wintrobe's Clinical Hematology, Tenth Edition, G. Lee, et al., eds. Williams & Wilkins, Baltimore, MD, (1999)).

Precise diagnosis of acute leukemia usually relies on a bone aspirate and biopsy. Aspirate smears are used for morphological, immunological and cytological assessments. The demonstration of lymphoblasts in the bone marrow is diagnostic of ALL. The presence of greater than 5% leukemic lymphoblast cells in the bone marrow confirms ALL diagnosis but most require greater than 25% for a definitive diagnosis. Lumbar punctures are used to diagnose central nervous system involvement. Serum uric acids levels and serum lactate dehydrogenase levels have been found to be elevated in ALL. (*See,* M. Kinney, et al., Classification and Differentiation of the Acute Leukemias, pp. 2209-2240, In Wintrobe's Clinical Hematology, Tenth Edition, G. Lee, et al., eds. Williams & Wilkins, Baltimore, MD (1999); J. Whitlock, et al., Acute Lymphocytic Leukemia, pp. 2241-2271; In Wintrobe's Clinical Hematology, Tenth Edition, G. Lee, et al., eds. Williams & Wilkins, Baltimore, MD, (1999)).

### 5.5.2.9. CHRONIC LYMPHOCYTIC LEUKEMIA (CLL)/SMALL B CELL LYMPHOCYTIC LYMPHOMA (SLL)

CLL/SLL is the most common type of leukemia. When the disease involves the peripheral blood and bone marrow it is referred to as CLL. However, when the lymph nodes and other tissues are infiltrated by cells that are immunologically and morphologically identical to those in CLL, but where leukemic characteristics of the disease are absent, then the disease is referred to as SLL. This disease largely afflicts the elderly with a greater incidence of the disease occurring in men than women. Painless lymphadenopathy is the most common finding at presentation. Hypogammaglobulinemia is common with most cases of CLL/SLL exhibiting reduced levels of all immunoglobulins rather than any particular subclass of immunoglobulins. Asymptomatic patients are frequently diagnosed during routine blood counts (lymphocyte count of over 5000x10⁹/L). As many as 20% of-CLL/SLL cases report B symptoms. An additional diagnostic feature is infiltration of the bone marrow by more than 30% by immature lymphocytes. Lymph node biopsies generally show infiltration of involved nodes with well-differentiated lymphocytes. Autoimmune phenomena are often associated with CLL/SLL including autoimmune hemolytic anemia and immune thrombocytopenia. (*See,* J. Gribben, et al., Small B cell Lymphocytic Lymphoma/Chronic Lymphocytic Leukemia and Prolymphocytic Leukemia, pp. 243-261, In Non-Hodgkin's Lymphomas, P. Mauch, et al., eds., Lippincott Williams & Wilkins, Philadelphia, PA (2004); K. Maclennan, Diffuse Indolent B cell Neoplasms, pp. 43-47, In Malignant Lymphoma, B. Hancock, et al., eds., Oxford University Press, New York, N.Y. (2000); Clinical Oncology, A. Neal, et al., Neal, Hoskin and Oxford University Press, co-publ., New York, NY (2003)).

In contrast with many of the low-grade B cell malignancies, nonrandom reciprocal translocations are rarely found in CLL/SLL. However, other cytogenetic abnormalities have been reported including deletions at 13q14, 11q22-23 and 17q13, with the latter two involving the p53 locus. Approximately 20% of cases exhibit trisomy 12. An elevated level of ß-2 microglobulin, higher levels of CD38 expression and the production of tumor necrosis factor-alpha are all characteristic of CLL/SLL. The immunophenotype of CLL/SLL is very diagnostic and includes weak expression of surface immunoglobulin usually IgM, or IgM and IgG, as well as expression of the cell antigens CD19, CD20 and usually CD5 and CD23. (*See,* J. Gribben, et al., Small B cell Lymphocytic Lymphoma/Chronic Lymphocytic Leukemia and Prolymphocytic Leukemia, pp. 243-261, In Non-Hodgkin's Lymphomas, P. Mauch, et al., eds., Lippincott Williams & Wilkins, Philadelphia, PA (2004); K. Maclennan, Diffuse Indolent B cell Neoplasms, pp. 43-47, In Malignant Lymphoma, B. Hancock, et al., eds., Oxford University Press, New York, N.Y. (2000)).

### 5.5.2.10. B CELL PROLYMPHOCYTIC LEUKEMIA (PLL)

PLL, once considered a variant of CLL, is now understood to be a distinct disease. PLL is generally a disease of elderly men and is characterized by a very high white blood cell count (greater than 200x10⁹/L) and splenomegaly. Additional symptoms include anemia and thrombocytopenia. Prolymphocytes in PLL comprise more than 55% of the cells in the blood and bone marrow. In contrast with CLL, autoimmune phenomena are rarely observed in PLL. (*See,* J. Gribben, et al., Small B cell Lymphocytic Lymphoma/Chronic Lymphocytic Leukemia and Prolymphocytic Leukemia, pp. 243-261, In Non-Hodgkin's Lymphomas, P. Mauch, et al., eds., Lippincott Williams & Wilkins, Philadelphia, PA (2004)).

The immunophenotype of PLL is characterized by expression of CD19, CD21, CD22, CD24 and FMC7. The cells of PLL do not express CD23 and most do not express CD5. PLL cells exhibit complex chromosomal abnormalities, with deletions at 13q14 and 11q23 being some of the most frequent. The pattern of p53 mutation in PLL cells is different from that observed for CLL. Differential diagnosis usually relies on complete blood count, histological, immunophenotypic, and genetic analyses. (*See,* J. Gribben, et al., Small B cell Lymphocytic Lymphoma/Chronic Lymphocytic Leukemia and Prolymphocytic Leukemia, pp. 243-261, In Non-Hodgkin's Lymphomas, P. Mauch, et al., eds., Lippincott Williams & Wilkins, Philadelphia, PA (2004)).

### 5.5.2.11 HAIRY CELL LEUKEMIA (HCL)

HCL is a rare, indolent chronic leukemia affecting more men than women and largely those of middle age. The typical symptoms include massive splenomegaly and pancytopenia. The peripheral blood and bone marrow contain the typical "hairy cells," which are B lymphocytes with cytoplasmic projections. Over 90% of HCL patients have bone marrow infiltration. (*See,* Clinical Oncology, A. Neal, et al., Neal, Hoskin and Oxford University Press, co-publ., New York, NY (2003); J. Johnston, Hairy Cell Leukemia, pp. 2428-2446, In Wintrobe's Clinical Hematology, Tenth Edition, G. Lee et al., eds. Williams & Wilkins, Baltimore, MD (1999)).

Cytogenetic analysis has shown that clonal abnormalities are present in 19% of cases and involve numerical and structural abnormalities of chromosomes 5, 7 and 14. The serum level of TNF-α is elevated in hairy cell leukemia and correlates with tumor burden. Hairy cell leukemia cells express surface immunoglobulins (IgG and IgM) and CD11c, CD19, CD20, CD22 and typically CD25. In addition, FMC7, HC-2 and CD103 are expressed. HCL cells do not express CD5 or CD10. Diagnosis generally involves the use of bone marrow aspirates, cytogenetics, blood smears and immunophenotyping. *(See,* Clinical Oncology, A. Neal, et al., Neal, Hoskin and Oxford University Press, co-publ., New York, NY (2003); J. Johnston, Hairy Cell Leukemia, pp. 2428-2446, In Wintrobe's Clinical Hematology, Tenth Edition, G. Lee et al., eds. Williams & Wilkins, Baltimore, MD (1999)).

### 5.5.2.12. PRECURSOR B CELL LYMPHOBLASTIC LYMPHOMA/PRE-B CELL ACUTE LYMPHOBLASTIC LEUKEMIA/LYMPHOBLASTIC LYMPHOMA

Precursor B cell lymphoblastic lymphoma/pre-B cell acute lymphoblastic leukemia/Lymphoblastic lymphoma is a disease of precursor T or B cells. The T and B cell lymphoblastic lymphomas are morphologically identical, but clinical distinctions may be made based on degree of bone marrow infiltration or bone marrow involvement. 85-90% of lymphoblastic lymphomas are T-cell derived with the remainder being B cell derived. Lymphoblastic lymphoma has a median age of 20 years with a male predominance. Peripheral lymph node involvement is a common feature at presentation, occurring especially in the cervical, supraclavicular and axillary regions. This disease frequently presents with bone marrow involvement. Central nervous system is less common at presentment but often appears in cases of relapse. Other sites of involvement can include liver, spleen, bone, skin, pharynx and testes *(See,* J. Sweetenham, et al., Precursor B- and T-Cell Lymphoblastic Lymphoma, pp. 503-513, In Non-Hodgkin's Lymphomas, P. Mauch, et al., eds., Lippincott Williams & Wilkins, Philadelphia, PA (2004)).

Precursor B cell lymphoblastic lymphomas express immature markers B cell markers such as CD99, CD34 and terminal deoxynucleotidyl transferase. These cells also express CD79a, CD19, and sometimes CD20 and typically lack expression of CD45 and surface immunoglobulin. Translocations at 11q23, as well as t(9;22)(q34;q11.2) and t(12;21)(p13;q22), have been associated with poor prognosis. Good prognosis is associated with hyperdiploid karyotype, especially that associated with trisomy 4, 10, and 17 and t(12;21)(p13;q22). *(See,* J. Sweetenham, et al., Precursor B- and T-Cell Lymphoblastic Lymphoma, pp. 503-513, In Non-Hodgkin's Lymphomas, P. Mauch, et al., eds., Lippincott Williams & Wilkins, Philadelphia, PA (2004)).

Diagnostic tests include lymph node biopsies, blood tests, x-rays, CT scans, and lumbar punctures to examine the cerebralspinal fluid for malignant cells.

### 5.5.2.13. PRIMARY MEDIASTINAL LARGE B CELL LYMPHOMA

Primary mediastinal, large B cell lymphoma is a diffuse large B cell lymphoma occurring predominantly in young women and characterized by a locally invasive anterior mediastinal mass originating in the thymus. Distant spread to peripheral nodes and bone marrow involvement is unusual. Systemic symptoms are common. While this disease resembles nodal large cell lymphomas, it has distinct genetic, immunological, and morphological characteristics.

The immunophenotype of tumor cells of primary mediastinal large B cell lymphoma are often surface immunoglobulin negative but do express such B cell associated antigens as CD 19, CD20, CD22, and CD79a. CD10 and *BCL6* are also commonly expressed. Expression of plasma cell associated markers CD15, CD30, epithelial membrane antigen (EMA) is rare. *BCL6* and *c-myc* gene arrangements are also uncommon. The presence of clonal immunoglobulin rearrangements, immunoglobulin variable region and gene hypermutation along with *BCL6* hypermutation suggest that this lymphoma derives from a mature germinal center or post-germinal center B cell. The chromosomal translocations that seem to be associated with tumors of this disease are similar to those observed in other forms of diffuse large cell lymphoma. (*See,* P. Zinzani, et al., Primary Mediastinal Large B cell Lymphoma, pp. 455-460, In Non-Hodgkin's Lymphomas, P. Mauch, et al., eds., Lippincott Williams & Wilkins, Philadelphia, PA (2004)).

The diagnostic evaluation for primary mediastinal large B cell lymphoma generally includes a complete physical examination, complete hematological and biochemical analysis, total-body computerized tomography and bone marrow biopsy. Gallium-67 scanning is a useful test for staging, response to treatment and for assessment of relapse. *(See,* P. Zinzani et al., Primary Mediastinal Large B cell Lymphoma, pp. 455-460, In Non-Hodgkin's Lymphomas, P. Mauch, et al., eds., Lippincott Williams & Wilkins, Philadelphia, PA (2004)).

### 5.5.2.14. LYMPHOPLASMACYTIC LYMPHOMA (LPL)/LYMPHOPLASMACYTIC IMMUNOCYTOMA/WALDSTRÖM'S MACROGLOBULINEMIA

LPL/Lymphoplasmacytic immunocytoma/Waldström's Macroglobulinemia is a nodal lymphoma that is usually indolent, and often involves bone marrow, lymph nodes and spleen. This is generally a disease of older adults with males slightly predominating. Most patients have monoclonal IgM paraprotein in their serum (>3g/dL) resulting in hyperviscosity of the serum. Tumor cells have a plasmacytic morphology. A subset of LPL is characterized by recurrent translocations between chromosomes 9 and 14, which involves the *PAX5* and immunoglobulin heavy-chain loci. LPL is characterized by SHM as well as ongoing SHM, and is believed to be derived from post-GC B cells. (*See,* A. Rohatiner, et al., Lymphoplasmacytic Lymphoma and Waldström's Macroglobulinemia, pp. 263-273, In Non-Hodgkin's Lymphomas, P. Mauch, et al., eds., Lippincott Williams & Wilkins, Philadelphia, PA (2004); K. Maclennan, Diffuse Indolent B cell Neoplasms, pp. 43-47, In Malignant Lymphoma, B. Hancock, et al., eds., Oxford University Press, New York, N.Y. (2000); A. Lal, et al., Role of Fine Needle Aspiration in Lymphoma, pp. 181-220, In W. Finn, et al., eds., Hematopathology in Oncology, Kluwer Academic Publishers, Norwell, MA (2004)).

The immunophenotype of this disease shows expression of the B cell associated antigens CD19, CD20, CD22, and CD79a and a lack of expression of CD5, CD10, and CD23. Presence of strong surface immunoglobulin and CD20, the lack of expression of CD5, and CD23 and the presence of cytoplasmic immunoglobulin are characteristics that aid in distinguishing this disease from chronic lymphocytic leukemia. Also diagnostic of this disease is t(9;14)(p13;q32). (*See,* A. Rohatiner, et al., Lymphoplasmacytic Lymphoma and Waldström's Macroglobulinemia, pp. 263-273, In Non-Hodgkin's Lymphomas, P. Mauch, et al., eds., Lippincott Williams & Wilkins, Philadelphia, PA (2004); K. Maclennan, Diffuse Indolent B cell Neoplasms, pp. 43-47, In Malignant Lymphoma, B. Hancock, et al., eds., Oxford University Press, New York, N.Y. (2000); R. Chaganti, et al., Cytogenetics of Lymphoma, pp. 809-824, In Non-Hodgkin's Lymphomas, P. Mauch, et al., eds., Lippincott Williams & Wilkins, Philadelphia, PA (2004)).
Diagnostic tests typically include a complete blood count, renal and liver function tests, CT scans, biopsy and aspiration of the bone marrow, protein electrophoresis to quantify and characterize the paraprotein and serum viscosity. Measurement of ß₂-microglobulin is used as a prognostic test. *(See,* A. Rohatiner, et al., Lymphoplasmacytic Lymphoma and Waldström's Macroglobulinemia, pp. 263-273, In Non-Hodgkin's Lymphomas, P. Mauch, et al., eds., Lippincott Williams & Wilkins, Philadelphia, PA (2004)).

### 5.5.2.15. NULL-ACUTE LYMPHOBLASTIC LEUKEMIA

Null-acute lymphoblastic leukemia is a subset of ALL which lacks B- or T-cell characteristics. Phenotypic analysis of leukemic blasts shows a typical null ALL pattern, i.e., CD10 (common ALL antigen)-negative, strongly HLA-DR-positive, and CD19 (B4)-positive (see Katz et al. (1988) Blood 71(5):1438-47).

### 5.5.2.16. HODGKIN'S LYMPHOMA

Hodgkin's lymphoma usually arises in the lymph nodes of young adults. It can be divided into classical subtype and a less common nodular lymphocytic predominant subtype. The classical type exhibits SHM, but not ongoing SHM, and does not have a GC B cell gene expression profile. The nodular lymphocyte predominant type, in contrast, is characterized by SHM and ongoing SHM and a GC B cell gene expression profile. While the two types differ clinically and biologically, they do share certain features such as a lack of neoplastic cells within a background of benign inflammatory cells. B. Schnitzer et al., Hodgkin Lymphoma, pp. 259-290, In W. Finn and L. Peterson, eds., Hematopathology in Oncology, Kluwer Academic Publishers, Norwell, MA (2004)).

The most common features at presentation are painless enlargement of lymph nodes, usually in the neck, but occasionally in the inguinal region. Waxing and waning of nodes is also characteristic of this disease. B symptoms are observed in about one-third of patients. Isolated extranodal involvement is rare and in cases where dissemination has occurred extranodal involvement is observed about 10-20% of the time. (*See,* P. Johnson et al., Hodgkin's Disease: Clinical Features, pp. 181-204, In Malignant Lymphoma, B. Hancock, et al., eds., Oxford University Press, New York, N.Y. (2000)).

Reed-Sternberg (RS) cells are the malignant cells of Hodgkin's lymphoma. RS cells and their variants express CD15, CD25, CD30 and transferrin receptor. In addition these cells express polyclonal cytoplasmic immunoglobulin. In most cases of Hodgkin's lymphoma the RS cells do not express CD45, a feature that aids in distinguishing this disease from non-Hodgkin's Lymphomas. Epstein Barr virus has been demonstrated to be present in Reed-Sternberg cells in about one-half of Hodgkin's lymphoma cases but its role is unclear.

Diagnosis is most frequently made by lymph node biopsy. Additional diagnostic tests include a full blood count (often hematological tests are normal; white blood cell counts of less than 1.0 x 10⁹/L are seen in about 20% of cases), erythrocyte sedimentation rate (often elevated in advanced stages of the disease), biochemical tests including electrolytes, urea, creatinine, urate, calcium (hypercalcemia is rare but when present is associated with extensive bone involvement), liver blood tests, lactate dehydrogenase (elevated levels often associated with advanced disease), albumin and beta₂-microglobulin (β2-M). Lymphanigiograms and chest x-rays and CT scans of the chest, abdomen and pelvis are important in identifying abnormal lymph nodes and the extent of extranodal involvement. Bone marrow biopsies are typically considered optional as bone marrow involvement is unusual and the results of such biopsies appear not to affect clinical management or prognosis. Splenechtomies are not usually performed today as it rarely influences management and CT or MRI imaging provides information on splenic status. Significantly elevated levels of p55, TNF and sICAM-1 are correlated to the stage of the disease, presence of symptoms and complete response rate. (*See,* P. Johnson, et al., Hodgkin's Disease: Clinical Features, pp. 181-204, In Malignant Lymphoma, B. Hancock, et al., eds., Oxford University Press, New York, N.Y. (2000); Clinical Oncology, A. Neal, et al., Neal, Hoskin and Oxford University Press, co-publ., New York, NY (2003); R. Stein, Hodgkin's Disease, pp. 2538-2571, In Wintrobe's Clinical Hematology, Tenth Edition, G. Lee et al., eds. Williams & Wilkins, Baltimore, MD (1999)).

### 5.5.2.17. MULTIPLE MYELOMA

Multiple myeloma is a malignancy of plasma cells. Neoplastic cells are located in the bone marrow, and osteolytic bone lesions are characteristic. Reciprocal chromosomal translocations between one of the immunoglobulin loci and a variety of other genes, *e.g*., cyclin D1, cyclin D3, c-MAF, MMSET (multiple myeloma SET-domain protein) or fibroblast growth factor receptor 3 are believed to be the primary oncogenic events. Multiple myeloma is characterized by SHM, and the putative cell of origin is a post-GC B cell. Multiple myeloma is typically first identified by symptoms such as recurrent infection, fatigue, pain, and kidney problems and is confirmed with clinical testing (*see,* for example, Cancer: Principles and Practice of Oncology. 6th edition. DeVita, V.T., Hellman, S. and Rosenberg, S. A. editors. 2001 Lippincott Williams and Wilkins Philadelphia, PA 1910.6 pp. 2465-2499).

Patients who are candidates for treatment by the compositions and antibodies for use in the methods of the invention can undergo further diagnostic tests on blood and/or urine to confirm the diagnosis or suspicion of multiple myeloma including, but not limited to, complete blood count (CBC) tests to determine if the types of cells reported in a CBC are within their normal ranges which are well known in the art, blood chemistry profile to determine whether levels of various blood components, such as albumin, blood urea nitrogen (BUN), calcium, creatinine, and lactate dehydrogenase (LDH), deviate from standard values. Serum levels of beta₂-microglobulin (ß₂-M) can also be examined, and surrogate markers for IL-6, a growth factor for myeloma cells. Urinalysis can be used to measure the levels of protein in the urine. Electrophoresis can be used to measure the levels of various proteins, including M protein in the blood (called serum protein electrophoresis, or SPEP) or urine (called urine electrophoresis, or UEP). An additional test, called immunofixation electrophoresis (IFE) or immunoelectrophoresis, may also be performed to provide more specific information about the type of abnormal antibody proteins present. Assessing changes and proportions of various proteins, particularly M protein, can be used to track the progression of myeloma disease and response to treatment regimens. Multiple myeloma is characterized by a large increase in M protein which is secreted by the myeloma tumor cells.

Diagnostic tests on bone can also be conducted to confirm the diagnosis or suspicion of multiple myeloma including, but not limited to, X-rays and other imaging tests-including a bone (skeletal) survey, magnetic resonance imaging (MRI), and computerized axial tomography (CAT), also known as computed tomography (CT) can assess changes in the bone structure and determine the number and size of tumors in the bone. Bone marrow aspiration or bone marrow biopsy can be used to detect an increase in the number of plasma cells in the bone marrow. Aspiration requires a sample of liquid bone marrow, and biopsy requires a sample of solid bone tissue. In both tests, samples are preferably taken from the pelvis (hip bone). The sturnum (breast bone) can also be used for aspiration of bone marrow.

Patients with multiple myeloma are typically categorized into the following three groups that help define effective treatment regimens. Monoclonal gammopathy of undetermined significance (MGUS) is typically characterized by a serum M protein level of less than 3 g/dL, bone marrow clonal plasma cells of less than 10%, no evidence of other B cell disorders, and no related organ or tissue impairment, such as hypercalcemia (increased serum calcium levels), impaired kidney function noted by increased serum creatinine, anemia, or bone lesions. Asymptomatic myelomas are typically stage I and includes smoldering multiple myeloma (SMM) and indolent multiple myeloma (IMM). SMM is characterized by serum M protein greater than or equal to 3 g/dL and IMM is characterized by bone marrow clonal plasma cells greater than or equal to 10% of the bone marrow cells. Symptomatic myeloma is characterized by M protein in serum and/or urine and includes Stage II multiple myeloma characterized by the presence of bone marrow clonal plasma cells or plasmacytoma and Stage III multiple myeloma characterized by related organ or tissue impairment.

Osteosclerotic myeloma is a component of the rare POEMS syndrome (polyneuropathy, organomegaly, endocrinopathy, monoclonal gammopathy and skin lesions). Peak incidence is at 40 to 50 years of age. Systemic features include skeletal lesions, marrow-plasma cells < 5%, a normal CBC, increased platelets, and organomegaly. The CSF has a high protein with no cells present. The M-protein levels are low (< 3g/dl, median = 1.1 g/dl); heavy chain class - usually α or γ; light chain class -usually λ; rare urine monoclonal and occasional cryoglobulinemia. Neuropathy occurs in 50% of the patients with weakness both proximal and distal, sensory loss is greater in larger than small fibers; and demyelination and long distal latency.

Smoldering multiple myeloma patients generally present with stable disease for months/years; no anemia, bone lesions, renal insufficiency or hypercalcemia; have >10% plasma cells in bone marrow and monoclonal serum protein. The criteria for smoldering multiple myeloma is compatible with the diagnosis of multiple myeloma; however, there is no evidence of progressive course. These are cases with a slow progression, the tumor cell mass is low at diagnosis and the percentage of bone marrow plasma cells in S phase is low (<0.5%). Characteristic clinical features include: serum M protein levels >3 g/dL and/or bone marrow plasma cells ≥10%; absence of anemia, renal failure, hypercalcemia, lytic bone lesions.

Indolent (or asymptomatic) multiple myeloma is a multiple myeloma diagnosed by chance in the absence of symptoms, usually after screening laboratory studies. Indolent multiple myeloma is similar to smoldering myeloma but with few bone lesions and mild anemia. Most cases of indolent multiple myeloma develop overt multiple myeloma within 3 years. Diagnostic criteria are the same as for multiple myeloma except: no bone lesions or one asymptomatic lytic lesion (X-ray survey); M component level <3 g/dL for IgG, 2 g/dL for IgA urine light chain < 4 g/24h; hemoglobin > 10 g/dl, serum calcium normal, serum creatinine <2 mg/dL, and no infections.

### 5.5.2.18. SOLITARY PLASMACYTOMA

Solitary plasmacytoma is one of a spectrum of plasma cell neoplasms which range from benign monoclonal gammopathy to solitary plasmacytoma to multiple myeloma. Approximately seventy per cent of all solitary plasmacytoma cases eventually result in multiple myeloma. These diseases are characterized by a proliferation of B cells which produce the characteristic paraprotein. Solitary plasmacytoma results in a proliferation of clonal plasma cells in a solitary site, usually a single bone or extramedullary tissue site. Diagnostic criteria of solitary plasmacytoma include a histologically confirmed single lesion, normal bone biopsy, negative skeletal survey, no anemia, normal calcium and renal function. Most cases exhibit minimally elevated serum M-protein (paraprotein). The median age at diagnosis is 50-55, about 5-10 years younger than the median age for multiple myeloma. *(See,* C. Wilson, The Plasma Cell Dycrasias, pp. 113-144, In W Finn and L. Peterson, eds., Hematopathology in Oncology, Kluwer Academic Publishers, Norwell, MA (2004), S. Chaganti, et al., Cytogenetics of Lymphoma, pp. 809-824, In Non-Hodgkin's Lymphomas, P. Mauch, et al., eds., Lippincott Williams & Wilkins, Philadelphia, PA, (2004)).

The immunophenotypic and genetic features of plasmacytoma appear to be similar to multiple myeloma.

### 5.5.2.19. LIGHT CHAIN DISEASE/LIGHT CHAIN DEPOSITION DISEASE (LCDD)

LCDD is a plasma cell dycrasias disorder caused by the over-synthesis of immunoglobulin light chains (usually kappa light chains) that are deposited in tissues. Patients commonly present with organ dysfunction, weakness, fatigue and weight loss. In approximately 80% of cases of LCDD a monoclonal immunoglobulin is detected. Detection of monoclonal kappa light chains using immunofluorescent techniques is limited by the tendency of light chains to give excess background staining, therefore, ultrastructural immunogold labeling may be necessary. *(See,* C. Wilson, The Plasma Cell Dycrasias; pp. 113-144, In W. Finn and L. Peterson, eds., Hematopathology in Oncology, Kluwer Academic Publishers, Norwell, MA (2004)).

### 5.5.2.20. PLASMA CELL LEUKEMIA (PCL),

PCL, a plasma cell dycrasias, is a rare aggressive variant of multiple myeloma. The criteria for plasma cell leukemia is a peripheral blood absolute plasma cell count of greater than 2x10⁹/L or plasma cells greater than 20% of white blood cells. Determination of the presence of a CD138⁺ population with cytoplasmic light chain restriction by flow cytometry. will distinguish PCL from lymphoid neoplasm with plasmacytic features. PCL cells are also characterized by the lack of surface light chain and CD 19 expression, and either no or weak expression of CD45. About 50 % of cases of PCL express CD20 and about 50% lack expression of CD56. The genetic abnormalities observed in PCL patients are the same as those observed for multiple myeloma patients but they are found at higher frequency in PCL. *(See,* C. Wilson, The Plasma Cell Dycrasias, pp. 113-144, In W. Finn and L. Peterson, eds., Hematopathology in Oncology, Kluwer Academic Publishers, Norwell, MA, (2004)).

Plasma cell leukemia has two forms: if initial diagnosis is based on leukemic phase of myeloma then the primary form is present, otherwise it is secondary. Primary plasma cell leukemia is associated with a younger age, hepatosplenomegaly, lymphadenopathy, and fewer lytic bone lesions but poorer prognosis than the secondary form. The peripheral blood of plasma cell leukemic patients has greater than 20% plasma cells with absolute count of 2000/ml or more.

### 5.5.2.21. MONOCLONAL GAMMOPATHY OF UNKNOWN SIGNIFICANCE (MGUS)

MGUS is a relatively common condition characterized by the presence of electrophoretically homogeneous immunoglobulins or benign M-components. The occurrence of this condition appears to increase with age. Most individuals carrying the M-components never develop malignant plasma cell dycrasias, such as multiple myeloma. However, some individuals with this condition have associated malignant conditions. When symptomatic, patients can have enlarged liver or spleen and pieuroneuropathy. *(See,* J. Foerster, Plasma Cell Dycrasias: General Considerations, pp. 2612-2630, In Wintrobe's Clinical Hematology, Tenth Edition, G. Lee et al., eds. Williams & Wilkins, Baltimore, MD (1999)).

MGUS can be differentiated from multiple myeloma by the presence of increased number of monoclonal plasma cells circulating in the peripheral blood. The serological characteristics of M-components are identical to other plasma cell dycrasias conditions, however, the total concentration of M-component is usually less than 30 g/L. The paraprotein is usually IgG; however multiple paraproteins may be present including IgG, IgA, IgM. The relative amount of each of the individual immunoglobulin classes is typically proportional to that found in normal serum. Proteinemia or proteinuria is rare. Serial measurements of M-protein levels in the blood and urine, and continued monitoring of the clinical and laboratory features (including protein electrophoresis) is the most reliable method of differentiating MGUS from early stage plasma cell dycrasias. *In* Wintrobe's Clinical Hematology, Tenth Edition, G. Lee et al., eds. Williams & Wilkins, Baltimore, MD (1999)).

### 5.5.2.22. MATURE B CELL MALIGNANCIES:

The inventors have shown that the inventive anti-CD19 compositions can deplete mature B cells. Thus, as another aspect, the invention can be practiced to treat mature B cell malignancies, including but not limited to follicular lymphoma, mantle-cell lymphomas, Burkitt's lymphoma, multiple myeloma, diffuse large B-cell lymphoma (DLBCL) including, germinal center B cell-like (GCB) DLBCL, activated B cell-like (ABC) DLBCL, and type 3 DLBCL, Hodgkin's lymphoma including classical and nodular lymphocyte pre-dominant type, lymphoplasmacytic lymphoma (LPL), marginal-zone lymphoma including gastric mucosal-associated lymphoid tissue (MALT) lymphoma, and chronic lymphocytic leukemia (CLL) including immunoglobulin-mutated CLL and immunoglobulin-unmutated CLL.

### 5.5.2.23. PRE-B CELL MALIGNANCIES:

Further, CD 19 is expressed earlier in B cell development than, for example, CD20, and is therefore particularly suited for treating pre-B cell and immature B cell malignancies, *e g.,* in the bone marrow. Representative pre-B cell and immature B cell malignancies include but are not limited to mantle cell lymphoma, pre-B cell acute lymphoblastic leukemia, precursor B cell lymphoblastic lymphoma, and other malignancies characterized by CD 19 expression.

### 5.5.3. DETERMINING CD19 DENSITY IN A SAMPLE OR SUBJECT

While not required, assays for CD 19 density can be employed to further characterize the patient's diagnosis. Methods of determining the density of antibody binding to cells are known to those skilled in the art (*See, e.g.,* Sato et al., J. Immunology 165:6635-6643 (2000); which discloses a method of assessing cell surface density of specific CD antigens) Other standard methods include Scatchard analysis. For example, the antibody or fragment can be isolated, radiolabeled, and the specific activity of the radiolabeled antibody determined. The antibody is then contacted with a target cell expressing CD19. The radioactivity associated with the cell can be measured and, based on the specific activity, the amount of antibody or antibody fragment bound to the cell determined.
Alternatively, fluorescence activated cell sorting (FACS) analysis can be employed. Generally, the antibody or antibody fragment is bound to a target cell expressing CD19. A second reagent that binds to the antibody is then added, for example, a flourochrome labeled anti-immunoglobulin antibody. Flourochrome staining can then be measured and used to determine the density of antibody or antibody fragment binding to the cell.

As another suitable method, the antibody or antibody fragment can be directly labeled with a detectable label, such as a fluorophore, and bound to a target cell. The ratio of label to protein is determined and compared with standard beads with known amounts of label bound thereto. Comparison of the amount of label bound to the cell with the known standards can be used to calculate the amount of antibody bound to the cell.

The present invention provides a method for detecting *in vitro* or *in vivo* the presence and/or density of CD 19 in a sample or individual. This can also be useful for monitoring disease and effect of treatment and for determining and adjusting the dose of the antibody to be administered. The *in vivo* method can be performed using imaging techniques such as PET (positron emission tomography) or SPECT (single photon emission computed tomography). Alternatively, one could label the anti-CD 19 antibody with Indium using a covalently attached chelator. The resulting antibody can be imaged using standard gamma cameras the same way as ZEVALIN™ (Indium labeled anti-CD20 mAb) (Biogen Idec) is used to image CD20 antigen.

The *in vivo* method can be performed by contacting a sample to be tested, optionally along with a control sample, with a human anti-CD19 antibody of the invention under conditions that allow for formation of a complex between an antibody of the invention and the human CD 19 antigen. Complex formation is then detected (*e.g*., using an FACS analysis or Western blotting). When using a control sample along with the test sample, a complex is detected in both samples and any statistically significant difference in the formation of complexes between the samples is indicative of the presence of human CD 19 in the test sample.

Mean florescence intensity can be used as a measure of CD 19 density. B cells may be removed from a patient and stained with CD 19 antibodies that have been labeled with a florescent label and the fluorescence intensity is measured using flow cytometry. Fluorescence intensities can be measured and expressed as an average of intensity per B cell. Using such methods, mean florescence intensities that are representative of CDC 19 density can be compared for a patient before and after treatment using the methods and compositions of the invention, or between patients and normal levels of hCD 19 on B cells.

In patients where the density of CD 19 expression on B cells has been determined, the density of CD 19 may influence the determination and/or adjustment of the dosage and/or treatment regimen used with the anti-CD 19 antibody of the compositions and antibodies for use in the methods of the invention. For example, where density of CD19 is high, it may be possible to use anti-CD19 antibodies that less efficiently mediate ADCC in humans. Where the patient treated using the compositions and antibodies for use in the methods of the invention has a low CD 19 density, a higher dosage of the anti-CD 19 antibody of the compositions and for use in the methods of the invention may be used. Where the patient treated using the compositions and antibodies for use in the methods of the invention has a low CD 19 density, a low dosage of the anti-CD 19 antibody of the compositions and for use in the methods of the invention may be used. Where the patient treated using the compositions and antibodies for use in the methods of the invention has a high CD 19 density, a lower dosage of the anti-CD 19 antibody of the compositions and for use in the methods of the invention may be used. CD 19 density can be compared to CD20 density in a patient, CD 19 density can be compared to an average CD 19 density for humans or for a particular patient population, or CD19 density can be compared to CD 19 levels in the patietn prior to therapy or prior to onset of a B cell disease or disorder. The patient treated using the compositions and antibodies for use in the methods of the invention may have a B cell malignancy where CD 19 is present on the surface of B cells.

### 5.6. IMMUNOTHERAPEUTIC PROTOCOLS

The anti-CD19 antibody compositions used in the therapeutic regimen/protocols, referred to herein as "anti-CD 19 immunotherapy" can be naked antibodies, immunoconjugates and/or fusion proteins. The compositions of the invention can be used as a single agent therapy or in combination with other therapeutic agents or regimens. The anti-CD19 antibodies or immunoconjugates can be administered prior to, concurrently with, or following the administration of one or more therapeutic agents. Therapeutic agents that can be used in combination therapeutic regimens with the compositions of the invention include any substance that inhibits or prevents the function of cells and/or causes destruction of cells. Examples, include, but are not limited to, radioactive isotopes, chemotherapeutic agents, and toxins such as enzymatically active toxins of bacterial, fungal, plant or animal origin, or fragments thereof.

The therapeutic regimens described herein, or any desired treatment regimen can be tested for efficacy using a transgenic animal model such as the mouse model described below in Section 6.2, which expresses human CD 19 antigen addition to or in place of native CD19 antigen. Thus, an anti-CD19 antibody treatment regimen can be tested in an animal model to determine efficacy before administration to a human.

The anti-CD19 antibodies of the compositions and for use in the methods of the invention can be used to treat B cell diseases, including B cell malignancies. The term "B cell malignancy" includes any malignancy that is derived from a cell of the B cell lineage. Exemplary B cell malignancies include, but are not limited to: B cell subtype non-Hodgkin's lymphoma (NHL) including low grade/follicular, NHL, small lymphocytic (SL) NHL, intermediate grade/follicular NHL, intermediate grade diffuse NHL, high grade immunoblastic NHL, high grade lymphoblastic NHL, high grade small non-cleaved cell NHL; mantle-cell lymphoma, and bulky disease NHL; Burkitt's lymphoma; multiple myeloma; pre-B acute lymphoblastic leukemia and other malignancies that derive from early B cell precursors; common acute lymphocytic leukemia (ALL); chronic lymphocytic leukemia (CLL) including including immunoglobulin-mutated CLL and immunoglobulin-unmutated CLL; hairy cell leukemia; Null-acute lymphoblastic leukemia; Waldenstrom's Macroglobulinemia; diffuse large B cell lymphoma (DLBCL) including germinal center B cell-like (GCB) DLBCL, activated B cell-like (ABC) DLBCL, and type 3 DLBCL; prolymphocytic leukemia; light chain disease; plasmacytoma; osteosclerotic myeloma; plasma cell leukemia; monoclonal gammopathy of undetermined significance (MGUS); smoldering multiple myeloma (SMM); indolent multiple myeloma (IMM); Hodgkin's lymphoma including classical and nodular lymphocyte pre-dominant type; lymphoplasmacytic lymphoma (LPL); and marginal-zone lymphoma including gastric mucosal-associated lymphoid tissue (MALT) lymphoma.

The inventors have shown that the inventive antibodies and compositions can deplete mature B cells. Thus, as another aspect, the invention can be employed to treat mature B cell malignancies (i.e., express Ig on the cell surface) including but not limited to follicular lymphoma, mantle-cell lymphoma, Burkitt's lymphoma, multiple myeloma, diffuse large B-cell lymphoma (DLBCL) including germinal center B cell-like (GCB), DLBCL, activated B cell-like (ABC) DLBCL, and type 3 DLBCL, Hodgkin's lymphoma including classical and nodular lymphocyte pre-dominant type, lymphoplasmacytic lymphoma (LPL), marginal-zone lymphoma including gastric mucosal-associated lymphoid tissue (MALT) lymphoma, and chronic lymphocytic leukemia (CLL) including immunoglobulin-mutated CLL and immunoglobulin-unmutated CLL.

Further, CD 19 is expressed earlier in B cell development than, for example, CD20, and is therefore particularly suited for treating pre-B cell and immature B cell malignancies (*i.e.,* do not express Ig on the cell surface), for example, in the bone marrow. Illustrative pre-B cell and immature B cell malignancies, include but are not limited to acute lymphoblastic leukemia

The invention may be practiced to treat extranodal tumors.

### 5.6.1. ANTI-CD19 IMMUNOTHERAPY

In accordance with the present invention "anti-CD 19 immunotherapy" encompasses the administration of any of the anti-CD 19 antibodies of the invention in accordance with any of the therapeutic regimens described herein. The anti-CD 19 antibodies can be administered as naked antibodies, or immunoconjugates or fusion proteins.

Anti-CD 19 immunotherapy encompasses the administration of the anti-CD 19 antibody as a single agent therapeutic for the treatment of a B cell malignancy. Anti-CD 19 immunotherapy encompasses methods of treating an early stage disease resulting from a B cell malignancy. Anti-CD19 immunotherapy encompasses methods of treating a B cell malignancy wherein the anti-CD19 antibody mediates ADCC. Anti-CD19 immunotherapy encompasses methods of treating a B cell malignancy wherein the anti-CD 19 antibody is administered before the patient has received any treatment for the malignancy, whether that therapy is chemotherapy, radio chemical based therapy or surgical therapy.

In a preferred embodiment, a human subject having a B cell malignancy can be treated by administering a humanized antibody that preferably mediates human ADCC. In cases of early stage disease, or single agent therapies, any anti-CD 19 antibody that preferably mediates ADCC can be used in the human subjects (including murine and chimeric antibodies); however, human and humanized antibodies are preferred.

Antibodies of the IgG1 or IgG3 human isotypes are preferred for therapy. However, the IgG2 or IgG4 human isotypes can be used, provided they mediate human ADCC. Such effector function can be assessed by measuring the ability of the antibody in question to mediate target cell lysis by effector cells *in vitro* or *in vivo*.

The dose of antibody used should be sufficient to deplete circulating B cells. Progress of the therapy can be monitored in the patient by analyzing blood samples. Other signs of clinical improvement can be used to monitor therapy.

Methods for measuring depletion of B cell that can be used in connection with the compositions and antibodies for use in the methods of the invention are well known in the art and include, but are not limited to the following. Circulating B cells depletion can be measured with flow cytometry using a reagent other than an anti-CD19 antibody that binds to B cells to define the amount of B cells. Antibody levels in the blood can be monitored using standard serum analysis. B cell depletion may be indirectly measured by defining the amount to an antibody known to be produced by B cells. The level of that antibody is then monitored to determine the depletion and/or functional depletion of B cells. B cell depletion can be measured by immunochemical staining to identify B cells. B cells extracted from patient tissues can be placed on microscope slides, labeled and examined for presence or absence. A comparison is made between B cells extracted prior to therapy and after to determine differences in the presence of B cells.

Tumor burden can be measured and used in connection with the compositions and antibodies for use in the methods of the invention. Methods for measuring tumor burden are well known in the art and include, but are not limited to the following. PET scans can be used to measure metabolic activity and identify areas of higher activity which are indicative of tumors. CT scans and MRI can also be used to examine soft tissue for the presence and size of tumors. Bone scans can be used to measure tumor volume and location. Tumor burden can be measured by examining the blood flow into and out of a tumor using doppler technology (e.g., ultrasound). Changes in blood flow over time or deviations from normal blood flow in the appropriate tissue of a patient can be used to calculate an estimate to tumor burden. Such methods for measuring tumor burden can be used prior to and following the methods of treatment of the invention.

As described herein, B cells are depleted and/or tumor burden is decreased while ADCC function is maintained.

Where the anti-CD19 antibody is administered as a single agent therapy, the invention contemplates use of different treatment regimens.

According to certain aspects of the invention, the anti-CD 19 antibody used in the compositions and for use in the methods of the invention, is a naked antibody. The dose of naked anti-CD19 antibody used may be at least about 0.1, 0.2, 0.3,0.4, 0.5, 0.6, 0,7, 0.8, 0.9, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5 mg/kg of body weight of a patient. The dose of naked anti-CD 19 antibody used may be at least about 1 to 10, 5 to 15, 10 to 20, or 15 to 25 mg/kg of body weight of a patient. The dose of naked anti-CD19 antibody used may be at least about 1 to 20, 3 to 15, or 5 to 10 mg/kg of body weight of a patient. The dose of naked anti-CD19 antibody used may be at least about 5, 6, 7, 8, 9, or 10 mg/kg of body weight of a patient.

The dose may comprise about 375 mg/m² of anti-CD19 antibody administered weekly for 4 to 8 consecutive weeks. The dose may be at least about 1, 2, 3, 4, 5, 6, 7, 8, 9, 10, 11, 12, 13, 14, or 15 mg/kg of body weight of the patient administered weekly for 4 to 8 consecutive weeks.

The exemplary doses of anti-CD19 antibody described above can be administered as described in Section 5.4.3. The above doses may be single dose injections. The doses may be administered over a period of time. The doses may be administered multiple times over a period of time. The period of time may be measured in days, months or weeks. Multiple doses of the anti-CD 19 antibody can be administered at intervals suitable to achieve a therapeutic benefit while balancing toxic side effects. For example, where multiple doses are used, it is preferred to time the intervals to allow for recovery of the patient's monocyte count prior to the repeat treatment with antibody. This dosing regimen will optimize the efficiency of treatment, since the monocyte population reflects ADCC function in the patient.

The compositions of the invention may be administered to a human patient as long as the patient is responsive to therapy. The compositions of the invention may be administered to a human patient as long as the patient's disease does not progress. The compositions of the invention may be administered to a human patient until a patient's disease does not progress or has not progressed for a period of time, then the patient is not administered the compositions of the invention unless the disease reoccurs or begins to progress again. For example, a patient can be treated with any of the above doses for about 4 to 8 weeks, during which time the patient is monitored for disease progression. If disease progression stops or reverses, then the patient will not be administered the compositions of the invention until that patient relapses, i.e., the disease being treated reoccurs or progresses. Upon this reoccurrence or progression, the patient can be treated again with the same dosing regimen initially used or using other doses described above.

The compositions of the invention can be administered as a loading dose followed by multiple lower doses (maintenance doses) over a period of time. The doses may be timed and the amount adjusted to maintain effective B cell depletion. The loading dose may be about 10, 11, 12, 13, 14, 15, 16, 17, or 18 mg/kg of patient body weight and the maintenance dose is at least about 5 to 10 mg/kg of patient body weight. The maintenance dose may be administered at intervals of every 7, 10, 14 or 21 days. The maintenance doses can be continued indefinitely, until toxicity is present, until platelet count decreases, until there is no disease progression, until the patient generates an immune response to the drug, or until disease progresses to a terminal state. The compositions of the invention may be administered to a human patient until the disease progresses to a terminal stage.

Where circulating monocyte levels of a patient are monitored as part of a treatment regimen, doses of anti-CD19 antibody administered may be spaced to allow for recovery of monocyte count. For example, a composition of the may be administered at intervals of every 8, 9, 10, 11, 12, 13, 14, 15, 16, 17, 18, 19, 20, 21, 22, 23, 24, 25, 26, 27, 28, 29, or 30 days.

Where an anti-CD19 antibody is conjugated to or administered in conjunction with a toxin, one skilled in the art will appreciate that the dose of anti-CD 19 antibody can be adjusted based on the toxin dose and that the toxin dose will depend on the specific type of toxin being used. Typically, where a toxin is used, the dose of anti-CD19 antibody will be less than the dose used with a naked anti-CD19 antibody. The appropriate dose can be determined for a particular toxin using techniques well known in the art. For example, a dose ranging study can be conducted to determine the maximum tolerated dose of anti-CD 19 antibody when administered with or conjugated to a toxin.

Where an anti-CD19 antibody is conjugated to or administered in conjunction with a radiotherapeutic agent, the dose of the anti-CD 19 antibody will vary depending on the radiotherapeutic used. Atwo step process may be used. First, the human patient is administered a composition comprising a naked anti-CD 19 antibody and about 6, 7, 8, 9, or 10 days later a small amount of the radiotherapeutic is administered. Second, once the tolerance, distribution, and clearance of the low dose therapy has been determined, the patient is administered a dose of the naked anti-CD19 antibody followed by a therapeutic amount of the radiotherapeutic is administered. Such treatment regimens are similar to those approved for treatment of Non-Hodgkin's lymphoma using ZEVALIN™ (Indium labeled anti-CD20 mAb) (Biogen Idec) or BEXXAR™ (GSK, Coulter Pharmaceutical).

### 5.6.2. COMBINATION WITH CHEMOTHERAPEUTIC AGENTS

Anti-CD 19 immunotherapy (using naked antibody, immunoconjugates, or fusion proteins) can be used in conjunction with other therapies including but not limited to, chemotherapy, radioimmunotherapy (RIT), chemotherapy and external beam radiation (combined modality therapy, CMT), or combined modality radioimmunotherapy (CMRIT) alone or in combination, etc. The anti-CD 19 antibody therapy of the present invention can be administered in conjunction with CHOP (Cyclophosphamide-Hydroxydoxorubicin-Oncovin (viricristine)-Prednisolone), the most common chemotherapy regimen for treating non-Hodgkin's lymphoma. As used herein, the term "administered in conjunction with" means that the anti-CD 19 immunotherapy can be administered before, during, or subsequent to the other therapy employed.

The anti-CD 19 immunotherapy may be in conjunction with a cytotoxic radionuclide or radiotherapeutic isotope. For example, an alpha-emitting isotope such as ²²⁵Ac, ²²⁴Ac, ²¹¹At, ²¹²Bi, ²¹³Bi, ²¹²Pb, ²²⁴Ra, or ²²³Ra. Alternatively, the cytotoxic radionuclide may a beta-emitting isotope such as ¹⁸⁶Re, ¹⁸⁸Re, ⁹⁰Y, ¹³¹I, ⁶⁷Cu, ¹⁷⁷Lu, ¹⁵³Sm, ¹⁶⁶Ho, or ⁶⁴Cu. Further, the cytotoxic radionuclide may emit Auger and low energy electrons and include the isotopes ¹²⁵I, ¹²³I or ⁷⁷Br. The isotope may be ¹⁹⁸Au, ³²P, and the like. The amount of the radionuclide administered to the subject may be between about 0.001 mCi/kg and about 10 mCi/kg.

The amount of the radionuclide administered to the subject may be between about 0.1 mCi/kg and about 1.0 mCi/kg. The amount of the radionuclide administered to the subject may be between about 0.005 mCi/kg and 0.1 mCi/kg.

The anti-CD 19 immunotherapy may be in conjunction with a chemical toxin or chemotherapeutic agent. Preferably the chemical toxin or chemotherapeutic agent is selected from the group consisting of an enediyne such as calicheamicin and esperamicin; duocarmycin, methotrexate, doxorubicin, melphalan, chlorambucil, ARA-C, vindesine, mitomycin C, cis-platinum, etoposide; bleomycin and 5-fluorouracil.

Suitable chemical toxins or chemotherapeutic agents that can be used in combination therapies with the anti-CD 19 immunotherapy include members of the enediyne family of molecules, such as calicheamicin and esperamicin. Chemical toxins can also be taken from the group consisting of duocarmycin (*see*, *e.g.,* U.S. Pat. No. 5,703,080 and U.S. Pat. No. 4,923,990), methotrexate, doxorubicin, melphalan, chlorambucil, ARA-C, vindesine, mitomycin C, cis-platinum, etoposide, bleomycin and 5-fluorouracil. Examples of chemotherapeutic agents also include Adriamycin, Doxorubicin, 5-Fluorouracil, Cytosine arabinoside ("Ara-C"), Cyclophosphamide, Thiotepa, Taxotere (docetaxel), Busulfan, Cytoxin, Taxol, Methotrexate, Cisplatin, Melphalan, Vinblastine, Bleomycin, Etoposide, Ifosfamide, Mitomycin C, Mitoxantrone, Vincreistine, Vinorelbine, Carboplatin, Teniposide, Daunomycin, Carminomycin, Aminopterin, Dactinomycin, Mitorinycins, Esperamicins (*see,* U.S. Pat. No. 4,675,187), Melphalan and other related nitrogen mustards.

"CVB" (1.5 g/m² cyclophosphamide, 200-400 mg/m² etoposide, and 150-200 mg/m² carmustine) can be used in the combination therapies of the invention. CVB is a regimen used to treat non-Hodgkin's lymphoma. Patti et al., Eur. J. Haematol. 51:18 (1993). Other suitable combination chemotherapeutic regimens are well-known to those of skill in the art. *See,* for example, Freedman et al., "Non-Hodgkin's Lymphomas," in CANCER MEDICINE, VOLUME 2, 3rd Edition, Holland et al. (eds.), pp. 2028-2068 (Lea & Febiger 1993). As an illustration, first generation chemotherapeutic regimens for treatment of intermediate-grade non-Hodgkin's lymphoma include C-MOPP (cyclophosphamide, vincristine, procarbazine and prednisone) and CHOP (cyclophosphamide, doxorubicin, vincristine, and prednisone). A useful second generation chemotherapeutic regimen is m-BACOD (methotrexate, bleomycin, doxorubicin, cyclophosphamide, vincristine, dexamethasone and leucovorin), while a suitable third generation regimen is MACOP-B (methotrexate, doxorubicin, cyclophosphamide, vincristine, prednisone, bleomycin and leucovorin). Additional useful drugs include phenyl butyrate and brostatin-1. In a preferred multimodal therapy, both chemotherapeutic drugs and cytokines are co-administered with an antibody, immunoconjugate or fusion protein according to the present invention. The cytokines, chemotherapeutic drugs and antibody, immunoconjugate or fusion protein can be administered in any order, or together.

Other toxins that are preferred for use in the compositions and with antibodies for use in the methods of the invention include poisonous lectins, plant toxins such as ricin, abrin, modeccin, botulina and diphtheria toxins. Of course, combinations of the various toxins could also be coupled to one antibody molecule thereby accommodating variable cytotoxicity. Illustrative of toxins which are suitably employed in the combination therapies of the invention are ricin, abrin, ribonuclease, DNase I, Staphylococcal enterotoxin-A, pokeweed antiviral protein, gelonin, diphtherin toxin, Pseudomonas exotoxin, and Pseudomonas endotoxin. *See,* for example, Pastan et al., Cell 47:641 (1986), and Goldenberg et al., Cancer Journal for Clinicians 44:43 (1994). Enzymatically active toxins and fragments thereof which can be used include diphtheria A chain, nonbinding active-fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. *See,* for example, WO 93/21232 published October 28, 1993.

Suitable toxins and chemotherapeutic agents are described in REMINGTON'S PHARMACEUTICAL SCIENCES, 19th Ed. (Mack Publishing Co. 1995), and in GOODMAN AND GILMAN'S THE PHARMACOLOGICAL BASIS OF THERAPEUTICS, 7th Ed. (MacMillan Publishing Co. 1985). Other suitable toxins and/or chemotherapeutic agents are known to those of skill in the art.

The anti-CD19 immunotherapy of the present invention may also be in conjunction with a prodrug-activating enzyme which converts a prodrug (*e.g*., a peptidyl chemotherapeutic agent, *see,* WO81/01145) to an active anti-cancer drug. *See,* for example, WO 88/07378 and U.S. Patent No. 4,975,278. The enzyme component of such combinations includes any enzyme capable of acting on a prodrug in such a way so as to covert it into its more active, cytotoxic form. The term "prodrug" as used in this application refers to a precursor or derivative form of a pharmaceutically active substance that is less cytotoxic to tumor cells compared to the parent drug and is capable of being enzymatically activated or converted into the more active parent form. *See, e.g.,* Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al. (ed.), pp. 247-267, Humana Press (1985). Prodrugs that can be used in combination with the anti-CD 19 antibodies of the invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, α-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

Administration of the compositions and antibodies for use in the methods of the invention may enable the postponement of toxic therapy and may help avoid unnecessary side effects and the risks of complications associated with chemotherapy and delay development of resistance to chemotherapy. Toxic therapies and/or resistance to toxic therapies may be delayed in patients administered the compositions and antibodies for use in the methods of the invention for up to about 6 months, 1, 2, 3, 4, 5, 6, 7, 8, 9, or 10 years.

### 5.6.3. COMBINATION WITH THERAPEUTIC ANTIBODIES

The anti-CD19 immunotherapy described herein may be administered in combination with other antibodies, including, but not limited to, anti-CD20 mAb, anti-CD52 mAb, anti-CD22 antibody (as described, for example, in U.S. Patent No. 5,484,892, U.S. patent publication number 2004/0001828 of U.S. application serial number 10/371,797, U.S. patent publication number 2003/0202975 of U.S. application serial number 10/372,481 and U.S. provisional application serial number 60/420,472), and anti-CD20 antibodies, such as RITUXAN™ (C2B8; RITUXIMAB™; Biogen Idec). Other examples of therapeutic antibodies that can be used in combination with the antibodies of the invention or used in the compositions of the invention include, but are not limited to, HERCEPTIN™ (Trastuzumab; Genentech), MYLOTARG™ (Gemtuzumab ozogamicin; Wyeth Pharmaceuticals), CAMPATH™ (Alemtuzumab; Berlex), ZEVALIN™ (Ipritumomab tiuxetan; Biogen Idec), BEXXAR™ (Tositumomab; GlaxoSmithKline Corixa), ERBITUX™ (Cetuximab; Imclone), and AVASTIN™ (Bevacizumab; Genentech).

The anti-CD 19 and anti-CD20 and/or anti-CD22 mAb can be administered, optionally in the same pharmaceutical composition, in any suitable ratio. To illustrate, the ratio of the anti-CD19 and anti-CD20 antibody can be a ratio of about 1000:1, 500:1, 250:1, 100:1, 90:1, 80:1, 70:1, 60;1, 50:1, 40:1, 30:1. 20:1, 19:1, 18:1, 17:1, ,16:1, 15:1, 14:1, 13:1, 12:1, 11:1, 10:1, 9:1, 8:1, 7:1, 6:1, 5:1, 4:1, 3:i, 2:1, 1:1, 1:2, 1:3,1:4, 1:5, 1:6, 1:7, 1:8, 1:9, 1:10, 1:11, 1:12, 1:13, 1:14, 1:15, 1:16, 1:17, 1:18, 1:19, 1:20, 1:30, 1:40, 1:50, 1:60, 1:70, 1:80, 1:90. 1:100, 1:250, 1:500 or 1:1000 or more. Likewise, the ratio of the anti-CD19 and anti-CD22 antibody can be a ratio of about 1000:1, 500:1, 250:1,100:1,90:1, 80:1, 70:1, 60;1, 50:1, 40:1, 30:1. 20:1, 19:1, 18:1, 17:1, 16:1, 15:1, 14:1, 13:1, 12:1, 11:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:30, 1:40, 1:50, 1:60, 1:70, 1:80, 1:90. 1:100, 1:250,1:500 or 1:1000 or more.

### 5.6.4. COMBINATION COMPOUNDS THAT ENHANCE MONOCYTE OR MACROPHAGE FUNCTION

In certain embodiments of the methods of the invention, a compound that enhances monocyte or macrophage function (*e.g.,* at least about 25%, 50%, 75%, 85%, 90%, 95% or more) can be used in conjunction with the anti-CD19 immunotherapy. Such compounds are known in the art and include, without limitation, cytokines such as interleukins (*e.g.,* IL-12), and interferons (*e.g.,* alpha or gamma interferon).

The compound that enhances monocyte or macrophage function or enhancement can be formulated in the same pharmaceutical composition as the antibody, immunoconjugate or antigen-binding fragment. When administered separately, the antibody/fragment and the compound can be administered concurrently (within a period of hours of each other), can be administered during the same course of therapy, or can be administered sequentially (*i.e.,* the patient first receives a course of the antibody/fragment treatment and then a course of the compound that enhances macrophage/monocyte function or vice versa). The compound that enhances monocyte or macrophage function is administered to the human subject prior to, concurrently with, or following treatment with other therapeutic regimens and/or the compositions of the invention. The human subject may have a blood leukocyte, monocyte, neutrophil, lymphocyte, and/or basophil count that is within the normal range for humans. Normal range for human blood leukocytes (total) is about 3.5- about 10.5 (10⁹/L). Normal range for human blood neutrophils is about 1.7- about 7.0 (10⁹/L), monocytes is about 0.3-about 0.9 (10⁹/L), lymphocytes is about 0.9- about 2.9 (10⁹/L), basophils is about 0- about 0.3 (10⁹/L), and eosinophils is about 0.05- about 0.5 (10⁹/L). The human subject may have a blood leukocyte count that is less than the normal range for humans, for example at least about 0.01, 0.05, 0.1, 0.2, 0.3, 0.4, 0.5, 0.6, 0.7, or 0.8 (10⁹/L) leukocytes.

This embodiment of the invention can be practiced with the antibodies, immunoconjugates or antibody fragments of the invention or with other antibodies known in the art and is particularly suitable for subjects that are resistant to anti-CD19, anti-CD20 and/or anti-CD22 antibody therapy (for example, therapy with existing antibodies such as C2B8), subjects that are currently being or have previously been treated with chemotherapy, subjects that have had a relapse in a B cell disorder, subjects that are immunocompromised, or subjects that otherwise have an impairment in macrophage or monocyte function. The prevalence of patients that are resistant to therapy or have a relapse in a B cell disorder may be attributable, at least in part, to an impairment in macrophage or monocyte function. Thus, the invention provides methods of enhancing ADCC and/or macrophage and/or monocyte function to be used in conjunction with the methods of administering anti-CD19 antibodies.

### 5.6.5. COMBINATION WITH IMMUNOREGULATORY AGENTS

The anti-CD 19 immunotherapy of the present invention may also be used in conjunction with an immunoregulatory agent. In this approach, the use of chimerized antibodies is preferred; the use of humanized anti-CD 19 antibody is most preferred. The term "immunoregulatory agent" as used herein for combination therapy refers to substances that act to suppress, mask, or enhance the immune system of the host. This would include substances that suppress cytokine production, downregulate or suppress self-antigen expression, or mask the MHC antigens. Examples of such agents include 2-amino-6-aryl-5-substituted pyrimidines (*see,* U.S. Pat. No. 4,665,077), azathioprine (or cyclophosphamide, if there is an adverse reaction to azathioprine); bromocryptine; glutaraldehyde (which masks the MHC antigens, as described in U.S. Pat. No. 4,120,649); anti-idiotypic antibodies for MHC antigens and MHC fragments; cyclosporin A; steroids such as glucocorticosteroids, e:g., prednisone, methylprednisolone, and dexamethasone; cytokine or cytokine receptor antagonists including anti-interferon-y, -β, or -α antibodies; anti-tumor necrosis factor-α antibodies; anti-tumor necrosis factor-β antibodies; anti-interleukin-2 antibodies and anti-IL-2 receptor antibodies; anti-L3T4 antibodies; heterologous anti-lymphocyte globulin; panT antibodies, preferably anti-CD3 or anti-CD4/CD4a antibodies; soluble peptide containing a LFA-3 binding domain (WO 90/08187 published Jul. 26, 1990); streptokinase; TGF-β; streptodornase; RNA or DNA from the host; FK506; RS-61443; deoxyspergualin; rapamycin; T-cell receptor (U.S. Pat. No. 5,114,721); T-cell receptor fragments (Offner et al., Science 251:430-432 (1991); WO 90/11294; and WO 91/01133); and T-cell receptor antibodies (EP 340,109) such as T10B9. Examples of cytokines include, but are not limited to lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor -α; mullerian-inhibiting substance; mouse gonadotropin-associated peptide; inhibin; activin; vascular endothelial growth factor; integrin; thrombopoiotin (TPO); nerve growth factors such as NGF-α; platelet-growth factor; transforming growth factors (TGFs) such as TGF-α and TGF- α; insulin-like growth factor-I and -II; erythropoietin (EPO); osteoinductive factors; interferons; colony stimulating factors (CSFs) such as macrophage-CSF (M-CSF); granulocyte-macrophage-CgP (GM-CSP); and granulocyte-CSF (G-CSF); interleukins (ILs) such as IL-1, IL-la, IL-2, IL-3, IL-4, IL-5, IL-6, IL-7, IL-8, IL-9, IL-11, IL-12; IL-15; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines. As described herein, the methods further include administering to the subject one or more immunomodulatory agents, preferably a cytokine. Preferred cytokines are selected from the group consisting of interleukin-1 (IL-1), IL-2, IL-3, IL-12, IL-15, IL-18, G-CSF, GM-CSF, thrombopoietin, and y interferon.

These immunoregulatory agents may be administered at the same time or at separate times from the anti-CD 19 antibodies of the invention, and are used at the same or lesser dosages than as set forth in the art. The preferred immunoregulatory agent will depend on many factors, including the type of disorder being treated, as well as the patient's history, but a general overall preference is that the agent be selected from cyclosporin A, a glucocorticosteroid (most preferably prednisone or methylprednisolone), OKT-3 monoclonal antibody, azathioprine, bromocryptine, heterologous anti-lymphocyte globulin, or.a mixture thereof.

### 5.6.6. COMBINATION WITH OTHER THERAPEUTIC AGENTS

Agents that act on the tumor neovasculature can also be used in conjunction with anti-CD 19 immunotherapy and include tubulin-binding agents such as combrestatin A4 (Griggs et al., Lancet Oncol. 2:82, (2001)) and angiostatin and endostatin (reviewed in Rosen, Oncologist 5:20, 2000, incorporated by reference herein). Immunomodulators suitable for use in combination with anti-CD19 antibodies include, but are not limited to, α-interferon, γ-interferon, and tumor necrosis factor alpha (TNFα). The therapeutic agents used in combination therapies using the compositions and antibodies for use in the methods of the invention are peptides.

The anti-CD19 immunotherapy may be in conjunction with one or more calicheamicin molecules. The calicheamicin family of antibiotics are capable of producing double-stranded DNA breaks at sub-picomolar concentrations. Structural analogues of calicheamicin which may be used include, but are not limited to, γ11, γ21, γ31, N-acetyl-γ11, PSAG and 011 (Hinman et al., Cancer Research 53:3336-3342 (1993) and Lode et al., Cancer Research 58: 2925-2928 (1998)).

Alternatively, a fusion protein comprising an anti-CD19 antibody of the invention and a cytotoxic agent may be made, *e.g.,* by recombinant techniques or peptide synthesis.

An anti-CD 19 antibody of the invention may be conjugated to a "receptor" (such as streptavidin) for utilization in tumor pretargeting wherein the antagonist-receptor conjugate is administered to the patient, followed by removal of unbound conjugate from the circulation using a clearing agent and then administration of a "ligand" (*e.g.,* biotin) which is conjugated to a therapeutic agent (*e.g.,* a radionucleotide).

A treatment regimen may include compounds that mitigate the cytotoxic effects of the anti-CD 19 antibody compositions of the invention. Such compounds include analgesics (*e.g.,* acetaminophen), bisphosphonates, antihistamines (*e.g.,* chlorpheniramine maleate), and steroids (*e.g*., dexamethasone, retinoids, deltoids, betamethasone, cortisol, cortisone, prednisone, dehydrotestosterone, glucocorticoids, mineralocorticoids, estrogen, testosterone, progestins).

The therapeutic agent used in combination with the anti-CD19 immunotherapy of the invention may be a small molecule (*i.e.,* inorganic or organic compounds having a molecular weight of less than about 2500 daltons). For example, libraries of small molecules may be commercially obtained from Specs and BioSpecs B.V. (Rijswijk, The Netherlands), Chembridge Corporation (San Diego, CA), Comgenex USA Inc. (Princeton, NJ), and Maybridge Chemicals Ltd. (Cornwall PL34 OHW, United Kingdom).

The anti-CD 19 immunotherapy can be administered in combination with an anti-bacterial agent. Non-limiting examples of anti-bacterial agents include proteins, polypeptides, peptides, fusion proteins, antibodies, nucleic acid molecules, organic molecules, inorganic molecules, and small molecules that inhibit and/or reduce a bacterial infection, inhibit and/or reduce the replication of bacteria, or inhibit and/or reduce the spread of bacteria to other cells or subjects. Specific examples of anti-bacterial agents include, but are not limited to, antibiotics such as penicillin, cephalosporin, imipenem, axtreonam, vancomycin, cycloserine, bacitracin, chloramphenicol, erythromycin, clindamycin, tetracycline, streptomycin, tobramycin, gentamicin, amikacin, kanamycin, neomycin, spectinomycin, trimethoprim, norfloxacin, rifampin, polymyxin, amphotericin B, nystatin, ketocanazole, isoniazid, metronidazole, and pentamidine.

The anti-CD 19 immunotherapy of the invention can be administered in combination with an anti-fungal agent. Specific examples of anti-fungal agents include, but are not limited to, azole drugs (*e.g.,* miconazole, ketoconazole (NIZORAL^{®}), caspofungin acetate (CANCIDAS^{®}), imidazole, triazoles (*e.g.,* fluconazole (DIFLUCAN^{®}), and itraconazole (SPORANOX^{®})), polyene (*e.g.,* nystatin, amphotericin B (FUNGIZONE^{®}), amphotericin B lipid complex ("ABLC")(ABELCET^{®}), amphotericin B colloidal dispersion ("ABCD")(AMPHOTEC^{®}), liposomal amphotericin B (AMBISONE^{®})), potassium iodide (KI), pyrimidine (*e.g.,* flucytosine (ANCOBON^{®})), and voriconazole (VFEND^{®}). Administration of anti-bacterial and anti-fungal agents can mitigate the effects or escalation of infectious disease that may occur in the methods of the invention where a patient's B cells are significantly depleted.

The anti-CD19 immunotherapy of the invention can be administered in combination with one or more of the agents described above to mitigate the toxic side effects that may accompany administration of the compositions of the invention. The anti-CD 19 immunotherapy of the invention can be administered in combination with one or more agents that are well known in the art for use in mitigating the side effects of antibody administration, chemotherapy, toxins, or drugs.

As described herein, where the anti-CD 19 immunotherapy of the invention is administered to treat multiple myeloma, the compositions of the invention may be administered .in combination with or in treatment regimens with high-dose chemotherapy (melphalan, melphalan/prednisone (MP), vincristine/doxorubicin/dexamethasone (VAD), liposomal doxorubicin/vincristine, dexamethasone (DVd), cyclophosphamide, etoposide/dexamethasone/cytarabine, cisplatin (EDAP)), stem cell transplants (e.g., autologous stem cell transplantation or allogeneic stem cell transplantation, and/or mini-allogeneic (non-myeloablative) stem cell transplantation), radiation therapy, steroids (*e.g.,* corticosteroids, dexamethasone, thalidomide/dexamethasone, prednisone, melphalan/prednisone), supportive therapy (*e.g.,* bisphosphonates, growth factors, antibiotics, intravenous immunoglobulin, low-dose radiotherapy, and/or orthopedic interventions), THALOMID™ (thalidomide, Celgene), and/or VELCADE™ (bortezomib, Millennium).

As described herein where the anti-CD 19 immunotherapy of the invention are administered in combination with another antibody or antibodies and/or agent, the additional antibody or antibodies and/or agents can be administered in any sequence relative to the administration of the antibody of this invention. For example, the additional antibody or antibodies can be administered before, concurrently with, and/or subsequent to administration of the anti-CD 19 antibody or immunoconjugate of the invention to the human subject. The additional antibody or antibodies can be present in the same pharmaceutical composition as the antibody of the invention, and/or present in a different pharmaceutical composition. The dose and mode of administration of the antibody of this invention and the dose of the additional antibody or antibodies can be the same or different, in accordance with any of the teachings of dosage amounts and modes of administration as provided in this application and as are well known in the art.

### 5.7. USE OF ANTI-CD19 ANTIBODIES IN DIAGNOSING B CELL MALIGNANCIES

The present invention also encompasses anti-CD19 antibodies, and compositions thereof, that immunospecifically bind to the human CD 19 antigen, which anti-CD 19 antibodies are conjugated to a diagnostic or detectable agent. In preferred embodiments, the antibodies are humanized anti-CD 19 antibodies. Such anti-CD 19 antibodies can be useful for monitoring or prognosing the development or progression of a B cell malignancy as part of a clinical testing procedure, such as determining the efficacy of a particular therapy. Such diagnosis and detection can be accomplished by coupling an anti-CD19 antibody that immunospecifically binds to the human CD 19 antigen to a detectable substance including, but not limited to, various enzymes, such as but not limited to, horseradish peroxidase, alkaline phosphatase, beta-galactosidase, or acetylcholinesterase; prosthetic groups, such as but not limited to, streptavidin/biotin and avidin/biotin; fluorescent materials, such as but not limited to, umbelliferone, fluorescein, fluorescein isothiocynate, rhodamine, dichlorotriazinylamine fluorescein, dansyl chloride or phycoerythrin; luminescent materials, such as but not limited to, luminol; bioluminescent materials, such as but not limited to, luciferase, luciferin, and aequorin; radioactive materials, such as but not limited to iodine (¹³¹I, ¹²⁵I, ¹²³I, ¹²¹I,), carbon (¹⁴C), sulfur (³⁵S) tritium (³H), indium (¹¹⁵In, ¹¹³In, ¹¹²In, ¹¹¹In) and technetium (⁹⁹Tc), thallium (²⁰¹Ti), gallium (⁶⁸Ga, ⁶⁷Ga), palladium (¹⁰³Pd), molybdenum (⁹⁹Mo), xenon (¹³³Xe), fluorine (¹⁸F), ¹⁵³Sm, ¹⁷⁷Lu, ¹⁵⁹Gd, ¹⁴⁹Pm, ¹⁴⁰La, ¹⁷⁵Yb, ¹⁶⁶Ho, ⁹⁰Y, ⁴⁷Sc, ¹⁸⁶Re, ¹⁸⁸Re, ¹⁴²Pr, ¹⁰⁵Rh, ⁹⁷Ru, ⁶⁸Ge, ⁵⁷Co, ⁶⁵Zn, ⁸⁵Sr, ³²P, ¹⁵³Gd, ¹⁶⁹Yb, ⁵¹Cr, ⁵⁴Mn, ⁷⁵Se, ¹¹³Sn, and ¹¹⁷Tin; positron emitting metals using various positron emission tomographies, noradioactive paramagnetic metal ions, and molecules that are radiolabelled or conjugated to specific radioisotopes. Any detectable label that can be readily measured can be conjugated to an anti-CD19 antibody and used in diagnosing B cell malignancies. The detectable substance may be coupled or conjugated either directly to an antibody or indirectly, through an intermediate (such as, for example, a linker known in the art) using techniques known in the art. *See, e.g.,* U.S. Patent No. 4,741,900 for metal ions which can be conjugated to antibodies for use as a diagnostics according to the present invention. As described herein, the present disclosure provides for diagnostic kits comprising an anti-CD19 antibody conjugated to a diagnostic or detectable agent.

### 5.8. KITS

The present disclosure provides a pharmaceutical pack or kit comprising one or more containers filled with a composition of the present disclosure for the prevention, treatment, management or amelioration of a B cell malignancy, or one or more symptoms thereof, potentiated by or potentiating a B cell malignancy.

The present disclosure provides kits that can be used in the above-described methods. A kit comprises a composition of the present disclosure, in one or more containers. In another embodiment, a kit comprises a composition of the invention, in one or more containers, and one or more other prophylactic or therapeutic agents useful for the prevention, management or treatment of a B cell malignancy, or one or more symptoms thereof, potentiated by or potentiating a B cell malignancy in one or more other containers. The kit further comprises instructions for preventing, treating, managing or ameliorating a B cell malignancy, as well as side effects and dosage information for method of administration. Optionally, associated with such container(s) can be a notice in the form prescribed by a governmental agency regulating the manufacture, use or sale of pharmaceuticals or biological products, which notice reflects approval by the agency of manufacture, use or sale for human administration.

### 6. EXAMPLES

In the examples below, a transgenic mouse model was used for evaluating human CD19 directed immunotherapies. These data show that antibodies that both bind the CD19 antigen and mediate ADCC are effective at inducing B cell depletion *in vivo,* in subjects having effector cells that express FcγR, (preferably, FcγRIII or FcγRIV) and carry out ADCC. Such antibodies can be used to induce a durable depletion of B cells *in vivo,* and in certain embodiments can eliminate virtually all B cells from the circulation, spleen and lymph nodes. Surprisingly, bone marrow B cells and their precursors that express relatively low densities of the CD19 antigen are depleted as well. The effectiveness of B cell depletion is not dependent on which region of human CD19 an anti-CD 19 antibody binds, but is influenced by CD19 density (in the patient sample). The efficiency of B cell clearance may correlate with the anti-CD 19 antibody's ability to mediate ADCC. The efficiency of B cell clearance using anti-CD 19 antibodies may also correlate with host effector FcγR expression/function.

### 6.1. MATERIALS AND METHODS

The murine HB12a and HB12b anti-CD 19 antibodies described herein are exemplary of antibodies that bind to human CD 19. Such antibodies can be used to engineer human, humanized, or chimeric anti-CD19 antibodies using the techniques described above in Section 5.1. Human, humanized, or chimeric anti-CD19 antibodies having the same specificity for human CD19 or portions thereof as the HB12a and HB12b antibodies are contemplated for use in the compositions and antibodies for use in the methods of the invention. In particular, human, humanized, or chimeric anti-CD19 antibodies having the same or similar heavy chain CDR1, CDR2, and/or CDR3 regions as the HB12a or HB12b are contemplated for use in the compositions and antibodies for use in the methods of the invention.

### 6.1.1. Materials and Methods

*Antibody Generation and Sequences Analysis.* The HB12a and HB12b antibodies were generated in Balb/c mice immunized with a mouse pre-B cell line that was transfected with cDNAs encoding human CD19 (Zhou et al., Mol. Cell Biol., 14:3884-94 (1994)). Both antibodies were submitted to the Fifth International Workshop and Conference on Human Leukocyte Differentiation Antigens that was held in Boston on November 3,-7, 1993.

Heavy chain gene utilization was determined using RNA extracted from 1-5 × 10⁶ hybridoma cells using the RNEASY^{®} Mini Kit (QIAGEN^{®}, Valencia, CA). First strand cDNA was synthesized in a volume of 20 µL from 2 µg of total RNA using 200 units of SUPERSCRIPT III^{®} reverse transcriptase and first strand cDNA, synthesis buffer from INVITROGEN^{®} (Carlsbad, CA), 20 ng random hexamer primers and 20 units of RNAse inhibitor from PROMEGA^{®} (Madison, WI), and 80 nmoles of dNTP from Denville (Metuchen, NJ). One µl of cDNA solution was used as template for PCR amplification of heavy chain (V_{H}) genes. PCR reactions were carried out in a 50-µl volume of a reaction mixture composed of 10 mM Tris-HCl (pH 8.3), 5 mM NH₄Cl, 50 mM KCl, 1.5 mM MgCl₂, 800 µM dNTP (Denville), 400 pmol of each primer, and 2.5 U of Taq DNA polymerase (Invitrogen) with 10% pfu proofreading polymerase (Stratagene, LaJolla, CA). For V_{L}, PCR reactions were carried out in a 50-µl volume of a reaction mixture composed of 20 mM Tris-HCl (pH 8.4), 50 mM KCl, 1.5 mM MgCl₂, 800 µM dNTP (Denville), 400 pmol of each primer, and 2.5 U of Taq DNA polymerase (Invitrogen) spiked with 10% pfu proofreading polymerase (Stratagene). After a 3 min denaturation step, amplification was for 32 cycles (94°C for 1 min, 58°C for 1 min, 72°C for 1 min) followed by a 10 minute extension at 72°C (Thermocycler, Perkin Elmer). Heavy chain cDNA was amplified using a promiscuous sense 5' V_{H} primer (MsV_{H}E; 5' GGG AAT TCG AGG TGC AGC TGC AGG AGT CTG G 3 ') (SEQ ID NO:19) as previously described (Kantor et al., J. Immunol., 158: 1175-1186 (1997)) and an antisense primer complementary to the Cγ coding region (primer Cγ1; 5' GAG TTC CAG GTC ACT GTC ACT GGC TCA GGG A3') (SEQ ID NO:20).

Light chain gene utilization was determined using cytoplasmic RNA extracted as described for heavy chain. The 5' variable region nucleotide sequence was obtained from cDNA that was generated using the GeneRacer™ kit (Invitrogen). Total RNA was dephosphorylated with calf intestinal phosphatase. The 5' cap structure was removed from intact, full-length mRNA with tobacco acid pyrophosphatase. A GeneRacer RNA oligo was ligated to the 5' end of the mRNA using T4 RNA ligase providing a known 5' priming site for GeneRacer PCR primers after the mRNA was transcribed into cDNA. The ligated mRNA was reverse transcribed with Superscript™III RT and the GeneRacer random primer. The first strand cDNA was amplified using the GeneRacer 5' primer (homologous to the GeneRacer RNA oligo) and a constant region specific antisense 3' primer (GAC TGA GGC ACC TCC AGA TGT TAA CTG) (SEQ ID NO:21). Touchdown PCR amplifications were carried out in a 50-µL volume with buffers as recommended by Invitrogen, using 2.5 U of Taq DNA polymerase (Invitrogen) with 10% pfu proofreading, polymerase (Stratagene) added. After a 2 main denaturation step, Taq and pfu was added and amplification was carried out in 3 steps: five cycles of 94°C for 30 s, 72°C for 60 s; 5 cycles of 94°C for 30 s, 72°C for 60 s; 20 cycles of 94°C for 30 s, 65°C for 30 s, 72°C for 60 s, followed by 10 min extension at 72°C. 2.5 U of Taq was added and the extension allowed to proceed for another 10 min to ensure intact 3' A-overhangs. Amplified PCR products were cloned into the pCR4-TOPO vector for sequencing and transformed into OneShot® TOP10 competent cells. DNA inserts from 8 clones was sequenced for each mAb light chain using the pCR4-TOPO vector specific "M 13 Forward" and "M 13 Reverse" primers, as described for heavy chain.

The purified heavy and light chain PCR products were sequenced directly in broth directions using an ABI 377 PRISM^{®} DNA sequencer after amplification using the Perkin Elmer Dye Terminator Sequencing system with AmpliTaq^{®} DNA polymerase and the same primers used for initial PCR amplification or pCR4-TOPO vector specific primers, as described for light chain. The HB 12a and HB 12b heavy and light chain regions were sequenced completely on both the sense and anti-sense DNA strands.

*Antibodies and Immunofluorescence Analysis.* Monoclonal mouse anti-CD19 antibodies that bind to the human CD 19 antigen used herein included HB12a (IgG1) and HB12b (IgG1), FMC63 (IgG2a, Chemicon International, Temecula, CA), B4 (IgG1, Beckman Coulter, Miami, FL) (Nadler et al., J. Immunol., 131:244-250 (1983)), and HD237 (IgG2b, Fourth International Workshop on Human Leukocyte Differentiation Antigens, Vienna, Austria, 1989), an isotype switch variant of the HD37 antibody (Pezzutto et al., J. Immunol., 138:2793-2799 (1987)). Other antibodies included: monoclonal mouse anti-CD19 antibody which binds to mouse CD 19, MB 19-1 (IgA) (Sato et al., J. Immunol., 157:4371-4378 (1996)); monoclonal mouse CD20-specific antibodies (Uchida et al., Intl. Immunol., 16:119-129 (2004)); B220 antibody RA3-6B2 (DNAX Corp., Palo Alto, CA); Thy1.2 antibody (CALTAG™ Laboratories, Burlingame, CA); and CD5, CD43 and CD25 antibodies (BD PHARMINGEN™, Franklin Lakes, NJ). Isotype-specific and anti-mouse Ig or IgM antibodies were from Southern Biotechnology Associates, Inc. (Birmingham, AL).

The mouse pre-B cell line, 300.19 (Alt et al., Cell, 27:381-388 (1981)), transfected with hCD19 cDNA (Tedder and Isaacs, J. Immunol., 143:712-717 (1989)), or single-cell leukocyte suspensions were stained on ice using predetermined optimal concentrations of each antibody for 20-30 minutes according to established methods (Zhou et al., Mol Cell. Biol., 14:3884-3894 (1994)). Cells with the forward and side light scatter properties of lymphocytes were analyzed on FACSCAN^{®} or FACSCALIBUR^{®} flow cytometers (Becton Dickinson, San Jose, CA). Background staining was determined using unreactive control antibodies (CALTAG™ Laboratories, Burlingame, CA) with gates positioned to exclude ≥ 98% of the cells. For each sample examined, ten-thousand cells with the forward and side light scatter properties of mononuclear cells were analyzed for each sample whenever possible, with fluorescence intensities shown on a four-decade log scale.

*Mice.* Transgenic mice expressing human CD19 (h19-1) and their wild-type (WT) littermates were produced as previously described (Zhou et al., Mol. Cell. Biol., 14:3884-3894 (1994)). TG-1 mice were generated from the original h19-1 founders (C57BL/6 x B6/SJL), and were crossed onto a C57BL/6 background for at least 7 generations. TG-2 mice were generated from the original h19-4 founders (C57BL/6 x B6/SJL). After multiple generations of backcrossing, TG-1^{+/+} mice were obtained the B cells of which expressed cell surface density of human CD 19 at about the same density found on human B cells. Human CD 19 expressing mice have been further described and used as a model in several studies (Engel et al., Immunity, 3:39-50 (1995); Sato et al., Proc. Natl. Acad. Sci. USA, 92:11558-11562 (1995); Sato et al., J. Immunol., 157:4371-4378 (1996); Tedder et al., Immunity, 6:107-118 (1997); Sato et al., J. Immunol., 158:4662-4669 (1997); Sato et al., J. Immunol., 159:3278-3287 (1997); Sato et al., Proc. Natl. Acad. Sci. USA, 94:13158-13162 (1997); Inaoki et al., J. Exp. Med., 186:1923-1931 (1997); Fujimoto et al., J. Immunol., 162:7088-7094 (1999); Fujimoto et al., Immunity, 11:191-200 (1999); Satterthwaite et al., Proc. Natl. Acad. Sci. USA, 97:6687-6692 (2000); Fujimoto et al., Immunity, 13:47-57 (2000); Sato et al., J. Immunol., 165:6635-6643 (2000); Zipfel et al., J. Immunol., 165:6872-6879 (2000); Qian et al., J. Immunol., 166:2412-2419 (2001); Hasegawa et al., J. Immunol., 167:2469-2478 (2001); Hasegawa et al., J. Immunol., 167:3190-3200 (2001); Fujimoto et al., J. Biol. Chem., 276:44820-44827 (2001); Fujimoto et al., J. Immunol., 168:5465-5476 (2002); Saito et al., J. Clin. Invest., 109:1453-1462 (2002); Yazawa et al., Blood, 102:1374-80 (2003); Shoham et al., J. Immunol., 171:4062-4072 (2003)). CD19-deficient (CD19^{-/-}) mice and their WT littermates are also as previously described (Engel et al., Immunity, 3:39-50 (1995)). Expression of human CD19 in transgenic mice has been shown to lower endogenous mouse CD19 expression (Sato et al., J. Immunol., 157:4371-4378 (1996); and Sato et al., J. Immunol., 158:4662-4669 (1997)) and hypotheses regarding this lowering of endogenous mouse CD19 expression have also been assessed (Shoham et al., J. Immunol., 171:4062-4072 (2003)). Densities of CD19 expression in transgenic mice expressing human CD19 have also been assessed (Sato et al., J. Immunol., 165:6635-6643 (2000)).

TG-1^{+/+} mice were bred with FcR (Fc receptor) common γ chain (FcRγ)-deficient mice (FcRγ^{-/-}, B6.129P2*-Fcerg1^{tm1})* from Taconic Farms (Germantown, NY) to generate hCD19^{+/-} FcRγ^{-/-} and WT littermates. Mice hemizygous for a c-Myc transgene (Eµ-cMycTG, C57B1/6J-TgN(IghMyc); The Jackson Laboratory, Bar Harbor, ME) were as described (Harris et al., J. Exp. Med., 167:353 (1988) and Adams et al., Nature, 318:533 (1985)). c-MycTG mice (B6/129 background) were crossed with hCD19TG-1^{+/+} mice to generate hemizygous hCD19TG-1^{+/-} cMycTG^{+/-} offspring as determined by PCR screening. Rag1^{-/-} (B6.129S7-*Rag1*^{tm1Mom}/J) mice were from The Jackson Laboratory. Macrophage-deficient mice were generated by tail vein injections of clodronate-encapsulated liposomes (0.1 mL/10 gram body weight; Sigma Chemical Co., St. Louis, MO) into C57BL/6 mice on day -2, 1 and 4 in accordance with standard methods (Van Rooijen and Sanders, J. Immunol. Methods, 174:83-93 (1994)). All mice were housed in a specific pathogen-free barrier facility and first used at 6-9 weeks of age.

*ELISAs.* Serum Ig concentrations were determined by ELISA using affinity-purified mouse IgM, IgG1, IgG2a, IgG2b, IgG3, and IgA (Southern Biotechnology Associates, Inc.) to generate standard curves as described (Engel et al., Immunity, 3:39 (1995)). Serum IgM and IgG autoantibody levels against dsDNA, ssDNA and histone were determined by ELISA using calf thymus double-stranded (ds) DNA (Sigma-Aldrich), boiled calf thymus DNA (which contains single-stranded (ss) DNA) or histone (Sigma-Aldrich) coated microtiter plates as described (Sato et al., J. Immunol., 157:4371 (1996)).

*Immunotherapy.* Sterile anti-CD19 and unreactive, isotype control antibodies (0.5-250 µg) in 200 µL phosphate-buffered saline (PBS) were injected through lateral tail veins. All experiments used 250 µg of antibody unless indicated otherwise. Blood leukocyte numbers were quantified by hemocytometer following red cell lysis, B220⁺ B cell frequencies were determined by immunofluorescence staining with flow cytometry analysis. Antibody doses in humans and mice were compared using the Oncology Tool Dose Calculator (www.fda.gov/cder/cancer/animalframe.htm).

*Immunizations.* Two-month old WT mice were immunized i.p. with 50 µg of 2,4,6-trinitrophenyl (TNP)-conjugated lipopolysaccharide (LPS) (Sigma, St. Louis, MO) or 25 µg 2,4-dinitrophenol-conjugated (DNP)-FICOLL^{®} (Biosearch Technologies, San Rafael, CA) in saline. Mice were also immunized i.p. with 100 µg of DNP-conjugated keyhole limpet hemocyanin (DNP-KLH, CALBIOCHEM^{®}-NOVABIOCHEM^{®} Corp., La Jolla, CA) in complete Freund's adjuvant and were boosted 21 days later with DNP-KLH in incomplete Freund's adjuvant. Mice were bled before and after immunizations as indicated. DNP- or TNP-specific anti-body titers in individual serum samples were measured in duplicate using ELISA plates coated with DNP-BSA (CALBIOCHEM^{®}-NOVABIOCHEM^{®} Corp., La Jolla, CA) or TNP-BSA (Biosearch Technologies, San Rafael, CA) according to standard methods (Engel et al., Immunity, 3:39-50 (1995)). Sera from TNP-LPS immunized mice were diluted 1:400, with sera from DNP-FICOLL^{®} and DNP-BSA immunized mice diluted 1:1000 for ELISA analysis.

*Tumor Studies.* Spontaneous lymph node tumor from a hCD19TG-^{1+/-} c-mycTG^{+/-} mouse was isolated and expanded *in vivo*. Tumor cells (10⁻⁵/mouse) were administered i.v. to Rag^{-/-} recipient mice on day 0, with FMC63 and isotype-matched control mAbs (250 µg/ml) given i.v. on days 1 and 7. Blood leukocytes from recipient mice were isolated weekly with the number of circulating mouse CD19⁺ B220⁺ cells quantified by immunofluorescent staining with flow cytometry analysis.

*Statistical Analysis*. All data are shown as means ± SEM. The Student's t-test was used to determine the significance of differences between sample means.

### 6.2. EXAMPLE 1: HUMAN CD 19 EXPRESSION IN TRANSGENIC MICE

The transgenic hCD 19TG mice described herein or other transgenic animals expressing human CD 19 can be used to assess different therapeutic regimens comprising human, humanized, or chimeric anti-CD19 antibodies, such as variations in dosing concentration, amount, or timing. The efficacy in human patients of different therapeutic regimens can be predicted using the two indicators described below, *i*.*e.,* B cell depletion in certain bodily fluids and/or tissues and the ability of a monoclonal human or humanized anti-CD19 antibody to bind B cells. Treatment regimens that are effective in human CD19 transgenic mice can be used with the compositions and antibodies for use in the methods of the invention to treat B cell malignancies in humans.

In order to determine whether human CD 19 was expressed on B cells from transgenic mice (hemizygous TG-1^{+/-}) expressing the human CD19 transgene, B cells were extracted from the bone marrow, blood, spleen and peritoneal lavage of these mice. Human CD 19 and mouse CD 19 expression were assessed in these cells by contacting the cells with mouse monoclonal anti-CD19 antibodies that bind CD19. Binding of the antibody to the B lineage cells was detected using two-color immunofluorescence staining with flow cytometry analysis.

The results are shown in **Fig. 1A** in graphs of the detected expression of murine CD 19 (mCD19) (x-axis) plotted against the detected expression of human CD19(hCD19) (y-axis) for bone marrow (BM), blood, spleen and peritoneal lavage (PL). The units of the axis represent a four decade log scale beginning with 1 on the lower left. The B4 anti-CD 19 antibody that binds to human CD19 (Beckman/Coulter) was used to visualize human CD19 expression and the 1D3 CD19 antibody that binds to mouse CD 19 (PharMingen) was used to visualize mouse CD19 expression (also used for **Figs. 1B** and **1C**). While human CD19 expression increases incrementally during human B cell development, murine CD 19 is expressed at high levels during mouse bone marrow B cell development. **Fig. 1A** shows that human CD19 expression parallels mouse CD19 expression on peripheral B cells found in blood, spleen and peritoneal lavage (PL) demonstrating that the mouse anti-hCD19 antibody (that binds human CD19) binds the peripheral B cell populations. In addition, a small population of bone marrow (BM) derived B cells express endogenous mouse CD 19 but not human CD19 (monoclonal mouse anti-CD19 antibody that binds to human CD19). Thus, bone marrow B cells fall into two categories in hemizygous TG-1^{+/-} mice, mature B lineage cells that are hCD19⁺mCD19⁺ and less mature B lineage cells that are only mCD19⁺ (**Fig**. **1A**). These results are consistent with the findings of Zhou et al. (Mol. Cell. Biol., 14:3884-3894 (1994)) which indicated that human CD19-expression in these transgenic mice correlates with B cell maturation. All mature B cells within the blood, spleen, and peritoneal cavity were both hCD19+ and mCD19⁺.

The relative expression levels of mCD19 and hCD 19, as assessed by measuring mean fluorescence intensity (mouse anti-CD 19 for hCD19 and mouse anti-CD19 for mCD19) respectively, are shown in **Fig. 1B**. Among TG-1 mice homozygous for the hCD19 transgene (TG-1^{+/+}), hCD19 expression on blood borne B cells was comparable to hCD19 expression on human B cells. To compare the relative densities of hCD19 and mCD19 expression in TG-1^{+/+}, TG-1^{+/-}, and TG-2^{+/+} transgenic mouse lines, blood derived B cells were extracted and assayed for CD19 expression as described above. The results are shown in **Fig. 1B** in histograms showing the percent human CD 19 expression for human blood B cells, TG-1^{+/+}, TG-1^{+/-}, and TG-2^{+/+} blood B cells from hCD19TG mice (left) and the percent mouse CD 19 expression for wild type (WT) mouse blood B cells, TG-1^{+/+}, TG-1^{+/-}, and TG-2^{+/+} CD19⁺ blood B cells from hCD19TG mice (right). The values (linear values of mean fluorescent intensity) represent the mean relative densities of CD19 expression (±SEM) compared to blood B cells from humans or wild-type (WT) mice (shown as 100%). The results show that in homozygous TG-1^{+/+} mice, blood B cells expressed hCD19 at densities as measured by mean fluorescence intensities about 72% higher than human blood B cells. Blood B cells in TG-1^{+/-} mice expressed hCD19 at densities similar to human blood B cells, while blood B cells in TG-2^{+/+} mice expressed hCD 19 at densities 65% lower than human blood B cells.

Further comparisons of the relative densities of hCD19 and mCD19 expression in B cells from TG-1 ^{+/-}mouse tissues are shown in **Fig. 1C** in histograms showing the mean fluorescence intensities (MFI±SEM) of anti-CD19 antibody staining for B cells from bone marrow, blood, spleen, lymph node, and PL for hCD19 (left) and mCD19 (right). The results demonstrate that in TG-1^{+/-} mice, hCD19 was expressed at increasing levels by B220⁺ cells in the bone marrow (63% of human blood levels) < blood (100%) < spleen (121%) = lymph node (120%) and < peritoneal cavity (177%). Human CD19 expression had a small influence on mCD19 expression. Levels of mRNA for hCD19 and mCD19 did not change.

To determine whether mouse anti-hCD19 antibodies (that bind to human CD19) of the IgG1 (HB12a, HB12b, B4), IgG2a (FMC63) and IgG2b (HD237) isotypes react differently, blood and spleen B220⁺ B cells were isolated from TG-1^{+/-} mice. The isolated cells were contacted *in vitro* with the above-mentioned anti-CD 19 antibodies and assessed for their ability to bind human CD 19 expressing transgenic mouse (hCD 19TG) B cells using monoclonal antibody staining which was visualized using isotype-specific PE-conjugated secondary antibodies with flow cytometry analysis.

The results are shown in **Fig. 1D** in graphs of the fluorescence intensity (x-axis) versus the relative B cell number (y-axis) for IgG2b (murine isotype), IgG2a (murine isotype), and IgG1 (murine isotype) anti-CD19 antibodies at 5 µg/mL. The fluorescence intensity of B220⁺ cells stained with anti-CD19 antibody are shown as solid lines and the fluorescence intensity of the isotype-matched control (CTL) is shown as a dashed line. Each antibody reached saturating levels of reactivity with spleen B cells at a concentration of 5 µg/mL. The results demonstrate that anti-CD 19 antibody binding density on mouse blood and spleen B220⁺ B cells from TG-1^{+/-} mice is uniform for the antibody isotypes tested and for both blood and spleen B cells.

To determine whether mean fluorescence intensities were independent of anti-CD 19 antibody isotype, the binding activity of individual anti-CD19 antibodies (at 5 µg/mL) was assessed by staining a mouse pre-B cell line, 300.19, transfected with a hCD 19 cDNA using the same anti-mouse Ig secondary antibody. Antibody staining (MFI±SEM) was visualized using mouse Ig-specific PE-conjugated secondary antibody with flow cytometry analysis. The results are shown in **Fig. 1E** in a histogram of anti-CD 19 antibody binding (as shown by staining intensity, y-axis) to hCD19 cDNA-transfected 300.19 cells, for HB12a, HB12b, B4, FMC63, HD237 anti-CD19 antibodies and a control antibody (CTL). Each antibody stained cells with characteristic mean fluorescence intensities that were independent of anti-CD19 antibody isotype, with HB12b showing the lowest levels of staining and HD237 demonstrating the highest. Thus, the results shown demonstrate that 300.19 cells are a model *in vitro* system for the comparison of the ability of anti-CD19 antibodies to bind CD19 *in vitro.*

Thus, taken together, the results shown in **Fig. 1** demonstrate that hCD 19TG mice and the 300.19 cells represent appropriate *in vitro* and *in vivo* model systems for assessing the ability of anti-hCD19 antibodies to bind B cells when hCD19 is expressed over a range of densities.

**Figs.** 1A-D represent results obtained with ≥ 3 mice of each genotype.

### 6.2. EXAMPLE 2: ANTI-CD19 ANTIBODY DEPLETION OF B CELLS IN VIVO

Mouse anti-CD19 antibodies (that bind to human CD19) were assessed for their ability to deplete hCD19TG (TG-1^{+/-}) blood, spleen, and lymph node B cells *in vivo.* Each antibody was given to mice at either 250 or 50 µg/mouse, a single dose about 10 to 50-fold lower than the 375 mg/m² dose primarily given four times for anti-CD20 therapy in humans (Maloney et al., J. Clin. Oncol., 15:3266-74(1997) and McLaughlin *et al.,* 12:1763-9 (1998)).

The results are shown in **Fig. 2A** in a plot of B cell amount 7 days following CD 19 or isotype-matched control (CTL) treatment with HB12a, HB12b,or FMC63 anti-CD 19 antibodies or a control. Separate plots are provided for lymph nodes, spleen and blood tissues for each anti-CD 19 antibody. The percentage of gated lymphocytes depleted at 7 days shown on each plot demonstrates representative B cell depletion from blood, spleen and lymph nodes of TG-1^{+/-} mice as determined by immunofluorescence staining with flow cytometry analysis. **Fig. 2B** shows mean numbers (±SEM per ml) of B220⁺ blood B cells following treatment with anti-CD 19 (closed circles) or isotype-control (open circles) antibodies. The value shown after time 0 represents data obtained at 1 hour. **Fig. 2C** and **Fig. 2D** show spleen and lymph node B cell numbers (±SEM), respectively, after treatment of TG-1^{+/-} mice with anti-CD 19 (filled bars) or control (open bars) antibody at the indicated doses. In **Figs. 2B-D**, significant differences between mean results for anti-CD19 or isotype-control antibody treated mice (≥ 3 mice per data point) are indicated; *p<0.05, **p<0.01, in comparison to controls.

Each antibody depleted the majority of circulating B cells within one hour of treatment (**Fig. 2B**), with potent depleting effects on spleen and lymph node B cell frequencies (**Fig. 2A**) and numbers (**Figs. 2C-D**) by day seven. The HB 12a antibody depleted 98% of blood B cells and 90-95% of splenic and lymph node B cells by day seven. Similarly, the HB12b, B4, FMC63, and HD237 antibodies depleted 99%, 96%, 99%, and 97% of blood B cells, respectively. The HB12b, B4, FMC63, and HD237 antibodies depleted 88-93%, 64-85%, 72-95%, and 88-90% of splenic and lymph node B cells, respectively. The few remaining peripheral B cells primarily represented phenotypically immature cells that were potential emigrants from the bone marrow. None of the CD 19 antibodies had significant effects when given to WT mice, and isotype-matched control antibodies given under identical conditions did not affect B cell numbers (**Figs. 2A-D**). Thus, anti-hCD19 antibodies effectively depleted B cells from the circulation, spleen and lymph nodes of hCD19TG mice by day seven. A summary of B cell depletion in TG-1^{+/-} mice is provided in **Table 1**.

**TABLE 1**

| **Tissue** | **B subset^{a}** | **Control mAb^{b}** | **CD19 mAb** | **% Depletion** |
|---|---|---|---|---|
| BM: | B220⁺ | 3.41±0.57 (11) | 0.82±0.13(11) | 76** |
| | Pro-B | 0.75±0.1 (5) | 0.97±0.22 (5) | 0 |
| | Pre-B | 1.74±0.58 (5) | 0.10±0.01 (5) | 94** |
| | immature | 0.70±0.16 (5) | 0.04±0.01 (5) | 93** |
| | mature | 0.86±0.14 (5) | 0.004±0.0004 (5) | 99** |
| Blood: | B220⁺ | 0.822±0.14 (11) | 0.004±0.0006 | 99** |
| Spleen: | B220⁺ | 25.2±2.2 (11) | 1.7±0.2 (11) | 93** |
| LN: | B220⁺ | 0.89±0.11 (11) | 0.06±0.01 (11) | 93** |
| Peritoneum: | B220⁺ | 1.16±0.11 (11) | 0.37±0.03 (11) | 68** |
| | B1a | 0.86±0.12 (5) | 0.31±006 (5) | 61** |
| | B2 | 0.34±0.06 (5) | 0.08±0.02 (5) | 73** |

| | | | | |
|---|---|---|---|---|
| ^{a}B cell subsets were: bone marrow (BM) pro-B (CD43⁺IgM⁻B22U^{lo}), pre-B (CD43⁻ IgM-B220^{lo}); immature B (IgM⁺B220^{lo}), mature B (IgM⁺B220^{hi}); peritoneal B1a (CD5⁺B220^{lo}), B2 (CD5⁻B22O^{hi}). ^{b}Values (±SEM) indicate cell numbers (x 10⁻⁶) present in mice seven days after antibody treatment (250 µg). BM values are for bilateral femurs. Blood numbers are per/ml. LN numbers are for bilateral inguinal and axillary nodes. Mouse numbers are indicated in parentheses. Significant differences between means are indicated; *p<0.05, **p<0.01. | | | | |

### 6.3.1. DEPLETION OF BONE MARROW B CELLS

Known anti-CD19 antibodies were tested in hCD19TG mice to determine whether such antibodies were effective in depleting B cells from various bodily fluids and tissues. The assays described herein can be used to determine whether other anti-CD 19 antibodies, for example, anti-CD 19 antibodies that bind to specific portions of the human CD19 antigen, will effectively deplete B cells. The results using anti-CD19 antibodies identified as capable of depleting B cells can be correlated to use in humans. Antibodies with properties of the identified antibodies can be used in the compositions and methods of the invention for use in the treatment of B cell malignancies in humans. **Figs. 3A-3F** depict bone marrow B cell depletion following CD 19 antibody treatment.

**Fig. 3A** shows graphs of the fluorescence intensity (x-axis) versus the relative B cell number (y-axis) for hCD19 and mCD19 expression by TG-1^{+/-} bone marrow B cell subpopulations assessed by four-color immunofluorescence staining with flow cytometry analysis of cells with the forward- and side-scatter properties of lymphocytes. Pro-B cells were defined as CD43⁺IgM⁻B220^{lo}, pre-B cells were CD43⁻IgM⁻B220^{lo}, immature B cells were IgM⁺B220^{lo} and mature B cells were IgM⁺B220^{hi}. Bar graphs (right) show relative **mean** MFI (±SEM) values for CD19 expression by each B cell subset (≥ 3 mice/data point). As in hCD19TG mice **(****Fig. 1A****),** CD19 expression is heterogeneous in humans as B cells mature and exit the bone marrow. Only a small fraction of pro-B cells (20%, CD43^{hi}IgM⁻ B220^{lo}) expressed hCD19 in TG-1^{+/-} mice, while most pre-B cells were hCD19⁺ and the majority of mature B cells in the bone marrow expressed hCD19 at relatively high levels. Halfofpro-B cells (55%, IgM⁻B220⁺) expressed mCD19 in TG-1^{+/-} mice, while mCD19 was expression by the majority of pre-B cells and mature B cells in the bone marrow at relatively high levels.

**Fig. 3B** shows depletion of hCD 19⁺ cells in hCD 19TG mice seven days following FMC63 or isotype-matched control antibody (250 µg) treatment assessed by two-color immunofluorescence staining with flow cytometry analysis. Numbers represent the relative frequency of cells within the indicated gates. Results represent those obtained with three littermate pairs of each mouse genotype. Following CD 19 antibody treatment, the vast majority of hCD19⁺ cells in the bone marrow of TG-1^{+/+}, TG-1^{+/-} and TG-2^{+/+} mice were depleted by the FMC63 antibody given at 250 µg/mouse.

**Fig. 3C** shows representative B220⁺ B cell depletion seven days following anti-CD 19 or isotype-matched control antibody (250 µg) treatment of TG-1^{+/-} mice. Bar graph values represent the total number (±SEM) of B220⁺ cells within the bilateral femurs of antibody treated mice. Significant differences between sample means (≥ 3 mice per group) are indicated; *p<0.05, **p<0.01. Unexpectedly, a large fraction of mCD19⁺ pre-B cells that expressed hCD19 at low to undetectable levels were also depleted from the bone marrow. Consistent with this, the FMC63, HB12a, HB12b, B4 and HD237 antibodies depleted the majority of bone marrow B220⁺ cells.

**Fig. 3D** shows representative bone marrow B cell subset depletion seven days following FMC63 or isotype-matched control antibody (250 µg) treatment of TG-1^{+/-} mice as assessed by three-color immunofluorescence staining. IgM⁻B220^{lo} pro-/pre-B cells were further subdivided based on CD43 expression (lower panels). **Fig. 3E** shows representative depletion or CD25⁺B220^{lo} pre-B cells of bone marrow seven days following FMC63 or isotype-matched control antibody (250 µg) treatment of hCD19TG mouse lines as assessed by two-color immunofluorescence staining. Results are from experiments carried out on different days so the gates were not identical. When the individual bone marrow subpopulations were analyzed, the majority ofCD43^{hi}IgM-B220^{lo} pro-B cells **(****Fig. 3D****)** were not affected by FMC63 antibody treatment in TG-1^{+/+}, TG-1^{+/-} or TG-2^{+/+} mice, while the majority of CD25⁺CD43^{lo}gM⁻B220^{lo} pre-B cells **(****Fig. 3E****)** were depleted. **Fig. 3F** shows bar graphs indicating numbers (±SEM) of pro-B, pre-B, immature, and mature B cells within bilateral femurs seven.days following FMC63 (closed bars) or control (open bars) antibody treatment of ≥ 3 littermate pairs. The results demonstrate, that the majority of immature and mature B cells were also depleted from the bone marrow of TG-1^{+/+}, TG-1^{+/-} and TG-2^{+/+} mice. Thus, most hCD19⁺ cells were depleted from the bone marrow by CD19 antibody treatment, including pre-B cells that expressed hCD19 at low levels.

### 6.3.2. DEPLETION OF PERITONEAL B CELLS

Peritoneal cavity B cells in TG-1^{+/-} mice express hCD19 at higher levels than other tissue B cells **(****Fig. 1A** and **Fig. 1C****),** primarily due to the presence of CD5⁺IgM^{hi}B220^{lo}-B1 cells that expressed hCD19 at approximately 25% higher densities than the CD5⁻ IgM^{lo}B220^{hi} subset of conventional (B2) B cells **(****Fig. 4A****).** **Figs. 4B-4C** demonstrate that peritoneal cavity B cells are sensitive to anti-CD 19 antibody treatment.

**Fig. 4A** shows plots of human and mouse CD 19 expression (x-axis) versus the relative number of peritoneal cavity CD5⁺B220⁺ B1a and CD5⁻B220^{hi} B2 (conventional) B cells (y-axis). Single-cell suspensions of peritoneal cavity lymphocytes were examined by three-color immunofluorescence staining with flow cytometry analysis. Bar graphs represent mean MFI (±SEM) values for CD 19 expression by 3 littermate pairs of TG-1^{+/-} mice.

**Fig. 4B** shows depletion of peritoneal cavity B220⁺ cells from TG-1^{+/-} mice treated with CD 19 (HB12a, HB12b, and FMC63 at 250 µg; B4 and HD237 at 50 µg) antibodies or control antibody (250 µg). Numbers represent the relative frequencies of B220⁺ cells within the indicated gates on day seven. Bar graph values represent the total number (±SEM) of B220⁺ cells within the peritoneum of antibody treated mice (≥ 3 mice per group). Significant differences between sample means are indicated; *p<0.05, **p<0.01. The results demonstrate that anti-CD19 antibody treatment at 250µg/mouse depleted a significant portion of peritoneal B220⁺ B cells by day seven. The results shown in **Fig. 4B** are in part explained by the depletion of both B1 and conventional B2 cells. When hCD19 was expressed at the highest densities in TG-1^{+/+} mice, the majority of B1 and B2 cells were depleted. However, CD19-mediated depletion of B1 and B2 cells was less efficient in TG-1^{+/-} and TG-2^{+/+} mice where hCD19 levels were lower. Thus, CD19 antibody treatment depleted peritoneal B1 and B2 cells depending on their density of CD19 expression as assessed using mean fluorescence intensity, although peritoneal B cells were more resistant to anti-CD19 antibody-mediated depletion than spleen and lymph node B cells.

**Fig. 4C** shows representative depletion of CD5⁺B220⁺ B1a and CD5⁻B220^{hi} B2 B cells seven days following anti-CD19 antibody or control antibody treatment of hCD19TG mice. Numbers represent the relative frequencies of each B cell subset within the indicated gates. Bar graph values represent the total number (±SEM) of each cell subset within the peritoneum of antibody treated mice (≥ 3 mice per group). Significant differences between sample means are indicated; *p<0.05, **p<0.01.

### 6.3.3. DISTINCT ANTI-CD19 ANTIBODIES MEDIATE B CELL CLEARANCE

In order to determine whether HB12a and HB12b anti-CD 19 antibodies are distinct from known anti-CD 19 antibodies, the amino acid sequence of each anti-CD 19 antibody variable region used herein was analyzed **(****Figs. 5A** and **5B****, 6A** and **6B, 7A** and **7B)**.

**Fig. 5A** depicts the nucleotide (SEQ ID NO:1) and predicted amino acid (SEQ ID NO:2) sequences for heavy chain V_{H}-D-J_{H} junctional sequences of the HB12a anti-CD19 antibody. Sequences that overlap with the 5' PCR primer are indicated by double underlining and may vary from the actual DNA sequence since redundant primers were used. Approximate junctional borders between V, D, and J sequences are designated in the sequences by vertical bars (|). Nucleotides in lower case letters indicate either nucleotide additions at junctional borders or potential sites for somatic hypermutation. The amino-terminal residue of the antibody (E) is marked as residue 1.

**Fig. 5B** depicts the nucleotide (SEQ ID NO:3) and predicted amino acid (SEQ ID NO:4) sequences for heavy chain V_{H}-D-J_{H} junctional sequences of the HB12b anti-CD19 **antibody.** Sequences that overlap with the 5' PCR primer are indicated by double underlining and may vary from the actual DNA sequence since redundant primers were used. Approximate junctional borders between V, D, and J sequences are designated in the sequences by vertical bars (|). Nucleotides in lower case letters indicate either nucleotide additions at junctional borders or potential sites for somatic hypermutation. The amino-terminal residue of the antibody (E) is marked as residue 1.

**Fig. 6A** depicts the nucleotide (SEQ ID NO:15) and predicted amino acid sequence (SEQ ID NO:16) sequences for light chain Vκ-Jκ junctional sequences of the HB12a anti-CD 19 antibody. **Fig. 6B** depicts the nucleotide (SEQ ID NO:17) and predicted amino acid (SEQ ID NO:18) sequences for the light chain V-J junctional sequences of the HB12b anti-CD 19 antibody. The amino-terminal amino acid of the mature secreted protein deduced by amino acid sequence analysis is numbered as number 1. Sequences that overlap with the 3' PCR primer are indicated by double underlining. Predicted junctional borders for the V-J-C regions are indicated (/) with J region nucleotides representing potential sites for somatic hypermutation in bold.

**Fig. 7A** and **7B** depict the amino acid sequence alignment of published mouse anti-CD 19 antibodies. **Fig. 7A** shows a sequence alignment for heavy chain V_{H}-D-J_{H} junctional sequences including a consensus sequence (SEQ ID NO:5), HB12a (SEQ ID NO:2), 4G7 (SEQ ID NO:6), HB12b (SEQ ID NO:4), HD37 (SEQ ID NO:7), B43 (SEQ ID NO:8), and FMC63 (SEQ ID NO:9). Amino acid numbering and designation of the origins of the coding sequences for each antibody V, D and J region are according to conventional methods (Kabat et al., Sequences of Proteins of Immunological. Interest., U.S. Government Printing Office, Bethesda, MD (1991)) where amino acid positions 1-94 and complementarity-determining regions CDR1 and 2 are encoded by a V_{H} gene. A dash indicates a gap inserted in the sequence to maximize alignment of similar amino acid sequences. A dot indicates identity between each anti-CD 19 antibody and the consensus amino acid sequence for all antibodies. CDR regions are highlighted for clarity. **Fig. 7B** shows light chain Vκ amino acid sequence analysis of anti-CD 19 antibodies. Consensus sequence (SEQ ID NO:10), HB12a (SEQ ID NO:16); HB12b (SEQ ID NO:18); HD37 (SEQ ID NO:11), B43 (SEQ ID NO:12), FMC63 (SEQ ID NO:13), and 4G7 (SEQ ID NO:14) are aligned. Amino acid numbering and designation of the origins of the coding sequence for each anti-CD 19 antibody is according to conventional methods (Kabat *et al.,* (1991) *Sequences of Proteins of Immunological Interest.,* U.S. Government Printing Office, Bethesda, MD). The amino acid following the predicted signal sequence cleavage site is numbered 1. A dash indicates a gap inserted in the sequence to maximize alignment of similar amino acid sequences. CDR regions are highlighted (boxed) for clarity.

Since each anti-CD 19 antibody examined in this study depleted significant numbers of B cells *in vivo*, the amino acid sequence of each anti-CD 19 antibody variable region was assessed to determine whether these antibodies differ in sequence and potentially bind to different CD 19 epitopes. Antibodies bind target antigens through molecular interactions that are mediated by specific amino acids within the variable regions of each antibody molecule. Thus, complex interactions between protein antigens and the antibodies that bind to specific epitopes on these antigens are almost unique to each antibody and its specific amino acid sequence. This level of complexity in antigen and antibody interactions is a reflection of a diverse antibody repertoire to most protein antigens. While antibody interactions with target antigens are primarily mediated by amino acids within complementarity-determining regions (CDR) of antibody molecules, framework amino acids are also critical to antigen-binding activity. Thus, structurally similar antibodies are likely to bind to the same antigens or region of a target molecule, while structurally dissimilar antibodies with different V and CDR regions are likely to interact with different regions of antigens through different molecular interactions.

Since antibodies that interact with and bind to the same molecular region (or epitope) of a target antigen are structurally similar by definition, the amino acid sequences of HB12a, HB12b, FMC63 and other published anti-CD19 antibodies were compared including the HD37 (Kipriyanov et al., J. Immunol. Methods, 196:51-62(1996); Le Gall et al., FEBS Letters, 453:164-168 (1999)), 2G7 (Meeker et al., Hybridoma, 3:305-320 (1984); Brandl et al., Exp. Hematol., 27:1264-1270 (1999)), and B43 (Bejcek et al., Cancer Res., 55:2346-2351 (1995)) antibodies. The heavy chains of the anti-CD19 antibodies were generated through different combinations of V(D)J gene segments with the V regions derived from the V1S39, V1S56, V1S136, or V2S1 gene segments, D regions derived from FL16.1 gene segments, and J regions derived from either J2 or J4 gene segments **(Table 2)**. The published heavy and light chain variable regions of the B43 and HD37 antibodies were virtually identical in amino acid sequence **(****Figs. 7A-B).** This level of conservation reflects the fact that each of these antibodies is also remarkably similar at the nucleotide level, having identical V_{H}(D)J_{H} and V_{L}J_{L} junctions, with most differences accounted for by the use of redundant primers to PCR amplify each cDNA sequence. This indicates that the HD37 and B43 and antibodies share a common, if not identical, origin and therefore bind to identical epitopes on the CD 19 protein. The HB12a and 4G7 antibodies were also distinct from other anti-CD19 antibodies. Although the heavy chain regions of the HB12a and 4G7 antibodies were similar and are likely to have derived from the same germline V_{H}(D)J_{H} gene segments, different junctional borders were used for D-J_{H} assembly (**Fig**. **7A**). The HB12b antibody utilized a distinct V_{H} gene segment (**Table 2**) and had distinctly different CDR3 sequences (**Fig**. **7A**) from the other anti-CD19 antibodies. The FMC63 antibody also had a very distinct amino acid sequence from the other anti-CD 19 antibodies.

**TABLE 2**

| | Heavy Chain | | | | Light Chain | | |
|---|---|---|---|---|---|---|---|
| | V^{a} | D | J | Accession #^{b} | V | J | Accession # |
| | | | | | V1-133* | J2* | |
| HB12a | V1S136 (12,8) | FL16.1 | J2 | | 01 | 01 | |
| | | | | | | J4 * | |
| HB12b | V1S56 (27,14) | FL16.1 | J2 | | V3-2*01 | 01 | |
| 4G7 | V1S136 (10,8) | F16.1 | J2 | AJ555622 | V2-137 | J5 | AJ555479 |
| B43 | V1S39 (37,17) | FL16.1 | J4 | S78322 | V3-4 | J1 | S78338 |
| HD37 | V1S39 (34,16) | FL16.1 | J4 | X99230 | V3-4 | J1 | X99232 |
| FMC63 | V2S1 (20,16) | FL16.1 | J4 | Y14283 | V10-96 | J2 | Y14284 |

| | | | | | | | |
|---|---|---|---|---|---|---|---|
| N.D., not determined. ^{a}Numbers in parenthesis indicate the number of nucleotide differences between the CD19 antibody encoding gene and the most homologous germline sequence identified in current databases, excluding regions overlapping with PCR primers. ^{b}GENBANK^{®} accession numbers for gene sequences. | | | | | | | |

As shown in **Fig. 7B**, the HB12a, HB12b, FMC63, 4G7, and HD37/B43 antibodies each utilize distinct light chain genes (**Fig. 7B**). Light chains were generated from multiple V and J gene segments. The lack of homogeneity among these six anti-CD19 antibodies H and L chain sequences suggests that these antibodies bind to several distinct sites on human CD19. A comparison of amino acid sequences of paired heavy and light chains further indicates that most of these anti-CD19 antibodies are structurally distinct and will therefore bind human CD 19 through different molecular interactions. Thus, the ability of anti-CD 19 antibodies to deplete B cells *in vivo* is not restricted to a limited number of antibodies that bind CD19 at identical sites, but is a general property of anti-CD19 antibodies as a class.

### 6.3.4. CD19 DENSITY INFLUENCES THE EFFECTIVENESS OF CD19 ANTIBODY-INDUCED B CELL DEPLETION

To determine whether an anti-CD19 antibody's ability to deplete B cells is dependent on CD 19 density, the HB 12b and FMC63 anti-CD 19 antibodies were administered to mice having varying levels of CD 19 expression. The results demonstrate that human CD 19 density on B cells and antibody isotype can influence the depletion of B cells in the presence of an anti-CD 19 antibody. The same assay can be used to determine whether other anti-CD19 antibodies can effectively deplete B cells and the results can be correlated to treatment of human patients with varying levels of CD19 expression. Thus, the methods for examining CD 19 presence and density in human subjects described in Section 5.5.3 can be used to identify patients or patient populations for which certain anti-CD 19 antibodies can deplete B cells and/or to determine suitable dosages.

The results presented above indicate that although all five anti-CD 19 antibodies tested were similarly effective in TG-1^{+/-} mice when used at 250 or 50 µg, the extent of B cell depletion for B cells from blood bone marrow and spleen appeared to correlate with antibody isotype, IgG2a>IgG1>IgG2b (**Figs. 2A-2D**). Therefore, the effectiveness of the HB12b (IgG1) and FMC63 (IgG2a) antibodies was compared in homozygous TG-1^{+/+}, heterozygous TG-1^{+/-} and homozygous TG-2^{+/+} mice that express CD19 at different densities (**Figs. 1A-E**).

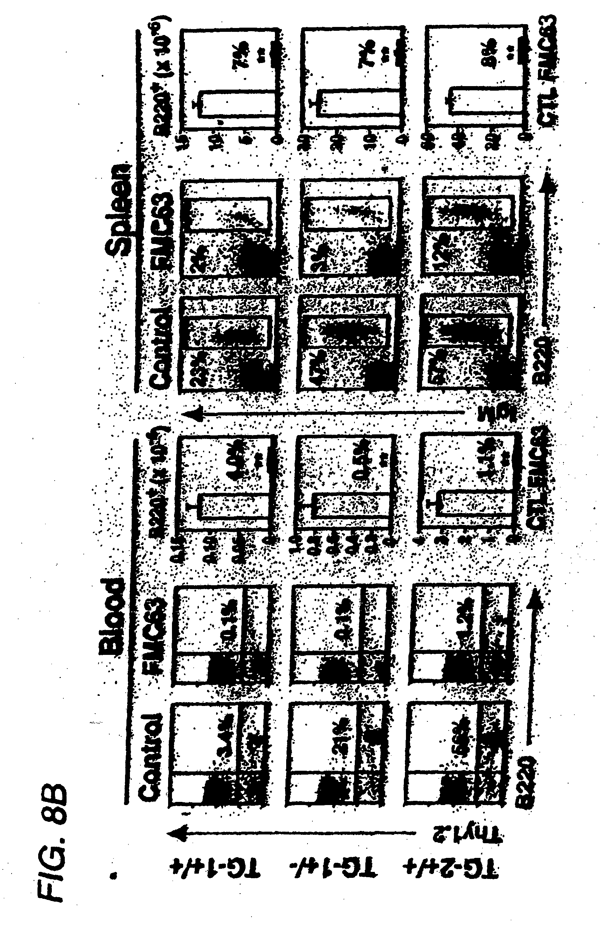

To determine whether CD 19 density influences the effectiveness of anti-CD19 antibody-induced B cell depletion representative blood and spleen B cell depletion was examined in hCD 19TG mice after HB 12b (**Fig. 8A**) or FMC63 **(****Fig. 8B**) antibody treatment (7 days, 250 µg/mouse). Numbers indicate the percentage of gated B220⁺ lymphocytes. Bar graphs indicate numbers (±SEM) of blood (per mL) or spleen (total number) B cells following treatment with anti-CD19 antibodies (closed bars) or isotype-control (open bars) antibodies. Significant differences between mean results for anti-CD 19 antibody or isotype-control antibody treated mice (≥ 3 mice per data point) are indicated; *p<0.05, **p<0.01.

The results presented in **Figs. 8A-8D** demonstrate that CD 19 density influences the efficiency of B cell depletion by anti-CD19 antibodies *in vivo.* Low-level CD19 expression in TG-2^{+/+} mice had a marked influence on circulating or tissue B cell depletion by the HB12b antibody on day seven (**Fig. 8A**). Differences in CD19 expression by TG-1^{+/+}, TG-1^{+/-} and TG-2^{+/+} mice also influenced circulating and tissue B cell depletion by the FMC63 antibody but did not significantly alter circulating B cell depletion (**Fig. 8B**).

To further verify that CD 19 density is an important factor in CD 19 mAb-mediated B cell depletion, the relative depletion rates of CD19TG-1^{+/+} and CD19TG-2^{+/+} B cells were compared directly. Splenocytes from CD19TG-1^{+/+} and CD19TG-2^{+/+} mice were differentially labeled with CFSE by labeling unfractionated splenocytes from hCD19TG-1^{+/+} and hCD19TG-2^{+/+} mice were labeled with 0.1 and 0.01 µM Vybrant™ CFDA SE (CFSE; Molecular Probes), respectively, according to the manufacture's instructions. The relative frequency of B220⁺ cells among CFSE-labeled splenocytes was determined by immunofluorescence staining with flow cytometry analysis. Subsequently, equal numbers of CFSE-labeled B220⁺ hCD19TG-1^{+/+} and hCD19TG-2^{+/+} splenocytes (2.5x10⁵) were injected into the peritoneal cavity of three wild type B6 mice. After 1 hour, the mice were given either FMC63 or control mAb (250 µg, i.p.). After 24 hours, the labeled lymphocytes were recovered with the relative frequencies of CFSE-labeled B220⁺ and B220⁻ cells assessed by flow cytometry. The gates in each histogram in **Fig. 8C** indicate the frequencies of B220⁺ cells within the CD19TG-1^{+/+} (CFSE^{high}) and CD19TG-2^{+/+}(CFSE^{low}) splenocyte populations. The bar graph indicates the number of CFSE labeled cell population present in anti-CD19 mAb treated mice relative to control mAb-treated mice. Results represent hCD19TG-1^{+/+} splenocytes (filled bars) and hCD19TG-2^{+/+} splenocytes (open bars) transferred into ≥3 wild type recipient mice, with significant differences between sample means (±SEM) indicated; **p<0.01.

B cell clearance was assessed 24 hours after anti-CD 19 or control mAb treatment of individual mice. CD19TG-1^{+/+} B220⁺ B cells were depleted at significantly faster rates (p<0.01) than CD19TG-2^{+/+} B cells in anti-CD19 mAb-treated mice compared with control mAb-treated mice (**Fig. 8C**). Furthermore, the relative frequency of CD19TG-1^{+/+} B220⁺ B cells to CD19TG-2^{+/+} B220⁺ B cells in anti-CD19 mAb treated mice was significantly lower (p<0.01) than the ratio of CD19TG-1^{+/+} B220⁺ B cells to CD19TG-2^{+/+} B220⁺ B cells in control mAb treated mice. Likewise, the numbers of CD19TG-1^{+/+} and CD19TG-2^{+/+} CFSE-labeled B220⁻ cells in anti-CD19 or control mAb mice were also comparable. Thus, CD19TG-1^{+/+} B cells that express high density CD19 were depleted at a faster rate than CD19TG-2^{+/+} B cells that express CD 19 at a low density.

**Fig. 8D** shows fluorescence intensity of B220⁺ cells stained with CD 19 (thick lines), CD20 (thin lines) or isotype-matched control (CTL, dashed lines) antibodies (5 µg/mL), with antibody staining visualized using isotype-specific, PE-conjugated secondary antibody with flow cytometry analysis. Results represent those obtained in 4 experiments. The results show the relative anti-hCD19 and anti-mCD20 antibody binding densities on spleen B220⁺ B cells from TG-1^{+/-} mice. The density of anti-mCD20 antibody binding was 10-64% as high as anti-CD 19 antibody binding irrespective of which antibody isotype was used for each antibody **(****Fig. 8D****).** Although mCD20 expression was generally lower than hCD19 expression, the levels of hCD19 expression in TG-1+/- mice are still comparable to levels of hCD19 expression found on human B cells **(****Fig. 1B****).** Thus, anti-CD 19 antibodies effectively depleted TG-2^{+/+} B cells that expressed hCD19 at relatively low densities **(****Fig. 1B****),** although high level CD19 expression by TG-1^{+/+} and TG-1^{+/-} B cells obfuscated the relative differences in effectiveness of IgG2a and IgG1 antibodies. Although there is a direct inverse correlation between numbers of B cells and density of hCD 19 expression in TG-1 and TG-2 transgenic mice, density of hCD19 is an important factor contributing to the depletion of B cells. Anti-CD19 antibody levels were saturated when administered at 250 µg/mouse (*see, also,* saturating levels in **Fig. 12**). Thus, free anti-CD19 antibody levels were in excess regardless of B cell number.

### 6.4. EXAMPLE 3: TISSUE B CELL DEPLETION IS FCγR-DEPENDENT

The following assays were used to determine whether B cell depletion by an anti-CD19 antibody was dependent on FcγR expression. Through a process of interbreeding hCD19tg with mice lacking expression of certain FcγR, mice were generated that expressed hCD19 and lacked expression of certain FcγR. Such mice were used in assays to assess the ability of anti-CD19 antibodies to deplete B cells through pathways that involve FcγR expression, *e.g.,* ADCC. Thus, anti-CD19 antibodies identified in these assays can be used to engineer chimeric, human or humanized anti-CD 19 antibodies using the techniques described above in Section 5.1. Such antibodies can in turn be used in the compositions and for use in the methods of the invention for the treatment of B cell malignanices in humans.

The innate immune system mediates B cell depletion following anti-CD20 antibody treatment through FcγR-dependent processes. Mouse effector cells express four different FcγR classes for IgG, the high-affinity FcγRI (CD64), and the low-affinity FcγRII (CD32), FcγRIII (CD16), and FcγRIV molecules. FcγRI, FcγRIII and FcγRIV are hetero-oligomeric complexes in which the respective ligand-binding α chains associate with a common γ chain (FcRγ). FcRγ chain expression is required for FcγR assembly and for FcγR triggering of effector functions, including phagocytosis by macrophages. Since FcRγ^{-/-} mice lack high-affinity FcγRI (CD64) and low-affinity FcγRIII (CD16) and FcγRIV molecules, FcRγ^{-/-} mice expressing hCD19 were used to assess the role of FcyR in tissue B cell depletion following anti-CD19 antibody treatment. **Fig. 9A** shows representative blood and spleen B cell depletion seven days after anti-CD19 or isotype-control antibody treatment of FcRγ^{+/-} or FcRγ^{-/-} littermates. Numbers indicate the percentage of B220⁺ lymphocytes within the indicated gates. **Fig. 9B** shows blood and tissue B cell depletion seven days after antibody treatment of FcRγ^{-/-} littermates on day zero. For blood, the value shown after time zero represents data obtained at 1 hour. Bar graphs represent mean B220⁺ B cell numbers (±SEM) after anti-CD19 (filled bars) or isotype-control (open bars) antibody treatment of mice (≥ 3 mice per group). Significant differences between mean results for anti-CD19 or isotype-control antibody treated mice are indicated; *p<0.05, **p<0.01. The results presented in **Figs. 9A** and **9B** demonstrate that B cell depletion following anti-CD19 antibody treatment is FcRγ-dependent. There were no significant changes in numbers of bone marrow, blood, spleen, lymph node and peritoneal cavity B cells in FcRγ^{-/-} mice following FMC63 antibody treatment when compared with FcRγ^{-/-} littermates treated with a control IgG2a antibody. By contrast, anti-CD19 antibody treatment depleted most B cells in FcRγ^{+/-} littermates. Thus, anti-CD19 antibody treatment primarily depletes blood and tissue B cells through pathways that require FcγRI and FcγRIII expression.

**Fig. 9C** shows representative B cell numbers in monocyte-depleted hCD19TG-1^{+/-} mice. Mice were treated with clodronate-liposomes on day -2, 1 and 4, and given FMC63 (n=9), isotype control (n=6), or CD20 (n=3) mAb (250 µg) on day 0. Mice treated with PBS-liposomes and FMC63 anti-CD19 antibody (n=3) served as controls. Representative blood and spleen B cell depletion is shown 7 days after antibody treatment with the percentage of lymphocytes within the indicated gates indicated.

**Fig. 9D** shows blood and tissue B cell depletion 7 days after antibody treatment as in (C). Bar graphs represent mean B220⁺ B cell numbers (±SEM) after antibody treatment of mice (≥ 3 mice per group). For blood, values indicate numbers of circulating B cells in PBS-treated mice with FMC63 anti-CD19 antibody (closed triangles), or monocyte-depleted mice treated with control antibody (open circles), CD20 antibody (closed squares), or FMC63 anti-CD19 antibody (closed circles). Significant differences between mean results for isotype-control mAb-treated mice and other groups are indicated; *p<0.05, **p<001.

The results presented in **Fig. 9** show B cell depletion following anti-CD19 antibody treatment is FcRγ and monocyte-dependent. Mice rendered macrophage-deficient by treatment with liposome-encapsulated clodronate did not significantly deplete circulating B cells 1 day after FMC63, anti-CD20 (MB20-11) or control anti-CD19 antibody treatment, while FMC63 antibody treatment eliminated circulating B cells in mice treated with PBS-loaded liposomes **(****Figs. 9C-D).** After 4-7 days, circulating B cell numbers were significantly depleted by both FMC63 and anti-CD20 antibody treatment, with anti-CD19 antibody treatment having more dramatic effects on B cell numbers in clodronate-treated mice. Similarly, anti-CD19 and anti-CD20 antibody treatment decreased bone marrow B220⁺ cell numbers by 55% in clodronate-treated mice on day 7 relative to control antibody treated littermates, while anti-CD 19 antibody treatment decreased bone marrow B220⁺ cell numbers by 88% in PBS-treated mice. Anti-CD19 antibody treatment decreased spleen B cell numbers by 52% in clodronate-treated mice on day 7 relative to control antibody treated littermates, while anti-CD20 antibody depleted B cells minimally, and anti-CD 19 antibody, treatment decreased spleen B cell numbers by 89% in PBS-treated mice. Both anti-CD19 and anti-CD20 antibody treatment decreased lymph node B cell numbers by 48-53% in clodronate-treated mice on day seven relative to control antibody treated littermates, while anti-CD19 antibody treatment decreased lymph node B cell numbers by 93% in PBS-treated mice. In blood, spleen and lymph nodes, anti-CD19 antibody treatment was significantly less effective in clodronate-treated mice than in PBS-treated littermates (p<0.01). These findings implicate macrophages as major effector cells for depletion of CD19⁺ and CD20⁺ B cells *in vivo,* and indicate that anti-CD 19 antibody therapy may be more effective than anti-CD20 antibody therapy when monocyte numbers or function are reduced.

### 6.4. EXAMPLE 4: ANTI-CD19 ANTIBODY-INDUCED B CELL DEPLETION IS DURABLE

In order to assess the efficacy and duration of B cell depletion, the hCD19TG mice were administered a single low dose 250 µg injection of anti-CD19 antibody. **Figs. 10A-****10C** demonstrate duration and dose response of B cell depletion following anti-CD19 antibody treatment. **Fig. 10A** shows numbers of blood B220⁺ B cells and Thy-1⁺ T cells following FMC63 or isotype-control antibody treatment of TG-1^{+/-} mice on day zero. Values represent mean (±SEM) results from six mice in each group. The results demonstrate that circulating B cells were depleted for 13 weeks with a gradual recovery of blood-borne B cells over the ensuing 13 weeks. Thy-1⁺ T cell representation was not altered as a result of anti-CD 19 treatment.

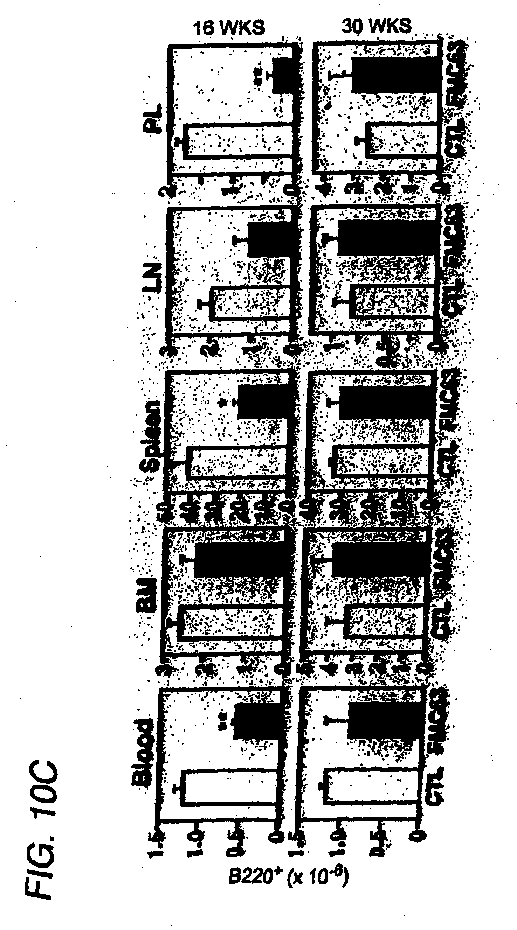

**Figs. 10B-10C** show representative tissue B cell depletion in the mice shown in **Fig. 10A** at 11, 16, and 30 weeks following antibody treatment. Numbers indicate the percentage of B220⁺ lymphocytes within the indicated gates. The results in **Fig. 10B** show that the bone marrow, blood, spleen, lymph node, and peritoneal cavity were essentially devoid of B cells 11 weeks after antibody treatment (significant differences between sample means are indicated; *p<0.05, **p<0.01). After the first appearance of circulating B cells, it took >10 additional weeks for circulating B cell numbers to reach the normal range. By week 16 post-antibody treatment, blood, spleen, LN and PL B cell numbers had begun to recover while the BM B cell compartment was not significantly different from untreated controls. as shown in **Fig. 10C**. By week 30, all tissues were repopulated with B cells at levels comparable to those in normal controls.

**Fig. 10D** shows anti-CD19 antibody dose responses for blood, bone marrow and spleen B cell depletion. Mice were treated with anti-CD19 antibodies on day zero with tissue B cells representation assessed on day seven. Results represent those obtained with three mice in each group for each antibody dose. Control antibody doses were 250 µg. Significant differences between sample means are indicated; *p<0.05, **p<0.01. A single FMC63 antibody dose as low as 2 µg/mouse depleted significant numbers of circulating B cells, while 10 µg the HB12b antibody was required to significantly reduce circulating B cell numbers (**Fig**. **10D**). Significant depletion of bone marrow and spleen B cells by day seven required 5-fold higher antibody doses of 10-50 µg/mouse. Thus, CD19 antibody treatment at relatively low doses can deplete the majority of circulating and tissue B cells for significant periods of time.

### 6.5.1. CD19 PERSISTS ON THE B CELL SURFACE AFTER ADMINISTRATION OF ANTI-CD19 ANTIBODY

Whether CD 19 internalization influenced B cell depletion *in vivo* was assessed by comparing cell-surface CD 19 expression following HB12a, HB12b and FMC63 antibody treatment (250 µg).

**Figs. 11A-11C** show cell surface CD19 expression and B cell clearance in TG-1^{+/-} mice treated with HB12a (**Fig. 11A**), HB12b (**Fig. 11B**)**,** FMC63 (**Fig. 11C**) or isotype-matched control antibody (250 µg) *in vivo.* At time zero (prior to anti-CD19 administration), and at 1, 4, and 24 hours post-antibody administration, spleen B cells were harvested and assessed for CD19 (thick line) and control (thin line) antibody binding by treating cells with isotype-specific secondary antibody *in vitro* with flow cytometry analysis. Isolated B cells were also treated *in vitro* with saturating concentrations of each CD19 antibody plus isotype-specifc secondary antibody *in vitro* with flow cytometry analysis to visualize total cell surface CD19 expression. Each time point represents results with one mouse. The results presented in **F**igs. 1**1A-11C** demonstrate that cell surface CD 19 is not eliminated from the cell surface following antibody binding *in vivo* and show that the majority of spleen B cells expressed uniform high levels of cell surface hCD19 for up to 24 hours after antibody treatment although a subset of B cells expressed reduced levels of hCD19 at 1 hour following FMC63 antibody treatment (**Fig**. **11C**). The results shown in **Figs**. 1**1A-11C** also demonstrate that the amount of CD19 on the surface of B cells is constant, indicating that the capability of the B cells to mediate ADCC is maintained.

The results demonstrate that CD19 surprisingly exhibited lower levels of internalization than expected following administration of anti-CD 19 antibodies. In particular, the results demonstrate that CD 19 unexpectedly persists on the cell surface following binding of an anti-CD 19 antibody, thus, the B cell remains accessible to the ADCC activity. These results demonstrate, in part, why the anti-CD 19 antibodies and treatment regimens of the invention are efficacious in treating B cell malignancies.

**Figs. 12A-12C** document the extent of B cell depletion and the ability of anti-hCD19 antibodies to bind hCD19 and thus inhibit the binding of other anti-hCD19 antibodies. The results in **Fig. 12A** demonstrate that a single administration of FMC63 (250 µg) to TG-1^{+/-} mice results in significant depletion of both blood and spleen B cells within 1 hour of antibody administration. In this experiment, blood and spleen cells were harvested and assessed for B cell frequencies prior to anti-CD19 antibody administration or at various times thereafter (1, 4, or 24 hours). Blood samples were stained with anti-Thy1.2 and anti-B220 to identify B cells in the lower right quadrant. Spleen cells were stained with anti-IgM and anti-B220 antibodies to identify B cells within the indicated gate. Each time point represents results with one mouse. Unexpectedly, blood B cells were cleared more rapidly than splenic B cells.

The B cell depletion described in **Fig. 12A** suggested that the administered antibody rapidly saturated available antibody-binding sites on hCD19 within hour of administration. To confirm this observation, mice were treated with either FMC63 (hCD 19 binding antibody) or isotype-control antibody. At various time thereafter blood and spleen B cells were stained with the fluorochrome-conjugated B4 antibody to identify unoccupied antibody binding sites on the surface of mCD19⁺ or mCD20⁺ B cells. The frequencies of cells within the upper and lower-right quadrants are indicated. Each time point represents results obtained from one mouse. The results indicate FMC63 treatment resulted in a progressive depletion of hCD 19 bearing cells over the course of the experiment with blood B cells being depleted more rapidly than spleen. Those B cells remaining at each time point could be identified by their expression of mCD 19 or mCD20, but were not stained by B4 suggesting that the administered FMC63 was bound to the remaining B cells. These finding confirm the ability of FMC63 to bind and deplete B cells in vivo. Moreover, FMC63 prevents B4 binding suggesting that these antibodies recognize overlapping epitopes on hCD19. The results in **Fig. 12C** confirm that HB12b antibody treatment (250 µg) also saturates antibody-binding sites on hCD 19 within 1 hour of administration and results in the depleting of hCD19 positive B cells. Unexpectedly, the HB12b antibody did not completely inhibit binding of the B4 antibody suggesting that unlike FMC63, HB12b recognizes an epitope on hCD19 that is distinct from that recognized by B4. The results shown in **Figs. 12B-12C** demonstrate that most anti-CD19 antibodies inhibit the binding of most other anti-CD19 antibodies, indicating that most anti-CD19 antibodies bind to similar, the same, or overlapping regions or epitopes on the CD19 protein. Alternatively, these observations may also result from the relatively small size of the CD 19 extracellular domain compared with the size of antibody molecules.

### 6.6. EXAMPLE 5: ANTI-CD19 ANTIBODY TREATMENT ABROGATES HUMORAL IMMUNITY AND AUTOIMMUNITY

The assays described in this example can be used to determine whether an anti-CD 19 antibody is capable of eliminating or attenuating immune responses. Anti-CD19 antibodies identified in these assays can be used to engineer chimeric, human or humanized anti-CD19 antibodies using the techniques described above in Section 5.1. Such antibodies can in turn be used in the compositions and for use in the methods of the invention for the treatment of B cell malignancies in humans.

The effect of anti-CD 19 antibody-induced B cell depletion on serum antibody levels was assessed by giving hCD19TG^{+/-} mice a single injection of anti-CD19 antibody. **Fig. 13A** shows CD 19 antibody treatment reduces serum immunoglobulin levels in TG-1^{+/-} mice. Two-month-old littermates were treated with a single injection of FMC63 (closed circles) or control (open circles) antibody (250 µg) on day 0. Antibody levels were determined by ELISA, with mean values (±SEM) shown for each group of ≥ 5 mice. Differences between CD19 or control mAb-treated mice were significant; *p<0.05, **p<0.01. The results show that after 1 to 2 weeks, serum IgM, IgG2b, IgG3, and IgA antibody levels were significantly reduced, and remained reduced for at least 10 weeks (**Fig**. **13A**). IgG1 and IgG2a serum levels were significantly below normal at 6 and 4 weeks posttreatment.

Since hCD19TG^{+/-} mice produce detectable autoantibodies after 2 mos of age (Sato et al., J. Immunol, 157:4371(1996)), serum autoantibody binding to ssDNA, dsDNA and histones was assessed. **Fig. 13B** shows anti-CD19 antibody treatment reduces autoantibody anti-dsDNA, anti-ssDNA and anti-histone autoantibody levels after anti-CD19 antibody treatment. The results show that anti-CD19 antibody treatment significantly reduced serum IgM autoantibody levels after 2 weeks and prevented the generation of isotype-switched IgG autoantibodies for up to 10 weeks (**Fig**. **13B**). Thus, B cell depletion substantially reduced acute and long-term antibody responses and attenuated class-switching of normal and pathogenic immune responses.

The influence of B cell depletion on T cell-independent type 1 (TI-1) and type 2 (TI-2) antibody responses was assessed by immunizing hCD 19TG^{+/-} mice with TNP-LPS or DNP-Ficoll (at day zero), 7 days after anti-CD19 antibody (FMC63) or control antibody treatment. Significant hapten-specific IgM, IgG, and IgA antibody responses were not observed in anti-CD 19 antibody-treated mice immunized with either antigen (**Figs. 14A** and **14B**). Antibody responses to the T cell-dependent (TD) Ag, DNP-KLH, were also assessed using mice treated with anti-CD19 antibody 7 days before immunization (**Fig. 14B**). **Fig**. **14C** shows that DNP-KLH immunized mice treated with anti-CD19 antibody showed reduced humoral immunity. Littermates were treated with FMC63 (closed circles) or control (open circles) antibody (250 µg) seven days before primary immunizations on day zero, with serum obtained on the indicated day. For DNP-KLH immunizations, all mice were challenged with 100 µg of DNP-KLH on day 21. All values are mean (±SEM) ELISA OD units obtained using sera from five mice of each group. Differences between anti-CD19 or control antibody-treated mice were significant; *p<0.05, **p<0.01. The results show that control antibody-treated littermates generated primary IgM antibody responses 7 days after DNP-KLH immunization and secondary responses after antigen challenge on day 21 (**Fig. 14C**). However, significant hapten-specific IgM, IgG or IgA antibody responses were not detected in CD 19 mAb-treated mice immunized or re-challenged with antigen. To assess the effect of B cell depletion on secondary antibody responses, mice were also immunized with DNP-KLH and treated with anti-CD19 antibody 14 days later (arrows) (**Fig. 14D**). By day 21, serum IgM, IgG, and IgA anti-DNP antibody responses had decreased in CD 19 mAb-treated mice to levels below those of immunized mice treated with control mAb. However, re-challenge of control mAb-treated mice with DNP-KLH on day 21 induced significant secondary antibody responses, while CD 19 mAb-treated mice did not produce anti-DNP antibodies after DNP-KLH-rechallenge. Thus, CD19 mAb-induced B cell depletion substantially reduced both primary and secondary antibody responses and prevented class-switching during humoral immune responses.

### 6.7. EXAMPLE 6: ANTI-CD19 ANTIBODY TREATMENT IN CONJUNCTION WITH ANTI-CD20 ANTIBODY TREATMENT

The assay described herein can be used to determine whether other combination or conjugate therapies, e.g., anti-CD19 antibodies in combination with chemotherapy, toxin therapy or radiotherapy, have beneficial effects, such as an additive or more that additive depletion of B cells. The results of combination therapies tested in animal models can be correlated to humans by means well-known in the art.

Anti-CD20 antibodies are effective in depleting human and mouse B cells *in vivo.* Therefore, the benefit of simultaneous treatment with anti-CD19 (FMC63) and anti-CD20 (MB20-11) antibodies was assessed to determine whether this enhanced B cell deletion Mice were treated with suboptimal 2 µg doses of each antibody individually, or a combination of both antibodies at 1 µg, or with combined 2 µg doses. **Fig. 15** shows the results of TG-1^{+/-} mice treated with control (250 µg), FMC63 (CD19, 2 µg), MB20-11 (CD20, 2 µg), FMC63+MB20-11 (1 µg each), or FMC63+MB20-11 (2 µg each) antibodies on day zero. Blood B cell numbers were measured at time zero, one hour, and on days one, four and seven. Tissue B cell numbers were determined on day seven. Values represent means (±SEM) from groups of three mice. The results shown in **Fig. 15** demonstrate that simultaneous anti-CD 19 and anti-CD20 antibody treatments are beneficial. B cell depletion in mice treated with a combination of both antibodies at 1 µg was intermediate or similar to depletion observed following treatment of mice with 2 µg of each individual antibody (**Fig. 15**). However, the simultaneous treatment of mice with both antibodies at 2 µg lead to significantly more B cell depletion than was observed with either antibody alone. Thus, combined anti-CD 19 and anti-CD20 antibody therapies had beneficial effects that enhanced B cell depletion. This likely results from the accumulation of more therapeutically effective antibody molecules on the surface of individual B cells.

### 6.8. EXAMPLE 7: SUBCUTANEOUS (S.C.) ANTI-CD19 ANTIBODY ADMINISTRATION IS THERAPEUTICALLY EFFECTIVE

The assay described herein can be used to determine whether a subcutaneous route of administration of an anti-CD19 antibody can effectively deplete B cells. The results of the efficacy of different delivery routes tested in animal models can be correlated to humans by means well-known in the art.

Since anti-CD 19 antibody given i.v. effectively depletes circulating and tissue B cells, it was assessed whether anti-CD19 antibody given s.c. or i.p. depleted B cells to an equivalent extent. Wild-type mice were treated with the FMC63 antibody at 250 µg either subcutaneous (s.c.), intraperitoneal (i.p.) or i.v. Values represent mean (±SEM) blood (per ml), bone marrow, spleen, lymph node, and peritoneal cavity B220+ B cell numbers on day seven (n≥3) as assessed by flow cytometry. Significant differences between mean results for each group of mice are indicated; *p<0.05, **p<0.01 in comparison to the control. The results in Fig. 16 demonstrate that subcutaneous (s.c.), intraperitoneal (i.p.) and i.v. administration of CD 19 antibody effectively depletes circulating and tissue B cells in vivo. The vast majority of circulating and tissue B cells were depleted in mice given anti-CD19 antibodies as 250 µg doses either i.v., i.p., or s.c. (Fig. 16). Unexpectedly, giving anti-CD19 antibody i.p. did not deplete peritoneal B cells significantly better than i.v: treatment. Accordingly, an anti-CD19 antibody can be used to effectively deplete both circulating and tissue B cells when given as ≤ 64 mg s.c. injections. Since anti-CD19 antibodies are effective down to 10 µg doses i.v. (Fig. 10D) even lower s.c. antibody doses are likely to be effective.

### 6.9. EXAMPLE 8: ANTI-CD19 ANTIBODY TREATMENT ABROGATES TUMOR GROWTH IN VIVO

Burkitt's lymphoma, a B cell malignancy in humans, is characterized by translocations of the *c-myc* proto-oncogene to Ig gene promoter regions, leading to aberrant c-Myc over-expression. Similarly, Eµ-cMyc transgenic (cMycTG) mice, in which the c-*myc* proto-oncogene is under the control of the Ig heavy chain enhancer, develop aggressive B cell-derived lymphomas at an early age, have about 90% mortality rate by 20 weeks of age, and have a median age of survival at about 12 weeks (Harris et al., J. Exp. Med. 167:353 (1988) and Adams et al., Nature 318:533(1985)). Tumors from c-MycTG mice are not restricted to a specific B cell developmental stage, but predominantly present with Ig gene rearrangements and phenotypes characteristic of pre-B or immature B cells (Adams et al., Nature 318:533(1985)). To assess the efficacy of CD19-directed immunotherapy *in vivo,* hCD19TG-1^{+/+} and cMycTG mice were crossed to generate hCD19TG-1^{+/-} cMycTG^{+/-} mice that developed aggressive B cell-derived lymphomas at an early age. Tumor cells derived from one mouse were isolated, expanded *in vitro,* and characterized phenotypically to be hCD19⁺ and mouse CD19⁺ CD20⁻ CD43⁻ IgM⁺ IgD⁻ B220⁺ lymphoblasts, which are typical of the pre-B/immature B cell tumors that develop in c-mycTG^{+/-} mice (Harris et al., J. Exp. Med. 167:353 (1988) and Adams et al., Nature 318:533 (1985)). Tumor cells (10⁵) from hCD19TG-1^{+/-} c-mycTG⁺ mice were transplanted i.v. into 20 Rag^{-/-} mice on day 0. Equal numbers of randomly selected mice were treated with FMC63 (filled circles) or control (open circles) antibody (250 µg) on days 1 and 7. Fig. 17A shows the numbers of circulating tumor cells (±SEM) quantified by flow cytometry over a 6 week period and Fig. 17B shows mouse percent survival over a 7 week period. Each value indicates the percentage of viable mice on each day they were examined. The results in Fig. 17 demonstrate that anti-CD19 antibody treatment prevents hCD19⁺ lymphoma growth *in vivo.* Transplantation of these tumor cells into twenty Rag^{-/-} mice resulted in the appearance of circulating mouse CD19⁺ and B220⁺ lymphoblasts by 2 weeks in ten randomly selected recipients that were treated with a control mAb, with death by 3.5 weeks. By contrast, treating ten mice with anti-CD19 antibody (day 1 and 7) following tumor transplantation prevented the appearance of circulating tumor cells in all 10 recipients for up to 7 weeks. One anti-CD 19 antibody-treated mouse died during blood harvest, but never displayed circulating tumor cells. Thus, anti-CD 19 antibody treatment may offer an effective therapy for treating patients with B cell lineage malignancies, especially those with tumors that do not express CD20 or express CD20 at low levels.

## Claims

1. A pharmaceutical composition comprising a monoclonal chimerized, or humanized anti CD 19 antibody that (a) is of the IgG1 or IgG3 human isotype, or (b) mediates human antibody-dependent cellular cytotoxicity (ADCC), in a pharmaceutically acceptable carrier, wherein the antibody comprises the sequences as set out as amino acids 33 to 37, amino acids 51 to 68 and amino acids 101 to 115 of SEQ ID NO:2 and the sequences as set out as amino acids 43 to 58, amino acids 74 to 80 and amino acids 113 to 121 of SEQ ID NO:16, or the sequences as set out as amino acids 33 to 37, amino acids 51 to 68 and amino acids 101 to 114 of SEQ ID NO:4 and the sequences as set out as amino acids 44 to 58, amino acids 74 to 80 and amino acids 113 to 121 of SEQ ID NO:18, wherein one amino acid substitution is introduced into one or more of said sequences and wherein the antibody is able to deplete CD 19 expressing B cells from the bone marrow.

2. The pharmaceutical composition of claim 1, wherein a therapeutically effective amount of the monoclonal chimerized anti-CD19 antibody of the IgG1 or IgG3 human isotype is less than about 1 mg/kg of patient body weight.

3. The pharmaceutical composition of claim 1, wherein a therapeutically effective amount of a monoclonal chimerized anti-CD19 antibody of the IgG1 or IgG3 human isotype is greater than about 2 mg/kg of patient body weight.

4. The composition of claim 1, wherein the anti-CD 19 antibody that mediates ADCC is of the IgG1, or IgG3 human isotype.

5. The pharmaceutical composition of any preceding claim, wherein the anti-CD19 antibody has a half-life of at least 4 to 7 days.

6. The pharmaceutical composition of any preceding claim, wherein the composition comprises a dose of 1500 mg/m2 or less of the anti-CD 19 antibody and wherein the anti-CD 19 antibody is administered by a parenteral, intraperitoneal, intramuscular, intravenous or subcutaneous route.

7. The pharmaceutical composition of claim 6, wherein the composition comprises a dose of 375 mg/m2 or less of the anti-CD19 antibody and wherein the anti-CD19 antibody is administered by a parenteral, intraperitoneal, intramuscular, intravenous or subcutaneous route.

8. The pharmaceutical composition of claim 7, wherein the composition comprises a dose of 1.5 mg/m2 or less of the anti-CD19 antibody and wherein the anti-CD19 antibody is administered by a parenteral, intraperitoneal, intramuscular, intravenous or subcutaneous route.

9. The pharmaceutical composition of claim 8, wherein the composition comprise a dose of 0.5 ug/m2 or less of the anti-CD19 antibody and wherein the anti-CD19 antibody is administered by a parenteral, intraperitoneal, intramuscular, intravenous or subcutaneous route.

10. The pharmaceutical composition of any preceding claim, wherein the anti-CD19 antibody is detectably labelled, is a naked antibody, is conjugated to a therapeutic compound, is conjugated to a cytotoxic agent, or is conjugated to a diagnostic agent.

11. The pharmaceutical composition of any preceding claim, wherein the anti-CD19 antibody is bispecific.

12. The pharmaceutical composition of claim 11, wherein the bispecific anti-CD19 antibody has specificity for binding effector cells.

13. The pharmaceutical composition of any preceding claim, wherein the ADCC function of the anti-CD 19 antibody is assessed by measuring the ability of the anti-CD19 antibody to mediate target cell lysis by effector cells *in vitro.*

14. The pharmaceutical composition of any preceding claim, wherein the anti-CD19 antibody comprises a heavy chain consisting of a sequence having at least 90% identity to SEQ ID NO:2 or SEQ ID NO:4.

15. The pharmaceutical composition of any preceding claim, wherein the anti-CD 19 antibody comprises the light chain consisting of a sequence having at least 95% identity to SEQ ID NO:16 or SEQ ID NO:18.

16. A monoclonal humanized anti-CD19 antibody that (a) is of the IgG1 or IgG3 human isotype, or (b) mediates human antibody-dependent cellular cytotoxicity (ADCC), wherein the antibody comprises the sequences as set out as amino acids 33 to 37, 51 to 68 and 101 to 115 of SEQ ID NO:2 and the sequences as set out as amino acids 43 to 58, 74 to 80 and 113 to 121 of SEQ ID NO:16, or the sequences as set out as amino acids 33 to 37, 51 to 68 and 101 to 114 of SEQ ID NO:4 and the sequences as set out as amino acids 44 to 58,74 to 80 and 113 to 121 of SEQ ID NO:18, wherein one amino acid substitution is introduced into one or more of said sequences, for use in treating a B cell malignancy in a human patient, wherein the antibody is able to deplete CD 19 expressing B cells from the bone marrow.

17. The antibody for use according to claim 16, wherein the anti-CD19 antibody comprises a heavy chain consisting of a sequence having at least 90% identity to SEQ ID NO:2 or SEQ ID NO:4.

18. The antibody for use according to claim 16 or 17, wherein the antibody comprises the light chain consisting of a sequence having at least 95% identity to SEQ ID NO:16 or SEQ ID NO:18.

19. The antibody for use according to any one of claims 16 to 18, wherein the antibody is able to deplete circulating B cells.

20. The antibody for use according to any one of claims 16 to 19, wherein the B cell malignancy is acute lymphoblastic leukaemia, mantle cell lymphoma, pre-B cell acute lymphoblastic leukaemia, or precursor B cell lymphoblastic lymphoma.

21. The antibody for use according to any one of claims 16 to 20, wherein the anti-CD 19 antibody that mediates ADCC is of the IgG1 or IgG3 human isotype.

22. The antibody for use according to any-one of claims 16 to 20, wherein the B cell malignancy is treated prior to the administration of the anti-CD 19 antibody.

23. The antibody for use according to any one of claims 16 to 20, wherein the B cell malignancy is treated with a therapy other than an anti-CD 19 antibody therapy subsequent to the administration of the anti-CD 19 antibody.

24. The antibody for use according to claim 22 or claim 23, wherein the treatment for the malignancy is chemotherapy, radioimmunotherapy, toxin therapy, prodrug-activating enzyme therapy, antibody therapy, monocyte or macrophage enhancing therapy, immunoregulatory therapy, tumor neovasculature satin therapy, calicheamicin-therapy; surgical-therapy,-or any combination thereof.

25. A monoclonal anti CD19 antibody that mediates human antibody-dependent cellular cytotoxicity (ADCC) comprising the sequences as set out as amino acids 33 to 37, amino acids 51 to 68 and amino acids 101 to 115 of SEQ ID NO:2 and the sequences as set out as amino acids 43 to 58, amino acids 74 to 80 and amino acids 113 to 121 of SEQ ID NO:16, or the sequences as set out as amino acids 33 to 37, amino acids 51 to 68 and amino acids 101 to 114 of SEQ ID NO:4 and the sequences as set out as amino acids 44 to 58, amino acids 74 to 80 and amino acids 113 to 121 of SEQ ID NO:18, wherein one amino acid substitution is introduced into one or more of said sequences and wherein the antibody is able to deplete CD 19 expressing B cells from the bone marrow for use in a regimen for (a) treating an early stage disease resulting from a B cell malignancy in a human patient, or (b) treating a B cell malignancy in a human subject wherein the human subject has not previously received treatment for the malignancy, the B cell malignancy is CD 19 positive or the human has a monocyte count of at least 1 per dl, wherein the regimen administered depletes circulating B cells.

26. The antibody for use according to claim 25, wherein the antibody comprises a heavy chain consisting of a sequence having at least 90% identity to SEQ ID NO:2 or SEQ ID NO:4.

27. The antibody for use according to any one of claims 25 to 26, wherein the anti-CD19 antibody comprises the light chain consisting of a sequence having at least 95% identity to SEQ ID NO:16 or SEQ ID NO:18.

28. The antibody for use according to any one of claims 25 to 27, wherein a therapy other than an anti-CD 19 antibody therapy is subsequently administered to the human.

29. The antibody for use according to claim 28, wherein the therapy is chemotherapy, radiotherapy, toxin based therapy, radiochemical based therapy or surgical therapy.

30. The antibody for use according to any one of claims 25 to 29, wherein the regimen comprises the administration of the antibody as a single therapeutic agent, in combination with another therapeutic agent, or in combination with an agent that reduces toxic side effects.

31. The antibody for use according to any one of claims 25 to 30, wherein the regimen comprises the repeat administration of the antibody.

32. The antibody for use according to any one of claims 25 to 30, wherein the anti-CD 19 antibody has a half-life that is at least 4 to 7 days.

33. The antibody for use according to any one of claims 25 to 32, wherein the anti-CD 19 antibody is a human IgG1 or IgG3 isotype antibody.

34. The antibody for use according to any one of claims 25 to 33, wherein the anti-CD19 antibody is a humanized antibody.

35. The antibody for use according to any one of claims 16 to 34, wherein the B cell malignancy is a B cell subtype non-Hodgkin's lymphoma (NHL) including low grade/follicular NHL, small lymphocytic (SL) NHL, intermediate grade/follicularNHL, intermediate grade diffuse NHL, high grade immunoblastic NHL, high grade lymphoblastic NHL, high grade small non-cleaved cell NHL and bulky disease NHL; Burkitt's lymphoma; multiple myeloma; pre-B acute lymphoblastic leukemia and other malignancies that derive from early B cell precursors; common acute lymphocytic leukemia; chronic lymphocytic leukemia; hairy cell leukemia; Null-acute lymphoblastic leukemia; Waldenstrom's Macroglobulinemia; and pro-lymphocytic leukemia; light chain disease; plasmacytoma; osteosclerotic myeloma; plasma cell leukemia; monoclonal gammopathy of undetermined significance (MGUS); smoldering multiple myeloma (SMM); indolent multiple myeloma (IMM); or Hodgkin's lymphoma.

36. The antibody for use according to any one of claims 25 to 35, wherein the regimen further comprises administering a compound that enhances monocyte or macrophage function.

37. The antibody for use according to claim 28, wherein the human is immunocompromised.

38. The antibody for use according to any one of claims 16 to 35, wherein the anti-CD 19 antibody is administered by a parenteral, intraperitoneal, or intramuscular route.

39. The antibody for use according to any one of claims 16 to 35, wherein the anti-CD 19 antibody is administered by an intravenous or subcutaneous route.

40. The antibody for use according to claim 39, wherein the anti-CD19 antibody is administered by a subcutaneous route in a dose of 37.5 mg/m2 or less.

41. The antibody for use according to claim 40, wherein the anri-CD19 antibody is administered in a dose of 1.5 mg/m2 or less.

42. The antibody for use according to any one of claims 25 to 41, wherein the regimen comprises more than one administration of the anti-CD19 antibody to the human.

43. The antibody for use according to any one of claims 25 to 42, wherein the regimen further comprises administering an anti-CD20 antibody, ayanti-CD22 antibody, an anti-CD52 antibody, or any combination thereof.

## Patentansprüche

1. Pharmazeutische Zusammensetzung, umfassend einen monoklonalen chimärisierten oder humanisierten anti-CD19-Antikörper, der (a) den menschlichen Isotyp IgG1 oder IgG3 aufweist, oder der (b) menschliche antikörperabhängige zelluläre Zytotoxizität (ADCC) vermittelt, in einem pharmazeutisch unbedenklichen Träger, wobei der Antikörper die Sequenzen, die als Aminosäuren 33 bis 37, Aminosäuren 51 bis 68 und Aminosäuren 101 bis 115 von SEQ ID NO: 2 angegeben sind und die Sequenzen, die als Aminosäuren 43 bis 58, Aminosäuren 74 bis 80 und Aminosäuren 113 bis 121 von SEQ ID NO: 16 angegeben sind, oder die Sequenzen, die als Aminosäuren 33 bis 37, Aminosäuren 51 bis 68 und Aminosäuren 101 bis 114 von SEQ ID NO: 4 angegeben sind, und die Sequenzen, die als Aminosäuren 44 bis 58, Aminosäuren 74 bis 80 und Aminosäuren 113 bis 121 von SEQ ID NO: 18 angegeben sind, umfasst, wobei eine Aminosäuresubstitution in eine oder mehrere der Sequenzen eingeführt ist und wobei der Antikörper fähig ist, CD19-exprimierende B-Zellen aus dem Knochenmark abzureichern.

2. Pharmazeutische Zusammensetzung nach Anspruch 1, wobei eine therapeutisch wirksame Menge des monoklonalen chimärisierten anti-CD19-Antikörpers des menschlichen Isotyps IgG1 oder IgG3 weniger als ungefähr 1 mg/kg des Körpergewichts des Patienten beträgt.

3. Pharmazeutische Zusammensetzung nach Anspruch 1, wobei eine therapeutisch wirksame Menge des monoklonalen chimärisierten anti-CD19-Antikörpers des menschlichen Isotyps IgG1 oder IgG3 mehr als ungefähr 2 mg/kg des Körpergewichts des Patienten beträgt.

4. Zusammensetzung nach Anspruch 1, wobei der anti-CD19-Antikörper, der die ADCC vermittelt, den menschlichen Isotyp IgG1 oder IgG3 aufweist.

5. Pharmazeutische Zusammensetzung nach einem vorhergehenden Anspruch, wobei der anti-CD19-Antikörper eine Halbwertszeit von mindestens 4 bis 7 Tagen aufweist.

6. Pharmazeutische Zusammensetzung nach einem vorhergehenden Anspruch, wobei die Zusammensetzung eine Dosis von 1500 mg/m2 oder weniger des anti-CD19-Antikörpers umfasst und wobei der anti-CD19-Antikörper auf einem parenteralen, intraperitonealen, intramuskulären, intravenösen oder subkutanen Weg verabreicht wird.

7. Pharmazeutische Zusammensetzung nach Anspruch 6, wobei die Zusammensetzung eine Dosis von 375 mg/m2 oder weniger des anti-CD19-Antikörpers umfasst und wobei der anti-CD19-Antikörper auf einem parenteralen, intraperitonealen, intramuskulären, intravenösen oder subkutanen Weg verabreicht wird.

8. Pharmazeutische Zusammensetzung nach Anspruch 7, wobei die Zusammensetzung eine Dosis von 1,5 mg/m2 oder weniger des anti-CD19-Antikörpers umfasst und wobei der anti-CD19-Antikörper auf einem parenteralen, intraperitonealen, intramuskulären, intravenösen oder subkutanen Weg verabreicht wird.

9. Pharmazeutische Zusammensetzung nach Anspruch 8, wobei die Zusammensetzung eine Dosis von 0,5 ug/m2 oder weniger des anti-CD19-Antikörpers umfasst und wobei der anti-CD19-Antikörper auf einem parenteralen, intraperitonealen, intramuskulären, intravenösen oder subkutanen Weg verabreicht wird.

10. Pharmazeutische Zusammensetzung nach einem vorhergehenden Anspruch, wobei der anti-CD19-Antikörper nachweisbar markiert ist, ein nackter Antikörper ist, mit einer therapeutischen Verbindung konjugiert ist, mit einem zytotoxischen Agens konjugiert ist oder mit einem diagnostischen Agens konjugiert ist.

11. Pharmazeutische Zusammensetzung nach einem vorhergehenden Anspruch, wobei der anti-CD19-Antikörper bispezifisch ist.

12. Pharmazeutische Zusammensetzung nach Anspruch 11, wobei der bispezifische anti-CD19-Antikörper eine Spezifität für die Bindung von Effektorzellen aufweist.

13. Pharmazeutische Zusammensetzung nach einem vorhergehenden Anspruch, wobei die ADCC-Funktion des anti-CD19-Antikörpers dadurch bestimmt wird, dass man die Fähigkeit des anti-CD19-Antikörpers, die Zielzell-Lyse durch Effektorzellen in vitro zu vermitteln, misst.

14. Pharmazeutische Zusammensetzung nach einem vorhergehenden Anspruch, wobei der anti-CD19-Antikörper eine schwere Kette bestehend aus einer Sequenz mit mindestens 90% Identität zu SEQ ID NO: 2 oder SEQ ID NO: 4 umfasst.

15. Pharmazeutische Zusammensetzung nach einem vorhergehenden Anspruch, wobei der anti-CD19-Antikörper die leichte Kette bestehend aus einer Sequenz mit mindestens 95% Identität zu SEQ ID NO: 16 oder SEQ ID NO: 18 umfasst.

16. Monoklonaler humanisierter anti-CD19-Antikörper, der (a) den menschlichen Isotyp IgG1 oder IgG3 aufweist, oder der (b) menschliche antikörperabhängige zelluläre Zytotoxizität (ADCC) vermittelt, wobei der Antikörper die Sequenzen, die als Aminosäuren 33 bis 37, 51 bis 68 und 101 bis 115 von SEQ ID NO: 2 angegeben sind und die Sequenzen, die als Aminosäuren 43 bis 58, 74 bis 80 und 113 bis 121 von SEQ ID NO: 16 angegeben sind, oder die Sequenzen, die als Aminosäuren 33 bis 37, 51 bis 68 und 101 bis 114 von SEQ ID NO: 4 angegeben sind und die Sequenzen, die als Aminosäuren 44 bis 58, 74 bis 80 und 113 bis 121 von SEQ ID NO: 18 angegeben sind, umfassen, wobei eine Aminosäuresubstitution in eine oder mehrere der Sequenzen eingeführt ist und wobei der Antikörper fähig ist, CD19-exprimierende B-Zellen aus dem Knochenmark abzureichern.

17. Antikörper zur Verwendung nach Anspruch 16, wobei der anti-CD19-Antikörper eine schwere Kette bestehend aus einer Sequenz mit mindestens 90% Identität zu SEQ ID NO: 2 oder SEQ ID NO: 4 umfasst.

18. Antikörper zur Verwendung nach einem der Ansprüche 16 bis 17, wobei der Antikörper die leichte Kette bestehend aus einer Sequenz mit mindestens 95% Identität zu SEQ ID NO: 16 oder SEQ ID NO: 18 umfasst.

19. Antikörper zur Verwendung nach einem der Ansprüche 16 bis 18, wobei der Antikörper fähig ist, zirkulierende B-Zellen abzureichern.

20. Antikörper zur Verwendung nach einem der Ansprüche 16 bis 19, wobei es sich bei der B-Zell-Malignität um akute Lymphoblastenleukämie, Mantelzell-Lymphom, prä-B-Zell akute Lymphoblastenleukämie oder Vorläufer-B-Zell lymphoblastisches Lymphom handelt.

21. Antikörper zur Verwendung nach einem der Ansprüche 16 bis 20, wobei der anti-CD19-Antikörper, der die ADCC vermittelt, den menschlichen Isotyp IgG1 oder IgG3 aufweist.

22. Antikörper zur Verwendung nach einem der Ansprüche 16 bis 20, wobei die B-Zell-Malignität vor der Verabreichung des anti-CD19-Antikörpers behandelt wird.

23. Antikörper zur Verwendung nach einem der Ansprüche 16 bis 20, wobei die B-Zell-Malignität anschließend an die Verabreichung des anti-CD19-Antikörpers mit einer Therapie behandelt wird, bei der es sich nicht um eine anti-CD19-Antikörpertherapie handelt.

24. Antikörper zur Verwendung nach Anspruch 22 oder Anspruch 23, wobei es sich bei der Behandlung für die Malignität um Chemotherapie, Radioimmuntherapie, Toxintherapie, Therapie mit einem Prodrug-aktivierenden Enzym, Antikörpertherapie, eine Monozyten oder Makrophagen verstärkende Therapie, immunregulatorische Therapie, Statintherapie der Gefäßneubildung von Tumoren, Calicheamicintherapie, chirurgische Therapie oder eine beliebige Kombination davon handelt.

25. Monoklonaler anti-CD19-Antikörper, der die menschliche antikörperabhängige zelluläre Zytotoxizität (ADCC) vermittelt, umfassend die Sequenzen, die als Aminosäuren 33 bis 37, Aminosäuren 51 bis 68 und Aminosäuren 101 bis 115 von SEQ ID NO: 2 angegeben sind und die Sequenzen, die als Aminosäuren 43 bis 58, Aminosäuren 74 bis 80 und Aminosäuren 113 bis 121 von SEQ ID NO: 16 angegeben sind, oder die Sequenzen, die als Aminosäuren 33 bis 37, Aminosäuren 51 bis 68 und Aminosäuren 101 bis 114 von SEQ ID NO: 4 angegeben sind, und die Sequenzen, die als Aminosäuren 44 bis 58, Aminosäuren 74 bis 80 und Aminosäuren 113 bis 121 von SEQ ID NO: 18 angegeben sind, umfasst, wobei eine Aminosäuresubstitution in eine oder mehrere der Sequenzen eingeführt ist und wobei der Antikörper fähig ist, CD19-exprimierende B-Zellen aus dem Knochenmark abzureichern zur Verwendung in einem Schema für (a) die Behandlung einer Krankheit im Frühstadium, die das Ergebnis einer B-Zell-Malignität in einem menschlichen Patienten ist, oder (b) die Behandlung einer B-Zell-Malignität in einem menschlichen Individuum, wobei das menschliche Individuum noch nicht für die Malignität behandelt worden ist, die B-Zell-Malignität CD19-positiv ist oder der Mensch eine Monozytenzahl von mindestens 1 pro dl aufweist, wobei das verabreichte Schema zirkulierende B-Zellen abreichert.

26. Antikörper zur Verwendung nach Anspruch 25, wobei der anti-CD19-Antikörper eine schwere Kette bestehend aus einer Sequenz mit mindestens 90% Identität zu SEQ ID NO: 2 oder SEQ ID NO: 4 umfasst.

27. Antikörper zur Verwendung nach einem der Ansprüche 25 bis 26, wobei der anti-CD19-Antikörper die leichte Kette bestehend aus einer Sequenz mit mindestens 95% Identität zu SEQ ID NO: 16 oder SEQ ID NO: 18 umfasst.

28. Antikörper zur Verwendung nach einem der Ansprüche 25 bis 27, wobei dem Menschen anschließend eine Therapie verabreicht wird, bei der es sich nicht um eine anti-CD19-Antikörpertherapie handelt.

29. Antikörper zur Verwendung nach Anspruch 28, wobei es sich bei der Therapie um Chemotherapie, Radiotherapie, Therapie auf Toxinbasis, Therapie auf Radiochemikalienbasis oder chirurgische Therapie handelt.

30. Antikörper zur Verwendung nach einem der Ansprüche 25 bis 29, wobei das Schema die Verabreichung des Antikörpers als einzelnes therapeutisches Agens, in Kombination mit einem anderen therapeutischen Agens oder in Kombination mit einem Agens, das toxische Nebenwirkungen verringert, umfasst.

31. Antikörper zur Verwendung nach einem der Ansprüche 25 bis 30, wobei das Schema die wiederholte Verabreichung des Antikörpers umfasst.

32. Antikörper zur Verwendung nach einem der Ansprüche 25 bis 30, wobei der anti-CD19-Antikörper eine Halbwertszeit von mindestens 4 bis 7 Tagen aufweist.

33. Antikörper zur Verwendung nach einem der Ansprüche 25 bis 32, wobei der anti-CD19-Antikörper ein menschlicher Isotyp IgG1 oder IgG3 ist.

34. Antikörper zur Verwendung nach einem der Ansprüche 25 bis 33, wobei der anti-CD19-Antikörper ein humanisierter Antikörper ist.

35. Antikörper zur Verwendung nach einem der Ansprüche 16 bis 34, wobei es sich bei der B-Zell-Malignität um ein B-Zell-Subtyp-Non-Hodgkin-Lymphom (NHL) einschließlich leichtes/follikuläres NHL, kleines lymphatisches (SL) NHL, mittelschweres/follikuläres NHL, mittelschweres diffuses NHL, schweres immunoblastisches NHL, schweres lymphoblastisches NHL, schweres kleinzelliges ungekerbtes NHL und Bulk-NHL; Burkitt-Lymphom; multiples Myelom; prä-B akute Lymphoblastenleukämie und andere Malignitäten, die von frühen B-Zell-Vorläufern abstammen; gewöhnliche akute lymphatische Leukämie; chronische lymphatische Leukämie; Haarzell-Leukämie; akute Lymphoblastenleukämie des Null-Typs; Waldenström-Makroglobulinämie; und prolymphatische Leukämie; L-Ketten-Krankheit; Plasmazytom; osteosklerotisches Myelom; Plasmazellleukämie; monoklonale Gammopathie unklarer Signifikanz (MGUS); schwelendes multiples Myelom (SMM); indolentes multiples Myelom (IMM) oder Hodgkin-Lymphom handelt.

36. Antikörper zur Verwendung nach einem der Ansprüche 25 bis 35, wobei das Schema weiterhin die Verabreichung einer Verbindung, die die Monozyten- oder Makrophagenfunktion verstärkt, umfasst.

37. Antikörper zur Verwendung nach Anspruch 28, wobei der Mensch immungeschwächt ist.

38. Antikörper zur Verwendung nach einem der Ansprüche 16 bis 35, wobei der anti-CD19-Antikörper auf einem parenteralen, intraperitonealen oder intramuskulären Weg verabreicht wird.

39. Antikörper zur Verwendung nach einem der Ansprüche 16 bis 35, wobei der anti-CD19-Antikörper auf einem intravenösen oder subkutanen Weg verabreicht wird.

40. Antikörper zur Verwendung nach Anspruch 39, wobei der anti-CD19-Antikörper auf einem subkutanen Weg in einer Dosis von 37,5 mg/m2 oder weniger verabreicht wird.

41. Antikörper zur Verwendung nach Anspruch 40, wobei der anti-CD19-Antikörper in einer Dosis von 1,5 mg/m2 oder weniger verabreicht wird.

42. Antikörper zur Verwendung nach einem der Ansprüche 25 bis 41, wobei das Schema mehr als eine Verabreichung des anti-CD19-Antikörpers an den Menschen umfasst.

43. Antikörper zur Verwendung nach einem der Ansprüche 25 bis 42, wobei das Schema weiterhin die Verabreichung eines anti-CD20-Antikörpers, eines anti-CD22-Antikörpers, eines anti-CD52-Antikörpers oder einer beliebigen Kombination davon umfasst.

## Revendications

1. Composition pharmaceutique comprenant un anticorps monoclonal chimérisé ou humanisé anti-CD19 qui (a) est de l'isotype humain IgG1 ou IgG3 ou (b) effectue la médiation d'une cytotoxicité cellulaire dépendante d'un anticorps humain (ADCC), dans un véhicule acceptable d'un point de vue pharmaceutique, dans laquelle l'anticorps comprend les séquences telles qu'indiquées en tant qu'acides aminés 33 à 37, acides aminés 51 à 68 et acides aminés 101 à 115 de la SEQ ID n° : 2 et les séquences telles qu'indiquées en tant qu'acides aminés 43 à 58, acides aminés 74 à 80 et acides aminés 113 à 121 de la SEQ ID n° : 16, ou les séquences telles qu'indiquées en tant qu'acides aminés 33 à 37, acides aminés 51 à 68 et acides aminés 101 à 114 de la SEQ ID n° : 4 et les séquences telles qu'indiquées en tant qu'acides aminés 44 à 58, acides aminés 74 à 80 et acides aminés 113 à 121 de la SEQ ID n° : 18, dans laquelle une substitution d'acide aminé est introduite dans une ou plusieurs desdites séquences et dans laquelle l'anticorps est capable de supprimer des lymphocytes B exprimant un CD19 de la moelle osseuse.

2. Composition pharmaceutique selon la revendication 1, dans laquelle une quantité efficace, d'un point de vue thérapeutique, de l'anticorps monoclonal chimérisé anti-CD19 de l'isotype humain IgG1 ou IgG3 est inférieure à 1 mg/kg environ du poids corporel du patient.

3. Composition pharmaceutique selon la revendication 1, dans laquelle une quantité efficace, d'un point de vue thérapeutique, d'un anticorps monoclonal chimérisé anti-CD19 de l'isotype humain IgG1 ou IgG3 est supérieure à 2 mg/kg environ du poids corporel du patient.

4. Composition selon la revendication 1, dans laquelle l'anticorps anti-CD19 qui effectue la médiation de l'ADCC est de l'isotype humain IgG1 ou IgG3.

5. Composition pharmaceutique selon l'une quelconque des revendications précédentes, dans laquelle l'anticorps anti-CD19 a une demi-vie d'au moins 4 à 7 jours.

6. Composition pharmaceutique selon l'une quelconque des revendications précédentes, dans laquelle la composition comprend une dose de 1 500 mg/m2 ou moins de l'anticorps anti-CD19 et dans laquelle l'anticorps anti-CD19 est administré par voie parentérale, intrapéritonéale, intramusculaire, intraveineuse ou sous-cutanée.

7. Composition pharmaceutique selon la revendication 6, dans laquelle la composition comprend une dose de 375 mg/m2 ou moins de l'anticorps anti-CD19 et dans laquelle l'anticorps anti-CD19 est administré par voie parentérale, intrapéritonéale, intramusculaire, intraveineuse ou sous-cutanée.

8. Composition pharmaceutique selon la revendication 7, dans laquelle la composition comprend une dose de 1,5 mg/m2 ou moins de l'anticorps anti-CD19 et dans laquelle l'anticorps anti-CD19 est administré par voie parentérale, intrapéritonéale, intramusculaire, intraveineuse ou sous-cutanée.

9. Composition pharmaceutique selon la revendication 8, dans laquelle la composition comprend une dose de 0,5 ug/m2 ou moins de l'anticorps anti-CD19 et dans laquelle l'anticorps anti-CD19 est administré par voie parentérale, intrapéritonéale, intramusculaire, intraveineuse ou sous-cutanée.

10. Composition pharmaceutique selon l'une quelconque des revendications précédentes, dans laquelle l'anticorps anti-CD19 est marqué de manière détectable, est un anticorps nu, est conjugué à un composé thérapeutique, est conjugué à un agent cytotoxique ou est conjugué à un agent diagnostique.

11. Composition pharmaceutique selon l'une quelconque des revendications précédentes, dans laquelle l'anticorps anti-CD19 est bispécifique.

12. Composition pharmaceutique selon la revendication 11, dans laquelle l'anticorps bispécifique anti-CD19 a une spécificité de liaison à des cellules effectrices.

13. Composition pharmaceutique selon l'une quelconque des revendications précédentes, dans laquelle la fonction d'ADCC de l'anticorps anti-CD19 est évaluée en mesurant la faculté de l'anticorps anti-CD19 à réaliser la médiation de la lyse *in vitro* d'une cellule cible par des cellules effectrices.

14. Composition pharmaceutique selon l'une quelconque des revendications précédentes, dans laquelle l'anticorps anti-CD19 comprend une chaîne lourde, constituée par une séquence ayant une identité, d'au moins 90%, avec la SEQ ID n° : 2 ou la SEQ ID n° : 4.

15. Composition pharmaceutique selon l'une quelconque des revendications précédentes, dans laquelle l'anticorps anti-CD19 comprend la chaîne légère, constituée par une séquence ayant une identité, d'au moins 95%, avec la SEQ ID n° : 16 ou la SEQ ID n° : 18.

16. Anticorps monoclonal humanisé anti-CD19 qui (a) est de 1'isotype humain IgG1 ou IgG3 ou (b) effectue la médiation d'une cytotoxicité cellulaire dépendante d'un anticorps humain (ADCC), dans laquelle l'anticorps comprend les séquences telles qu'indiquées en tant qu'acides aminés 33 à 37, 51 à 68 et 101 à 115 de la SEQ ID n° : 2 et les séquences telles qu'indiquées en tant qu'acides aminés 43 à 58, 74 à 80 et 113 à 121 de la SEQ ID n° : 16, ou les séquences telles qu'indiquées en tant qu'acides aminés 33 à 37, 51 à 68 et 101 à 114 de la SEQ ID n° : 4 et les séquences telles qu'indiquées en tant qu'acides aminés 44 à 58, 74 à 80 et 113 à 121 de la SEQ ID n° : 18, dans laquelle une substitution d'acide aminé est introduite dans une ou plusieurs desdites séquences, pour être utilisé dans le traitement d'une malignité à lymphocytes B chez un patient humain, dans laquelle l'anticorps est capable de supprimer des lymphocytes B exprimant un CD19 de la moelle osseuse.

17. Anticorps destiné à être utilisé selon la revendication 16, dans laquelle l'anticorps anti-CD19 comprend une chaîne lourde, constituée par une séquence ayant une identité, d'au moins 90%, avec la SEQ ID n° : 2 ou la SEQ ID n° : 4.

18. Anticorps destiné à être utilisé selon la revendication 16 ou la 17, dans laquelle 1"anticorps comprend la chaîne légère, constituée par une séquence ayant une identité, d'au moins 95%, avec la SEQ ID n° : 16 ou la SEQ ID n° : 18.

19. Anticorps destiné à être utilisé selon l'une quelconque des revendications 16 à 18, dans laquelle l'anticorps est capable de supprimer les lymphocytes B circulants.

20. Anticorps destiné à être utilisé selon l'une quelconque des revendications 16 à 19, dans laquelle la malignité à lymphocytes B est une leucémie aigüe lymphoblastique, le lymphome à cellules du manteau, la leucémie aigüe lymphoblastique à pré-lymphocytes B ou un lymphome lymphoblastique à lymphocytes B précurseurs.

21. Anticorps destiné à être utilisé selon l'une quelconque des revendications 16 à 20, dans laquelle l'anticorps anti-CD19 qui effectue la médiation de l'ADCC est de l'isotype humain IgG1 ou IgG3.

22. Anticorps destiné à être utilisé selon l'une quelconque des revendications 16 à 20, dans laquelle la malignité à lymphocytes B est traitée avant d'administrer l'anticorps anti-CD19.

23. Anticorps destiné à être utilisé selon l'une quelconque des revendications 16 à 20, dans laquelle la malignité à lymphocytes B est traitée avec une thérapie autre qu'une thérapie par anticorps anti-CD19, après l'administration de l'anticorps anti-CD19.

24. Anticorps destiné à être utilisé selon la revendication 22 ou la revendication 23, dans laquelle le traitement de la malignité est une : chimiothérapie, radio-immunothérapie, thérapie par toxine, thérapie enzymatique activant un pro-médicament, thérapie par anticorps, thérapie renforçant les monocytes ou macrophages, thérapie par immuno-régulation, thérapie de néovascularisation tumorale par statine, thérapie par calichéamicine, thérapie chirurgicale ou n'importe quelle combinaison de ceux-ci.

25. Anticorps monoclonal anti-CD19 qui effectue la médiation d'une cytotoxicité cellulaire dépendante d'un anticorps humain (ADCC), comprenant les séquences telles qu'indiquées en tant qu'acides aminés 33 à 37, acides aminés 51 à 68 et acides aminés 101 à 115 de la SEQ ID n° : 2 et les séquences telles qu'indiquées en tant qu'acides aminés 43 à 58, acides aminés 74 à 80 et acides aminés 113 à 121 de la SEQ ID n° : 16, ou les séquences telles qu'indiquées en tant qu'acides aminés 33 à 37, acides aminés 51 à 68 et acides aminés 101 à 114 de la SEQ ID n° : 4 et les séquences telles qu'indiquées en tant qu'acides aminés 44 à 58, acides aminés 74 à 80 et acides aminés 113 à 121 de la SEQ ID n° : 18, dans laquelle une substitution d'acide aminé est introduite dans une ou plusieurs desdites séquences et dans laquelle l'anticorps est capable de supprimer des lymphocytes B exprimant un CD19 de la moelle osseuse, destiné à être utilisé dans un régime pour (a) le traitement d'une maladie à un stade précoce résultant d'une malignité à lymphocytes B chez un patient humain ou (b) le traitement d'une malignité à lymphocytes B chez un sujet humain, dans laquelle le sujet humain n'a pas reçu préalablement de traitement pour la malignité, la malignité à lymphocytes B est positive aux CD19 ou l'être humain a un dénombrement des monocytes d'au moins 1 par dl, dans laquelle le régime administré supprime les lymphocytes B circulants.

26. Anticorps destiné à être utilisé selon la revendication 25, dans laquelle l'anticorps comprend une chaîne lourde, constituée par une séquence ayant une identité, d'au moins 90%, avec la SEQ ID n° : 2 ou la SEQ ID n° : 4.

27. Anticorps destiné à être utilisé selon l'une quelconque des revendications 25 à 26, dans laquelle l'anticorps anti-CD19 comprend la chaîne légère, constituée par une séquence ayant une identité, d'au moins 95%, avec la SEQ ID n° : 16 ou la SEQ ID n° : 18.

28. Anticorps destiné à être utilisé selon l'une quelconque des revendications 25 à 27, dans laquelle une thérapie autre qu'une thérapie par anticorps anti-CD19 est administrée par la suite à l'être humain.

29. Anticorps destiné à être utilisé selon la revendication 28, dans laquelle la thérapie est une : chimiothérapie, radiothérapie, thérapie basée sur une toxine, thérapie basée sur une substance radiochimique ou thérapie chirurgicale.

30. Anticorps destiné à être utilisé selon l'une quelconque des revendications 25 à 29, dans laquelle le régime comprend l'administration de l'anticorps en tant qu'agent thérapeutique seul, en combinaison avec un autre agent thérapeutique ou en combinaison avec un agent qui réduit les effets secondaires toxiques.

31. Anticorps destiné à être utilisé selon l'une quelconque des revendications 25 à 30, dans laquelle le régime comprend l'administration répétée de l'anticorps.

32. Anticorps destiné à être utilisé selon l'une quelconque des revendications 25 à 30, dans laquelle l'anticorps anti-CD19 a une demi-vie qui est d'au moins 4 à 7 jours.

33. Anticorps destiné à être utilisé selon l'une quelconque des revendications 25 à 32, dans laquelle l'anticorps anti-CD19 est un anticorps humain de l'isotype IgG1 ou IgG3.

34. Anticorps destiné à être utilisé selon l'une quelconque des revendications 25 à 33, dans laquelle l'anticorps anti-CD19 est un anticorps humanisé.

35. Anticorps destiné à être utilisé selon l'une quelconque des revendications 16 à 34, dans laquelle la malignité à lymphocytes B est un lymphome non Hodgkinien (LNH) d'un sous-type de lymphocytes B incluant : un LNH de faible grade/folliculaire, un LNH lymphocytaire à petites cellules (LS), un LNH de grade intermédiaire/folliculaire, un LNH diffus de grade intermédiaire, un LNH immunoblastique de grade élevé, un LNH lymphoblastique de grade élevé, un LNH de grade élevé à petites cellules non coupées et un LNH à un stade avancé ; le lymphome de Burkitt ; un myélome multiple ; une leucémie aigüe lymphoblastique à pré-lymphocytes B ainsi que d'autres malignités qui sont dérivées de précurseurs des lymphocytes B précoces ; la leucémie aiguë lymphocytaire commune ; la leucémie lymphocytaire chronique ; la leucémie à cellules "chevelues" ; la leucémie aiguë lymphoblastique des cellules de Null ; la macroglobulinémie de Waldenstrom ; et la leucémie pro-lymphocytaire ; une maladie des chaînes légères ; un plasmocytome ; un myélome ostéosclérotique ; la leucémie à plasmocytes ; une gammapathie monoclonale de signification indéterminée (GMSI) ; le myélome multiple latent (MML) ; le myélome multiple indolent (MMI) ; ou un lymphome de Hodgkin.

36. Anticorps destiné à être utilisé selon l'une quelconque des revendications 25 à 35, dans laquelle le régime comprend en outre l'administration d'un composé qui amplifie la fonction des monocytes ou macrophages.

37. Anticorps destiné à être utilisé selon la revendication 28, dans laquelle l'être humain est immuno-compromis.

38. Anticorps destiné à être utilisé selon l'une quelconque des revendications 16 à 35, dans laquelle l'anticorps anti-CD19 est administré par voie parentérale, intrapéritonéale ou intramusculaire.

39. Anticorps destiné à être utilisé selon l'une quelconque des revendications 16 à 35, dans laquelle l'anticorps anti-CD19 est administré par voie intraveineuse ou sous-cutanée.

40. Anticorps destiné à être utilisé selon la revendication 39, dans laquelle l'anticorps anti-CD19 est administré par voie sous-cutanée en une dose de 37,5 mg/m2 ou moins.

41. Anticorps destiné à être utilisé selon la revendication 40, dans laquelle l'anticorps anti-CD19 est administré en une dose de 1,5 mg/m2 ou moins.

42. Anticorps destiné à être utilisé selon l'une quelconque des revendications 25 à 41, dans laquelle le régime comprend plus d'une administration de l'anticorps anti-CD19 à l'être humain.

43. Anticorps destiné à être utilisé selon l'une quelconque des revendications 25 à 42, dans laquelle le régime comprend en outre l'administration d'un anticorps anti-CD20, d'un anticorps anti-CD22, d'un anticorps anti-CD52 ou d'une quelconque combinaison de ceux-ci.
